# EUROPEAN PATENT APPLICATION

(11) **EP 4 837 590 A1**
(43) Date of publication of application: **11.11.2026**
(21) Application number: 25736411.7
(22) Date of filing: 03.01.2025
(51) Int. Cl.: A61K 47/68, C07K 16/28, A61P 35/00

(54) **ANTIBODY, AND IMMUNOCONJUGATE COMPRISING SAME AND TLR7/8 AGONIST AND USE THEREOF**

(30) Priority: 05.01.2024 CN 202410022814
(71) Applicant: Fortvita Biologics Inc., 1205 Grand Cayman (KY)
(72) Inventor: XIONG, Huizhong, Suzhou, Jiangsu 215123 (CN); YOU, Fei, Suzhou, Jiangsu 215123 (CN); LI, Li, Suzhou, Jiangsu 215123 (CN); XIONG, Yao, Suzhou, Jiangsu 215123 (CN); CHEN, Ye, Suzhou, Jiangsu 215123 (CN)
(74) Representative: Sagittarius IP
(86) International application number: PCT/CN2025/070576
(87) International publication number: WO 2025/146173

(57) **Abstract**

The present invention relates to a TLR7/8 agonist and an immunoconjugate comprising the same and Trop2-targeting antibodies and FRα-targeting antibodies, and further provides therapeutic and diagnostic use of said immunoconjugates.

## Description

### Field of Invention

The present invention belongs to the fields of tumor treatment and targeted treatment. Specifically, the present invention provides novel anti-TROP2 antibodies, novel FRα antibodies, and novel TLR (e.g., TLR7/8) agonists, as well as immunoconjugates (ISACs) comprising said novel antibodies and/or novel TLR agonists and compositions comprising said conjugates; the invention further provides therapeutic and diagnostic uses of said ISACs.

### Background Of The Invention

TROP2, a trophoblast cell-surface antigen, also known as tumor-associated calcium signal transducer (TACSTD2), is overexpressed in a variety of human epithelial cancers, including breast cancer, lung cancer, gastric cancer, colorectal cancer, pancreatic cancer, prostate cancer, cervical cancer, head and neck cancer, ovarian cancer, etc. (Yeshe Cheng et al., Frontiers in Oncology, Dec 23, 2022; 12:951589). Pre-clinical and clinical studies have used anti-TROP2 antibody-drug conjugates (ADCs) for the treatment of cancer, however, the clinical results were only mediocre. For example, Trodelvy (anti-human TROP2 antibody-SN-38 conjugate) has achieved considerable therapeutic effect in the treatment of refractory solid tumors, for example, the objective response rate (ORR) in drug-resistant triple-negative breast cancer (TNBC) patients reaches 33%. One of the important mechanisms of action of Trodelvy is that SN-38 (payload) is linked by a pH-sensitive linker, which is capable of cleaving and specifically releasing SN-38 in an acidic tumor microenvironment. However, the linker of Trodelvy is not stable enough, the maleimide-mediated linker undergoes cleavage via thiol exchange under physiological conditions, resulting the half-life of Trodelvy in serum is relatively short (about 1 day). Thus, Trodelvy may have relatively high off-target effects. Although another anti-TROP2 antibody-drug conjugate, Ds-1062a (Dato-DXd, AstraZeneca and Daiichi Sankyo), has demonstrated significant efficacy in treating patients with non-mutated lung cancer and triple-negative breast cancer, there are still problems such as uneven DAR distribution that affects drug efficacy and produces target toxicity in normal tissues.

Folate receptor α (FRα), also known as folate receptor 1 (FOLR1), belongs to the folate receptor family and is found as a folate-binding protein. However, in addition to transporting folate, it is also involved in regulating the proliferation and metastasis of tumor cells. Since folic acid is an important nutrient necessary for maintaining cell growth and metabolism, this molecule plays a key role in the synthesis of DNA and RNA. In some cancer cells that proliferate rapidly (e.g., breast cancer, ovarian cancer, lung cancer, etc.), due to the higher requirement for folic acid, the expression level of folate receptor α is significantly increased, so that the cancer cells maintain an abnormally high proliferation rate and metabolic activity. At present, there are a plurality of anti-tumor therapeutic strategies targeting FOLR1, including monoclonal antibodies, bispecific antibodies, ADC drugs, CAR-T therapies, and the like.

Toll-like receptors (TLRs) are pattern recognition receptors (PRRs) that are expressed primarily on dendritic cells, macrophages, monocytes, natural killer cells, and T lymphocytes. TLRs bind to pathogen-associated molecular patterns (PAMPS) from bacteria, fungi, protozoa and viruses and serve as a first line of defense against invading pathogens. There are 10 known members of the TLR family in humans, which are type I transmembrane proteins characterized by a leucine-rich extracellular domain and a cytoplasmic tail comprising a conserved Toll/interleukin (IL)-1 receptor (TIR) domain. Within this family, TLR3, TLR7, TLR8 and TLR9 are located within the endosome.

TLR7 is expressed primarily on plasmacytoid cells and also on B-cells, which can be activated by binding to specific small molecule ligands (i.e. TLR7 agonists) or their natural ligands (i.e. single-stranded RNA, ssRNA). The altered immune cell responsiveness may be related to a decrease in the innate immune response during chronic viral infection. Agonist-induced TLR7 activation therefore might represent a new approach for the treatment of chronic viral infections (D. J Connolly and L. AJ O'Neill, Current Opinion in Pharmacology 2012, 12:510-518, P. A. Roethle et al., J. Med. Chem. 2013, 56, 7324-7333).

Toll-like receptors (TLRs) can also alter the tumor microenvironment and initiate adaptive anti-tumor immunity.

However, TLR agonists are poorly tolerated due to toxicity associated with broad immune activation following systemic administration. There is a very urgent clinical need to develop effective and safe TLR agonists, particularly TLR7/8 agonists, as new antiviral and anti-tumor immunotherapies to provide more therapeutic regimens.

### Summary Of The Invention

The present invention first provides novel TLR agonists, in particular TLR7/8 agonists.

The present invention also provides novel high-affinity TROP2 antibodies, or antigen binding fragments thereof.

The present invention also provides novel high-affinity FRα antibodies, or antigen-binding fragments thereof.

The present invention also provides a novel immunoconjugate (ISAC), which comprises a TLR7/8 agonist conjugated to a tumor-targeting antibody. The antibody-immuno agonist conjugate of the present invention is well-tolerated *in vivo* when administered systemically and triggers strong activation of intratumoral myeloid cells, resulting in tumor clearance and subsequent immunological memory.

In a first aspect, the present invention provides an antibody or an antigen-binding fragment thereof that specifically binds to TROP2 (e.g., human TROP2). In some embodiments, the method comprises:
(i) three complementarity determining regions HCDR1, HCDR2 and HCDR3 contained in the VH set forth in SEQ ID NO: 7, and/or three complementarity determining regions LCDR1, LCDR2 and LCDR3 contained in the VL set forth in SEQ ID NO: 8; or
(ii) three complementarity determining regions HCDR1, HCDR2, and HCDR3 contained in the VH set forth in SEQ ID NO: 12, and three complementarity determining regions LCDR 1, LCDR2, and LCDR3 contained in the VL set forth in SEQ ID NO: 14.

In some embodiments of the anti-TROP2 antibody or the antigen-binding fragment thereof of the present invention, the anti-TROP2 antibody or the antigen-binding fragment thereof comprises a first heavy chain complementarity determining region (HCDR1), a second heavy chain complementarity determining region (HCDR2), a third heavy chain complementarity determining region (HCDR3), and a first light chain complementarity determining region (LCDR1), a second light chain complementarity determining region (LCDR2), and a third light chain complementarity determining region (LCDR3), wherein the HCDR1, the HCDR2, the HCDR3, the LCDR1, the LCDR2, and the LCDR3 comprise or consist of amino acid sequences set forth in SEQ ID NO: 1, SEQ ID NO: 2, SEQ ID NO: 3, SEQ ID NO: 4, SEQ ID NO: 5, and SEQ ID NO: 6, respectively.

In some embodiments, the anti-TROP2 antibody or the antigen-binding fragment thereof of the present invention binds to TROP2 (e.g., human TROP2) with sufficient affinity, for example, with an equilibrium dissociation constant (K_{D}) of binding to TROP2, which is≤ 100 nM, ≤ 10 nM, ≤ 1 nM, ≤ 0.55 nM, ≤ 0.5 nM, ≤ 0.4 nM or ≤ 0.35 nM, e.g., 10⁻⁸M or less, for example, 10⁻⁷M to 10⁻¹⁰M, e.g., between about 0.1 nM-1 nM, between greater than 0.3-0.7 nM, between about 0.4-0.6 nM, between about 0.4-0.5 nM, between about 0.5-0.6 nM, e.g., as measured via ForteBio. In some embodiments, the TROP2 is human, mouse, or cynomolgus TROP2. In some embodiments, the binding affinity of the antibody is determined using biological optical interferometry.

In some embodiments, the anti-TROP2 antibody or the antigen-binding fragment thereof of the present invention binds to TROP2 expressed on the surface of a cell.

In some embodiments, the anti-TROP2 antibody or the antigen-binding fragment thereof of the present invention can induce endocytosis, e.g., antibody-dependent cellular phagocytosis (ADCP). In some embodiments, the antibody or the antigen-binding fragment thereof of the present invention can inhibit and/or reduce the growth and/or volume of a tumor in vivo.

In some embodiments, the anti-TROP2 antibody or the antigen-binding fragment thereof comprises a heavy chain variable region VH and/or a light chain variable region VL, wherein the VH
(i) comprises or consists of an amino acid sequence having at least 90%, 91%, 92%, 93%, 94%, 95%, 96%, 97%, 98%, or 99% identity to an amino acid sequence set forth in SEQ ID NO: 7 or 12; or
(ii) comprises or consists of an amino acid sequence set forth in SEQ ID NO: 7 or 12; or
(iii) comprises or consists of an amino acid sequence having one or more (preferably no more than 10, and more preferably no more than 5, 4, 3, 2, or 1) amino acid changes (preferably amino acid substitutions, and more preferably conservative amino acid substitutions) compared with the amino acid sequence set forth in SEQ ID NO: 7 or 12, wherein preferably, the amino acid changes do not occur in the CDRs; and/or
wherein the VL
(i) comprises or consists of an amino acid sequence having at least 90%, 91%, 92%, 93%, 94%, 95%, 96%, 97%, 98%, or 99% identity to an amino acid sequence set forth in SEQ ID NO: 8 or 14; or
(ii) comprises or consists of an amino acid sequence set forth in SEQ ID NO: 8 or 14; or
(iii) comprises or consists of an amino acid sequence having one or more (preferably no more than 10, and more preferably no more than 5, 4, 3, 2, or 1) amino acid changes (preferably amino acid substitutions, and more preferably conservative amino acid substitutions) compared with the amino acid sequence set forth in SEQ ID NO: 8 or 14, wherein preferably, the amino acid changes do not occur in the CDRs.

In some embodiments, the anti-TROP2 antibody or the antigen-binding fragment thereof of the present invention comprises VH and VL, wherein the VH comprises or consists of an amino acid sequence set forth in SEQ ID NO: 7, and the VL comprises or consists of an amino acid sequence set forth in SEQ ID NO: 8.

In some embodiments, the anti-TROP2 antibody or the antigen-binding fragment thereof of the present invention further comprises a heavy chain constant region and/or a light chain constant region.

In some embodiments, the anti-TROP2 antibody or the antigen-binding fragment thereof of the present invention is an IgG antibody.

In some embodiments, the heavy chain constant region of the anti-TROP2 antibody or the antigen-binding fragment thereof of the present invention is derived from IgG1, IgG2, IgG3, or IgG4, e.g., IgG1.

In some embodiments, the anti-TROP2 antibody or the antigen-binding fragment thereof of the present invention comprises a heavy chain and a light chain, wherein the heavy chain
(i) comprises or consists of an amino acid sequence having at least 85%, 90%, 91%, 92%, 93%, 94%, 95%, 96%, 97%, 98%, or 99% identity to an amino acid sequence set forth in SEQ ID NO: 9 or 15;
(ii) comprises or consists of an amino acid sequence set forth in SEQ ID NO: 9 or 15; or
(iii) comprises or consists of an amino acid sequence having one or more (preferably no more than 20 or 10, and more preferably no more than 5, 4, 3, 2, or 1) amino acid changes (preferably amino acid substitutions, and more preferably conservative amino acid substitutions) compared with the amino acid sequence set forth in SEQ ID NO: 9 or 15; and/or
the light chain
(i) comprises or consists of an amino acid sequence having at least 85%, 90%, 91%, 92%, 93%, 94%, 95%, 96%, 97%, 98%, or 99% identity to an amino acid sequence set forth in SEQ ID NO: 10 or 16;
(ii) comprises or consists of an amino acid sequence set forth in SEQ ID NO: 10 or 16; or
(iii) comprises or consists of an amino acid sequence having one or more (preferably no more than 20 or 10, and more preferably no more than 5, 4, 3, 2, or 1) amino acid changes (preferably amino acid substitutions, and more preferably conservative amino acid substitutions) compared with the amino acid sequence set forth in SEQ ID NO: 10 or 16.

In some embodiments, the anti-TROP2 antibody or the antigen-binding fragment thereof of the present invention comprises a heavy chain and a light chain, wherein the heavy chain comprises or consists of an amino acid sequence set forth in SEQ ID NO: 9, and the light chain comprises or consists of an amino acid sequence set forth in SEQ ID NO: 10.

In some embodiments, the anti-TROP2 antibody or the antigen-binding fragment thereof of the present invention comprises a heavy chain and a light chain, wherein the heavy chain comprises or consists of an amino acid sequence set forth in SEQ ID NO: 15, and the light chain comprises or consists of an amino acid sequence set forth in SEQ ID NO: 16.

In some embodiments, the anti-TROP2 antibody or the antigen-binding fragment thereof of the present invention is a chimeric antibody or a humanized antibody.

In some embodiments, the antigen-binding fragment of the anti-TROP2 antibody of the present invention is, for example, Fv, Fab, Fab', Fab'-SH, or F(ab').₂; a domain antibody (dAb); a linear antibody; a single-chain antibody (e.g., scFv); a single-domain antibody (e.g., VHH); a bivalent antibody or a fragment thereof; or a camelid antibody.

In some embodiments, the present invention also provides a fusion protein comprising the anti-TROP2 antibody or the antigen-binding fragment thereof of the present invention.

In some embodiments, the present invention also provides an immunoconjugate comprising the anti-TROP2 antibody or the antigen-binding fragment thereof of the present invention and a payload coupled to the antibody or the antigen-binding fragment thereof. In some embodiments, the payload is a toxin, a small molecule drug/agent, a cytotoxic agent, an apoptotic agent, a chelating agent, an immunomodulator/immunostimulatory agent/immunoagonist, an oligonucleotide, a polypeptide, a peptide epitope, a radionuclide, a prodrug, etc. In some preferred embodiments, the payload is an immunostimulatory agent/immune activator. In some more preferred embodiments, the load is a TLR agonist. In some more preferred embodiments, the load is a TLR7/8 agonist.

In some embodiments, the present invention relates to an isolated nucleic acid encoding the anti-TROP2 antibody or the antigen-binding fragment thereof of the present invention.

In some embodiments, the present invention relates to a vector comprising the nucleic acid of the present invention, preferably the vector is an expression vector.

In some embodiments, the present invention relates to a host cell comprising the nucleic acid or vector of the present invention, preferably, the host cell is prokaryotic or eukaryotic, and more preferably, the host cell is selected from yeast cells, mammalian cells (e.g., 293 cells or CHO cells, such as CHO-K cells or HEK293 cells), or other cells suitable for preparing an antibody or an antigen-binding fragment thereof.

In some embodiments, the present invention relates to a method for preparing an anti-TROP2 antibody or an antigen-binding fragment thereof, which comprises
a) culturing the host cell of the present invention under conditions suitable for expressing a nucleic acid encoding the anti-TROP2 antibody or the antigen-binding fragment thereof of the present invention,
b) optionally isolating the antibody or antigen binding fragment thereof,
c) optionally, the method further comprises isolating the anti-TROP2 antibody or the antigen-binding fragment thereof from the host cell; optionally, the antibody is purified, e.g., by ProteinA.

In another aspect, the present invention provides an anti-FRα antibody or an antigen-binding fragment thereof.

In some embodiments, the anti-FRα antibody or the antigen-binding fragment thereof of the present invention specifically binds to FRα (e.g., human FRα or cynomolgus FRα). In some embodiments, the K_{D} values of the binding affinity for FRα of the anti-FRα antibody or antigen-binding fragment thereof of the present invention (e.g., human FRα) less than or equal to about 10 nM, 9 nM, 8 nM, 7 nM, 6 nM, 5 nM, 4 nM, 3 nM, or 2 nM, or greater than about 0.5 nM or 1 nM, or in between said values. In some embodiments, the binding affinity of the antibody of the present invention is determined via bio-layer interferometry, e.g., ForteBio Octet.

In some embodiments, the anti-FRα antibody or the antigen-binding fragment thereof of the present invention is capable of effectively binding to FRα, e.g., human FRα.

In some embodiments, the anti-FRα antibody or the antigen-binding fragment thereof of the present invention comprises 3 complementarity determining regions from a heavy chain variable region (HCDRs): HCDR1, HCDR2, and HCDR3. In some embodiments, the anti-FRα antibody or the antigen-binding fragment thereof of the present invention comprises 3 complementarity determining regions from a light chain variable region (LCDRs): LCDR1, LCDR2 and LCDR3. In some embodiments, the anti-FRα antibody or the antigen-binding fragment thereof of the present invention comprises 3 complementarity determining regions from a heavy chain variable region (HCDRs) and 3 complementarity determining regions from a light chain variable region (LCDRs).

In some aspects, the anti-FRα antibody or the antigen-binding fragment thereof of the present invention comprises a heavy chain variable region (VH). In some aspects, the anti-FRα antibody or the antigen-binding fragment thereof of the present invention comprises a light chain variable region (VL). In some aspects, the anti-FRα antibody or the antigen-binding fragment thereof of the present invention comprises a heavy chain variable region and a light chain variable region. In some embodiments, the heavy chain variable region comprises 3 complementarity determining regions (CDRs) from the heavy chain variable region: HCDR1, HCDR2, and HCDR3. In some embodiments, the light chain variable region comprises 3 complementarity determining regions (CDRs) from the light chain variable region: LCDR1, LCDR2, and LCDR3.

In some embodiments, the anti-FRα antibody or the antigen-binding fragment thereof of the present invention further comprises an antibody heavy chain constant region. In some embodiments, the anti-FRα antibody or the antigen-binding fragment thereof of the present invention further comprises an antibody light chain constant region. In some embodiments, the anti-FRα antibody or the antigen-binding fragment thereof of the present invention further comprises a heavy chain constant region and a light chain constant region.

In some embodiments, the heavy chain variable region of the present invention:
(i) comprises or consists of an amino acid sequence having at least 90%, 91%, 92%, 93%, 94%, 95%, 96%, 97%, 98%, or 99% identity to the amino acid sequence set forth in SEQ ID NO: 33; or
(ii) comprises or consists of the amino acid sequence set forth in SEQ ID NO: 33;
   or
(iii) comprises or consists of an amino acid sequence having one or more (preferably no more than 10, and more preferably no more than 5, 4, 3, 2, or 1) amino acid changes (preferably amino acid substitutions, and more preferably conservative amino acid substitutions) compared with the amino acid sequence set forth in any one of (i)-(ii), wherein preferably, the amino acid changes do not occur in the CDRs.

In some embodiments, the light chain variable region of the present invention
(i) comprises or consists of an amino acid sequence having at least 90%, 91%, 92%, 93%, 94%, 95%, 96%, 97%, 98%, or 99% identity to the amino acid sequence selected from SEQ ID NO: 34; or
(ii) comprises or consists of the amino acid sequence selected from SEQ ID NO: 34;
   or
(iii) comprises or consists of an amino acid sequence having one or more (preferably no more than 10, and more preferably no more than 5, 4, 3, 2, or 1) amino acid changes (preferably amino acid substitutions, and more preferably conservative amino acid substitutions) compared with the amino acid sequence selected from any one of (i) to (ii), wherein preferably, the amino acid changes do not occur in the CDRs.

In some embodiments, the 3 complementarity determining regions from the heavy chain variable region (HCDRs) of the present invention, HCDR1, HCDR2, and HCDR3, are selected from
(i) the three complementarity determining regions HCDR1, HCDR2, and HCDR3 contained in the VH set forth in SEQ ID NO: 33; or
(ii) a sequence comprising a total of at least one and no more than 5, 4, 3, 2, or 1 amino acid change (preferably amino acid substitutions, and more preferably conservative substitutions) in the three HCDRs relative to any sequence of (i),
wherein the HCDRs may be determined according to any scheme for CDR definitions, e.g., according to Kabat, AbM, Chothia, Contact, or IMGT, or a combination thereof;

For example, the HCDR1 is determined according to the combined ranges of Kabat and Chothia schemes, and the HCDR2 and the HCDR3 are determined according to the Kabat scheme, respectively.

In some embodiments, the 3 complementarity determining regions from the light chain variable region (LCDRs) of the present invention, LCDR1, LCDR2, and LCDR3, are selected from
(i) three complementarity determining regions LCDR1, LCDR2, and LCDR3 contained in the VL set forth in SEQ ID NO: 34, or
(i) a sequence comprising a total of at least one and no more than 5, 4, 3, 2, or 1 amino acid change (preferably amino acid substitutions, and more preferably conservative substitutions) in the three LCDRs relative to any sequence of (i).
wherein the LCDRs may be determined according to any scheme for CDR definitions, e.g., according to Kabat, AbM, Chothia, Contact, IMGT, or a combination thereof;

For example, the LCDR1, the LCDR2, and the LCDR3 are determined according to the Kabat scheme, respectively.

In some embodiments, the anti-FRα antibody or the antigen-binding fragment thereof of the present invention comprises:
three complementarity determining regions HCDR1, HCDR2 and HCDR3 contained in a VH set forth in SEQ ID NO: 33, and three complementarity determining regions LCDR1, LCDR2 and LCDR3 contained in a VL set forth in SEQ ID NO: 34;
wherein the HCDRs and LCDRs may be determined according to any scheme for determining CDRs, e.g., according to Kabat, AbM, Chothia, Contact, or IMGT, or a combination thereof;

For example, the HCDR1 is determined according to combined ranges of Kabat and Chothia schemes, the HCDR2 and the HCDR3 are determined according to the Kabat scheme, and the LCDR1, LCDR2, and LCDR3 are determined according to the Kabat scheme.

In some embodiments, the HCDR1 comprises or consists of an amino acid sequence set forth in SEQ ID NO: 27, or the HCDR1 comprises an amino acid sequence having one, two, or three amino acid changes (preferably amino acid substitutions, and more preferably conservative substitutions) compared with the amino acid sequence set forth in SEQ ID NO: 27.

In some embodiments, the HCDR2 comprises or consists of an amino acid sequence set forth in SEQ ID NO: 28, or the HCDR2 comprises an amino acid sequence having one, two, or three amino acid changes (preferably amino acid substitutions, and more preferably conservative substitutions) compared with the amino acid sequence set forth in SEQ ID NO: 28.

In some embodiments, the HCDR3 comprises or consists of an amino acid sequence set forth in SEQ ID NO: 29, or the HCDR3 comprises an amino acid sequence having one, two, or three amino acid changes (preferably amino acid substitutions, and more preferably conservative substitutions) compared with the amino acid sequence set forth in SEQ ID NO: 29.

In some embodiments, the LCDR1 comprises or consists of an amino acid sequence set forth in SEQ ID NO: 30, or the LCDR1 comprises an amino acid sequence having one, two, or three amino acid changes (preferably amino acid substitutions, and more preferably conservative substitutions) compared with the amino acid sequence set forth in SEQ ID NO: 30.

In some embodiments, the LCDR2 comprises or consists of an amino acid sequence set forth in SEQ ID NO: 31, or the LCDR2 comprises an amino acid sequence having one, two, or three amino acid changes (preferably amino acid substitutions, and more preferably conservative substitutions) compared with the amino acid sequence set forth in SEQ ID NO: 31.

In some embodiments, the LCDR3 comprises or consists of an amino acid sequence set forth in SEQ ID NO: 32, or the LCDR3 comprises an amino acid sequence having one, two, or three amino acid changes (preferably amino acid substitutions, and more preferably conservative substitutions) compared with the amino acid sequence set forth in SEQ ID NO: 32.

In some specific embodiments, the anti-FRα antibody of the present invention comprises a first heavy chain complementarity determining region (HCDR1), a second heavy chain complementarity determining region (HCDR2), a third heavy chain complementarity determining region (HCDR3), and a first light chain complementarity determining region (LCDR1), a second light chain complementarity determining region (LCDR2), and a third light chain complementarity determining region (LCDR3), wherein
the HCDR1, HCDR2, HCDR3, LCDR1, LCDR2, and LCDR3 comprise or consist of amino acid sequences set forth in SEQ ID NO: 27, SEQ ID NO: 28, SEQ ID NO: 29, SEQ ID NO: 30, SEQ ID NO: 31, and SEQ ID NO: 32, respectively.

In some embodiments, the VH of the present invention comprises an HCDR1, an HCDR2 and an HCDR3, and the VL comprises an LCDR1, an LCDR2 and an LCDR3, wherein
the HCDR1, HCDR2, HCDR3, LCDR1, LCDR2, and LCDR3 comprise or consist of amino acid sequences set forth in SEQ ID NO: 27, SEQ ID NO: 28, SEQ ID NO: 29, SEQ ID NO: 30, SEQ ID NO: 31, and SEQ ID NO: 32, respectively.

In some embodiments, the anti-FRα antibody or the antigen-binding fragment thereof of the present invention comprises:
a VH comprising or consisting of an amino acid sequence set forth in SEQ ID NO: 33 or an amino acid sequence having at least 90%, 91%, 92%, 93%, 94%, 95%, 96%, 97%, 98%, or 99% identity thereto, and/or a VL comprising or consisting of an amino acid sequence set forth in SEQ ID NO: 34 or an amino acid sequence having at least 90%, 91%, 92%, 93%, 94%, 95%, 96%, 97%, 98%, or 99% identity thereto.

In some embodiments, the anti-FRα antibody or the antigen-binding fragment thereof of the present invention comprises a heavy chain variable region and a light chain variable region, wherein the heavy chain variable region comprises or consists of an amino acid sequence set forth in SEQ ID NO: 33, and the light chain variable region comprises or consists of an amino acid sequence set forth in SEQ ID NO: 34.

In one embodiment of the present invention, the amino acid changes described herein include amino acid substitutions, insertions, or deletions. In some embodiments, the amino acid changes described herein are conservative amino acid changes. Preferably, the amino acid changes described herein are amino acid substitutions, preferably conservative substitutions. In a preferred embodiment, the amino acid changes described herein occur in regions outside the CDRs (e.g., in FRs). More preferably, the amino acid changes described herein occur in a region outside the heavy chain variable region and/or outside the light chain variable region.

In some embodiments, the anti-FRα antibody or the antigen-binding fragment thereof of the present invention further comprises an antibody heavy chain. In some embodiments, the anti-FRα antibody or the antigen-binding fragment thereof of the present invention further comprises an antibody light chain. In some embodiments, the anti-FRα antibody or the antigen-binding fragment thereof of the present invention further comprises a heavy chain and a light chain. In some embodiments, the antibody heavy chain of the present invention comprises or consists of a heavy chain variable region and a heavy chain constant region. In some embodiments, the antibody light chain of the present invention comprises or consists of a light chain variable region and a light chain constant region. In some embodiments, the antibody of the present invention comprises or consists of two heavy chains and two light chains. In some embodiments, the antibody of the present invention comprises or consists of two identical heavy chains and two identical light chains.

In some embodiments, the heavy chain constant region of the anti-FRα antibody of the present invention is a heavy chain constant region of IgG1, IgG2, IgG3, or IgG4, e.g., a constant region of human IgG1, IgG2, IgG3, or IgG4. In some embodiments, the heavy chain constant region is an IgG heavy chain constant region, e.g., an IgG1, IgG2, IgG3, or IgG4 heavy chain constant region. In some embodiments, the heavy chain constant region is an IgG1 heavy chain constant region, e.g., comprising an amino acid sequence set forth in SEQ ID NO: 46 or an amino acid sequence having at least 90%, 91%, 92%, 93%, 94%, 95%, 96%, 97%, 98%, or 99% sequence identity to the amino acid sequence set forth in SEQ ID NO: 46.

In some embodiments, the light chain constant region of the anti-FRα antibody of the present invention is a lambda or Kappa light chain constant region, preferably a Kappa light chain constant region, e.g., a human lambda or Kappa light chain constant region. In some embodiments, the light chain constant region is a (human) lambda light chain constant region. In some embodiments, the Lambda light chain constant region comprises the amino acid sequence set forth in SEQ ID NO: 38 or an amino acid sequence having at least 90%, 91%, 92%, 93%, 94%, 95%, 96%, 97%, 98%, or 99% sequence identity to the amino acid sequence set forth in SEQ ID NO: 38 and not comprising cysteine mutations. In some embodiments, the light chain constant region is a (human) Kappa light chain constant region. In some embodiments, the Kappa light chain constant region comprises an amino acid sequence set forth in SEQ ID NO: 45 or an amino acid sequence having at least 90%, 91%, 92%, 93%, 94%, 95%, 96%, 97%, 98%, or 99% sequence identity to the amino acid sequence set forth in SEQ ID NO: 45.

In some embodiments, the anti-FRα antibody or the antigen-binding fragment thereof of the present invention is an antibody or an antigen-binding fragment thereof having a cysteine mutation in the light chain constant region, wherein the antibody or the antigen-binding fragment thereof comprises one or two lambda light chain constant regions and comprises a cysteine substitution at position 160 (EU numbering) of the lambda light chain constant region (LLC160C or LLC160) and/or a cysteine substitution at position 166 (EU numbering) of the lambda light chain constant region (LLC166C or LLC166).

In some embodiments, relative to the wild-type lambda light chain constant region, e.g., the amino acid sequence set forth in SEQ ID NO: 38, the Anti-FRα light chain constant region of the antibody or the antigen-binding fragment thereof has a cysteine mutation at position 160 (Eu numbering).

In some embodiments, the Lamda light chain constant region having a cysteine mutation at position 160 comprises an amino acid sequence set forth in SEQ ID NO: 39, or an amino acid sequence having at least 90%, 91%, 92%, 93%, 94%, 95%, 96%, 97%, 98%, or 99% sequence identity to the amino acid sequence set forth in SEQ ID NO: 39 and comprising an amino acid sequence VKAGVCTTTPS (SEQ ID NO: 42).

In some embodiments, relative to the wild-type lambda light chain constant region, e.g., the amino acid sequence set forth in SEQ ID NO: 38, the light chain constant region of the anti-FRα antibody or the antigen-binding fragment thereof has a cysteine mutation at position 166 (Eu numbering).

In some embodiments, the Lamda light chain constant region having a cysteine mutation at position 166 comprises an amino acid sequence set forth in SEQ ID NO: 40, or an amino acid sequence having at least 90%, 91%, 92%, 93%, 94%, 95%, 96%, 97%, 98%, or 99% sequence identity to the amino acid sequence set forth in SEQ ID NO: 40 and comprising the amino acid sequence TTTPSCQSNNK (SEQ ID NO: 43).

In some embodiments, relative to the wild-type lambda light chain constant region, e.g., the amino acid sequence set forth in SEQ ID NO: 38, the light chain constant region of the Anti-FRα antibody or the antigen-binding fragment thereof has cysteine mutations at positions 160 and 166 (Eu numbering).

In some embodiments, the Lamda light chain constant region having cysteine mutations at positions 160 and 166 comprises an amino acid sequence set forth in SEQ ID NO: 41, or has at least 90%, 91%, 92%, 93%, 94%, 95%, 96%, 97%, 98%, or 99% sequence identity to the amino acid sequence set forth in SEQ ID NO: 41 and comprises the amino acid sequence VKAGVCTTTPSCQSNNK (SEQ ID NO: 44).

In some embodiments, the antibody or the antigen-binding fragment thereof disclosed herein comprises:
(i) one or two Lamda light chain constant regions having a cysteine mutation at position 160 (Eu numbering); and/or
(ii) one or two Lamda light chain constant regions having a cysteine mutation at position 166 (Eu numbering).

In some embodiments, the Anti-FRα antibody or the antigen-binding fragment thereof of the present invention may also comprise a modification that alters the binding affinity for one or more Fc receptors in the heavy chain constant region or the Fc region contained therein. In one embodiment, the Fc receptor is an Fcγ receptor, particularly a human Fcγ receptor. In some embodiments, the Fc region comprises a mutation that reduces binding to the Fcγ receptor. In still other preferred embodiments, the Fc fragment may have a mutation that results in an increased serum half-life, e.g., a mutation that improves the binding of the Fc fragment to FcRn.

The Anti-FRα antibody or the antigen-binding fragment thereof of the present invention may be in any form of an antibody or an antigen-binding fragment thereof known in the art, such as a monoclonal, chimeric, humanized, fully human, bispecific, or multispecific antibody or an antibody fragment thereof.

In some embodiments, the heavy chain of the anti-FRα antibody of the present invention comprises
comprises or consists of an amino acid sequence having at least 85%, 90%, 91%, 92%, 93%, 94%, 95%, 96%, 97%, 98%, or 99% identity to the amino acid sequence selected from SEQ ID NO: 35;
comprises or consists of an amino acid sequence selected from SEQ ID NO: 35; or
comprises or consists of an amino acid sequence having one or more (preferably no more than 20 or 10, and more preferably no more than 5, 4, 3, 2, or 1) amino acid changes (preferably amino acid substitutions, and more preferably conservative amino acid substitutions) compared with the amino acid sequence selected from SEQ ID NO: 35.

In some embodiments, the light chain of the anti-FRα antibody of the present invention comprises
comprises or consists of an amino acid sequence having at least 85%, 90%, 91%, 92%, 93%, 94%, 95%, 96%, 97%, 98%, or 99% identity to an amino acid sequence selected from SEQ ID NO: 36 or 37;
comprises or consists of an amino acid sequence selected from SEQ ID NO: 36 or 37; or
comprises or consists of an amino acid sequence having one or more (preferably no more than 20 or 10, and more preferably no more than 5, 4, 3, 2, or 1) amino acid changes (preferably amino acid substitutions, and more preferably conservative amino acid substitutions) compared with the amino acid sequence selected from SEQ ID NO: 36 or 37.

In some embodiments, the anti-FRα antibody or the antigen-binding fragment thereof of the present invention comprises two identical heavy chains and two identical light chains, wherein
the heavy chain comprises the VH as described herein or the HCDR1, the HCDR2 and the HCDR3 as described herein, and the Fc region and the CH1 as described herein, or the heavy chain constant region as described herein; and/or
the light chain comprises the VL described herein or the LCDR1, the LCDR2, and the LCDR3 as described herein, and the Kappa light chain constant region as described herein.

In some embodiments, the anti-FRα antibody or the antigen-binding fragment thereof of the present invention comprisies two identical heavy chains and two identical light chains, wherein
the heavy chain comprises or consists of an amino acid sequence having at least 85%, 90%, 91%, 92%, 93%, 94%, 95%, 96%, 97%, 98%, or 99% identity to an amino acid sequence set forth in SEQ ID NO: 35, and/or
the light chain comprises or consists of an amino acid sequence having at least 85%, 90%, 91%, 92%, 93%, 94%, 95%, 96%, 97%, 98%, or 99% identity to an amino acid sequence set forth in SEQ ID NO: 36.

In some embodiments, the anti-FRα antibody or the antigen-binding fragment thereof of the present invention comprises two identical heavy chains and two identical light chains, wherein
the heavy chain comprises or consists of an amino acid sequence set forth in SEQ ID NO: 35, and/or
the light chain comprises or consists of an amino acid sequence set forth in SEQ ID NO: 36.

In some embodiments, the anti-FRα antibody or the antigen-binding fragment thereof of the present invention comprises two identical heavy chains and two identical light chains, wherein
the heavy chain comprises the VH as described herein or the HCDR1, HCDR2, and HCDR3 as described herein, and the Fc region and the CH1 as described herein, or the heavy chain constant region as described herein;
the light chain comprises the VL as described herein or the LCDR1, LCDR2, and LCDR3 as described herein, and the Lambda light chain constant region comprising LLC160C and/or 166C as described herein.

In one specific embodiment, the anti-FRα antibody or the antigen-binding fragment thereof of the present invention comprises two identical heavy chains and two identical light chains, wherein
the heavy chain comprises or consists of an amino acid sequence having at least 85%, 90%, 91%, 92%, 93%, 94%, 95%, 96%, 97%, 98%, or 99% identity to an amino acid sequence set forth in SEQ ID NO: 35, and/or
the light chain comprises or consists of an amino acid sequence having at least 85%, 90%, 91%, 92%, 93%, 94%, 95%, 96%, 97%, 98%, or 99% identity to an amino acid sequence set forth in SEQ ID NO: 37.

In one more specific embodiment, the anti-FRα antibody or the antigen-binding fragment thereof of the present invention comprises two identical heavy chains and two identical light chains, wherein
the heavy chain comprises or consists of an amino acid sequence set forth in SEQ ID NO: 35, and/or
the light chain comprises or consists of an amino acid sequence set forth in SEQ ID NO: 37.

In some embodiments, the anti-FRα antibody or the antigen-binding fragment thereof of the present invention is a chimeric antibody or a humanized antibody.

In some embodiments, the antigen-binding fragment of the anti-FRα antibody of the present invention is, for example, Fv, Fab, Fab', Fab'-SH, or F(ab')₂; a domain antibody (dAb); a linear antibody; a single-chain antibody (e.g., scFv); a single-domain antibody (e.g., VHH); a bivalent antibody or a fragment thereof; or a camelid antibody.

In one embodiment, the anti-FRαThe antibody is a full-length antibody.

In one embodiment, the anti-FRαThe antibody also encompasses multispecific antibodies such as bispecific antibodies.

In some embodiments, the anti-FRα antibody or the antigen-binding fragment thereof of the present invention has relatively good endocytic activity on FRα-positive cells, e.g., FRα-positive tumor cells.

In some embodiments, the present invention also provides a fusion protein comprising the anti-FRα antibody or the antigen-binding fragment thereof of the present invention.

In some embodiments, the present invention also provides an immunoconjugate comprising the anti-FRα antibody or the antigen-binding fragment thereof of the present invention, and further comprising a payload coupled to the antibody or the antigen-binding fragment thereof. In some embodiments, the payload is a toxin, a small molecule drug/agent, a cytotoxic agent, an apoptotic agent, a chelating agent, an immunomodulator/immunostimulatory agent/immunoagonist, an oligonucleotide, a polypeptide, a peptide epitope, a radionuclide, a prodrug, etc. In some preferred embodiments, the payload is an immunostimulatory agent/immune activator. In some more preferred embodiments, the load is a TLR agonist. In some more preferred embodiments, the load is a TLR7/8 agonist.

In some embodiments, the present invention relates to an isolated nucleic acid encoding the anti-FRα antibody or the antigen-binding fragment thereof of the present invention.

In some embodiments, the present invention relates to a vector comprising the nucleic acid of the present invention, preferably the vector is an expression vector.

In some embodiments, the present invention relates to a host cell comprising the nucleic acid or vector of the present invention, preferably, the host cell is prokaryotic or eukaryotic, and more preferably, the host cell is selected from yeast cells, mammalian cells (e.g., 293 cells or CHO cells, such as CHO-K cells or HEK293 cells), or other cells suitable for preparing an antibody or an antigen-binding fragment thereof.

In some embodiments, the present invention relates to a method for preparing an anti-FRα antibody or an antigen-binding fragment thereof, which comprises
a) culturing the host cell of the present invention under conditions suitable for expressing a nucleic acid encoding the anti-FRα antibody or the antigen-binding fragment thereof of the present invention,
b) optionally isolating the antibody or antigen binding fragment thereof,
c) optionally, the method further comprises isolating the anti-FRα antibody or the antigen-binding fragment thereof from the host cell; optionally, the antibody is purified, e.g., by ProteinA.

In another aspect, the present invention provides a novel TLR agonist, especially a TLR 7/8 agonist, selected from compounds of the following formula or a pharmaceutically acceptable salt or solvate thereof:
(1) a compound of formula (D-1): 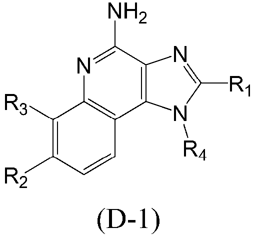 wherein:
   R₁ is C₁-C₆alkyl which is unsubstituted or substituted by one or more substituents selected from: hydroxy, halogen, cyano, C₃-C₆cycloalkyl, amino, NH(C₁-C₆alkyl), N(C₁-C₆alkyl)₂, C₁-C₆alkoxy and C₃-C₆cycloalkoxy;
   R₂ and R₃ are independently selected from hydrogen, C₁-C₆alkyl, hydroxy, halogen, cyano, amino, C₁-C₆alkoxy, arylC₁-C₆alkoxy and C₁-C₆alkoxycarbonyl, wherein said aryl is optionally substituted by one or more groups selected from C₁-C₆alkyl, hydroxy, halogen, cyano, amino and C₁-C₆alkoxy;
   R₄ is C₁-C₆alkyl which is unsubstituted or substituted by one or more substituents selected from: hydroxy, halogen, cyano, C₃-C₆cycloalkyl, arylC₁-C₆alkyl, amino, NH(C₁-C₆alkyl), N(C₁-C₆alkyl)₂, C₁-C₆alkoxy, NHR₅-substituted C₁-C₆alkoxy and NHR₅-substituted C₁-C₆alkylcarbonyloxy; wherein said aryl group is optionally substituted by one or more groups selected from C₁-C₆alkyl, hydroxy, halogen, cyano, amino, C₁-C₆alkoxy and C₁-C₆alkylamino;
   R₅ is selected from C₁-C₆alkyl and C₁-C₆alkylcarbonyl, wherein said C₁-C₆alkyl and C₁-C₆alkylcarbonyl are optionally substituted with one or more substituents selected from the group consisting of hydroxy, halo, amino, NH(C₁-C₆alkyl), N(C₁-C₆alkyl)₂, C₁-C₆alkoxy and C₃-C₆cycloalkoxy;
(2) a compound of formula (D-2): 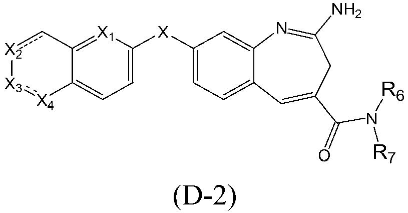 wherein:
   X is absent, or is -NH-(C=O)-, -(C=O)-NH-, or C₁-C₆alkylene, wherein said C₁-C₆alkylene is optionally substituted with one or more substituents selected from the group consisting of hydroxy and halogen, and wherein one or more carbon atoms therein may be replaced with a group selected from the group consisting of -NH- and -(C=O)-;
   X₁ is selected from CH or N;
   X₂ is selected from CH₂ or N;
   X₃ is NR₈, wherein R₈ is selected from H and C₁-C₆alkyl, and said C₁-C₆alkyl is optionally substituted with hydroxy, C₁-C₆alkoxy and amino substituted C₁-C₆alkoxy; X₄ is CH₂ or C(=O);
   R₆ and R₇ are each independently selected from H and C₁-C₆alkyl, and said C₁-C₆alkyl is optionally substituted by one or more groups selected from hydroxy, halogen, cyano, C₃-C₆cycloalkyl, amino, NH(C₁-C₆alkyl), N(C₁-C₆alkyl)₂ and C₁-C₆alkoxy; and
   - - - - - - represents a single or double bond;
(3) a compound of formula (D-3): 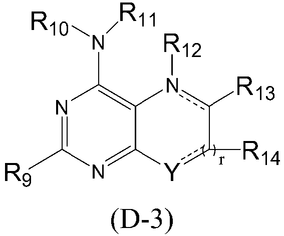 wherein:
   R₉ is selected from hydroxyl, amino, NH(C₁-C₆alkyl), N(C₁-C₆alkyl)₂ and C₁-C₆alkoxy;
   R₁₀ and R₁₁ are independently selected from hydrogen and C₁-C₈alkyl, and said C₁-C₈alkyl is optionally substituted with one or more substituents selected from the group consisting of hydroxy, halogen, C₁-C₆alkoxy and NR₁₅R₁₆, wherein R₁₅ and R₁₆ are independently selected from H, C₁-C₆acyl and hydroxy-substituted C₁-C₆acyl;
   R₁₂ is absent, or is selected from hydrogen and arylC₁-C₆alkyl, wherein said aryl is optionally substituted with one or more substituents selected from hydroxy, halogen, C₁-C₆alkoxy and heterocyclylC₁-C₆alkyl, and said heterocyclyl is optionally substituted with one or more substituents selected from C₁-C₆alkyl, C₁-C₆alkoxy-C₁-C₆alkyl and C₁-C₆acyl;
   R₁₃ is selected from hydrogen, halogen, hydroxy and oxo (=O);
   R₁₄ is selected from hydrogen, halogen, hydroxyl, C₁-C₆alkyl and C₁-C₆alkoxy;
   r is 0 or 1;
   Y is selected from C and NR₁₇, wherein R₁₇ is selected from hydrogen or C₁-C₆alkyl optionally substituted with aryl or heterocyclyl, wherein said aryl and heterocyclyl are optionally substituted with one or more substituents selected from C₁-C₆acyl, C₁-C₆alkyl, C₁-C₆alkoxy-C₁-C₆alkyl, hydroxyl-C₁-C₆alkyl and NR₁₈R₁₉-C₁-C₆alkyl, wherein R₁₈ and R₁₉ are independently selected from H, C₁-C₆alkyl and C₁-C₆acyl; and
   - - - - - - represents a single or double bond.

In one embodiment, the present invention provides a compound having a structure of formula (D-1) as described above, which has a structure represented by formula (D-1'): 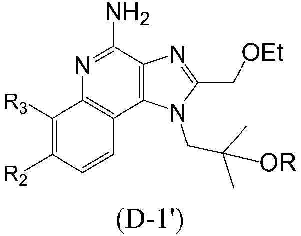 wherein:
R₂ and R₃ are as defined above; and
R is selected from H and 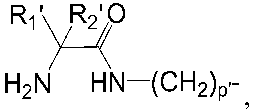
wherein R₁' and R₂' are independently selected from hydrogen and C₁-C₆alkyl, and p' is an integer selected from 1-6.

In one embodiment, the present invention provides a compound having a structure of formula (D-2) as described above, wherein R₆ and R₇ are selected from C₁-C₆alkyl, preferably n-propyl.

In one embodiment, the present invention provides a compound having a structure of formula (D-3) as described above, which has a structure represented by formula (D-3'): 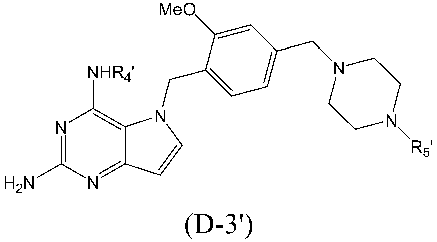 wherein
R₄' represents a C₃-C₆alkyl optionally substituted by one or more groups selected from hydroxy, halogen, amino and C₁-C₆alkoxy, preferably n-pentyl;
R₅' is selected from H, C₁-C₆alkyl, C₁-C₆alkoxy-C₁-C₆alkyl, C₁-C₆acyl, preferably selected from H, acetyl, and methoxyethyl.

In one embodiment, the present invention provides a compound having a structure of formula (D-3) as described above, which has a structure represented by formula (D-3"): 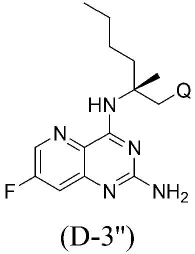 wherein:
Q is selected from OR₆' and NR₇'R₈';
wherein R₆' is selected from H and C₁-C₆alkyl;
R₇' and R₈' are independently selected from H, C₁-C₆acyl, hydroxy-substituted C₁-C₆acyl, preferably, R₇' and R₈' are both H, or one of R₇' and R₈ ' is H and the other is acetyl or hydroxyacetyl.

In one embodiment, the present invention provides a compound having a structure of formula (D-3) as described above, which has a structure represented by formula (D-3"'): 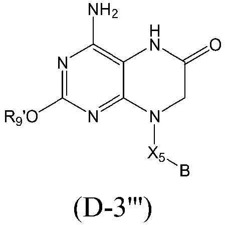 wherein:
R₉' is C₁-C₆alkyl, preferably straight-chain C₃-C₆alkyl, e.g. n-butyl;
r is 0 or 1;
X₅ is selected from C₁-C₆alkylene, e.g., -CH₂-, -CH₂-CH₂-, -CH₂-CH₂-CH₂-, - CH₂-CH₂-CH₂-CH₂-, -CH₂-CH₂-CH₂-CH₂-CH₂-, -CH₂-CH₂-CH₂-CH₂-CH₂-CH₂-;
B is selected from the group consisting of aryl and heterocyclyl, e.g., phenyl, tetrahydroisoquinolinyl, and piperidinyl, wherein said aryl and heterocyclyl are optionally substituted with C₁-C₆acyl, C₁-C₆alkyl, C₁-C₆alkoxy-C₁-C₆alkyl, hydroxyl-C₁-C₆alkyl or NR'R"-C₁-C₆alkyl, wherein R' and R" are independently selected from H, C₁-C₆alkyl and C₁-C₆acyl.

In one embodiment, the present disclosure provides a compound having a structure of formula (D-1) as described above, which has a structure represented by formula (D-1"): 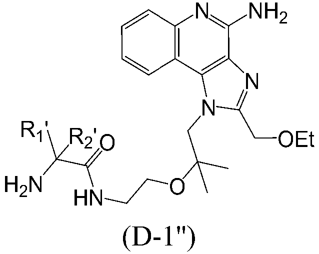 wherein R₁' and R₂' are independently selected from hydrogen and C₁-C₆alkyl, preferably, R₁' and R₂' are both methyl, or R₁' is hydrogen and R₂' is 3,3-dimethylbutyl.

In one embodiment, the present disclosure provides the compound as defined above, which is selected from the following compounds: 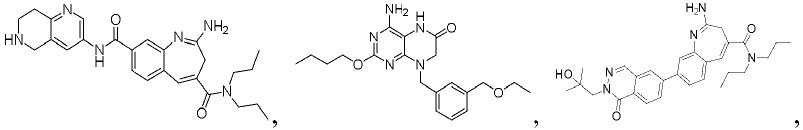 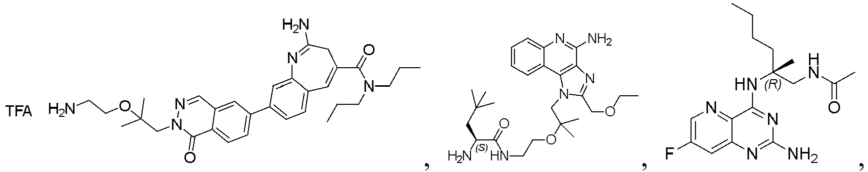 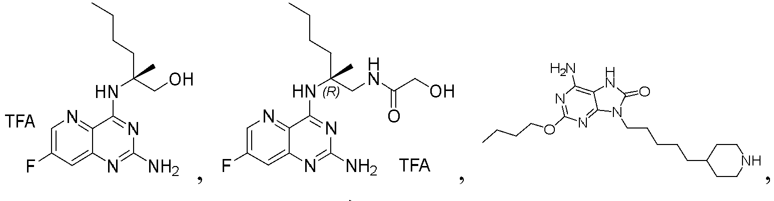 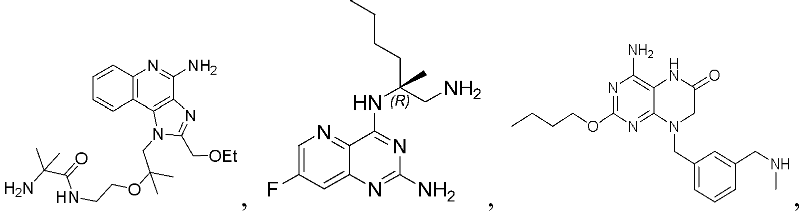 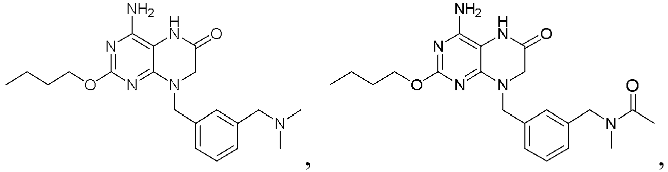 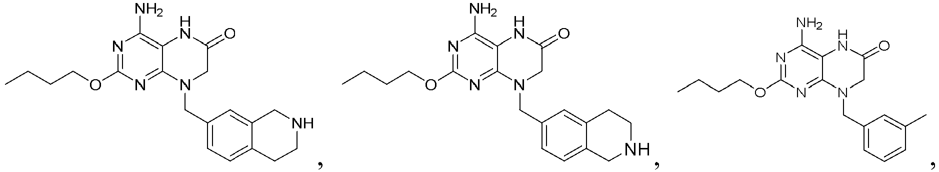 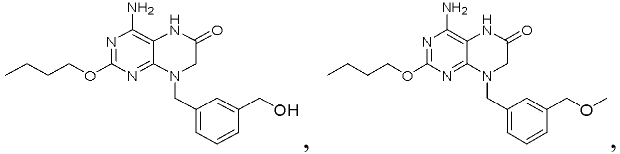 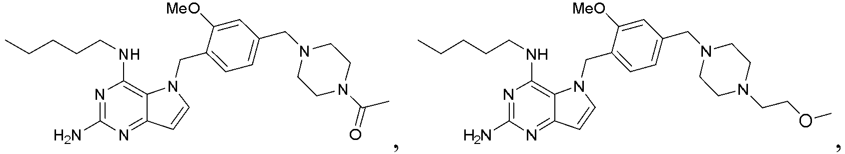 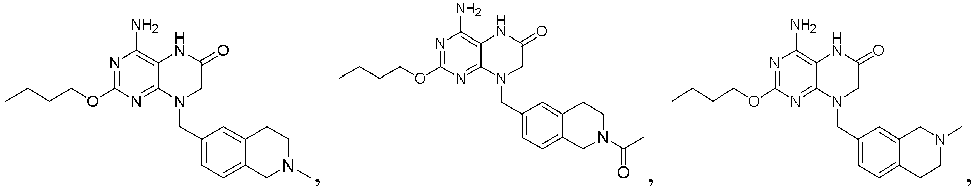 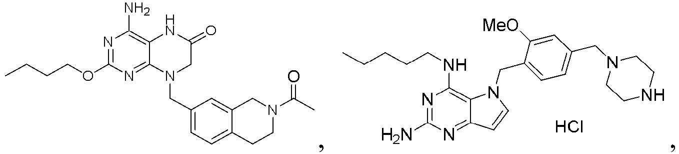 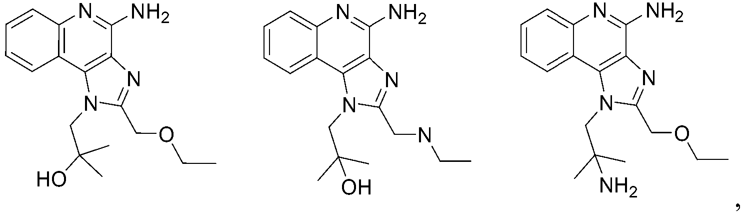 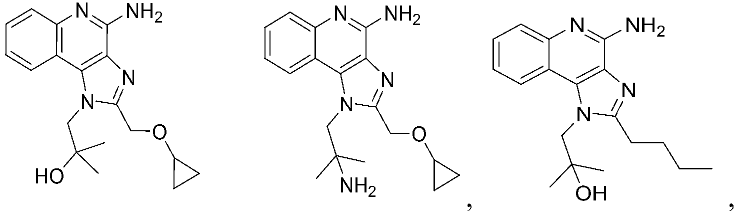 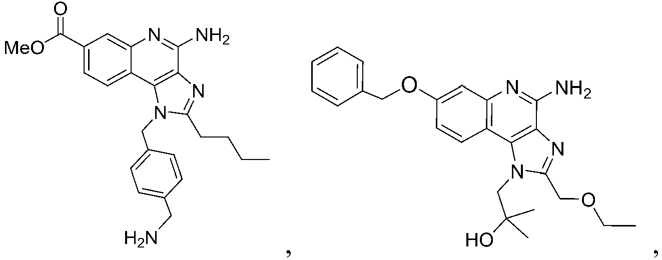 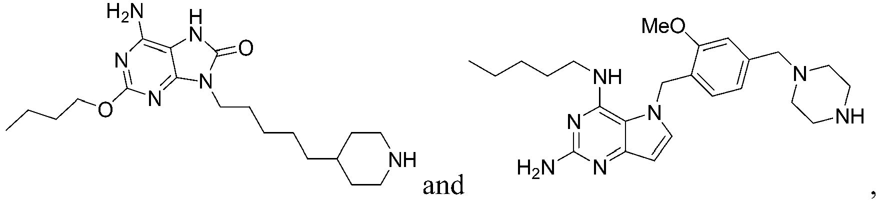 preferably, the compound is selected from the following compounds:

| **Compound No.** | **Structural formula** | **MW.** |
|---|---|---|
| Compound A1 | 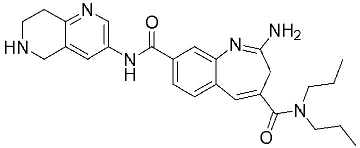 | 460.58 |
| Compound A2 | 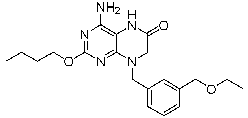 | 385.47 |
| Compound A3 | 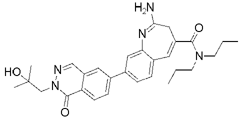 | 501.63 |
| Compound A4 | 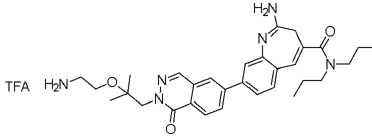 | 641.72 |
| Compound A5 | 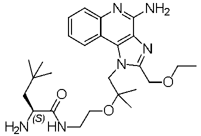 | 484.64 |
| Compound A6 | 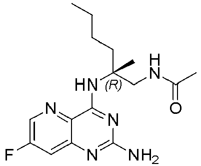 | 431.42 |
| Compound A7 | 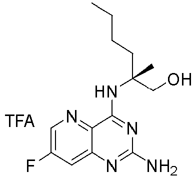 | 390.36 |
| Compound A8 | 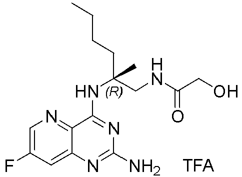 | 448.18 |
| Compound A9 | 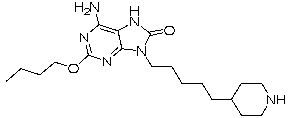 | 376.51 |
| Compound A10 | 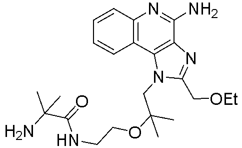 | 442.3 |
| Compound A11 | 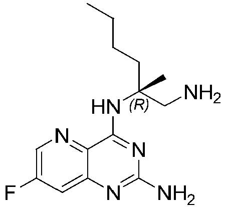 | 292.18 |
| Compound A12 | 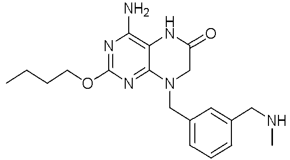 | 370.457 |
| Compound A13 | 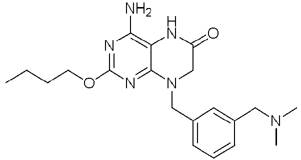 | 384.22 |
| Compound A14 | 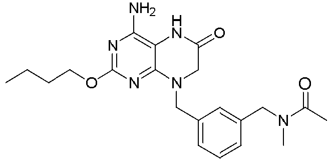 | 412.494 |
| Compound A15 | 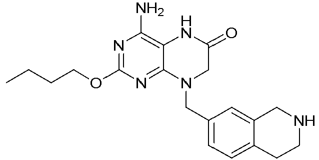 | 418.926 |
| Compound A16 | 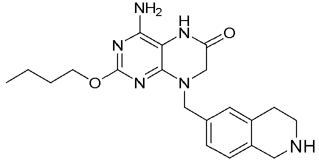 | 418.926 |
| Compound A17 | 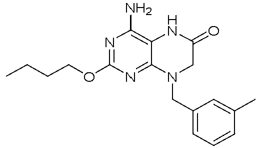 | 341.415 |
| Compound A18 | 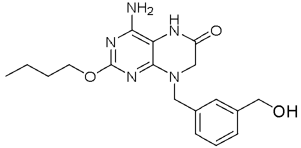 | 357.414 |
| Compound A19 | 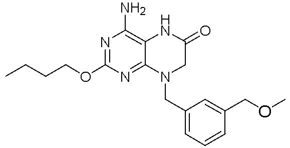 | 371.441 |
| Compound A20 | 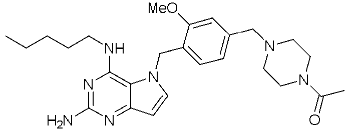 | 479.629 |
| Compound A21 | 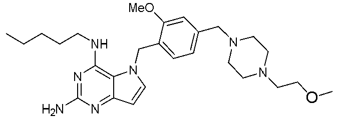 | 495.672 |
| Compound A22 | 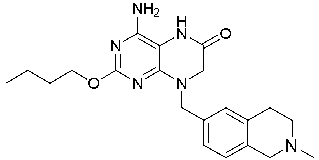 | 396.495 |
| Compound A23 | 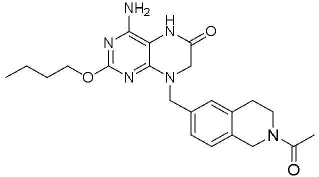 | 424.505 |
| Compound A24 | 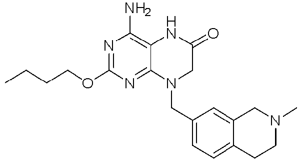 | 396.495 |
| Compound A25 | 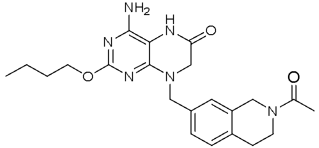 | 424.505 |
| Compound A26 | 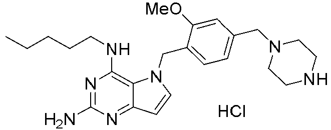 | 474.05 |

In some embodiments, the compound of the present invention has balanced TLR7 and TLR8 stimulatory activity.

In a third aspect, the invention provides a TLR agonist linked to a linker, selected from a compound of formula (II), or a pharmaceutically acceptable salt or solvate thereof:

L-(D)ₘ (II)

wherein:
D is a TLR agonist as defined in the second aspect;
L has the following structure:

Z'-L₁₋E-L₂-L₃-

wherein L₃ is linked to D;
Z' is selected from 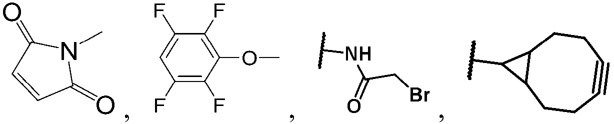 and 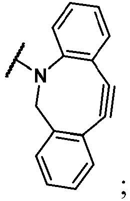
L₁ is -(CH₂)ₙ-C(=O)- or -(CH₂)ₙ-(O(CH₂)₂)ₜ(CH₂)ₙ-C(=O)-;
E is absent, or is a peptide residue comprising 2 to 10 amino acids, wherein the amino acid residues is a natural amino acid residue or a non-natural amino acid residue and is optionally substituted by C₁-C₆alkyl, and the C-terminus of the peptide residue is covalently linked to L₂;
L₂ is absent, or is 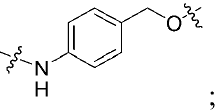
L₃ is absent, or is -C(=O)-*, -C(=O)((CH₂)ₙO)ₜ(CH₂)ₙ-* or -NH(CH₂)-*, wherein * indicates that the terminus is covalently linked to D;
n is an integer from 0 to 10,
t is an integer from 1 to 10,
m is an integer from 1 to 6.

In one embodiment, the present invention provides the compound of formula (II) as described above, or a pharmaceutically acceptable salt or solvate thereof, wherein
E has a structure represented by the following formula:

   -X₁-X₂-
wherein X₁ represents -(N(R⁰)C(R⁰)₂C(=O))ₛ-, wherein R⁰ independently of one another represent H or C₁-C₆alkyl; s = 0-10, preferably 2, 4, 6 and 8;
X₂ is absent, or is selected from

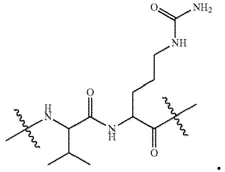

In one embodiment, the present invention provides the compound of formula (II) as described above, or a pharmaceutically acceptable salt or solvate thereof, wherein
E is selected from 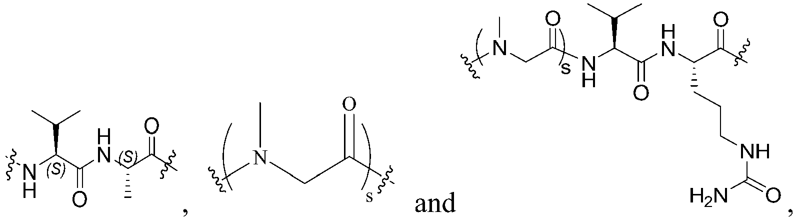
where s = 0-8.

In one embodiment, the present invention provides the compound of formula (II) as described above, or a pharmaceutically acceptable salt or solvate thereof, wherein
L- has a structure selected from: 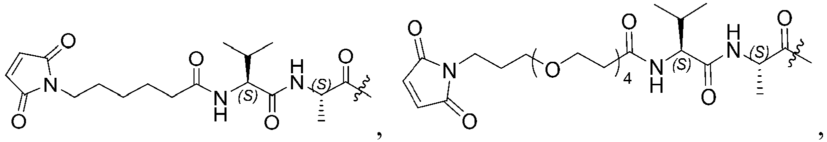 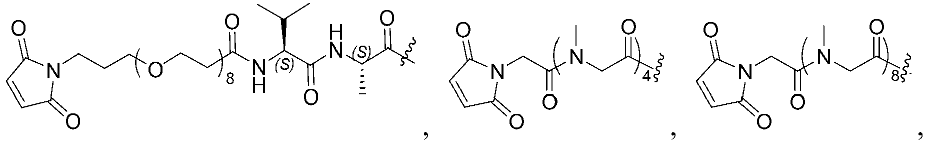 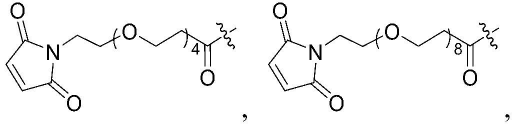 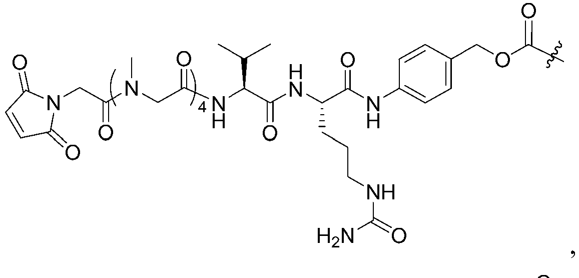 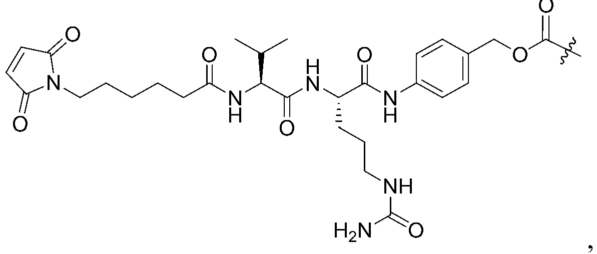 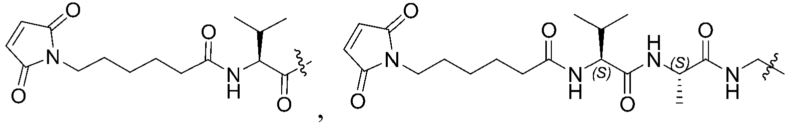 and

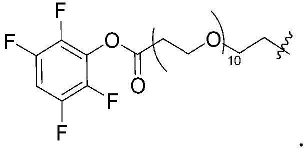

In one embodiment, the present invention provides the compound of formula (II) as described above, or a pharmaceutically acceptable salt or solvate thereof, selected from the group consisting of:
In one embodiment, the compound of formula (II) is selected from the following compounds

| **MW.** | **Compound No.** | **Structural formula** |
|---|---|---|
| 677.79 | Compound 1 | 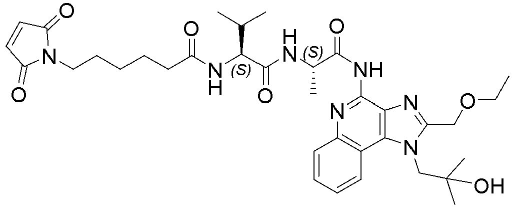 |
| 811.92 | Compound 2 | 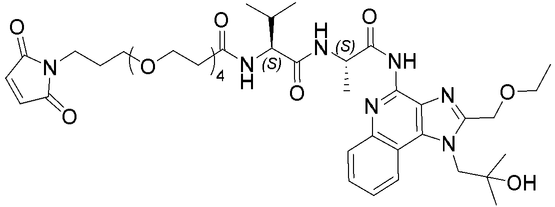 |
| 988.15 | Compound 3 | 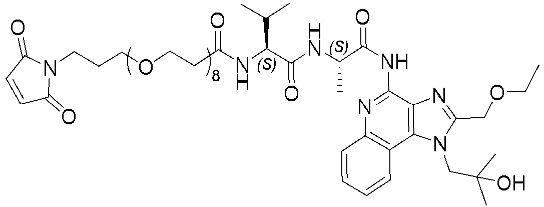 |
| 811.98 | Compound 4 | 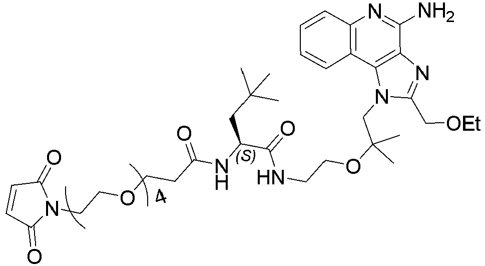 |
| 906.06 | Compound 5 | 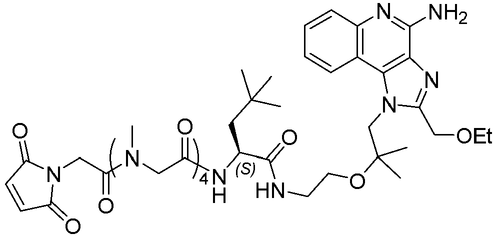 |
| 1190.37 | Compound 6 | 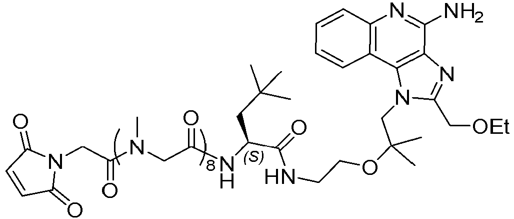 |
| 1310.52 | Compound 7 | 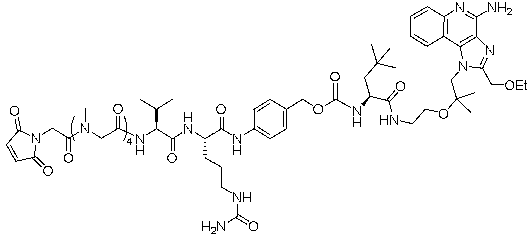 |
| 1083.30 | Compound 8 | 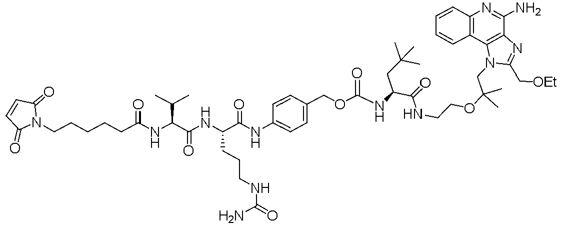 |
| 1041.22 | Compound 9 | 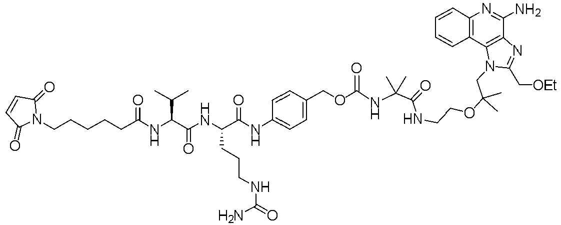 |
| 787.92 | Compound 10 | 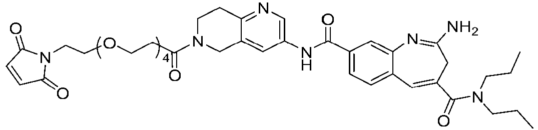 |
| 964.13 | Compound 11 | 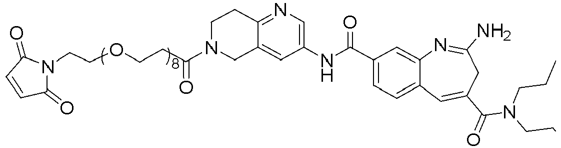 |
| 881.99 | Compound 12 | 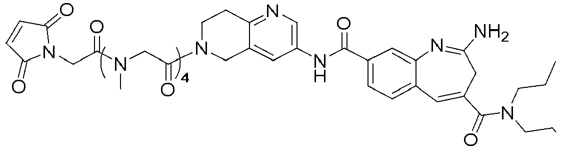 |
| 706.85 | Compound 13 | 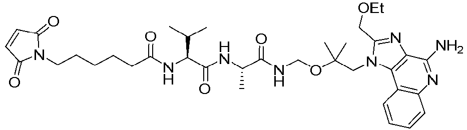 |
| 975.16 | Compound 14 | 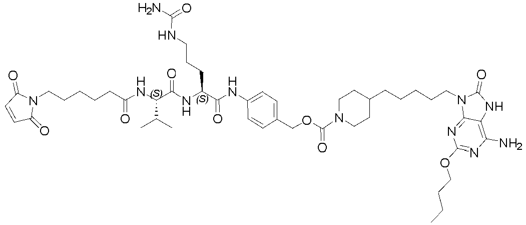 |
| 981.125 | Compound 15 | 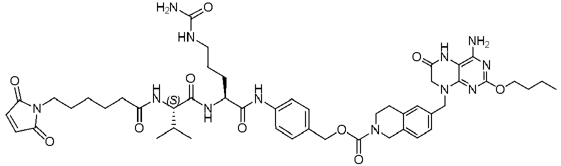 |
| 1059.23 | Compound 16 | 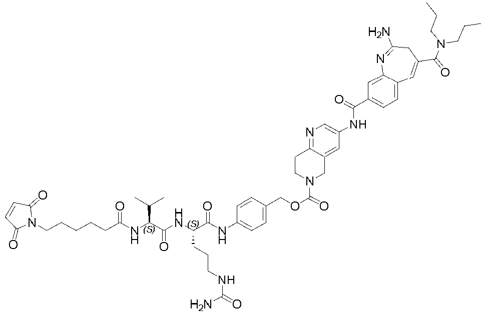 |
| 1036.25 | Compound 17 | 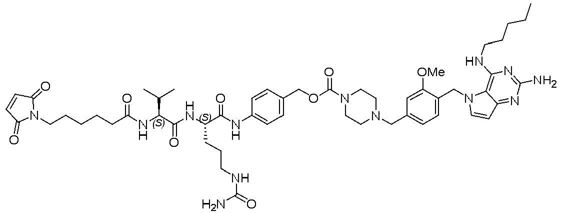 |
| 706.845 | Compound 18 | 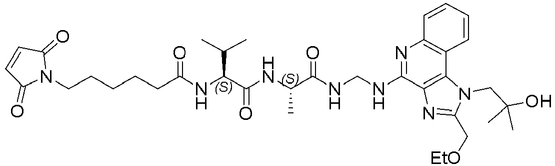 |
| 1098.5 | Compound 19 | 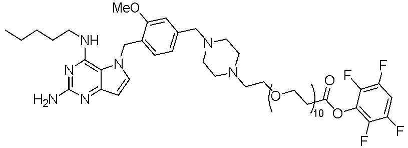 |

In some embodiments, the present invention also provides an immunoconjugate comprising a targeting moiety, the TLR agonist of the present invention, and/or the linker for linking the targeting moiety to the TLR agonist of the present invention. In some embodiments, the targeting moiety is a specific antigen-binding protein or a fragment thereof, e.g., a molecule having an antibody or immunoglobulin structure, a free receptor, a cyclic peptide, and the like. In some preferred embodiments, the targeting moiety is an antibody or an antigen-binding fragment thereof. In some preferred embodiments, the targeting moiety (e.g., an antibody) causes and/or promotes the activation of immune cells, e.g., by ADCP effect.

In some preferred embodiments, the immunoconjugate of the present invention comprises TLR agonists (e.g., a TLR 7/8 agonist of the invention as defined above), preferably conjugated to a tumor targeting antibody. The immunoconjugates of the present invention are well tolerated in vivo when administered systemically and elicit strong activation of intratumoral myeloid cells, leading to tumor clearance and subsequent immunological memory.

In some embodiments, the present invention provides an immunoconjugate having a structure of formula (I),

Ab-(L-(D)ₘ)ₚ (I)

or a pharmaceutically acceptable salt or solvate thereof,
wherein:
Ab is a specific antigen-binding protein or a fragment thereof, preferably a monoclonal antibody or a fragment thereof;
L is a linker;
D is a TLR agonist, preferably a TLR 7/8 agonist; and
m is an integer from 1 to 6; and
p is a number from 1 to 16, e.g. 2 to 10. In some embodiments, D in the formula (I) is the compound as defined in the second aspect of the present invention.

In some embodiments, L-D in formula (I) is the compound of formula (II) as defined in the third aspect of the present invention.

In some embodiments, -L- in formula (I) has a structure selected from: 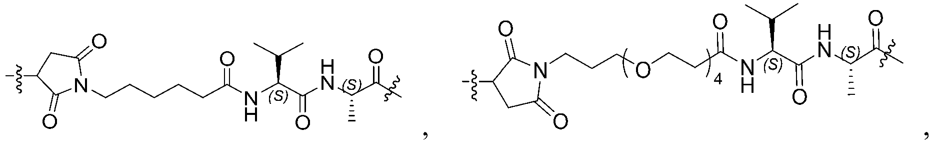 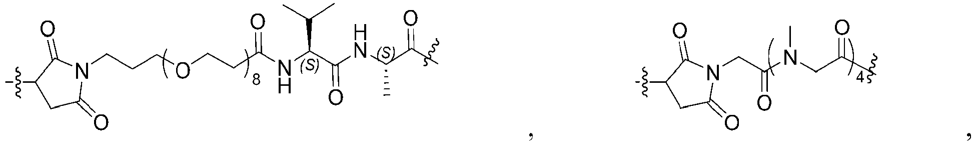 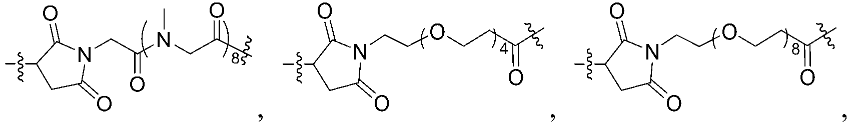 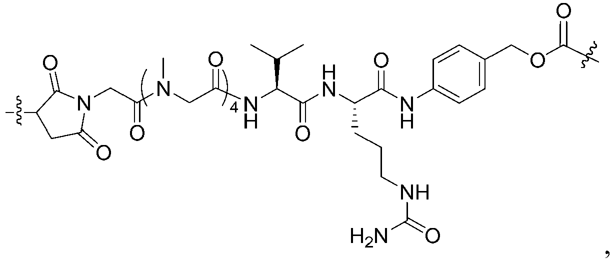 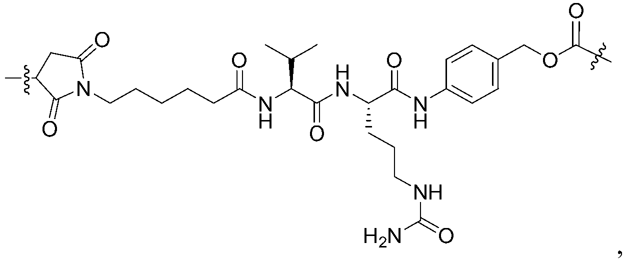 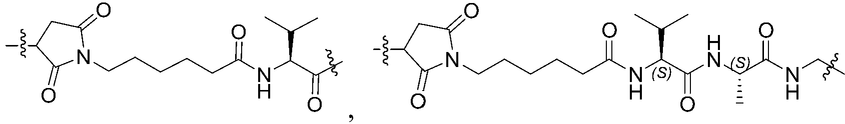 and 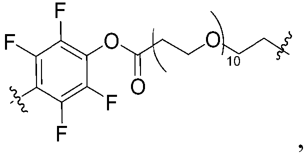 wherein the left side of the structure is linked to Ab, and the right side is linked to D.

In some embodiments, in formula (I), L-(D)ₘ has a structure selected from: 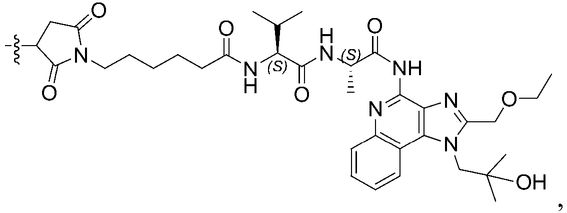 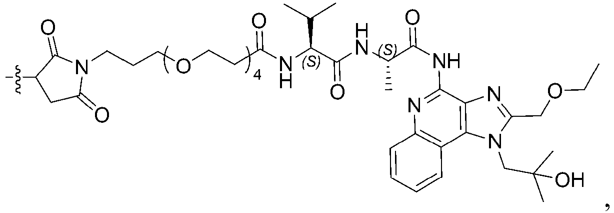 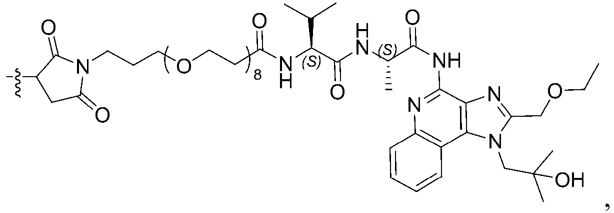 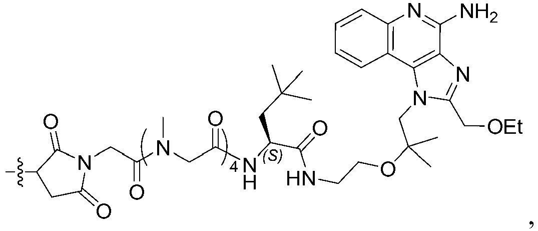 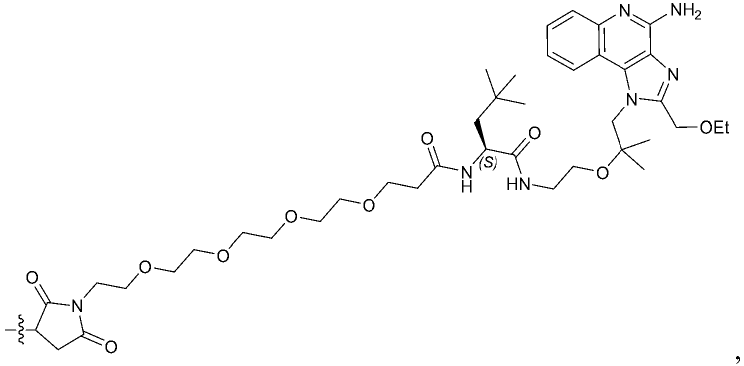 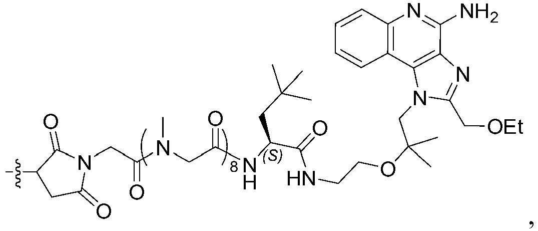 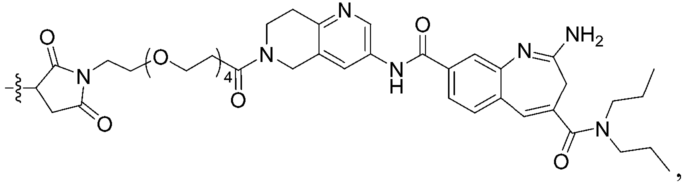 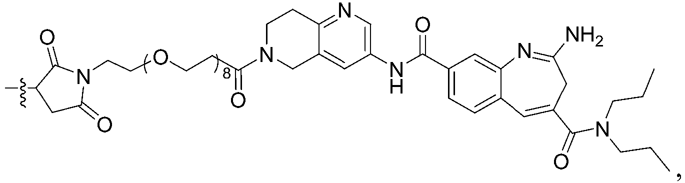 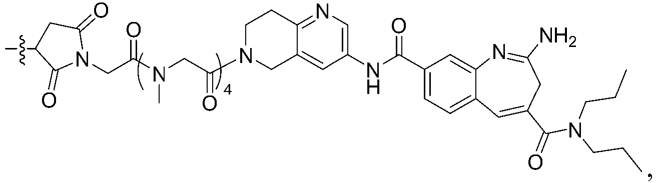 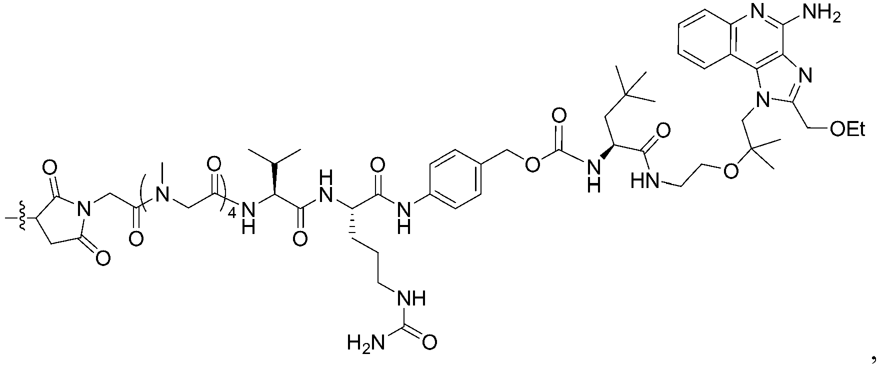 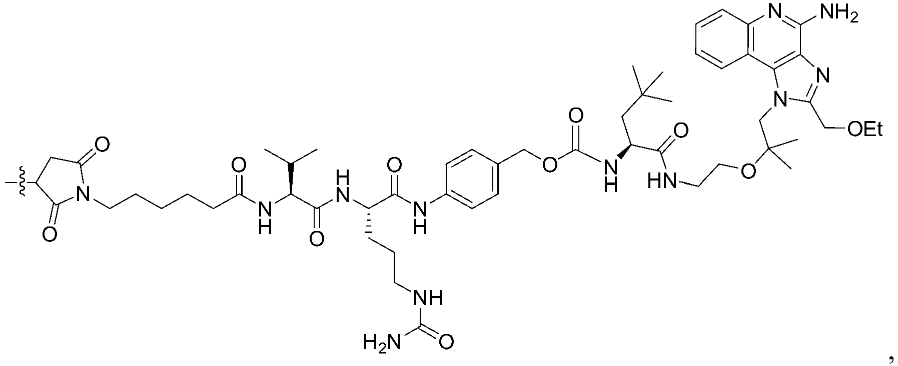 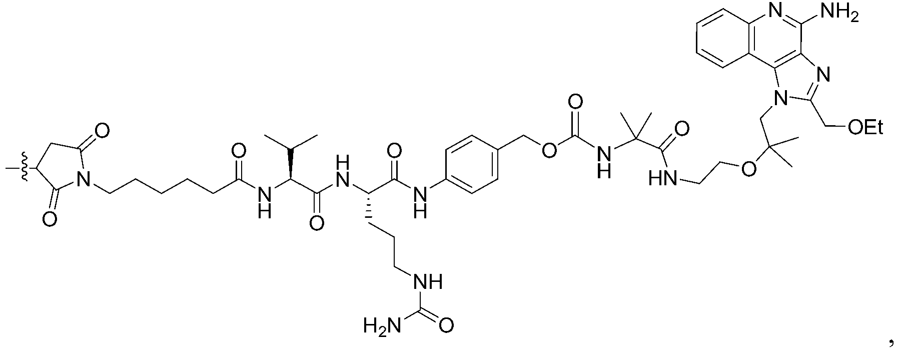 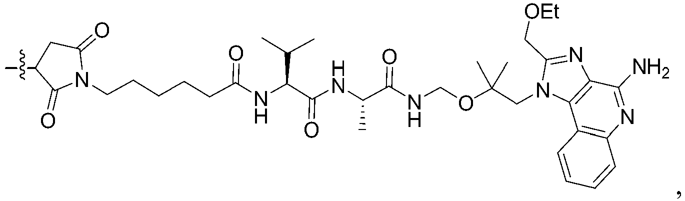 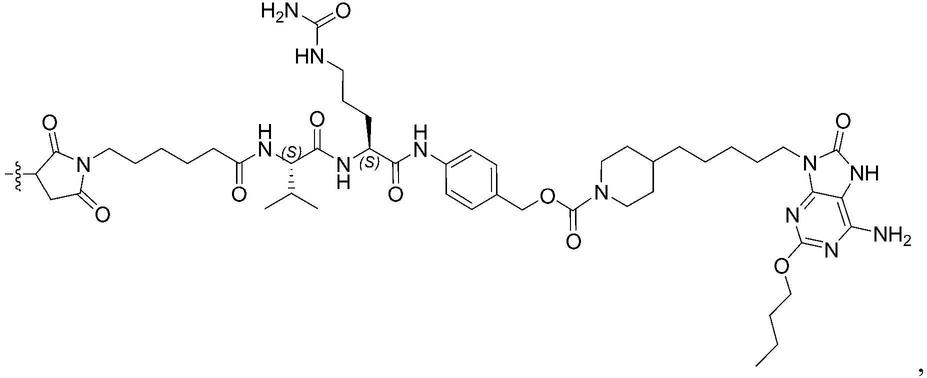 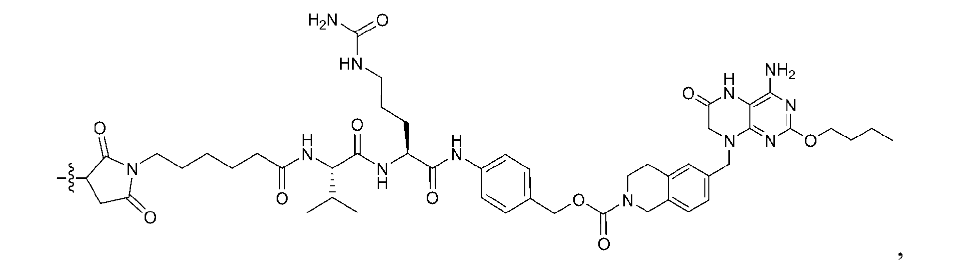 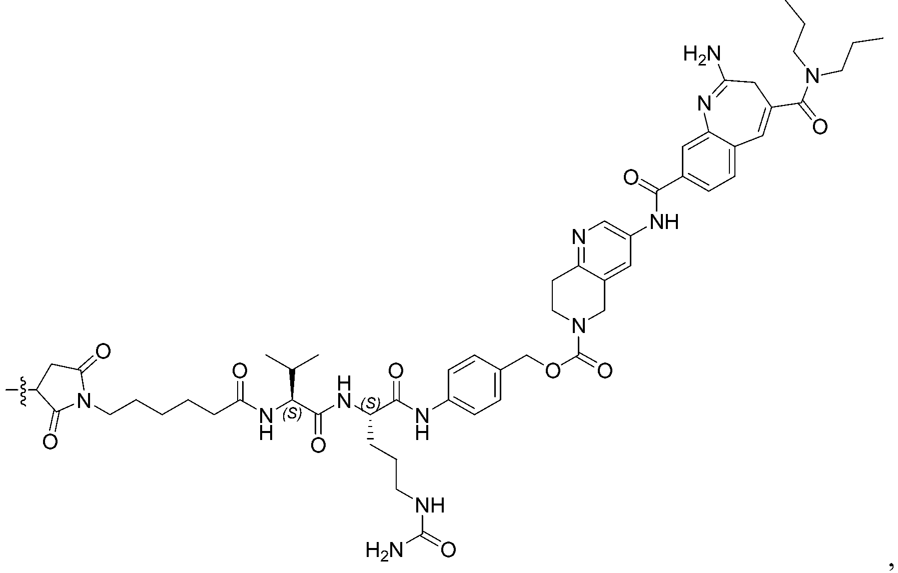 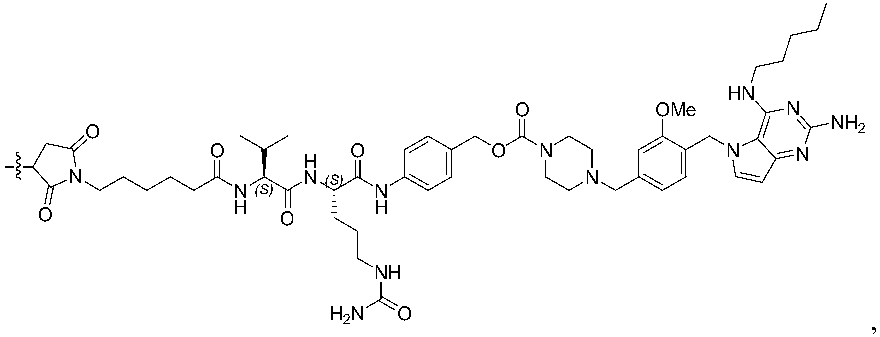 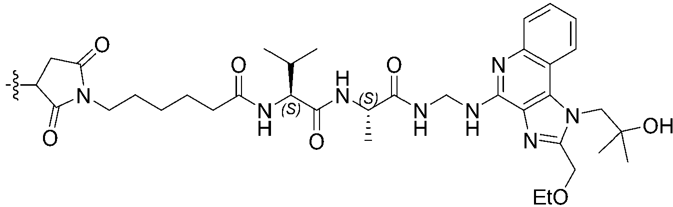 and

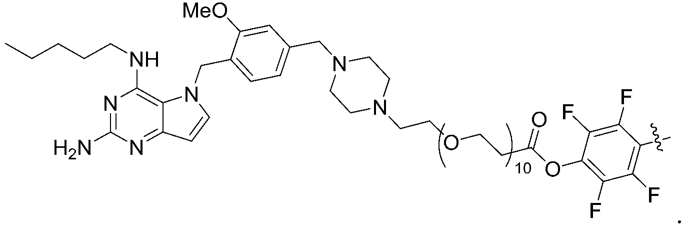

In some embodiments, the antibody-immunoagonist conjugate of the present invention or a pharmaceutically acceptable salt or a solvate thereof has a DAR value of 1 to 10, e.g., 2 to 8.

In some embodiments, the antibody-immunoagonist conjugate is selected from 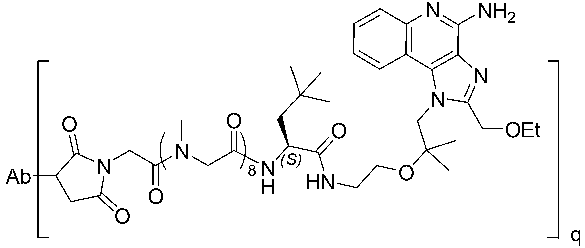 wherein Ab is an antibody or an antigen-binding fragment thereof that binds to TROP2 (e.g., human TROP2) or FRα (e.g., human FRα), preferably as defined above; q represents DAR, e.g., 1 to 10, e.g., 2 to 8, e.g., about 3, 4, 5, or 6.

In some embodiments, Ab in formula (I) is a specific antigen-binding protein or a fragment thereof. In some embodiments, Ab is an antibody. In some embodiments, Ab is an antibody fragment. In some embodiments, Ab is a monoclonal antibody or a fragment thereof. In some preferred embodiments, the Ab causes and/or promotes the activation of immune cells, e.g., by ADCP. In some preferred embodiments, Ab specifically binds to TROP2. In some preferred embodiments, Ab specifically binds to FRα. In some preferred embodiments, Ab has a cysteine mutation in its constant region.

In some embodiments, the antibody-immunoagonist conjugate of formula (I) or the subformula thereof of the present invention, or a composition comprising one or more of the antibody-immunoagonist conjugates (e.g., TROP2-ISAC or FRα-ISAC) has an average DAR of any value selected from the following ranges: 1 ± 0.4, 2 ± 0.4, 3 ± 0.4, or 4 ± 0.4.

In some preferred embodiments, Ab in the formula (I) is an antibody or an antigen-binding fragment thereof that specifically binds to TROP2. In some preferred embodiments, the antibody that specifically binds to TROP2 binds to a different epitope than a TROP2 cytotoxic ADC.

In some preferred embodiments, Ab in the formula (I) is an antibody or an antigen-binding fragment thereof that specifically binds to FRα. In some preferred embodiments, the antibody that specifically binds to FRα binds to a different epitope than a FRα cytotoxic ADC.

In some embodiments, in the antibody-immunoagonist conjugate comprising the anti-FRα antibody or an antigen-binding fragment thereof of the present invention, the cysteine mutation enables the anti-FRα antibody or the antigen-binding fragment thereof to be conjugated to the linker via the mutated cysteine, for example, to achieve site-directed conjugation of the immuneagonist.

In another aspect, the present invention provides a pharmaceutical composition comprising the antibody or the fragment thereof, the nucleic acid, the vector, the host cell, the fusion protein, the immunoconjugate or the pharmaceutically acceptable salt or the solvate thereof described above, and optionally one or more additional therapeutic agents, e.g., a chemotherapeutic agent, an angiogenesis inhibitor, a cytokine, a cytotoxic agent, an additional antibody, a small molecule drug or an immunomodulatory agent, and optionally pharmaceutical supplementary material. In another aspect, the present invention provides a pharmaceutical combination comprising the antibody or the fragment thereof, the nucleic acid, the vector, the host cell, the fusion protein, the immunoconjugate or the pharmaceutically acceptable salt or the solvate thereof, or the pharmaceutical composition described above, and one or more additional therapeutic agents, such as a chemotherapeutic agent, an angiogenesis inhibitor, a cytokine, a cytotoxic agent, an additional antibody, a small molecule drug, or an immunomodulatory agent.

In another aspect, the present invention provides a method for preventing or treating a tumor in a subject, comprising administering to the subject an effective amount of the antibody or the fragment thereof, the nucleic acid, the vector, the host cell, the fusion protein, the immunoconjugate or the pharmaceutically acceptable salt or the solvate thereof described above, or the pharmaceutical composition described above, or the pharmaceutical combination described above.

In another aspect, the present invention provides use of the antibody or the fragment thereof, the nucleic acid, the vector, the host cell, the fusion protein, the immunoconjugate or the pharmaceutically acceptable salt or solvate thereof of the present invention, or the pharmaceutical composition described above, or the pharmaceutical combination described above, in preparing an anti-tumor agent.

In some embodiments, the tumor is a cancer; preferably, the cancer has an elevated level (e.g., at the nucleic acid or protein level) of TROP2 or FRα, for example, as compared to a healthy individual or healthy tissue adjacent to the patient's cancer tissue.

In some embodiments, the cancer is selected from lung cancer, melanoma, head and neck tumor, prostate cancer, esophageal cancer, cervical cancer, kidney cancer, bladder cancer, ovarian cancer, pancreatic cancer or breast cancer.

In some embodiments, the method further comprises administering to the patient one or more therapies, e.g., therapeutic modalities and/or additional therapeutic agents; preferably, the therapeutic modalities comprise radiation therapy or surgery, the therapeutic agents comprise a chemotherapeutic agent, an angiogenesis inhibitor, a cytokine, a cytotoxic agent, an additional antibody, a small molecule drug, or an immunomodulatory agent.

The anti-TROP2 antibody and/or the immunoconjugate of the present invention has at least the following advantages:
(1) binding to human and cynomolgus TROP2 with high affinity;
(2) having excellent in vivo anti-tumor efficacy;
(3) exhibiting good safety in animal experiments, such as mice and monkeys; and
(4) targeting non-competitive epitopes compared to other immunoconjugates, such as TROP2 ADCs, making it favorable for combination/combination therapy.

The immunoconjugate of the present invention particularly has the following advantages:
(1) As a TROP2 ISAC, it has more significant monotherapy efficacy and has good safety in mice and monkeys compared with other ISACs with similar efficacy.
(2) It utilizes polysarcosine/polyethylene glycol (PEG) as a stable linker to avoid high toxicity associated with the released TLR7/8 agonist payload and avoid immunogenicity of the PEG linker.
(3) As a monotherapy, the TROP2-TLR7/8 agonist ISAC exhibits potent anti-tumor activity in the treatment of various TROP2+ xenograft tumor models, including, for example, gastric cancer and pancreatic cancer.

The anti-FRα antibody and/or the immunoconjugate of the present invention has at least the following advantages:
(1) binding to human, cynomolgus and mouse FRα with high affinity;
(2) having excellent in vivo anti-tumor efficacy;
(3) exhibiting good safety in animal experiments, for example, in mice;
   and
(4) targeting non-competitive epitopes compared to other immunoconjugates, such as FRα ADCs, making it favorable for combination/combination therapy.

The immunoconjugate of the present invention particularly has the following advantages:
(1) As an FRα ISAC, it has more significant monotherapy efficacy and has good safety in mice compared with other ISACs with similar efficacy.
(2) It utilizes polysarcosine/polyethylene glycol (PEG) as a stable linker to avoid high toxicity associated with the released TLR7/8 agonist payload and avoid immunogenicity of the PEG linker.
(3) As a monotherapy, the FRα-TLR7/8 agonist ISAC exhibits potent anti-tumor activity in the treatment of FRα+ xenograft tumor models, e.g., various types of ovarian cancer.
(4) The immune agonist is conjugated to the antibody via a mutated cysteine site, thereby
   (i) the conjugate is more hydrophilic,
   (ii) the conjugate is more stable in plasma, and the small molecule toxin does not easily detach;
   (iii) the conjugate has stronger efficacy in animals, for example, ISAC with an average DAR of 4 demonstrates efficacy comparable to that of a conjugate of an antibody with a DAR of 8 without cysteine mutation engineering.

### BRIEF DESCRIPTION OF THE DRAWINGS:

FIGs. 1A-1C show the results of antibody-dependent cellular phagocytosis (ADCP) functional assay.
FIG. 2 shows the results of antibody-dependent cellular phagocytosis (ADCP) functional assay of humanized antibodies.
FIG. 3 shows the results of macrophage endocytosis functional assay of humanized antibodies.
FIGs. 4A-4D show the assay results for the activity of the compounds of the present invention in H-TLR7 NFKB Reporter 293 and H-TLR8 NFKB Reporter 293 cell lines, wherein FIGs. 4A-4B show the TLR7 activity of the compounds, and FIGs. 4C-4D show the TLR8 activity of the compounds.
FIG. 5 shows the detection results of TROP2-ISAC of the present invention in a co-culture system of PBMCs and tumor cells.
FIGs. 6A to 6D show the anti-tumor effect of TROP2-ISAC of the present invention in the NCI-N87 model, wherein FIGs. 6A and 6C show the change in tumor volume over time, and FIGs. 6B and 6D show the change in animal body weight over time.
FIGs. 7A and 7B show the anti-tumor effect of TROP2-ISAC of the present invention in the BxPC3 model, wherein FIG. 7A shows the change in tumor volume over time, and FIG. 7B shows the change in animal body weight over time.
FIGs. 8A and 8B show the results of toxicity test of TROP2-ISAC of the present invention in BALB/C mice.
FIG. 9 shows the results of toxicity test of TROP2-ISAC of the present invention in hTROP2 KI mice.
FIGs. 10A to 10C show the anti-tumor effect of TROP2-ISAC of the present invention in the CT26-hTROP2 model, wherein FIG. 10A shows the change in tumor volume over time, FIG. 10B shows the change in animal body weight over time, FIG. 10C shows the change in animal survival rate over time.
Figs. 11A and 11B show the anti-tumor effect of TROP2-ISAC of the present invention in combination with TROP2-ADC in BxPC3 model, wherein FIG. 11A shows the change in tumor volume over time, and FIG. 11B shows the change in animal body weight over time.
Figs. 12A and 12B show the anti-tumor effect of TROP2-ISAC of the present invention in combination with TROP2-ADC in LK-2 model, wherein FIG. 12A shows the change in tumor volume over time, and FIG. 12B shows the change in animal body weight over time.
Figs. 13A and 13B show the results of high-dose toxicity test of TROP2-ISAC of the present invention in BALB/C mice.
FIG. 14 shows the ADCP activity of anti-FRα antibody clone BC1254-A1 in comparison with other antibodies.
FIG. 15 shows the macrophage ADCP activity of anti-FRα antibody clone BC1254-A1.
Figure 16 shows average DAR values of BC1254-A1 V2-Compound 6 conjugates determined by RP-HPLC.
Figure 17 shows average DAR values of BC1254-A1-Compound 6 conjugates determined by HIC-HPLC.
FIG. 18 shows the comparison of the ADCP activities of anti-FRα antibody clone BC1254-A1 before and after cysteine mutation engineering, and before and after conjugation to an immune activator.
FIG. 19 shows the immunostimulatory effect of anti-FRα-ISAC in a co-culture system with PBMCs and target tumor cells as assessed by measuring TNFα secretion.
FIG. 20 shows the immunostimulatory effect of anti-FRα-ISAC in a co-culture system with PBMCs and target tumor cells as assessed by measuring IFNα secretion.
FIGs. 21A and 21B show line graphs illustrating changes in tumor volume and mouse body weight over time of model mice with subcutaneous OV90 tumor formation after receiving treatment with FRα-ISAC, respectively.
FIGs. 22A and 22B show line graphs illustrating changes in tumor volume and mouse body weight over time of model mice with subcutaneous A2780cisR-FRα tumor formation after receiving treatment with FRα-ISAC, respectively.
FIGs. 23A and 23B show line graphs illustrating changes in tumor volume and mouse body weight over time of model mice with subcutaneous A2780cisR-FRα tumor formation after receiving combined treatment of FRα-ISAC and FRα-ADC, respectively.
FIG. 24 shows the assessment of toxicity of FRα-ISAC in mice by monitoring changes in body temperature.

### Detailed Description Of The Invention

### I. DEFINITONS

Before the present invention is described in detail below, it is to be understood that this invention is not limited to the particular methodology, protocols, and reagents described herein, as these may vary. It should also be understood that the terms used herein are merely intended to describe specific embodiments rather than limit the scope of the present invention, which will be limited only by the appended claims. Unless otherwise defined, all technical and scientific terms used herein have the same meaning as commonly understood by those of ordinary skill in the art to which the present invention belongs.

For the purpose of explaining this specification, the following definitions will be used, and wherever appropriate, terms used in the singular form may also include the plural form, and vice versa. It is to be understood that the terminology used herein is for the purpose of describing particular embodiments only and is not intended to be limiting.

The term "about" used in combination with a numerical value is intended to encompass the numerical values in a range from a lower limit less than the specified numerical value by 5% to an upper limit greater than the specified numerical value by 5%.

As used herein, the term "and/or" refers to any one of the options or any two or more of the options.

As used herein, the term "comprise" or "include" is intended to mean that the described elements, integers or steps are included, but not to the exclusion of any other elements, integers or steps. The term "comprise" or "include" used herein, unless otherwise indicated, also encompasses the situation where the entirety consists of the described elements, integers or steps. For example, when referring to an antibody variable region "comprising" a particular sequence, it is also intended to encompass an antibody variable region consisting of the particular sequence.

The terms "TROP2" and "TACD2" are used interchangeably herein and refer to a human trophoblast cell surface glycoprotein antigen, which is a type I cell surface glycoprotein highly expressed in human cancers.It was originally identified as an antigen present on tumors of the human gastrointestinal tract, and is the second of two members of the family. The sequences of mouse and cynomolgus TROP2 have high homology to their human counterparts. In some embodiments of the present invention, TROP2 is human TROP2. In some embodiments, TROP2 is a protein under UniProtDatabase Accession No.P09758 .

The term "anti-TROP2 antibody", "anti-TROP2", "TROP2 antibody" or "anti-TROP2 antibody" as used herein refers to an antibody or an antigen-binding fragment thereof capable of binding to a TROP2 protein with sufficient affinity. The antibody can be used as a diagnostic agent and/or a therapeutic agent targeting TROP2, or used to construct an immunoconjugate, e.g., an antibody-immunoagonist conjugate.

The terms "FOLR1" and "FRα" are used interchangeably herein and refer to folate receptor α (FRα), also known as folate receptor 1 (FOLR1), which belongs to the folate receptor family and is a cell surface glycoprotein with a molecular weight of 38-40 kD. In certain rapidly proliferating cancer cells, the expression level of FRα is significantly increased. In some embodiments of the present invention, FRα is human FRα. In some embodiments, FRα is found in UniProt Database under Accession No.P15328 .

The term "anti-FRα antibody", "anti-FRα", "FRα antibody" or "FRα-targeting antibody" as used herein refers to an antibody or an antigen-binding fragment thereof capable of binding to FRα protein with sufficient affinity. The antibody can be used as a diagnostic and/or therapeutic agent targeting FRα, or in the construction of an immunoconjugate, e.g., an antibody-immunoagonist conjugate.

The term "antigen" refers to a molecule that elicits an immune response. This immune response may involve antibody production or activation of specific immune cells, or both. The skilled artisan will appreciate that any macromolecule, including substantially all proteins or peptides, can be used as an antigen. Further, antigens may be derived from recombinant or genomic DNA. As used herein, the term "epitope" refers to a moiety of an antigen (e.g., TROP2) that specifically interacts with an antibody molecule.

The terms "complete antibody", "whole antibody" and "full-length antibody" are used interchangeably herein to refer to an antibody molecule having the structure of a native immunoglobulin molecule. In the case of a conventional four-chain IgG antibody, a full-length antibody comprises two heavy chains (H) and two light chains (L) interconnected by disulfide bonds. In the case of a heavy chain antibody having only heavy chains but lacking light chains, a full length antibody comprises two heavy chains (H) interconnected by disulfide bonds. For a conventional four-chain IgG antibody, the full-length antibody heavy chain generally consists of a heavy chain variable region (abbreviated herein as VH) and a heavy chain constant region, wherein the heavy chain constant region comprises at least 3 domains CH1, CH2, and CH3.The full-length antibody light chain consists of a light chain variable region (abbreviated herein as VL) and a light chain constant region, wherein the light chain constant region consists of one domain CL. Each heavy chain variable region VH or each light chain variable region consists of three CDRs and 4 FRs arranged from amino-terminus to carboxyl-terminus in the following order: FR1, CDR1, FR2, CDR2, FR3, CDR3, and FR4. The term "antibody fragment" includes a portion of an intact antibody. In a preferred embodiment, the antibody fragment is an antigen binding fragment.

The term "antigen-binding fragment" of an antibody is a molecule different from a full-length antibody, which comprises a portion of the full-length antibody, but is capable of binding to an antigen of the full-length antibody or competes with the full-length antibody (i.e., a full-length antibody from which the antigen-binding fragment is derived) for binding to an antigen. An antigen-binding fragment may be prepared by recombinant DNA techniques, or by enzymatic or chemical cleavage of an intact antibody. The antigen-binding fragment includes, but is not limited to, Fv, Fab, Fab', Fab'-SH, F(ab')2, a domain antibody (dAb), a linear antibody, a single-chain antibody (e.g., scFv), a single-domain antibody (e.g., VHH), a bivalent antibody or a fragment thereof, a camelid antibody, a diabody, a single-domain antibody (sdAb), and a nanobody. For example, an Fab fragment can be obtained by papain digestion of a full-length antibody. In addition, the F(ab')2, a dimer of the Fab', is a bivalent antibody fragment produced by pepsin digestion of a portion below disulfide bonds in a hinge region of a complete antibody. The F(ab')2 can be reduced by disrupting the disulfide bond in the hinge region under neutral conditions, thereby converting the F(ab')2 dimer into an Fab' monomer. The Fab' monomer is essentially a Fab fragment with a hinge region. The Fv fragment consists of the VL and VH domains of a single arm of an antibody. The two domains VL and VH of the Fv fragment can be encoded by separate genes, but the two domains can also be linked, using recombinant methods, by a synthetic linker peptide so that they are produced as a single protein chain in which the VL and VH regions pair to form a single-chain Fv (scFv).

The term "single-chain antibody (scAb)" is used herein in the broadest sense and specifically covers antibodies with monospecificity or multispecificity (e.g., bispecificity) that are initially produced as a single continuous polypeptide chain. Such single chain antibodies include, but are not limited to, antibodies having two VL regions and a VH region joined. In one embodiment, the single-chain antibody is an scFv.

"Diabodies" are bivalent, small antibodies constructed by gene fusion, e.g., dimers consisting of two polypeptide chains. The VL and VH domains of each polypeptide chain of the diabody are joined by a linker such that the VL and VH domains encoded in the same polypeptide chain form a dimer with different single chain variable region fragments. Diabodies generally have two antigen-binding sites.

"Complementarity determining region" or "CDR region" or "CDR" is a region in an antibody variable domain that is highly variable in sequence and forms a structurally defined loop ("hypervariable loop") and/or comprises antigen-contacting residues ("antigen contact site"). The CDRs are primarily responsible for binding to antigenic epitopes. The CDRs of heavy and light chains are generally referred to as CDR1, CDR2, and CDR3, and are numbered sequentially from the N-terminus. The CDRs located in a heavy chain variable domain of an antibody are referred to as HCDR1, HCDR2, and HCDR3, whereas the CDRs located in a light chain variable domain of an antibody are referred to as LCDR1, LCDR2, and LCDR3.In a given amino acid sequence of a light chain variable region or a heavy chain variable region, the exact amino acid sequence boundary of each CDR can be determined using any one or a combination of many well-known antibody CDR assignment systems including, e.g., Chothia based on the three-dimensional structure of antibodies and the topology of the CDR loops (Chothia et al. (1989) Nature 342: 877-883; al-Lazikani et al., "Standard conformations for the canonical structures of immunoglobulins", Journal of Molecular Biology, 273: 927-948 (1997)), Kabat based on antibody sequence variability (Kabat et al., Sequences of Proteins of Immunological Interest, 4th Ed., U.S. Department of Health and Human Services, National Institutes of Health (1987)), AbM (University of Bath), Contact (University College London), International ImMunoGeneTics database (IMGT) (imgt.cines.fr/ on the World Wide Web), and North CDR definition based on the affinity propagation clustering using a large number of crystal structures (North et al., A New Clustering of Antibody CDR Loop Conformations, Journal of Molecular Biology, 406, 228-256 (2011)).

The following are the regional ranges of the CDRs defined using the Kabat, AbM, Chothia, Contact, and IMGT schemes.

| CDR | Kabat scheme | AbM scheme | Chothia scheme | Contact scheme | IMGT scheme |
|---|---|---|---|---|---|
| LCDR1 (Kabat and Chothia numbering systems) | L24-L34 | L24-L34 | L26-L32 | L30-L36 | L27-L32 |
| LCDR2 (Kabat and Chothia numbering systems) | L50-L56 | L50-L56 | L50-L52 | L46-L55 | L50-L52 |
| LCDR3 (Kabat and Chothia numbering systems) | L89-L97 | L89-L97 | L91-L96 | L89-L96 | L89-L96 |
| HCDR1 (Kabat numbering system) | H31-H35B | H26-H35B | H26-H32 | H30-H35B | H26-H35B |
| HCDR1 (Chothia numbering system) | H31-H35 | H26-H35 | H26-H32 | H30-H35 | H26-H35 |
| HCDR2 (Kabat and Chothia numbering systems) | H50-H65 | H50-H58 | H53-H55 | H47-H58 | H51-H57 |
| HCDR3 (Kabat and Chothia numbering systems) | H95-H102 | H95-H102 | H96-H101 | H93-H101 | H93-H102 |

CDRs can also be determined based on having the same Kabat numbering positions as a reference CDR sequence (e.g., any of the exemplary CDRs of the present invention).

Unless otherwise stated, the term "CDR" or "CDR sequence" used herein encompasses CDR sequences determined by any one of the schemes described above.

Unless otherwise stated, residue positions of an antibody variable region (including heavy chain variable region residues and light chain variable region residues) used herein are numbered according to the Kabat numbering system (Kabat et al., Sequences of Proteins of Immunological Interest, 5th Ed. Public Health Service, National Institutes of Health, Bethesda, Md. (1991)).

In one embodiment, the heavy chain variable region CDRs of the antibody of the present invention are determined according to the following rules:
VH CDR1 According to Kabat&Chothia and VH CDR2 and VH CDR3 are both determined according to the Kabat scheme.

In one embodiment, the light chain variable region CDRs of the antibody of the present invention are determined according to the Kabat scheme.

In one embodiment, the heavy chain variable region CDRs of the antibody of the present invention are determined according to the following rules: VH CDR1 is determined according to the Kabat & chothia rules, and VH CDR2 and VH CDR3 are both determined according to the Kabat rules; and the light chain variable region CDRs are determined according to the Kabat rules.

It should be noted that boundaries of CDRs of variable regions of the same antibody obtained based on different assignment systems may differ. That is, the CDR sequences of the variable regions of the same antibody defined under different assignment systems are different. Thus, when it comes to defining an antibody with specific CDR sequences defined in the present invention, the scope of the antibody also encompasses such antibodies whose variable region sequences comprise the specific CDR sequences, but having claimed CDR boundaries different from the specific CDR boundaries defined by the present invention due to a different scheme (e.g., different assignment system rules or their combinations) applied.

The "antibody that binds to the same or overlapping epitope" as a reference antibody refers to an antibody that blocks 50%, 60%, 70%, 80%, 90%, or 95% or more of the binding of the reference antibody to its antigen in a competition assay. Conversely, the reference antibody blocks 50%, 60%, 70%, 80%, 90%, or 95% or more of the binding of the antibody to its antigen in a competition assay. Accordingly, two antibodies or derivatives thereof bind to non-competing epitopes if they bind to non-overlapping epitopes, and preferably do not exhibit cross-blocking and/or interference with binding to the antigen in competition assays. Numerous types of competitive binding assays can be used to determine whether an antibody competes with another, such as direct or indirect solid-phase radioimmunoassay (RIA), direct or indirect solid-phase enzyme immunoassay (EIA), and sandwich competition assay.

An antibody that shows the same or similar binding affinity and/or specificity as a reference antibody refers to an antibody that is capable of having at least 50%, 60%, 70%, 80%, 90%, or 95% or more of the binding affinity and/or specificity of the reference antibody. This can be determined by any method known in the art for determining binding affinity and/or specificity.

The term "chimeric antibody" is an antibody molecule in which: (a) a constant region or a portion thereof is modified, substituted, or exchanged such that antigen-binding sites are linked to constant regions of different or modified classes, effector functions, and/or species, or disparate molecules imparting new properties (e.g., enzymes, toxins, hormones, growth factors, and drugs) to chimeric antibodies, etc.; or (b) a variable region or a portion thereof is modified, substituted, or exchanged by variable regions with different or modified antigen-binding specificities. For example, a mouse antibody can be modified by substituting its constant region with a constant region from a human immunoglobulin. Due to the substitution with a human constant region, the chimeric antibody can retain its specificity for recognizing antigens, while having reduced immunogenicity in humans as compared to the original mouse antibody.

"Humanized antibody" is an antibody that retains the antigen-specific reactivity of a non-human antibody (e.g., a mouse monoclonal antibody) and is less immunogenic, e.g., when administered to humans as a therapeutic agent. This can be achieved, for example, by retaining non-human antigen-binding sites and substituting the remainder of the antibodies with their human counterparts (i.e., the portions of the constant and variable regions not involved in binding are substituted with the corresponding parts of human antibodies).

The term "Fc region" is used herein to define a C-terminus region of an immunoglobulin heavy chain, which comprises at least a portion of a constant region. The term includes Fc regions of native sequences and variant Fc regions.A native immunoglobulin "Fc domain" comprises two or three constant domains, i.e., a CH2 domain, a CH3 domain, and an optional CH4 domain. For example, in native antibodies, an immunoglobulin Fc domain comprises the second and the third constant domains (CH2 domain and CH3 domain) derived from two heavy chains of IgG, IgA, and IgD antibodies; or comprises the second, the third, and the fourth constant domains (CH2 domain, CH3 domain, and CH4 domain) derived from two heavy chains of IgM and IgE antibodies. Unless otherwise stated herein, amino acid residues in the Fc region or heavy chain constant region are numbered according to the EU numbering scheme (also known as the EU index) described in, for example, Kabat et al., Sequences of Proteins of Immunological Interes, 5th Ed., Public Health Service, National Institutes of Health, Bethesda, MD, 1991. Herein, the term "Fc region" does not comprise a heavy chain variable region VH and a light chain variable region VL as well as a heavy chain constant region CH1 and a light chain constant region CL of an immunoglobulin, but in some cases, it may comprise a hinge region at the N-terminus of the heavy chain constant region.

The term "amino acid substitution" or "amino acid mutation" refers to the replacement of at least one amino acid residue in a predetermined parent amino acid sequence with a different "substituted" amino acid residue. The substituted residue or residues may be "naturally occurring amino acid residues" (i.e., encoded by the genetic code) and selected from: alanine (Ala); arginine (Arg); asparagine (Asn); aspartic acid (Asp); cysteine (Cys); glutamine (Gln); glutamic acid (Glu); glycine (Gly); histidine (His); isoleucine (Ile): leucine (Leu); lysine (Lys); methionine (Met); phenylalanine (Phe); proline (Pro); serine (Ser); threonine (Thr); tryptophan (Trp); tyrosine (Tyr); and valine (Val).

The amino acid position to be mutated into cysteine is generally indicated by "chain type, mutation position". Herein, unless otherwise specified, LLC represents a lambda light chain, LC represents a kappa light chain, and HC represents a heavy chain. Thus, "LLC160C" means that the amino acid at EU position 160 of the lambda light chain is substituted with cysteine (C).

When referring to a heavy chain amino acid position in the present invention, unless otherwise specifically stated, it refers to an amino acid position according to the IgG1 heavy chain numbering, i.e., it encompasses amino acid positions based on the IgG1 heavy chain numbering, as well as amino acid positions on other heavy chains corresponding to the amino acid position.

'Sequence identity' is defined as the percentage of identical residues in amino acid sequence variants after aligning the sequences and introducing gaps, if necessary, to achieve the maximum percent sequence identity.

"Parental protein (e.g., parental antibody or parental constant region or parental Fc region)" refers to a protein comprising an amino acid sequence in which one or more amino acid residues are to be replaced with one or more other amino acid residues, e.g., cysteine residues. The parent protein may comprise a native or wild-type sequence. The parent protein may have existing amino acid sequence modifications (such as additions, deletions, and/or substitutions) relative to other native, wild-type, or modified forms of the protein. The parent antibody may be directed against the target antigen of interest, e.g., a biologically important polypeptide.

As used herein, the term "binding" or "specific binding" means that the binding effect is selective for antigens and can be distinguished from unwanted or nonspecific interactions. The ability of an antigen-binding site to bind to a particular antigen may be determined by enzyme-linked immunosorbent assay (ELISA) or conventional binding assays known in the art, such as radioimmunoassay (RIA), bio-layer interferometry, MSD assay, or surface plasmon resonance (SPR).

"Immunoconjugate" herein refers to the linkage of a payload to an antibody or an antigen-binding fragment thereof via a linker, such that the antibody or the antigen-binding fragment thereof can serve as a carrier to target the payload to a target site. The term "payload" refers to an active moiety conjugated to the antibody or antibody fragment of the present invention and may include any moiety used to attach to the antibody or antibody fragment. In some embodiments, the payload may be a drug, e.g., a small molecule drug, a radionuclide, DNA, RNA, an enzyme, or a polypeptide, among others. In some embodiments, the immunoconjugate encompasses an antibody-drug conjugate (ADC), an antibody immunostimulatory agent (agonist) drug conjugate (ISAC), an antibody oligonucleotide conjugate (AOC), an antibody-polypeptide drug conjugate (APC), an antibody-nuclide drug conjugate (RDC), an antibody-degradation drug conjugate (ADeC), or the like. Payloads or active moieties suitable for linking to antibodies include, for example, cytotoxic agents, chemotherapeutic agents, innate immune agonists (e.g., Toll-like receptor agonist (TLR) ISAC drugs SBT6050, SBT6290, and BDC-1001; sTING agonist ISAC drug XMT-2056, Treg cell-regulating ISAC drug ADCT-301, etc.), immunomodulators, therapeutic oligonucleotides (siRNA, PMO, etc.), radionuclides, or the like.In some embodiments, the immunoconjugate disclosed herein is an antibody immunoconjugate.AgonistConjugates were ISAC.

As used herein, an "Antibody Immunoagonist Conjugates (ISAC)"means a structure acquired by linking an antibody to an immune agonist (preferably TLR7/8 agonist).

The term "linker" refers to a structural fragment that links a drug (e.g., a small molecule drug such as a TLR7/8 agonist) to an antibody moiety. It should be understood that the linker has a functional group that can form a bond with a functional group of the antibody or the antigen-binding fragment thereof before being linked to the antibody or the antigen-binding fragment thereof.

The term "linker-payload" refers to compound formed by linking a payload, e.g., a drug (e.g., a small molecule drug such asTLR7/8 Agonist) to a linker.

The term "site-specific conjugation" as used herein refers to the conjugation of a drug to a specific site of an antibody via a linker.

As used herein, the term "alkyl" refers to a fully saturated branched or unbranched hydrocarbon group. The alkyl group preferably contains 1-16 carbon atoms, e.g., 1-12 carbon atoms, 1-10 carbon atoms, 1-6 carbon atoms, or 1-4 carbon atoms. For example, C₁-C₆alkyl refers to a fully saturated branched or unbranched hydrocarbon group containing from 1 to 6 carbon atoms. Representative examples of alkyl include, but are not limited to, methyl, ethyl, n-propyl, iso-propyl, n-butyl, sec-butyl, iso-butyl, tert-butyl, n-pentyl, isopentyl, neopentyl, n-hexyl, 3-methylhexyl, 2,2-dimethylpentyl, 2,3-dimethylpentyl, n-heptyl, n-octyl, n-nonyl, n-decyl and the like.

The term "C₁-C₆alkoxy" as used herein refers to a radical of the formula -O-(C₁-C₆alkyl), wherein the alkyl group is as defined above. Representative examples of C₁-C₆alkoxy include, but are not limited to, methoxy, ethoxy, n-propoxy, isopropoxy, n-butoxy, isobutoxy, and tert-butoxy.

The term "C₁-C₆acyl" as used herein refers to a radical of the formula -C(=O)-(C₁-C₆alkyl), wherein the alkyl group is as defined above. Representative examples of C₁-C₆acyl include, but are not limited to, formyl, acetyl, propionyl, and the like.

The term "heterocyclyl" as used herein refers to a monovalent saturated or partially unsaturated monocyclic or bicyclic ring system having 3 to 10 ring atoms, which comprises 1 to 5 ring heteroatoms selected from N, O, and S, with the remaining ring atoms being carbon. Representative examples of heterocyclyl include, but are not limited to, aziridinyl, oxiranyl, azetidinyl, oxetanyl, pyrrolidinyl, tetrahydrofuranyl, tetrahydrothienyl, pyrazolidinyl, imidazolidinyl, piperidinyl, tetrahydropyranyl, piperazinyl, morpholinyl, thiomorpholinyl, dioxothiomorpholinyl, azepanyl, diazepanyl, tetrahydroquinolinyl, tetrahydroisoquinolinyl, and the like.

The term "aryl" refers to monocyclic or fused aromatic hydrocarbon groups having a conjugated π-electron system, such as phenyl, naphthyl and the like.

The term "alkenyl" refers to a linear or branched hydrocarbon group containing 2-16 carbon atoms and containing at least one double bond and no triple bonds. Alkenyl groups preferably contain 2-12 carbon atoms, 2-10 carbon atoms, 2-8 carbon atoms, 2-6 carbon atoms, or 2-4 carbon atoms. Representative examples of alkenyl include, but are not limited to, ethenyl, propenyl, butenyl, pentenyl, hexenyl and the like.

The term "alkynyl" refers to a linear or branched hydrocarbon group containing 2-16 carbon atoms and comprising at least one triple bond. Alkynyl groups preferably contain 2-12 carbon atoms, 2-10 carbon atoms, 2-8 carbon atoms, 2-6 carbon atoms, or 2-4 carbon atoms. Representative examples of alkynyl include, but are not limited to, ethynyl, propynyl, butynyl, pentynyl, hexynyl and the like.

The term "halogen" or "halo" refers to fluorine (-F), chlorine (-Cl), bromine (-Br), and iodine (-I).

The term "haloalkyl" refers to an alkyl group as defined herein substituted with one or more halo groups as defined herein. Haloalkyl can preferably be monohaloalkyl, dihaloalkyl or polyhaloalkyl including perhaloalkyl. Monohaloalkyl groups may contain one iodo, bromo, chloro or fluoro within the alkyl. Dihaloalkyl and polyhaloalkyl groups can contain two or more of the same halo atoms or a combination of different halo groups within the alkyl. Preferably the polyhaloalkyl contains up to 12, 10, or 8, or 6, or 4, or 3, or 2 halo groups. Non-limiting examples of haloalkyl include fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl and dichloropropyl. A perhaloalkyl refers to an alkyl having all hydrogen atoms replaced with halo atoms.

The term "haloalkenyl" refers to an alkenyl group, as defined herein, substituted with one or more halo groups, as defined herein. The term "haloalkynyl" refers to an alkynyl group, as defined herein, substituted with one or more halo groups, as defined herein. The "halo" defined for "haloalkyl" is applicable to "haloalkenyl" and "haloalkynyl".

The term "polyol group" refers to an alkyl group as defined above containing multiple (e.g., 2-10, e.g., 3, 4, 5, 6, 7, or 8) hydroxyl groups, which optionally contains one or more (e.g., 2, 3, or 4) other groups (e.g., amino and carbonyl). Non-limiting examples of "polyol group" include, for example,

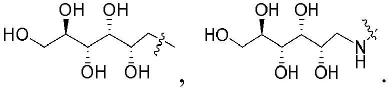

The term "amino acid" refers to naturally occurring and synthetic amino acids. The amino acids may be L or D isomers. The conventional amino acids referred to herein are written in accordance with conventional usage. See, e.g., Immunology-A Synthesis (2nd Edition, E. S. Golub and D. R. Gren, Eds., Sinauer Associates, Sunderland, Mass. (1991)), herein incorporated by reference. Moreover, in the present disclosure, amino acids are generally represented by single-letter and three-letter abbreviations known in the art. For example, glycine may be represented by Gly, alanine may be represented by Ala, valine may be represented by Val, glutamine may be represented by Gln, glutamic acid may be represented by Glu, phenylalanine may be represented by Phe, and leucine may be represented by Leu.

The term "optional" or "optionally" means that the subsequently described event or circumstance occurs or does not occur, and that the description includes instances where the event or circumstance occurs and instances where it does not. For example, when a group or structure is "optionally substituted", the group or structure may be substituted or unsubstituted.

The term "pharmaceutically acceptable salt" refers to a salt that retains the biological effects and properties of the ISAC conjugate of the present invention and is not biologically or otherwise undesired. The ISAC conjugates of the invention can exist in their pharmaceutically acceptable salt form including acid addition salts and base addition salts. In the present invention, pharmaceutically acceptable non-toxic acid addition salts refer to salts formed from the ISAC conjugates of the present invention and organic or inorganic acids including, but not limited to, hydrochloric acid, sulfuric acid, hydrobromic acid, hydroiodic acid, phosphoric acid, nitric acid, perchloric acid, acetic acid, oxalic acid, maleic acid, fumaric acid, tartaric acid, benzenesulfonic acid, methanesulfonic acid, salicylic acid, succinic acid, citric acid, lactic acid, propionic acid, benzoic acid, p-toluenesulfonic acid, malic acid, etc. Pharmaceutically acceptable non-toxic base addition salts refer to salts formed by the ISAC conjugates of the present invention with organic or inorganic bases including, but not limited to, alkali metal salts, e.g., lithium, sodium, or potassium salts; alkaline earth metal salts, e.g., calcium or magnesium salts; organic base salts, e.g., ammonium salts formed with N group-containing organic bases.

The term "solvate" refers to an association formed by one or more solvent molecules and the antibody-immunoagonist conjugate of the present invention. Solvents for forming solvates include, but are not limited to, water, methanol, ethanol, isopropanol, ethyl acetate, tetrahydrofuran, N,N-dimethylformamide, dimethylsulfoxide, and the like.

"Pharmaceutically acceptable" and "pharmaceutical" are used interchangeably herein, provided that there is no contradiction according to the context.

The term "drug-to-antibody ratio" or "DAR" refers to the average ratio of payload moieties (D) to Ab moieties conjugated to the immunoconjugates described herein (e.g., ISAC). In other words, the DAR value reflects the average number of payload molecules carried by each ISAC molecule based on a single antibody molecule (Ab moiety) in a population of immunoconjugates.

In some embodiments described herein, most ISAC molecules in a population of immunoconjugates carry the same number of payload molecules, and in this case, the DAR may be approximately determined by m and p in formula (I); for example, the DAR may be 1 to 16, e.g. 2-16, 4-16, 5-12, 6-10, 2-8, 3-8, 2-6, 4-6, 6-10, e.g. 2, 3, 4, 5, 6, 7, 8, 9, 10, 11, 12, 13, 14 or 15. DAR may also be determined by the overall ratio of the drug moiety (D) conjugated to the Ab moiety described herein to the Ab moiety in the product as measured by detection methods (e.g., by conventional methods such as mass spectrometry, ELISA assay, electrophoresis, and/or HPLC), for example, when the number of payload molecules (e.g., m × p) carried by a single ISAC molecule in a population of immunoconjugates differs greatly. In some embodiments, the DAR value of the conjugate of the present invention is 1 to 16, e.g. 2-16, 4-16, 5-12, 6-10, 2-8, 3-8, 2-6, 4-6, 6-10, for example, 1.0-8.0, 2.0-6.0, e.g., 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8.0, 7.9, 8, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, or 10.0, and ranges with two of these values as endpoints.

The term "therapeutic agent" described herein encompasses any substance that is effective in preventing or treating a tumor, e.g., cancer, including a chemotherapeutic agent, a cytokine, an angiogenesis inhibitor, a cytotoxic agent, other antibody, a small molecule drug, or an immunomodulatory agent (e.g., an immunosuppressant).

The term "cytotoxic agent" used herein refers to a substance that inhibits or prevents cell functions and/or causes cell death or cell destruction.

"Chemotherapeutic agents" include chemical compounds useful in the treatment of cancer or immune system disease.

The term "drug" refers to an organic compound capable of modulating biological processes, particularly altering or preventing pathological processes.

The term "prodrug" refers to an active or inactive compound that is chemically modified and, after being administered to an individual, becomes an active drug through in vivo physiological actions (e.g., hydrolysis, metabolism, etc.). Techniques for making and using prodrugs are well known to those skilled in the art.

The term "small molecule drug" refers to a low molecular weight organic compound capable of modulating biological processes, particularly altering or preventing pathological processes. 'Small molecule' is defined as a molecule with a molecular weight of less than 10 kD, usually less than 2 kD and preferably less than 1 kD, more preferably less than 500 D. Small molecule drugs include, but are not limited to, organic molecules, organic molecules containing inorganic components, molecules containing radioactive atoms, synthetic molecules, peptidomimetics, and antibody mimetics. As therapeutic agents, small molecules penetrate cells better, are less susceptible to degradation, and are less likely to elicit an immune response than large molecules.

"Anti-tumor compound" is a pharmaceutically active compound that has an effect on a tumor, including but not limited to cytotoxic agents or chemotherapeutic agents, such as the cytotoxic agents disclosed in WO2021/173773, the camptothecin compounds exatecan (topoisomerase I inhibitor exatecan), DXd (a novel topoisomerase I inhibitor exatecan derivative), auristatin compounds such as monomethyl auristatin E (MMAE), or maytansinoid compounds such as small molecule microtubule inhibitor DM1. It should be understood that the anti-tumor compound may be substituted with isotopes including, but not limited to, deuterium, tritium, and the like. For example, after a deuterium substitution is performed to replace a carbon-hydrogen bond with a carbon-deuterium bond, since the later is more stable than the former, the substitution can directly affect properties such as absorption, distribution, metabolism, and excretion of certain drugs, thereby improving the efficacy, safety, and tolerability of the drugs. Thus, the "anti-tumor compound" of the present application may encompass the deuterium-substituted compounds.

"Substituted with deuterium" refers to the replacement of hydrogen in the molecule with deuterium, for example, the replacement of one or more hydrogens, such as 1-10 (e.g., 1, 2, 3, 4, 5, 6, 7, 8, 9, or 10) hydrogens, with deuterium.

The term "immunomodulatory agent" used herein refers to a natural or synthetic active agent or drug that inhibits or modulates (e.g., activates) an immune response. The immune response may be a humoral response or a cellular response. Immunomodulatory agents include immunosuppressants. In some embodiments, the immunomodulator of the present invention includes an immune checkpoint inhibitor or an immune checkpoint agonist.

The term "effective amount" refers to an amount or dosage of the antibody, fragment, composition, or combination of the present invention which generates expected effects in a patient in need of treatment or prevention after administration to the patient in a single or multiple doses. "Therapeutically effective amount" and "prophylactically effective amount" may be included according to the desired effect.

"Therapeutically effective amount" refers to an amount effective to achieve a desired therapeutic result at a necessary dose for a necessary period of time. The therapeutically effective amount is also such an amount that any toxic or undesired effect of the antibody, fragment thereof, composition, or combination is inferior to the therapeutically beneficial effect. The "therapeutically effective amount" preferably inhibits a measurable parameter (e.g., tumor volume) by at least about 30%, and even more preferably by at least about 40%, 45%, 50%, 55%, 60%, 65%, 70%, 75%, 80%, 85%, 90%, or even 100%, relative to untreated subjects.

"Prophylactically effective amount" refers to an amount effective to achieve a desired prophylactic result at a necessary dose for a necessary period of time. Generally, since a prophylactic dose is used in subjects prior to or at an earlier stage of a disease, the prophylactically effective amount will be less than the therapeutically effective amount.

The terms "host cell", "host cell line", and "host cell culture" are used interchangeably and refer to cells into which exogenous nucleic acids are introduced, including progenies of such cells. Host cells include "transformants" and "transformed cells", which include primary transformed cells and progenies derived therefrom, regardless of the number of passages. Progeny may not be exactly the same as parent cells in terms of nucleic acid content, and may contain mutations. Mutant progenies having the same function or biological activity that are screened or selected from the initially transformed cells are included herein.

The term "label" used herein refers to a compound or composition that is conjugated or fused directly or indirectly to an agent, such as a polynucleotide probe or an antibody, and facilitates the detection of the agent to which it is conjugated or fused. The label may itself be detectable (e.g., radioisotope labels or fluorescent labels) or, in the case of an enzymatic label, may catalyze chemical alteration of a substrate compound or composition which is detectable. The term is intended to encompass direct labeling of a probe or an antibody by coupling (i.e., physically linking) a detectable substance to the probe or antibody and indirect labeling of a probe or an antibody by reacting with another reagent which is directly labeled.

"Individual" or "subject" includes mammals. Mammals include, but are not limited to, domesticated animals (e.g., cows, sheep, cats, dogs, and horses), primates (e.g., human and non-human primates such as monkeys), rabbits, and rodents (e.g., mice and rats). In some embodiments, the individual or subject is a human.

An "isolated" antibody or other molecule (e.g., an ISAC molecule) is an antibody or molecule that has been separated from its natural environment or a component of the environment in which it is expressed. In some embodiments, the antibody or ISAC molecule is purified to a purity greater than 95% or 99% as determined by, for example, electrophoresis (e.g., SDS-PAGE, isoelectric focusing (IEF), and capillary electrophoresis) or chromatography (e.g., ion exchange or reverse-phase HPLC).

The term "anti-tumor effect" refers to a biological effect that can be demonstrated by a variety of means, including but not limited to, for example, decrease in tumor volume, decrease in number of tumor cells, decrease in tumor cell proliferation, or decrease in tumor cell viability.

The terms "cancer" and "cancerous" refer to or describe the physiological condition in mammals that is typically characterized by unregulated cell growth. The cancer may be at an early, intermediate or advanced stage or be a metastatic cancer. Cancers suitable for treatment by the molecules of the invention include, but are not limited to, lung cancer, melanoma, head and neck tumors, prostate cancer, esophageal cancer, cervical cancer, kidney cancer, bladder cancer, ovarian cancer, pancreatic cancer, or breast cancer, including metastatic forms of those cancers.

The term "tumor" refers to all neoplastic cell growth and proliferation, whether malignant or benign, and all pre-cancerous and cancerous cells and tissues. "Tumor" encompasses solid tumors and hematological tumors as well as metastatic lesions. The terms "cancer", "cancerous", and "tumor" are not mutually exclusive when referred to herein.

The term "pharmaceutical supplementary material" refers to diluents, adjuvants (e.g., Freund's adjuvants (complete and incomplete)), excipients, carriers, stabilizers, or the like, which are administered with the active substance.

The term "pharmaceutical composition" refers to a composition that exists in a form allowing the biological activity of active ingredients contained therein to be effective and does not contain additional ingredients having unacceptable toxicity to a subject to which the composition is administered.

The term "pharmaceutical combination" refers to a non-fixed combination product or a fixed combination product, including but not limited to a kit and a pharmaceutical composition. The term "non-fixed combination" means that the active ingredients (e.g., (i) the antibody and/or the ISAC molecule of the present invention, and (ii) an additional therapeutic agent) are administered, either simultaneously or sequentially (without specific time limitation or at identical or different time intervals), to a patient as separate entities, wherein such administration provides two or more prophylactically or therapeutically effective active agents in the patient. In some embodiments, the antibody and/or the ISAC molecule of the present invention and the additional therapeutic agent used in the pharmaceutical combination are administered at levels that do not exceed their levels when used alone. The term "fixed combination" means that two or more active agents are administered to a patient simultaneously in the form of a single entity. The dosage and/or time interval of two or more active agents is preferably selected such that the combined use of the components can result in a therapeutic effect on the disease or disorder greater than that achieved by the use of either component alone. The ingredients may each be in a separate formulation form that may or may not be identical.

The term "combination therapy" refers to the administration of two or more therapeutic agents or modalities (e.g., radiotherapy or surgery) to treat the diseases described herein. Such administration includes co-administration of these therapeutic agents in a substantially simultaneous manner, e.g., in a single capsule having a fixed ratio of active ingredients. Alternatively, such administration includes co-administration of the active ingredients in a variety of or separate containers (e.g., tablets, capsules, powder and liquid). The powder and/or liquid may be reconstituted or diluted to a desired dose prior to administration. In addition, such administration also includes using each type of therapeutic agents in a sequential manner at approximately the same time or at different times. In any case, the therapeutic regimen will provide the beneficial effect of the drug combination in treating the disorders or conditions described herein.

As used herein, "treatment" refers to slowing, interrupting, arresting, alleviating, stopping, reducing, or reversing the progression or severity of an existing symptom, disorder, condition, or disease.

As used herein, "prevention" (or "prevent" or "preventing") includes the inhibition of the development or progression of symptoms of a disease or disorder, or a specific disease or disorder. In some embodiments, subjects with family history of cancer are candidates for preventive regimens. Generally, in the context of cancer, the term "prevention" refers to the administration of a drug prior to the onset of signs or symptoms of cancer, particularly in subjects at risk of cancer.

The term "vector" as used herein refers to a nucleic acid molecule capable of proliferating another nucleic acid to which it is linked. The term includes vectors that serve as self-replicating nucleic acid structures as well as vectors that incorporate into the genome of a host cell into which they have been introduced. Some vectors are capable of directing the expression of a nucleic acid to which they are operably linked. Such vectors are referred to herein as "expression vectors."

"Subject/patient/individual sample" refers to a collection of cells or fluids obtained from a patient or a subject. The source of the tissue or cell sample may be solid tissue as from a fresh, frozen and/or preserved organ or tissue sample or biopsy or puncture sample; blood or any blood component; bodily fluids such as cerebrospinal fluids, amniotic fluids, peritoneal fluids, or interstitial fluids; and cells from a subject at any time during pregnancy or development. Tissue samples may comprise compounds that are naturally not mixed with tissues, such as preservatives, anticoagulants, buffers, fixatives, nutrients, antibiotics, and the like.

### II. Antibody, Nucleic Acid, Host Cell

In some embodiments, the anti-TROP2 antibody or the antigen-binding fragment thereof of the present invention comprises 3 complementarity determining regions from a heavy chain variable region (HCDRs): HCDR1, HCDR2, and HCDR3.

In some embodiments, the anti-TROP2 antibody or the antigen-binding fragment thereof of the present invention comprises 3 complementarity determining regions from a light chain variable region (LCDRs): LCDR1, LCDR2, and LCDR3.

In some embodiments, the anti-TROP2 antibody or the antigen-binding fragment thereof of the present invention comprises 3 complementarity determining regions from a heavy chain variable region (HCDRs) and 3 complementarity determining regions from a light chain variable region (LCDRs).

In some aspects, the anti-TROP2 antibody or the antigen-binding fragment thereof of the present invention comprises a heavy chain variable region (VH). In some aspects, the anti-TROP2 antibody or the antigen-binding fragment thereof of the present invention comprises a light chain variable region (VL). In some aspects, the anti-TROP2 antibody or the antigen-binding fragment thereof of the present invention comprises a heavy chain variable region and a light chain variable region. In some embodiments, the heavy chain variable region comprises 3 complementarity determining regions (CDRs) from the heavy chain variable region: HCDR1, HCDR2, and HCDR3. In some embodiments, the light chain variable region comprises 3 complementarity determining regions (CDRs) from the light chain variable region: LCDR1, LCDR2, and LCDR3.

In some embodiments, the anti-TROP2 antibody or the antigen-binding fragment thereof of the present invention further comprises an antibody heavy chain constant region. In some embodiments, the anti-TROP2 antibody or the antigen-binding fragment thereof of the present invention further comprises an antibody light chain constant region. In some embodiments, the anti-TROP2 antibody or the antigen-binding fragment thereof of the present invention further comprises a heavy chain constant region and a light chain constant region.

In some embodiments, the heavy chain variable region of the present invention:
(i) comprises or consists of an amino acid sequence having at least 90%, 91%, 92%, 93%, 94%, 95%, 96%, 97%, 98%, or 99% identity to the amino acid sequence selected from SEQ ID NO: 7 and SEQ ID NO: 12; or
(ii) comprises or consists of the amino acid sequence selected from SEQ ID NO: 7 and SEQ ID NO: 12; or
(iii) comprises or consists of an amino acid sequence having one or more (preferably no more than 10, and more preferably no more than 5, 4, 3, 2, or 1) amino acid changes (preferably amino acid substitutions, and more preferably conservative amino acid substitutions) compared with the amino acid sequence selected from SEQ ID NO: 7 and SEQ ID NO: 12, wherein preferably, the amino acid changes do not occur in the CDRs.

In some embodiments, the light chain variable region of the present invention
(i) comprises or consists of an amino acid sequence having at least 90%, 91%, 92%, 93%, 94%, 95%, 96%, 97%, 98%, or 99% identity to the amino acid sequence selected from SEQ ID NO: 8 and SEQ ID NO: 14; or
(ii) comprises or consists of the amino acid sequence selected from SEQ ID NO: 8 and SEQ ID NO: 14; or
(iii) comprises or consists of an amino acid sequence having one or more (preferably no more than 10, and more preferably no more than 5, 4, 3, 2, or 1) amino acid changes (preferably amino acid substitutions, and more preferably conservative amino acid substitutions) compared with the amino acid sequence selected from SEQ ID NO: 8 and SEQ ID NO: 14, wherein preferably, the amino acid changes do not occur in the CDRs.

In some embodiments, the 3 complementarity determining regions from the heavy chain variable region (HCDRs) of the present invention, HCDR1, HCDR2, and HCDR3, are selected from
(i) three complementarity determining regions HCDR1, HCDR2 and HCDR3 contained in the VH set forth in SEQ ID NO: 7 or 12;
(ii) a sequence comprising a total of at least one and no more than 5, 4, 3, 2, or 1 amino acid change (preferably amino acid substitutions, and more preferably conservative substitutions) in the three HCDRs relative to any sequence of (i).
wherein the HCDR1-3 are determined according to any CDR scheme, e.g., Kabat, AbM, Chothia, or IMGT, or a combination thereof;

preferably, the HCDR1 is determined according to the AbM scheme, and the HCDR2 and the HCDR3 are determined according to the Kabat scheme, respectively.

In some embodiments, the 3 complementarity determining regions from the light chain variable region (LCDRs) of the present invention, LCDR1, LCDR2, and LCDR3, are selected from
(i) three complementarity determining regions LCDR1, LCDR2 and LCDR3 contained in a VL set forth in SEQ ID NO: 8 or 14, or
(ii) a sequence comprising a total of at least one and no more than 5, 4, 3, 2, or 1 amino acid change (preferably amino acid substitutions, and more preferably conservative substitutions) in the three LCDRs relative to any sequence of (i).

wherein the LCDR1-3 are determined according to any CDR scheme, e.g., Kabat, AbM, Chothia, or IMGT, or a combination thereof;
preferably, the LCDR1, the LCDR2, and the LCDR3 are determined according to the Kabat scheme, respectively.

In some embodiments, the HCDR1 comprises or consists of the amino acid sequence set forth in SEQ ID NO: 1, or the HCDR1 comprises an amino acid sequence having one, two, or three changes (preferably amino acid substitutions, and more preferably conservative substitutions) compared with the amino acid sequence set forth in SEQ ID NO: 1.

In some embodiments, the HCDR2 comprises or consists of the amino acid sequence set forth in SEQ ID NO: 2, or the HCDR2 comprises an amino acid sequence having one, two, or three changes (preferably amino acid substitutions, and more preferably conservative substitutions) compared with the amino acid sequence set forth in SEQ ID NO: 2.

In some embodiments, the HCDR3 comprises or consists of the amino acid sequence set forth in SEQ ID NO: 3, or the HCDR3 comprises an amino acid sequence having one, two, or three changes (preferably amino acid substitutions, and more preferably conservative substitutions) compared with the amino acid sequence set forth in SEQ ID NO: 3.

In some embodiments, the LCDR1 comprises or consists of the amino acid sequence set forth in SEQ ID NO: 4, or the LCDR1 comprises an amino acid sequence having one, two, or three changes (preferably amino acid substitutions, and more preferably conservative substitutions) compared with the amino acid sequence set forth in SEQ ID NO: 4.

In some embodiments, the LCDR2 comprises or consists of the amino acid sequence set forth in SEQ ID NO: 5, or the LCDR2 comprises an amino acid sequence having one, two, or three changes (preferably amino acid substitutions, and more preferably conservative substitutions) compared with the amino acid sequence set forth in SEQ ID NO: 5.

In some embodiments, the LCDR3 comprises or consists of the amino acid sequence set forth in SEQ ID NO: 6, or the LCDR3 comprises an amino acid sequence having one, two, or three changes (preferably amino acid substitutions, and more preferably conservative substitutions) compared with the amino acid sequence set forth in SEQ ID NO: 6.

In some embodiments, the antibody heavy chain constant region of the present invention is a heavy chain constant region of IgG1, IgG2, IgG3, or IgG4, preferably a heavy chain constant region of IgG4. In some embodiments, the antibody light chain constant region of the present invention is a lambda or Kappa light chain constant region, preferably a Kappa light chain constant region.

In some embodiments, the heavy chain constant region of the antibody of the present invention comprises an Fc region or a mutated Fc region.

In some embodiments, the Fc region is an IgG Fc from a human, e.g., an Fc from human IgG1, an Fc from human IgG2, an Fc from human IgG3, or an Fc from human IgG4.In one embodiment, the Fc region is from human IgG1.

In some specific embodiments, the anti-TROP2 antibody of the present invention comprises a first heavy chain complementarity determining region (HCDR1), a second heavy chain complementarity determining region (HCDR2), a third heavy chain complementarity determining region (HCDR3), and a first light chain complementarity determining region (LCDR1), a second light chain complementarity determining region (LCDR2), and a third light chain complementarity determining region (LCDR3), wherein the HCDR1, HCDR2, HCDR3, LCDR1, LCDR2, and LCDR3 comprise or consist of amino acid sequences set forth in SEQ ID NO: 1, SEQ ID NO: 2, SEQ ID NO: 3, SEQ ID NO: 4, SEQ ID NO: 5, and SEQ ID NO: 6, respectively; or

In some embodiments, the VH of the present disclosure comprises HCDR1, HCDR2, and HCDR3, and the VL comprises LCDR1, LCDR2, and LCDR3, wherein the HCDR1, HCDR2, and HCDR3 comprise or consist of amino acid sequences set forth in SEQ ID NO: 1, SEQ ID NO: 2, and SEQ ID NO: 3, respectively; the LCDR1, LCDR2, and LCDR3 comprise or consist of amino acid sequences set forth in SEQ ID NO: 4, SEQ ID NO: 5, and SEQ ID NO: 6, respectively.

In some embodiments, the antibody or the antigen-binding fragment thereof disclosed herein comprises:
(i) a VH comprising or consisting of an amino acid sequence set forth in SEQ ID NO: 7 or an amino acid sequence having at least 90%, 91%, 92%, 93%, 94%, 95%, 96%, 97%, 98%, or 99% identity thereto, and/or a VL comprising or consisting of an amino acid sequence set forth in SEQ ID NO: 8 or an amino acid sequence having at least 90%, 91%, 92%, 93%, 94%, 95%, 96%, 97%, 98%, or 99% identity thereto;
(ii) a VH comprising or consisting of an amino acid sequence set forth in SEQ ID NO: 12 or an amino acid sequence having at least 90%, 91%, 92%, 93%, 94%, 95%, 96%, 97%, 98%, or 99% identity thereto, and/or a VL comprising or consisting of an amino acid sequence set forth in SEQ ID NO: 14 or an amino acid sequence having at least 90%, 91%, 92%, 93%, 94%, 95%, 96%, 97%, 98%, or 99% identity thereto.

In some embodiments, the antibody or the antigen-binding fragment thereof of the present invention comprises a heavy chain variable region and a light chain variable region, wherein
(i)the heavy chain variable region comprises or consists of an amino acid sequence set forth in SEQ ID NO: 7, and the light chain variable region comprises or consists of an amino acid sequence set forth in SEQ ID NO: 8; or
(ii) the heavy chain variable region comprises or consists of an amino acid sequence set forth in SEQ ID NO: 12, and the light chain variable region comprises or consists of an amino acid sequence set forth in SEQ ID NO: 14.

In some embodiments, the anti-TROP2 antibody or the antigen-binding fragment thereof of the present invention further comprises an antibody heavy chain. In some embodiments, the anti-TROP2 antibody or the antigen-binding fragment thereof of the present invention further comprises an antibody light chain. In some embodiments, the anti-TROP2 antibody or the antigen-binding fragment thereof of the present invention further comprises a heavy chain and a light chain.

In some embodiments, the heavy chain comprises
(i) comprises or consists of an amino acid sequence having at least 85%, 90%, 91%, 92%, 93%, 94%, 95%, 96%, 97%, 98%, or 99% identity to an amino acid sequence set forth in SEQ ID NO: 9 or 15;
(ii) comprises or consists of the amino acid sequence selected from SEQ ID NO: 9 and 15; or
(iii) comprises or consists of an amino acid sequence having one or more (preferably no more than 20 or 10, and more preferably no more than 5, 4, 3, 2, or 1) amino acid changes (preferably amino acid substitutions, and more preferably conservative amino acid substitutions) compared with the amino acid sequence selected from SEQ ID NO: 9 and 15.

In some embodiments, the light chain comprises
(i) comprises or consists of an amino acid sequence having at least 85%, 90%, 91%, 92%, 93%, 94%, 95%, 96%, 97%, 98%, or 99% identity to the amino acid sequence selected from SEQ ID NO: 10 and 16;
(ii) comprises or consists of the amino acid sequence selected from SEQ ID NO: 10 or 16; or
(iii) comprises or consists of an amino acid sequence having one or more (preferably no more than 20 or 10, and more preferably no more than 5, 4, 3, 2, or 1) amino acid changes (preferably amino acid substitutions, and more preferably conservative amino acid substitutions) compared with the amino acid sequence selected from SEQ ID NO: 10 or 16.

In some embodiments, the antibody or the antigen-binding fragment thereof disclosed herein comprises a heavy chain and a light chain, wherein the heavy chain comprises or consists of an amino acid sequence set forth in SEQ ID NO: 9, and the light chain comprises or consists of an amino acid sequence set forth in SEQ ID NO: 10.

In some embodiments, the antibody or the antigen-binding fragment thereof disclosed herein comprises a heavy chain and a light chain, wherein the heavy chain comprises or consists of an amino acid sequence set forth in SEQ ID NO: 15, and the light chain comprises or consists of an amino acid sequence set forth in SEQ ID NO: 16.

In some embodiments, the antibody or the antigen-binding fragment thereof of the present invention comprises or consists of two heavy chains and two light chains, optionally, the two heavy chains are identical and/or the two light chains are identical.

In one embodiment of the present invention, the amino acid changes described herein include amino acid substitutions, insertions, or deletions. Preferably, the amino acid changes described herein are amino acid substitutions, preferably conservative substitutions.

In some embodiments, the anti-TROP2 antibody or the antigen-binding fragment thereof of the present invention has one or more of the following properties:
(i) showing the same or similar binding affinity and/or specificity for TROP2 as the antibody of the present invention;
(ii) inhibiting (e.g., competitively inhibiting) the binding of the antibody of the present invention to TROP2;
(iii) binding to the same or overlapping epitope as the antibody of the present invention;
(iv) competing with the antibody of the present invention for binding to TROP2;
(v) having one or more biological properties of the antibody of the present invention.

In some embodiments, the anti-TROP2 antibody of the present invention is an antibody in the form of IgG1, IgG2, IgG3, or IgG4, preferably an antibody in the form of IgG1.

In some embodiments, the anti-TROP2 antibody is a monoclonal antibody.

In some embodiments, the anti-TROP2 antibody is humanized.

In some embodiments, the anti-TROP2 antibody is a chimeric antibody.

In one embodiment, the anti-TROP2 antibody of the present invention also encompasses an antibody fragment (e.g., an antigen-binding fragment) thereof, preferably an antibody fragment selected from: Fab, Fab', Fab'-SH, Fv, a single-chain antibody (e.g., scFv), (Fab')2, a single-domain antibody (e.g., VHH), a domain antibody (dAb), a bivalent antibody, or a linear antibody.

In one embodiment, the anti-TROP2 antibody is a full-length antibody.

In one embodiment, the anti-TROP2 antibody also encompasses multispecific antibodies such as bispecific antibodies.

In some embodiments, the anti-FRα antibody or the antigen-binding fragment thereof of the present invention comprises 3 complementarity determining regions from a heavy chain variable region (HCDRs): HCDR1, HCDR2, and HCDR3. In some embodiments, the anti-FRα antibody or the antigen-binding fragment thereof of the present invention comprises 3 complementarity determining regions from a light chain variable region (LCDRs): LCDR1, LCDR2 and LCDR3. In some embodiments, the anti-FRα antibody or the antigen-binding fragment thereof of the present invention comprises 3 complementarity determining regions from a heavy chain variable region (HCDRs) and 3 complementarity determining regions from a light chain variable region (LCDRs).

In some aspects, the anti-FRα antibody or the antigen-binding fragment thereof of the present invention comprises a heavy chain variable region (VH). In some aspects, the anti-FRα antibody or the antigen-binding fragment thereof of the present invention comprises a light chain variable region (VL). In some aspects, the anti-FRα antibody or the antigen-binding fragment thereof of the present invention comprises a heavy chain variable region and a light chain variable region.In some embodiments, the heavy chain variable region comprises 3 complementarity determining regions (CDRs) from the heavy chain variable region: HCDR1, HCDR2, and HCDR3. In some embodiments, the light chain variable region comprises 3 complementarity determining regions (CDRs) from the light chain variable region: LCDR1, LCDR2, and LCDR3.

In some embodiments, the anti-FRα antibody or the antigen-binding fragment thereof of the present invention further comprises an antibody heavy chain constant region. In some embodiments, the anti-FRα antibody or the antigen-binding fragment thereof of the present invention further comprises an antibody light chain constant region. In some embodiments, the anti-FRα antibody or the antigen-binding fragment thereof of the present invention further comprises a heavy chain constant region and a light chain constant region.

In some embodiments, the heavy chain variable region of the present invention:
(i) comprises or consists of an amino acid sequence having at least 90%, 91%, 92%, 93%, 94%, 95%, 96%, 97%, 98%, or 99% identity to the amino acid sequence set forth in SEQ ID NO: 33; or
(ii) comprises or consists of the amino acid sequence set forth in SEQ ID NO: 33;
   or
(iii) comprises or consists of an amino acid sequence having one or more (preferably no more than 10, and more preferably no more than 5, 4, 3, 2, or 1) amino acid changes (preferably amino acid substitutions, and more preferably conservative amino acid substitutions) compared with the amino acid sequence set forth in any one of (i)-(ii), wherein preferably, the amino acid changes do not occur in the CDRs.

In some embodiments, the light chain variable region of the present invention
(i) comprises or consists of an amino acid sequence having at least 90%, 91%, 92%, 93%, 94%, 95%, 96%, 97%, 98%, or 99% identity to the amino acid sequence selected from SEQ ID NO: 34; or
(ii) comprises or consists of the amino acid sequence selected from SEQ ID NO: 34; or
(iii) comprises or consists of an amino acid sequence having one or more (preferably no more than 10, and more preferably no more than 5, 4, 3, 2, or 1) amino acid changes (preferably amino acid substitutions, and more preferably conservative amino acid substitutions) compared with the amino acid sequence selected from any one of (i) to (ii), wherein preferably, the amino acid changes do not occur in the CDRs.

In some embodiments, the 3 complementarity determining regions from the heavy chain variable region (HCDRs) of the present invention, HCDR1, HCDR2, and HCDR3, are selected from
(i) three complementarity determining regions HCDR1, HCDR2, and HCDR3 contained in the VH set forth in SEQ ID NO: 33; or
(ii) a sequence comprising a total of at least one and no more than 5, 4, 3, 2, or 1 amino acid change (preferably amino acid substitutions, and more preferably conservative substitutions) in the three HCDRs relative to any sequence of (i),
wherein the HCDRs may be determined according to any scheme for determining CDRs, e.g., according to Kabat, AbM, Chothia, Contact, or IMGT, or a combination thereof;

For example, the HCDR1 is determined according to combined ranges of Kabat and Chothia schemes, and the HCDR2 and the HCDR3 are determined according to the Kabat scheme, respectively.

In some embodiments, the 3 complementarity determining regions from the light chain variable region (LCDRs) of the present invention, LCDR1, LCDR2, and LCDR3, are selected from
(i) three complementarity determining regions LCDR1, LCDR2, and LCDR3 contained in the VL set forth in SEQ ID NO: 34, or
(ii) a sequence comprising a total of at least one and no more than 5, 4, 3, 2, or 1 amino acid change (preferably amino acid substitutions, and more preferably conservative substitutions) in the three LCDRs relative to any sequence of (i).
wherein the LCDRs may be determined according to any scheme for determining CDRs, e.g., according to Kabat, AbM, Chothia, Contact, IMGT, or a combination thereof;

For example, the LCDR1, the LCDR2, and the LCDR3 are determined according to the Kabat scheme, respectively.

In some embodiments, the anti-FRα antibody or the antigen-binding fragment thereof of the present invention comprises:
three complementarity determining regions HCDR1, HCDR2 and HCDR3 contained in the VH set forth in SEQ ID NO: 33, and three complementarity determining regions LCDR1, LCDR2 and LCDR3 contained in the VL set forth in SEQ ID NO: 34;
wherein the HCDRs and LCDRs may be determined according to any scheme for determining CDRs, e.g., according to Kabat, AbM, Chothia, Contact, or IMGT, or a combination thereof;

For example, the HCDR1 is determined according to combined ranges of Kabat and Chothia schemes, the HCDR2 and the HCDR3 are determined according to the Kabat scheme, and the LCDR1, LCDR2, and LCDR3 are determined according to the Kabat scheme.

In some embodiments, the HCDR1 comprises or consists of an amino acid sequence set forth in SEQ ID NO: 27, or the HCDR1 comprises an amino acid sequence having one, two, or three amino acid changes (preferably amino acid substitutions, and more preferably conservative substitutions) compared with the amino acid sequence set forth in SEQ ID NO: 27.

In some embodiments, the HCDR2 comprises or consists of an amino acid sequence set forth in SEQ ID NO: 28, or the HCDR2 comprises an amino acid sequence having one, two, or three amino acid changes (preferably amino acid substitutions, and more preferably conservative substitutions) compared with the amino acid sequence set forth in SEQ ID NO: 28.

In some embodiments, the HCDR3 comprises or consists of an amino acid sequence set forth in SEQ ID NO: 29, or the HCDR3 comprises an amino acid sequence having one, two, or three amino acid changes (preferably amino acid substitutions, and more preferably conservative substitutions) compared with the amino acid sequence set forth in SEQ ID NO: 29.

In some embodiments, the LCDR1 comprises or consists of an amino acid sequence set forth in SEQ ID NO: 30, or the LCDR1 comprises an amino acid sequence having one, two, or three amino acid changes (preferably amino acid substitutions, and more preferably conservative substitutions) compared with the amino acid sequence set forth in SEQ ID NO: 30.

In some embodiments, the LCDR2 comprises or consists of an amino acid sequence set forth in SEQ ID NO: 31, or the LCDR2 comprises an amino acid sequence having one, two, or three amino acid changes (preferably amino acid substitutions, and more preferably conservative substitutions) compared with the amino acid sequence set forth in SEQ ID NO: 31.

In some embodiments, the LCDR3 comprises or consists of an amino acid sequence set forth in SEQ ID NO: 32, or the LCDR3 comprises an amino acid sequence having one, two, or three amino acid changes (preferably amino acid substitutions, and more preferably conservative substitutions) compared with the amino acid sequence set forth in SEQ ID NO: 32.

In some specific embodiments, the anti-FRα antibody of the present invention comprises a first heavy chain complementarity determining region (HCDR1), a second heavy chain complementarity determining region (HCDR2), a third heavy chain complementarity determining region (HCDR3), and a first light chain complementarity determining region (LCDR1), a second light chain complementarity determining region (LCDR2), and a third light chain complementarity determining region (LCDR3), wherein
the HCDR1, HCDR2, HCDR3, LCDR1, LCDR2, and LCDR3 comprise or consist of amino acid sequences set forth in SEQ ID NO: 27, SEQ ID NO: 28, SEQ ID NO: 29, SEQ ID NO: 30, SEQ ID NO: 31, and SEQ ID NO: 32, respectively.

In some embodiments, the VH of the present invention comprises an HCDR1, an HCDR2 and an HCDR3, and the VL comprises an LCDR1, an LCDR2 and an LCDR3, wherein
the HCDR1, HCDR2, HCDR3, LCDR1, LCDR2, and LCDR3 comprise or consist of amino acid sequences set forth in SEQ ID NO: 27, SEQ ID NO: 28, SEQ ID NO: 29, SEQ ID NO: 30, SEQ ID NO: 31, and SEQ ID NO: 32, respectively.

In some embodiments, the anti-FRα antibody or the antigen-binding fragment thereof of the present invention comprises:
a VH comprising or consisting of the amino acid sequence set forth in SEQ ID NO: 33 or an amino acid sequence having at least 90%, 91%, 92%, 93%, 94%, 95%, 96%, 97%, 98%, or 99% identity thereto, and/or a VL comprising or consisting of the amino acid sequence set forth in SEQ ID NO: 34 or an amino acid sequence having at least 90%, 91%, 92%, 93%, 94%, 95%, 96%, 97%, 98%, or 99% identity thereto.

In some embodiments, the anti-FRα antibody or the antigen-binding fragment thereof of the present invention comprises a heavy chain variable region and a light chain variable region, wherein the heavy chain variable region comprises or consists of an amino acid sequence set forth in SEQ ID NO: 33, and the light chain variable region comprises or consists of an amino acid sequence set forth in SEQ ID NO: 34.

In one embodiment of the present invention, the amino acid changes described herein include amino acid substitutions, insertions, or deletions. In some embodiments, the amino acid changes described herein are conservative amino acid changes. Preferably, the amino acid changes described herein are amino acid substitutions, preferably conservative substitutions. In a preferred embodiment, the amino acid changes described herein occur in regions outside the CDRs (e.g., in FRs). More preferably, the amino acid changes described herein occur in a region outside the heavy chain variable region and/or outside the light chain variable region.

In some embodiments, the anti-FRα antibody or the antigen-binding fragment thereof of the present invention further comprises an antibody heavy chain. In some embodiments, the anti-FRα antibody or the antigen-binding fragment thereof of the present invention further comprises an antibody light chain. In some embodiments, the anti-FRα antibody or the antigen-binding fragment thereof of the present invention further comprises a heavy chain and a light chain. In some embodiments, the antibody heavy chain of the present invention comprises or consists of a heavy chain variable region and a heavy chain constant region. In some embodiments, the antibody light chain of the present invention comprises or consists of a light chain variable region and a light chain constant region. In some embodiments, the antibody of the present invention comprises or consists of two heavy chains and two light chains. In some embodiments, the antibody of the present invention comprises or consists of two identical heavy chains and two identical light chains.

In some embodiments, the heavy chain constant region of the anti-FRα antibody of the present invention is a heavy chain constant region of IgG1, IgG2, IgG3, or IgG4, e.g., a constant region of human IgG1, IgG2, IgG3, or IgG4. In some embodiments, the heavy chain constant region is an IgG heavy chain constant region, e.g., an IgG1, IgG2, IgG3, or IgG4 heavy chain constant region. In some embodiments, the heavy chain constant region is an IgG1 heavy chain constant region, e.g., comprising an amino acid sequence set forth in SEQ ID NO: 46 or an amino acid sequence having at least 90%, 91%, 92%, 93%, 94%, 95%, 96%, 97%, 98%, or 99% sequence identity to the amino acid sequence set forth in SEQ ID NO: 46.

In some embodiments, the light chain constant region of the anti-FRα antibody of the present invention is a lambda or Kappa light chain constant region, preferably a Kappa light chain constant region, e.g., a human lambda or Kappa light chain constant region. In some embodiments, the light chain constant region is a (human) lambda light chain constant region.In some embodiments, the Lambda light chain constant region comprises the amino acid sequence set forth in SEQ ID NO: 38 or an amino acid sequence having at least 90%, 91%, 92%, 93%, 94%, 95%, 96%, 97%, 98%, or 99% sequence identity to the amino acid sequence set forth in SEQ ID NO: 38 and not comprising cysteine mutations. In some embodiments, the light chain constant region is a (human) Kappa light chain constant region. In some embodiments, the Kappa light chain constant region comprises an amino acid sequence set forth in SEQ ID NO: 45 or an amino acid sequence having at least 90%, 91%, 92%, 93%, 94%, 95%, 96%, 97%, 98%, or 99% sequence identity to the amino acid sequence set forth in SEQ ID NO: 45.

In some embodiments, the Anti-FRα antibody or the antigen-binding fragment thereof of the present invention is an antibody or an antigen-binding fragment thereof having a cysteine mutation in the light chain constant region, wherein the antibody or the antigen-binding fragment thereof comprises one or two lambda light chain constant regions and comprises a cysteine substitution at position 160 (EU numbering) of the lambda light chain constant region (LLC160C or LLC160) and/or a cysteine substitution at position 166 (EU numbering) of the lambda light chain constant region (LLC166C or LLC166).

In some embodiments, relative to the wild-type lambda light chain constant region, e.g., the amino acid sequence set forth in SEQ ID NO: 38, the light chain constant region of the anti-FRα antibody or the antigen-binding fragment thereof of the present invention has a cysteine mutation at position 160 (Eu numbering).

In some embodiments, the Lamda light chain constant region having a cysteine mutation at position 160 comprises an amino acid sequence set forth in SEQ ID NO: 39, or an amino acid sequence having at least 90%, 91%, 92%, 93%, 94%, 95%, 96%, 97%, 98%, or 99% sequence identity to the amino acid sequence set forth in SEQ ID NO: 39 and comprising an amino acid sequence VKAGVCTTTPS (SEQ ID NO: 42).

In some embodiments, relative to the wild-type lambda light chain constant region, e.g., the amino acid sequence set forth in SEQ ID NO: 38, the light chain constant region of the anti-FRα antibody or the antigen-binding fragment thereof of the present invention has a cysteine mutation at position 160 (Eu numbering).

In some embodiments, the Lamda light chain constant region having a cysteine mutation at position 166 comprises an amino acid sequence set forth in SEQ ID NO: 40, or an amino acid sequence having at least 90%, 91%, 92%, 93%, 94%, 95%, 96%, 97%, 98%, or 99% sequence identity to the amino acid sequence set forth in SEQ ID NO: 40 and comprising the amino acid sequence TTTPSCQSNNK (SEQ ID NO: 43).

In some embodiments, relative to the wild-type lambda light chain constant region, e.g., the amino acid sequence set forth in SEQ ID NO: 38, the light chain constant region of the anti-FRα antibody or the antigen-binding fragment thereof of the present invention has cysteine mutations at positions 160 and 166 (EU numbering).

In some embodiments, the Lamda light chain constant region having cysteine mutations at positions 160 and 166 comprises an amino acid sequence set forth in SEQ ID NO: 41, or has at least 90%, 91%, 92%, 93%, 94%, 95%, 96%, 97%, 98%, or 99% sequence identity to the amino acid sequence set forth in SEQ ID NO: 41 and comprises the amino acid sequence VKAGVCTTTPSCQSNNK (SEQ ID NO: 44).

In some embodiments, the antibody or the antigen-binding fragment thereof disclosed herein comprises:
(i) one or two Lamda light chain constant regions having a cysteine mutation at position 160 (Eu numbering); and/or
(ii) one or two Lamda light chain constant regions having a cysteine mutation at position 166 (Eu numbering).

The inventive anti-FRα antibody or the antigen-binding fragment thereof may be any form of an antibody or an antigen-binding fragment thereof known in the art, such as a monoclonal, chimeric, humanized, fully human, bispecific, or multispecific antibody or an antibody fragment thereof.

In some embodiments, the heavy chain of the anti-FRα antibody of the present invention
comprises or consists of an amino acid sequence having at least 85%, 90%, 91%, 92%, 93%, 94%, 95%, 96%, 97%, 98%, or 99% identity to the amino acid sequence selected from SEQ ID NO: 35;
comprises or consists of an amino acid sequence selected from SEQ ID NO: 35; or
comprises or consists of an amino acid sequence having one or more (preferably no more than 20 or 10, and more preferably no more than 5, 4, 3, 2, or 1) amino acid changes (preferably amino acid substitutions, and more preferably conservative amino acid substitutions) compared with the amino acid sequence selected from SEQ ID NO: 35.

In some embodiments, the light chain of the anti-FRα antibody of the present invention comprises or consists of an amino acid sequence having at least 85%, 90%, 91%, 92%, 93%, 94%, 95%, 96%, 97%, 98%, or 99% identity to an amino acid sequence selected from SEQ ID NO: 36 and 37;
comprises or consists of an amino acid sequence selected from SEQ ID NO: 36 and 37; or
comprises or consists of an amino acid sequence having one or more (preferably no more than 20 or 10, and more preferably no more than 5, 4, 3, 2, or 1) amino acid changes (preferably amino acid substitutions, and more preferably conservative amino acid substitutions) compared with the amino acid sequence selected from SEQ ID NO: 36 and 37.

In some embodiments, the anti-FRα antibody or the antigen-binding fragment thereof of the present invention comprises two identical heavy chains and two identical light chains, wherein
the heavy chain comprises the VH described herein or the HCDR1, the HCDR2 and the HCDR3 described herein, and the Fc region and the CH1 described herein, or the heavy chain constant region described herein; and/or
the light chain comprises the VL described herein or the LCDR1, the LCDR2, and the LCDR3 described herein, and the Kappa light chain constant region described herein.

In some embodiments, the anti-FRα antibody or the antigen-binding fragment thereof of the present invention comprises two identical heavy chains and two identical light chains, wherein
the heavy chain comprises or consists of an amino acid sequence having at least 85%, 90%, 91%, 92%, 93%, 94%, 95%, 96%, 97%, 98%, or 99% identity to the amino acid sequence set forth in SEQ ID NO: 35, and/or
the light chain comprises or consists of an amino acid sequence having at least 85%, 90%, 91%, 92%, 93%, 94%, 95%, 96%, 97%, 98%, or 99% identity to the amino acid sequence set forth in SEQ ID NO: 36.

In some embodiments, the anti-FRα antibody or the antigen-binding fragment thereof of the present invention comprises two identical heavy chains and two identical light chains, wherein
the heavy chain comprises or consists of an amino acid sequence set forth in SEQ ID NO: 35, and/or
the light chain comprises or consists of an amino acid sequence set forth in SEQ ID NO: 36.

In some embodiments, the anti-FRα antibody or the antigen-binding fragment thereof of the present invention comprises two identical heavy chains and two identical light chains, wherein
the heavy chain comprises the VH described herein or the HCDR1, HCDR2, and HCDR3 described herein, and the Fc region and the CH1 described herein, or the heavy chain constant region described herein;
the light chain comprises the VL described herein or the LCDR1, LCDR2, and LCDR3 described herein, and the Lambda light chain constant region comprising LLC160C and/or 166C described herein.

In one specific embodiment, the anti-FRα antibody or the antigen-binding fragment thereof of the present invention comprises two identical heavy chains and two identical light chains, wherein
the heavy chain comprises or consists of an amino acid sequence having at least 85%, 90%, 91%, 92%, 93%, 94%, 95%, 96%, 97%, 98%, or 99% identity to an amino acid sequence set forth in SEQ ID NO: 35, and/or
the light chain comprises or consists of an amino acid sequence having at least 85%, 90%, 91%, 92%, 93%, 94%, 95%, 96%, 97%, 98%, or 99% identity to an amino acid sequence set forth in SEQ ID NO: 37.

In one more specific embodiment, the anti-FRα antibody or the antigen-binding fragment thereof of the present invention comprises two identical heavy chains and two identical light chains, wherein
the heavy chain comprises or consists of an amino acid sequence set forth in SEQ ID NO: 35, and/or
the light chain comprises or consists of an amino acid sequence set forth in SEQ ID NO: 37.

In some embodiments, the anti-FRα antibody or the antigen-binding fragment thereof of the present invention is a chimeric antibody or a humanized antibody.

In some embodiments, the antigen-binding fragment of the anti-FRα antibody of the present invention is, for example, Fv, Fab, Fab', Fab'-SH, or F(ab')₂; a domain antibody (dAb); a linear antibody; a single-chain antibody (e.g., scFv); a single-domain antibody (e.g., VHH); a bivalent antibody or a fragment thereof; or a camelid antibody.

In one embodiment, the anti-FRαThe antibody is a full-length antibody.

In one embodiment, the anti-FRαThe antibody also encompasses multispecific antibodies such as bispecific antibodies.

In some embodiments, the anti-FRα antibody or the antigen-binding fragment thereof of the present invention has relatively good endocytic activity on FRα-positive cells, e.g., FRα-positive tumor cells.

In some embodiments, the present invention also provides a fusion protein comprising the anti-FRα antibody or the antigen-binding fragment thereof of the present invention.

In some embodiments, the present invention also provides an immunoconjugate comprising the anti-FRα antibody or the antigen-binding fragment thereof of the present invention, and further comprising a payload coupled to the antibody or the antigen-binding fragment thereof. In some embodiments, the payload is a toxin, a small molecule drug/agent, a cytotoxic agent, an apoptotic agent, a chelating agent, an immunomodulator/immunostimulatory agent/immunoagonist, an oligonucleotide, a polypeptide, a peptide epitope, a radionuclide, a prodrug, etc. In some preferred embodiments, the payload is an immunostimulatory agent/immune activator. In some more preferred embodiments, the payload is a TLR agonist. In some more preferred embodiments, the payload is a TLR7/8 agonist.

Thus, the present invention provides an antibody targeting FRα or an antigen-binding fragment thereof and an antibody-immunoagonist conjugate (ISAC) targeting FRα.

In one aspect, the antibody or the antigen-binding fragment thereof of the present invention is subjected to cysteine engineering, so that the antibody or the antigen-binding fragment thereof mutates one or more amino acids to cysteine on its heavy or light chain. Site-specific conjugated ISAC molecules can be prepared by conjugating a cysteine to a small molecule having a maleimide linker (e.g., an immune agonist, particularly a TLR agonist, more particularly a TLR7/8 agonist) via a thiol group on the cysteine in a nucleophilic reaction.

In some embodiments, Ab in the ADC of the present invention (including those of formula (II) or (III)) comprises cysteine. In some embodiments, in the ADC of the present invention, Ab is linked to a linker via cysteine (e.g., naturally occurring and/or introduced by cysteine mutation), for example, the linker is conjugated to Ab via a sulfhydryl group of cysteine.

In some embodiments, the Ab is conjugated to a linker via a natural cysteine. For example, the linker is conjugated (e.g., randomly conjugated) to the Ab via a sulfhydryl group of a disulfide-bonded cysteine.

In some embodiments, Ab is conjugated to a linker via a cysteine introduced by a cysteine mutation thereof; for example, Ab is conjugated (e.g., site-directed conjugation) to a linker via a cysteine residue that is unpaired or otherwise becomes part of an intramolecular or intermolecular disulfide bond (e.g., cysteine obtained by cysteine mutation), or for example, the linker is conjugated (e.g., random conjugation or site-directed conjugation) to Ab via a sulfhydryl group of a cysteine whose disulfide bond is opened.

In some embodiments, in the ADC molecule of the present invention, the Ab is conjugated (e.g., site-directed conjugation) to one toxin via a cysteine introduced by mutation of the cysteine of the Ab and conjugated (e.g., random conjugation) to another toxin via the natural cysteine of the Ab.

In some embodiments, Ab in the ADC molecule of the present invention comprises a cysteine introduced by a cysteine mutation, such that the linker is conjugated to the antibody via a sulfhydryl group of the mutated cysteine. In some embodiments, Ab comprises mutation of one or more non-cysteines into cysteines on the heavy or light chain thereof, such that the linker is conjugated to the cysteine, e.g., site-directed conjugation. The antibody or the antigen-binding fragment thereof comprising the cysteine mutation suitable for the ADC of the present invention is described in detail herein, wherein the cysteine obtained after the mutation is coupled to a linker, for example, site-directed coupling.

In some embodiments, in the ISAC molecule of the present invention, cysteine obtained by mutating Ab as follows is conjugated to the linker:
(i) a cysteine obtained by mutation of cysteine at position 160 in one or two Lamda light chain constant regions; and/or
(ii) a cysteine obtained by mutation of cysteine at position 166 in one or two Lamda light chain constant regions.

Cysteine obtained by mutation of any of the antibodies or the antigen-binding fragments thereof mentioned herein with any of the cysteines obtained herein can be used for conjugation with a linker, e.g., site-directed conjugation, to obtain the immunostimulatory molecule of the present invention.

In one aspect, the antibody or the antigen-binding fragment thereof of the present invention has cysteine mutations at sites that are cryptic in the constant regions, thereby bringing better stability and/or hydrophilicity to an immunoconjugate (e.g., ISAC) comprising same. "Cysteine mutation" as defined herein refers to the substitution of an amino acid that was not cysteine in the protein with cysteine.

In one aspect, the present invention provides a nucleic acid encoding any one of the chains or any monomer or domain of the antibody or the antigen-binding fragment thereof disclosed herein.Polynucleotide sequences encoding each strand can be generated using methods well known in the art.For example, a polypeptide encoded by the nucleic acid is capable of exhibiting human TROP2 antigen-binding ability when expressed in a suitable expression vector.For example, in some embodiments, the nucleic acid encoding the variable region of the heavy chain and/or the light chain is operably linked to the nucleic acid encoding the constant region of the heavy chain and/or the light chain in a reading frame, thereby producing the nucleic acid encoding the heavy chain and/or the light chain of the antibody when expressed in a suitable expression vector.

In one aspect, the present invention provides a nucleic acid encoding any of the anti-TROP2 antibodies or the fragments thereof described herein.The nucleic acid may comprise a nucleic acid encoding an amino acid sequence of the light chain variable region and/or heavy chain variable region of the antibody, or a nucleic acid encoding an amino acid sequence of the light chain and/or heavy chain of the antibody.

For example, the nucleic acid of the present invention comprises a nucleic acid encoding an amino acid sequence selected from any one of SEQ ID NOs: 1-10, 12, and 14-16, or a nucleic acid encoding an amino acid sequence having at least 85%, 90%, 91%, 92%, 93%, 94%, 95%, 96%, 97%, 98%, or 99% identity to the amino acid sequence selected from any one of SEQ ID NOs: 1-10, 12, and 14-16. As will be appreciated by those skilled in the art, each antibody or polypeptide amino acid sequence can be encoded by a variety of nucleic acid sequences because of codon degeneracy. The nucleic acid sequence encoding the molecule of the present invention can be generated using methods well known in the art, for example, by de novo solid-phase DNA synthesis or PCR amplification. For ease of production and purification, a secretory signal peptide can be fused to the N-terminus of the heavy chain and/or the light chain of the antibody, and/or a tag peptide to facilitate purification.

In another aspect, the invention provides a polynucleotide encoding any of the anti-FRα antibodies or fragments thereof as described herein. The nucleic acid may comprise a nucleic acid encoding an amino acid sequence of the light chain variable region and/or heavy chain variable region of the antibody, or a nucleic acid encoding an amino acid sequence of the light chain and/or heavy chain of the antibody.

For example, the nucleic acid of the present invention includes a nucleic acid encoding an amino acid sequence selected from any one of SEQ ID NOs: 27-46, or a nucleic acid encoding an amino acid sequence having at least 85%, 90%, 91%, 92%, 93%, 94%, 95%, 96%, 97%, 98%, or 99% identity to the amino acid sequence selected from any one of SEQ ID NOs: 27-46. As will be appreciated by those skilled in the art, each antibody or polypeptide amino acid sequence can be encoded by a variety of nucleic acid sequences because of codon degeneracy. The nucleic acid sequence encoding the molecule of the present invention can be generated using methods well known in the art, for example, by de novo solid-phase DNA synthesis or PCR amplification. For ease of production and purification, a secretory signal peptide can be fused to the N-terminus of the heavy chain and/or the light chain of the antibody, and/or a tag peptide to facilitate purification.

Polynucleotides encoding the polypeptide chains of the antibody disclosed herein can be inserted into one or more vectors for further cloning and/or expression in host cells.Methods which are well known to those skilled in the art can be used to construct expression vectors. Once an expression vector comprising one or more nucleic acid molecules of the invention has been prepared for expression, the expression vector can be transfected or introduced into a suitable host cell. Various techniques can be used for this purpose, for example, protoplast fusion, calcium phosphate precipitation, electroporation, retroviral transduction, viral transfection, biolistics, liposome-based transfection, or other conventional techniques.

The invention also provides a vector comprising a nucleic acid of the invention.In one embodiment, the vector is an expression vector, e.g., a eukaryotic expression vector. The vector includes, but is not limited to, a virus, a plasmid, a cosmid, a lambda phage, or a yeast artificial chromosome (YAC).In a preferred embodiment, the expression vector of the present invention is a pcDNA vector, e.g., a pcDNA3.1 expression vector. In other preferred embodiments, the expression vector of the present invention is a pTT5 vector or an expression vector derived from pTT5.

In one embodiment, provided is a host cell comprising the vector. The invention also provides a host cell comprising the nucleic acid or the vector. Host cells suitable for replicating and supporting the expression of the antibody of the present invention are well known in the art. Such cells can be transfected or transduced with a particular expression vector, and a large number of vector-containing cells can be grown for inoculation in large-scale fermenters, so as to obtain sufficient antibodies for clinical application. Suitable host cells for cloning or expressing the vector encoding the antibody include prokaryotic or eukaryotic cells described herein. For example, the antibody can be produced in bacteria, particularly when glycosylation and Fc effector functions are not required. After expression, the antibody can be isolated from the bacterial cell paste in a soluble fraction and can be further purified.

In one embodiment, the host cell is eukaryotic.In another embodiment, the host cell is selected from yeast cells, mammalian cells (e.g., CHO cells (e.g., CHO-S or CHO-K) or 293 cells (e.g., 293F or HEK293 cells)), or other cells suitable for preparing an antibody or a fragment thereof.In one embodiment, the host cell is prokaryotic, e.g., a bacterium, such as E. coli.

For example, eukaryotic microorganisms such as filamentous fungi or yeast are suitable cloning or expression hosts for the vector encoding the antibody.For example, fungal and yeast strains in which the glycosylation pathway has been "humanized" result in the production of antibodies having a partially or fully human glycosylation pattern.Suitable host cells for the expression of glycosylated antibodies are also derived from multicellular organisms (invertebrates and vertebrates).Vertebrate cells may also be used as hosts.For example, mammalian cell lines engineered to be suitable for growth in suspension may be used.Other examples of useful mammalian host cell lines are monkey kidney CV1 lines (COS-7) transformed with SV40, human embryonic kidney lines (HEK293, 293F, or 293T cells), and the like.Other useful mammalian host cell lines include Chinese hamster ovary (CHO) cells, including DHFR-CHO cells, CHO-S cells, ExpiCHO, and the like; and myeloma cell lines such as Y0, NS0, and Sp2/0.Mammalian host cell lines suitable for antibody production are known in the art.

### III. Immunoconjugates

The present invention provides an antibody-immunoagonist conjugates of formula (I):

Ab-(L-D)ₚ (I)

or a pharmaceutically acceptable salt or solvate thereof,
wherein:
Ab is an antibody or a fragment thereof that binds to TROP2 (e.g., human TROP2);
L is a linker;
D is a drug, preferably an anti-tumour compound; and
p is 1-16, e.g., 1-10, 1-9, 2-8, 4-10, 3-7, 4-6 or 2-6, e.g., 1, 2, 3, 4, 5, 6, 7, 8, 9, 10 or 12.

In some embodiments, Ab in formula (I) of the present invention is the aforementioned anti-TROP2 antibody or antigen-binding fragment thereof, or anti-FRα antibody or antigen-binding fragment thereof.

In some embodiments, D in formula (I) of the present invention may be any TLR7/8 agonist compounds, e.g. compounds of formulae (D-1) to (D-5) as defined above, e.g. compounds of formula (D-1'), e.g. compounds selected from the group consisting of

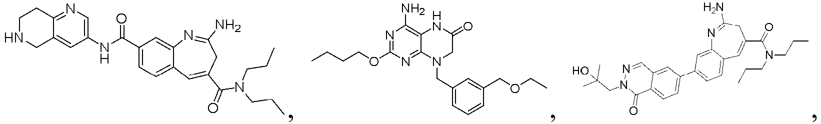

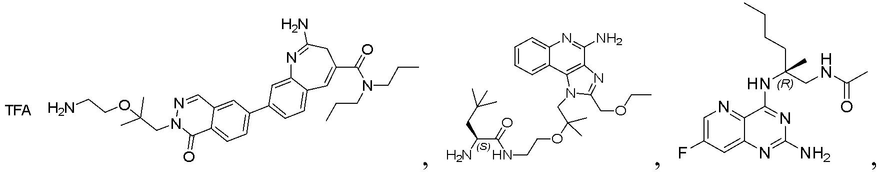

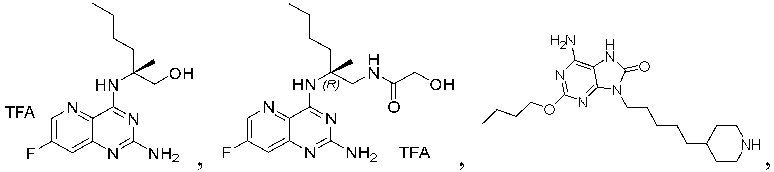

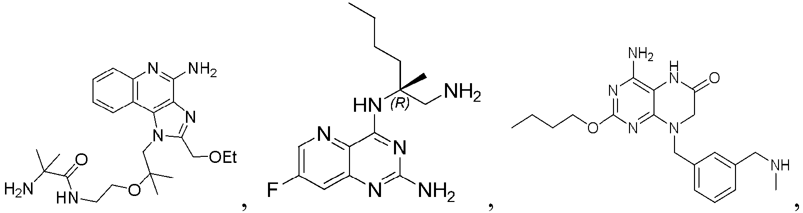

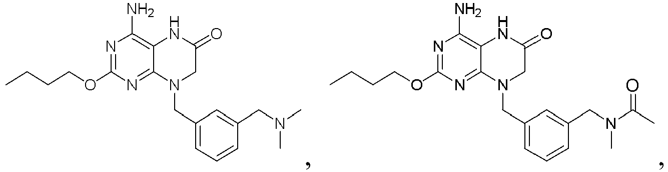

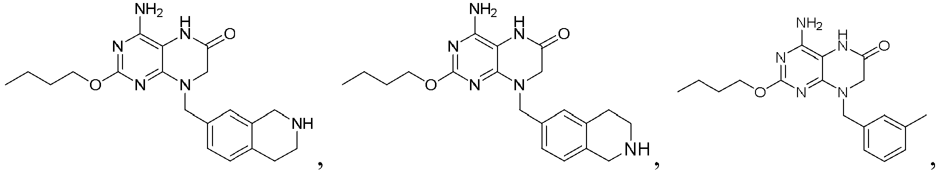

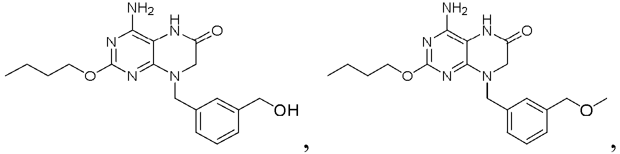

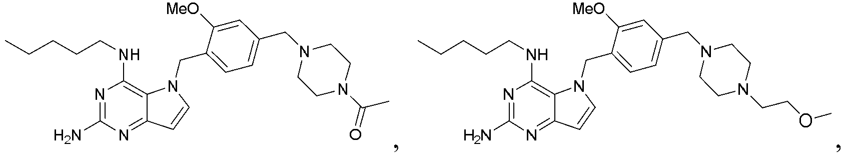

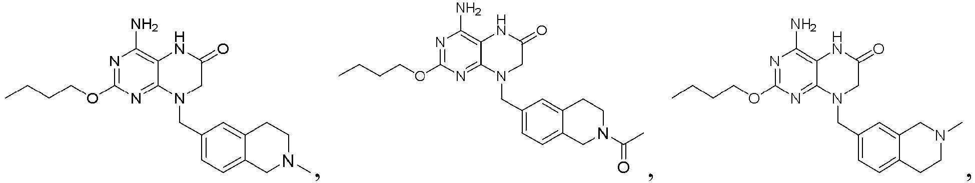

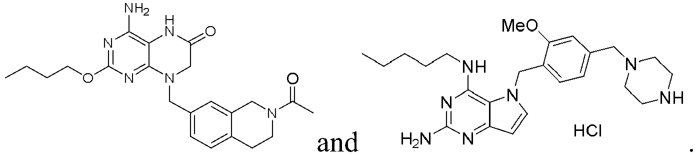

### IV. Preparation of Antibodies and ISAC Molecules of the Invention

The present invention provides a method for preparing an antibody, e.g., preparing the anti-TROP2 antibody or the antigen-binding fragment thereof or the anti-FRα antibody or the antigen-binding fragment thereof, to prepare Abs for the ISAC of the invention.

In one embodiment, the method comprises culturing a host cell comprising a nucleic acid encoding the antibody (e.g., any one and/or more polypeptide chains) or an expression vector comprising the nucleic acid, as provided above, under conditions suitable for expressing the antibody or a peptide chain thereof, and optionally recovering the antibody from the host cell (or a host cell medium).

The Abs prepared as described herein can be purified by known prior art such as high performance liquid chromatography, ion exchange chromatography, gel electrophoresis, affinity chromatography, size exclusion chromatography, and the like.The actual conditions used to purify a particular protein also depend on factors such as net charge, hydrophobicity, and hydrophilicity, and these will be apparent to those skilled in the art.

In some embodiments, the present invention relates to a method for preparing an anti-TROP2 or FRα antibody or an antigen-binding fragment thereof, which comprises
a) culturing the host cell of the present invention under conditions suitable for expressing a nucleic acid encoding the anti-TROP2 or FRα antibody or the antigen-binding fragment thereof of the present invention,
b) optionally isolating the antibody or antigen binding fragment thereof,
c) optionally, the method further comprises isolating the anti-TROP2 or FRα antibody or the antigen-binding fragment thereof from the host cell; optionally, the antibody is purified, e.g., by ProteinA.

Another aspect of the present invention provides a method for preparing an ISAC using the antibody of the present invention."ISAC" in the present invention is defined as an antibody conjugated to an active substance (D) having biological and/or pharmaceutical activity via a linker (L).The method comprises conjugating the antibody (Ab) of the present invention to one or more active substances D via one or more linkers (L) defined herein.Preferably, the linker-active substance is site-specifically conjugated to the antibody.

In some embodiments, the method comprises the following steps:
(a) adding the antibody Ab to a buffer,Addition of reducing agentfollowed by incubation;
(b) adding the linker-payload to the reaction solution in step (a) for coupling to give a crude product; and
(c) optionally purifying the crude product to obtain the antibody-drug conjugate of the present invention;
wherein Ab is as defined above.

It should be understood that the reaction of the linker-payload with Ab provides the moiety -L-D in the compound of formula (I), and the structure of the linker-payload can be determined when -L-D is clearly defined.

In some embodiments, the buffer solution in step a) is a PBS buffer, preferably with a pH of 5.0-9.0, e.g., 6.0-8.0.

In some embodiments, the reducing agent of step a) is TCEP.

In some embodiments, the linker-payload has a structure of the following formula (II):

L-(D)ₘ (II)

where D and L are as defined above.

In some embodiments, the step is performed under the specific reaction conditions disclosed in the examples.

It should be noted that ranges of specific reaction conditions disclosed in the examples, or embodiments resulting from a 100%, 80%, 60%, 40%, 20%, or 10% variation of the specific values, are also contemplated by the present invention.

### V. Pharmaceutical compositions

In some embodiments, the present invention provides a composition comprising any of the antibodies or the fragments thereof, the ISAC molecules, or the pharmaceutically acceptable salts thereof described herein, wherein preferably, the composition is a pharmaceutical composition or a pharmaceutical formulation. In one embodiment, the composition further comprises a pharmaceutical supplementary material. In one embodiment, the composition, e.g., the pharmaceutical composition, comprises a combination of the antibody or the fragment thereof, the ISAC molecule of the present invention, and one or more additional therapeutic agents.

The present invention further comprises a composition (including a pharmaceutical composition) comprising the antibody or the fragment thereof, the ISAC molecule, or the pharmaceutically acceptable salt thereof of the present invention. These compositions may further comprise a suitable pharmaceutical supplementary material, such as a pharmaceutical carrier and a pharmaceutical excipient known in the art, including buffers.

As used herein, the "pharmaceutical carrier" includes any and all solvents, dispersion media, isotonic agents and absorption delaying agents, and the like that are physiologically compatible.

For use and application of pharmaceutical excipients, see Handbook of Pharmaceutical Excipients, 8th Ed., R. C. Rowe, P. J. Seskey and S. C. Owen, Pharmaceutical Press, London, Chicago.

The composition of the present invention may be in a variety of forms. These forms include, for example, liquid, semi-solid, and solid dosage forms, such as liquid solutions (e.g., injectable solutions and infusible solutions), powders or suspensions, liposomes, and suppositories. The preferred form depends on the intended mode of administration and therapeutic use.

The medicament, preferably in the form of a lyophilized formulation or an aqueous solution, comprising the ISAC described herein can be prepared by mixing the antibody or the fragment thereof or the ISAC molecule of the present invention having desired purity with one or more optional pharmaceutical supplementary materials.

The pharmaceutical composition or formulation of the present invention can further comprise more than one active ingredient required for the specific indication treated, preferably active ingredients having complementary activities without adversely affecting one another. For example, it may be desirable to further provide additional therapeutic agents, including chemotherapeutic agents, angiogenesis inhibitors, cytokines, cytotoxic agents, other antibodies, small molecule drugs, immunomodulatory agents (e.g., immune checkpoint inhibitors or agonists), or the like. The active ingredients are suitably present in combination in amounts that are effective for the purpose intended.

Sustained release preparation can be prepared. Suitable examples of the sustained release preparation include a semipermeable matrix of a solid hydrophobic polymer containing an antibody. The matrix is in the form of a shaped article, such as a film or a microcapsule.

### VI. Pharmaceutical combination and kit

In some embodiments, the present invention further provides a pharmaceutical combination or a pharmaceutical combination product comprising the antibody or the fragment thereof or the ISAC molecule of the present invention, and one or more additional therapeutic agents (e.g., therapeutic agents, including chemotherapeutic agents, angiogenesis inhibitors, cytokines, cytotoxic agents, other antibodies, small molecule drugs, immunomodulatory agents (e.g., immune checkpoint inhibitors or agonists), or the like).

In some embodiments, the additional therapeutic agent is a TROP2-targeted antibody-drug conjugate or a stereoisomer thereof, or a pharmaceutically acceptable salt or solvate thereof.

In some embodiments, the additional therapeutic agent is an antibody-drug conjugate or a pharmaceutically acceptable salt or solvate disclosed in PCT/CN2024/121813.

In some embodiments, the additional therapeutic agent is an antibody-drug conjugate of the following formula, or a stereoisomer, a pharmaceutically acceptable salt, or a solvate thereof: 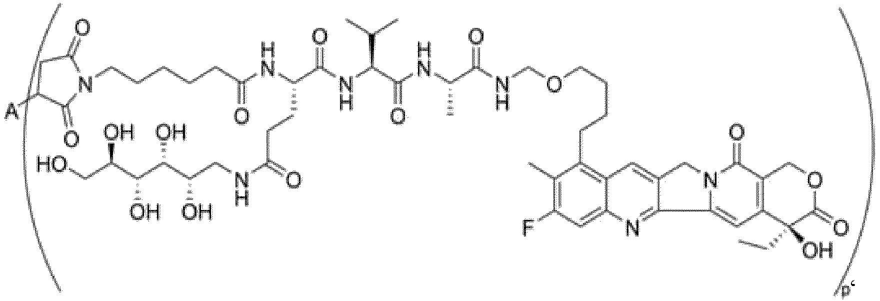 wherein A represents a TROP2-targeted antibody or a fragment thereof:
p' is 1-15, e.g., a value between 2-10, 2-8, 2-6, 2-5, 3-5, or 3.5-4.5.

In some embodiments, the TROP2-targeted antibody or the fragment thereof (e.g., antigen-binding fragment) suitable for use in constructing the antibody-drug conjugate described above may be a TROP2-targeted antibody or antibody fragment of mammalian origin (e.g., human or mouse), humanized, or chimeric origin.In some embodiments, the TROP2-targeting antibody or the fragment thereof suitable for use in the construction of the antibody-drug conjugate described above is the anti-TROP2 antibody or the fragment thereof such as an antigen-binding fragment defined above.

In some embodiments, the TROP2-targeted antibody or the fragment thereof suitable for use in the construction of the antibody-drug conjugate described above comprises a first heavy chain complementarity determining region (HCDR1), a second heavy chain complementarity determining region (HCDR2), a third heavy chain complementarity determining region (HCDR3), a first light chain complementarity determining region (LCDR1), a second light chain complementarity determining region (LCDR2), and a third light chain complementarity determining region (LCDR3), wherein
the HCDR1 comprises or consists of an amino acid sequence set forth in SEQ ID NO: 17;
the HCDR2 comprises or consists of an amino acid sequence set forth in SEQ ID NO: 18;
the HCDR3 comprises or consists of an amino acid sequence set forth in SEQ ID NO: 19;
the LCDR1 comprises or consists of an amino acid sequence set forth in SEQ ID NO: 20;
the LCDR2 comprises or consists of an amino acid sequence set forth in SEQ ID NO: 21;
the LCDR3 comprises or consists of an amino acid sequence set forth in SEQ ID NO: 22.

In some embodiments, the TROP2-targeting antibody or the fragment thereof suitable for use in the construction of the antibody-drug conjugate described above comprises a heavy chain variable region and a light chain variable region, wherein the heavy chain variable region comprises HCDR1, HCDR2, and HCDR3, and the light chain variable region comprises LCDR1, LCDR2, and LCDR3, wherein the HCDR1, HCDR2, HCDR3, LCDR1, LCDR2, and LCDR3 comprise or consist of amino acid sequences set forth in SEQ ID NO: 17, SEQ ID NO: 18, SEQ ID NO: 19, SEQ ID NO: 20, SEQ ID NO: 21, and SEQ ID NO: 22, respectively.

In some embodiments, the TROP2-targeted antibody or the fragment thereof suitable for use in the construction of the antibody-drug conjugate described above comprises a heavy chain variable region and a light chain variable region, wherein the heavy chain variable region comprises or consists of an amino acid sequence set forth in SEQ ID NO: 23 or an amino acid sequence having at least 90%, 91%, 92%, 93%, 94%, 95%, 96%, 97%, 98% or 99% identity to the amino acid sequence set forth in SEQ ID NO: 23, and the light chain variable region comprises or consists of an amino acid sequence set forth in SEQ ID NO: 24 or an amino acid sequence having at least 90%, 91%, 92%, 93%, 94%, 95%, 96%, 97%, 98% or 99% identity to the amino acid sequence set forth in SEQ ID NO: 24.

In some embodiments, the TROP2-targeted antibody or the fragment thereof suitable for use in the construction of the antibody-drug conjugate described above comprises a heavy chain variable region and a light chain variable region, wherein the heavy chain variable region comprises or consists of an amino acid sequence set forth in SEQ ID NO: 23 and the light chain variable region comprises or consists of an amino acid sequence set forth in SEQ ID NO: 24.In some embodiments, the TROP2-targeting antibody or the fragment thereof suitable for use in the construction of the antibody-drug conjugate described above comprises a heavy chain variable region having a sequence of SEQ ID NO: 23 and a light chain variable region having a sequence of SEQ ID NO: 24.

In some embodiments, the TROP2-targeting antibody or the fragment thereof suitable for use in the construction of the antibody-drug conjugate described above comprises a heavy chain and a light chain, wherein the heavy chain variable region comprises or consists of an amino acid sequence set forth in SEQ ID NO: 25 or an amino acid sequence having at least 90%, 91%, 92%, 93%, 94%, 95%, 96%, 97%, 98% or 99% identity to the amino acid sequence set forth in SEQ ID NO: 25, and the light chain variable region comprises or consists of an amino acid sequence set forth in SEQ ID NO: 26 or an amino acid sequence having at least 90%, 91%, 92%, 93%, 94%, 95%, 96%, 97%, 98% or 99% identity to the amino acid sequence set forth in SEQ ID NO: 26.

In some embodiments, the TROP2-targeted antibody or the fragment thereof suitable for use in the construction of the antibody-drug conjugate described above comprises a heavy chain and a light chain, wherein the heavy chain comprises or consists of an amino acid sequence set forth in SEQ ID NO: 25, and the light chain comprises or consists of an amino acid sequence set forth in SEQ ID NO: 26.In some embodiments, the TROP2-targeting antibody or the fragment thereof suitable for use in the construction of the antibody-drug conjugate described above comprises a heavy chain having a sequence of SEQ ID NO: 25 and a light chain having a sequence of SEQ ID NO: 24.

In some specific embodiments, the TROP2-targeting antibody suitable for use in the construction of the antibody-drug conjugate described above is a full-length antibody.In a specific embodiment, the antibody targeting TROP2 comprises or consists of two heavy chains and two light chains as defined above.

In some specific embodiments, the TROP2-targeting antibody or the fragment thereof suitable for use in the construction of the antibody-drug conjugate described above is a multispecific antibody such as a bispecific antibody.In some specific embodiments, the TROP2-targeting antibody suitable for use in the construction of the antibody-drug conjugate described above is a monoclonal antibody.In some specific embodiments, the TROP2-targeting antibody suitable for use in the construction of the antibody-drug conjugate described above is a chimeric antibody or a humanized antibody.In some specific embodiments, the TROP2-targeted antibody fragment suitable for use in the construction of the antibody-drug conjugate described above is selected from Fab, Fab', Fab'-SH, Fv, single-chain antibodies (e.g., scFv), and (Fab')₂, single-domain antibodies such as VHH, domain antibodies (dAbs), bivalent antibodies, or linear antibodies.

In some embodiments, the TROP2-targeting antibody or the fragment thereof is the TROP2-targeting antibody or the fragment thereof of PCT/CN2024/121813, for example, hRS7 antibody. The Patent Application PCT/CN2024/121813 is incorporated herein in its entirety.

In some embodiments, the additional therapeutic agent targetsFRαan antibody-drug conjugate, or a stereoisomer, a pharmaceutically acceptable salt, or a solvate thereof. In some specific embodiments, the FRα targeting antibody or the fragment thereof is mirvetuximab from ImmunoGen in U.S. Patent Application Publication No. US20200362029A1. In some specific embodiments, the FRα targeting antibody or the fragment thereof is FR57 from ImmunoGen in U.S. Patent Application Publication No. US20200362029A1. In some specific embodiments, the FRα targeting antibody -drug conjugate was Elahere^{®}. In some specific embodiments, the FRα targeting antibody-drug conjugate is FR57-Dxd. The Patent Application US20200362029A1 is incorporated herein in its entirety.

The above antibody-drug conjugate can be prepared according to the methods described in the following examples or the methods described in related applications PCT/CN2024/121813 and US20200362029A1.

Another object of the present invention is to provide a kit of parts comprising the pharmaceutical combination of the present invention, wherein preferably, the kit is in the form of a pharmaceutical dose unit. Dose units may thus be provided according to the dosing regimen or the interval between drug administrations.

In one embodiment, the kit of parts of the present invention comprises in the same package:
- a first container containing a pharmaceutical composition comprising the antibody or the fragment thereof, and/or the ISAC molecule of the present invention;
- a second container containing a pharmaceutical composition comprising an additional therapeutic agent.

### VII. Use and method

In one aspect, the present invention provides a method for preventing or treating a tumor (e.g., cancer) in a subject, comprising administering to the subject an effective amount of the antibody and/or the ISAC molecule, the pharmaceutical composition, the pharmaceutical combination, or the kit of the present invention.

In some embodiments, the tumor, e.g., cancer, includes solid tumors and hematological tumors as well as metastatic lesions. In one embodiment, examples of the solid tumor include a malignant tumor. The cancer may be at an early, intermediate or advanced stage or be a metastatic cancer.

In some embodiments, the tumor, e.g., cancer, is TROP2-positive, e.g., it comprises tumor cells expressing TROP2. In some embodiments, the patient's tumor comprises a tumor cell expressing TROP2. In some embodiments, the tumor cells in the patient express TROP2, e.g., moderately express TROP2, and preferably highly express TROP2. In some embodiments, the patient with the tumor (e.g., cancer) has TROP2 in the tumor tissue (e.g., at an elevated level, e.g., at a nucleic acid or protein level), e.g., as compared to the level of TROP2 in the same tissue of a healthy individual or in a healthy tissue adjacent to the tumor tissue of the patient. In some embodiments, the tumor cells of the patient have TROP2 (e.g., at an elevated level, e.g., at a nucleic acid or protein level), e.g., as compared to the level of TROP2 in the same cells of a healthy individual or in healthy cells adjacent to the tumor cells of the patient.

In a specific embodiment, the antibody of the present invention and/orISAC Moleculesbeing capable of killing tumor cells, and/or inhibiting tumor cell proliferation, e.g., tumor cells expressing TROP2, such as lung cancer cells, head and neck tumor cells, prostate cancer cells, melanoma cells, pancreatic cancer cells, breast cancer cells, esophageal cancer cells, cervical cancer cells, kidney cancer cells, bladder cancer cells, or ovarian cancer cells.

In some embodiments, the tumor, e.g., cancer, is FRα-positive, e.g., it comprises tumor cells expressing FRα. In some embodiments, the patient's tumor comprises tumor cells expressing FRα. In some embodiments, the tumor cells of the patient express FRα, e.g., moderately express FRα, preferably highly express FRα. In some embodiments, the tumor tissue of the patient with the tumor (e.g., cancer) has FRα (e.g., at an elevated level, e.g., at a nucleic acid or protein level), e.g., as compared to the level of FRα in the same tissue of a healthy individual or in a healthy tissue adjacent to the tumor tissue of the patient.In some embodiments, the tumor cells of the patient have FRα (e.g., at an elevated level, e.g., at a nucleic acid or protein level), e.g., as compared to the level of FRα in the same cells of a healthy individual or in healthy cells adjacent to the tumor cells of the patient.

In a specific embodiment, the antibody and/or ISAC molecule of the present invention being capable of killing tumor cells, and/or inhibiting tumor cell proliferation, e.g., tumor cells expressing FRα, such as lung cancer cells, head and neck tumor cells, prostate cancer cells, melanoma cells, pancreatic cancer cells, breast cancer cells, esophageal cancer cells, cervical cancer cells, kidney cancer cells, bladder cancer cells, or ovarian cancer cells.

In some embodiments, the tumor is tumor immune escape.

In some embodiments, the tumor is a cancer, e.g., lung cancer, head and neck tumor, prostate cancer, melanoma, pancreatic cancer, breast cancer, esophageal cancer, cervical cancer, renal cancer, bladder cancer, or ovarian cancer.

The subject may be a mammal, e.g., a primate, preferably a higher primate, e.g., a human (e.g., an individual suffering from or at risk of suffering from the disease described herein).In one embodimentwherein the subject has or is at risk of having a disease described herein (e.g., cancer). In some embodiments, the subject is receiving or has received other therapies, such as chemotherapy and/or radiation therapy.In some embodiments, the subject has previously received or is receiving immunotherapy.

In other aspects, the present invention provides use of the antibody and/or the ISAC molecules or the pharmaceutical composition, the pharmaceutical combination or the kit described above in the manufacture or preparation of a medicament, for the use described herein, e.g., preventing or treating the related diseases or conditions mentioned herein.

In some embodiments, the antibody of the present invention and/or ISAC molecules or the pharmaceutical composition, the pharmaceutical combination, or the kit delays the onset of the condition and/or symptoms associated with the condition.

In some embodiments, the antibody of the present invention and/or ISAC molecules or the pharmaceutical composition may also be administered in combination with one or more additional therapies, e.g., therapeutic modalities and/or additional therapeutic agents, for the use described herein, e.g., for use in preventing and/or treating the related diseases or conditions mentioned herein.

In some embodiments, the therapeutic modality includes surgery, radiotherapy, partial irradiation, focused irradiation, or the like.

In some embodiments, the therapeutic agent is selected from a chemotherapeutic agent, an angiogenesis inhibitor, a cytokine, a cytotoxic agent, an additional antibody, a small molecule drug, or an immunomodulatory agent (e.g., an immune checkpoint inhibitor or an agonist).

Exemplary other antibodies include antibodies that specifically bind to immune checkpoints.

The combination therapies of the invention encompass combined administration (e.g., two or more therapeutic agents are contained in the same formulation or separate formulations), and separate administration, in which the invention can occur prior to, concurrently with, and/or after the administration of other therapeutic agents and/or pharmaceuticals. Antibodies and/orAdministration of the ISAC Molecule.

The route of administration of the pharmaceutical composition is in accordance with known methods, for example, oral, by intravenous injection, intraperitoneal, intracerebral (intraparenchymal), intraventricular, intramuscular, intraocular, intraarterial, intraportal or intralesional routes; by sustained release systems or by implanted devices. In certain embodiments, the composition can be administered by bolus injection or by continuous infusion, or by an implanted device.

The compositions may also be administered topically via an implanted membrane, sponge, or another suitable material that absorbs or encapsulates the desired molecule.In certain embodiments, when an implanted device is used, the device can be implanted into any suitable tissue or organ, and the desired molecule can be delivered via diffusion, a timed release bolus, or continuous administration.

These and other aspects and embodiments of the invention are described in the accompanying drawings and the following detailed description, and are exemplified in the following examples.Any or all of the features described above and throughout the present application may be combined in various embodiments of the present invention.The following examples further illustrate the present invention. However, it should be understood that the examples are described by way of illustration rather than limitation, and various modifications may be made by those skilled in the art.

### Example.

### Example 1.1. Preparation of Anti-TROP2 Antibodies by Hybridomas

### 1.1.1 Immunization

Human TROP2 extracellular domain protein (Acro, Cat#TR2-H5223), and TiterMax (Sigma, Cat# T2684). Balb/c mice (purchased from Beijing Vital River Laboratory Animal Technology Co., Ltd.) were immunized by subcutaneous injection once every two weeks (50 µg of protein per mouse) for a total of four times.

### 1.1.2 Cell fusion and high-throughput screening

When the serum titer met the requirement, the spleen of the mouse was collected to prepare a B lymphocyte suspension, which was electrofused with SP2/0 myeloma cells (ATCC).The fused cells were diluted to 1-2×10⁴ cells/mL, and then were plated in a 96-well plate, and 100 µL of cell suspension was added to each well. On day 7 after the fusion, the screening medium (1640 medium containing 10% FBS and 1× HT) was replaced. On day 10 (or later, depending on the cell growth state) of the culture, the supernatant was collected for detection.

Hybridoma cells specifically expressing the anti-TROP2 antibody were screened out by a flow cytometer (FACS). The cells to be detected (huTROP2/GS-CHO & cynoTROP2/GS-CHO, constructed in-house) were counted and diluted to 1 × 10⁶ cells/mL, and added into a U-bottom 96-well plate at 100 µL/well. The mixture was centrifuged at 500 g for 5 min, and the cell medium was removed. Then the hybridoma culture supernatant in the 96-well plate was added to the U-shaped plate at 100 µL/well, and the cells were resuspended and left to stand on ice for 30 mins. The mixture was centrifuged at 500 g for 5 min, and the supernatant was removed. The cells were washed once with PBS. The mixture was centrifuged at 500 g for 5 min, and PBS was removed. 100 µL of anti-mouse Fab (Jackson Immunoresear, Cat#: 115-545-006, FITC-labeled secondary antibody, 1:500 dilution in PBS) was added to each well, and 100 µL of PE-labeled secondary antibody against anti-human Fc (Biolegend, Cat#: 409304) was added into the group of positive control antibody. The cells were incubated in the dark on ice for 30 min. The mixture was centrifuged at 500 g for 5 min, and the supernatant was removed. The cells were washed once with PBS. The cells were resuspended in 50 µL of 1× PBS, and the suspension was assayed by FACS. Clones positive for cell binding were subjected to an affinity assay. The final clones were subcloned by limiting dilution, and monoclonal cells were picked.

### 1.1.3 Subcloning of positive hybridoma cells

The subcloning steps by the limiting dilution method were as follows: a medium was added to a 96-well plate at 200 µL/well: compared with the screening medium, this medium had the same ingredients except that HAT was replaced with HT (Gibco, Cat #11067-030). Cells in the positive wells selected by fusing were prepared into a cell suspension, the cell suspension was then added to the first row at 100 µL/well, then the cell suspension of the first row was added to the second row at 100 µL/well after being well mixed, and then the cell suspension of the second row was added to the next row at 100 µL/well after being well mixed: the above steps were repeated: the 96-well plate was left to stand for 30 min, and the cells were observed and counted under a microscope. A corresponding volume containing about 100 cells was added to 20 mL of medium for well mixing and plating at 200 µL/well. One week later, the cells were observed under a microscope, and monoclonal wells were identified and marked..

When the cell confluence in each well reached 50% or more, upon a detection via the high-throughput screening method described above, cells in target positive wells were picked out and subjected to cell cryopreservation after expansion culture, and meanwhile, the clone supernatants were assayed for affinity,. The affinity assay (ForteBio) was performed according to the existing protocol (Estep, P et al., High throughput solution Based measurement of antibody-antigen affinity and epitope binning. MAbs, 2013.5(2): 270-8). Briefly, an AHQ (Pall, 1506091) sensor was equilibrated offline in an assay buffer for 30 min, and was equilibrated online for 60 seconds to establish a baseline. The purified antibodies obtained as described above were loaded online onto the AHQ sensor (ForteBio) for the ForteBio affinity assay. The sensor with the loaded antibodies was then exposed to the antigen TROP2 before transferring the sensor to the assay buffer for dissociation rate measurement. The K_{D} value is analyzed using ForteBio analysis software. The affinity assay results are the same as those shown in Table 1:

**Table 1. Binding Kinetic Constants of candidate TROP2 Hybridoma clones**

| **Clone ID** | **FACS** | | **Human TROP2** | **Cyno TROP2** | **Mouse TROP2** |
|---|---|---|---|---|---|
| | **GS-huTROP2** | **GS-cynoTROP2** | **K_{D} (M)** | **K_{D} (M)** | **K_{D} (M)** |
| 1D9B10 | 1282.33 | 1356.21 | 1.16E-09 | 3.51E-09 | N.B |
| Sacituzumab | N.D | N.D | 3.57E-09 | 6.45E-09 | N.B |

### Example.1.2. Preparation of chimeric antibodies

Antibody light and heavy chain gene sequences of the hybridoma candidate clones obtained in Example 1 were extracted and used to construct a human-mouse chimeric antibody. The heavy and light chain sequences of the chimeric antibody are set forth in SEQ ID NOs: 15 and 16, respectively.

About 5 × 10⁶ RNA freshly-cultured cells were taken, and RNA (Macherey-Nagel, Cat #740984.250) was extracted. Reverse transcription was performed by using PrimeScript II 1st Strand cDNA Synthesis Kit (Takara) to obtain cDNA. Gene fragments of antibody light and heavy chain variable regions were amplified by designing an upstream primer using a base sequence located in FR1 region at the 5' end and designing a downstream primer using a base located in the antibody constant region or FR4 region. The gene fragments were ligated into a T vector (Mighty TA-cloning Kit, Takara), monoclones were picked out for sequencing, and the sequencing results were analyzed and compared using MEGA7 software.

By alignment, the clones with correct and paired antibody light and heavy chain variable region sequences were selected, and the gene fragments of the light and heavy chain variable regions thereof were respectively ligated to a pcDNA3.1 vector by homologous recombinases from Nanjing Vazyme Biotech Co., Ltd. (Exnase^{®} II, Cat #C112-01), wherein an IgG1 subtype was selected as the constant region, and expression plasmids of light chain and heavy chain antibodies were obtained.

The light chain plasmid and heavy chain plasmid of the same antibody were then mixed in a molar ratio of 1:1, and transfected into 293F cells by polyethyleneimine (PEI) (Polysciences, Cat #23966). After 5-7 days of culture, the cell culture supernatant was collected and purified by a Protein A affinity column when the cell viability was below 60% to obtain a monoclonal antibody.

### Example 1.3. In Vitro Screening of Chimeric Antibodies

### 1.3.1 Affinity assay

The equilibrium dissociation constants (K_{D}) for binding of the antibodies of the present invention to human, cynomolgus and murine TROP2 was determined by bio-layer interferometry (ForteBio). The ForteBio affinity assay was performed according to existing protocol (Estep, P, et al., High throughput solution Based measurement of antibody-antigen affinity and epitope binning. MAbs, 2013.5(2): 270-8).

Briefly, an AHQ (Pall, 1506091) sensor was equilibrated offline in an assay buffer for 30 min, and was equilibrated online for 60 s to establish a baseline. The purified antibodies obtained as described above were loaded online onto the AHQ sensor (ForteBio) for the ForteBio affinity assay. The sensor with the loaded antibodies was then exposed to the antigen TROP2 before transferring the sensor to the assay buffer for dissociation rate measurement. The K_{D} value is analyzed using ForteBio analysis software. The sources of antigens were: human (Acro, Cat# TR2-H5223), monkey (Sino, Cat# 50922-M08H), and mouse (Sino, Cat# 90893-C08H).

The results of the antibody affinity assay are shown in Table 2:

**Table 2. Binding kinetic constants of TROP2 chimeric antibodies**

| **Clone ID** | **Human TROP2** | | | **Cyno TROP2** | | | **Mouse TROP2** | | |
|---|---|---|---|---|---|---|---|---|---|
| | **ka (1/Ms)** | **kd (1/s)** | **KD (M)** | **ka (1/Ms)** | **kd (1/s)** | **KD (M)** | **ka (1/Ms)** | **kd (1/s)** | **KD (M)** |
| ch1D9B10 | 3.64E +05 | 2.00E-04 | 5.50E-10 | 2.34E +05 | 2.00E-04 | 8.54E-10 | N.B | | |
| hTINA1 | 1.58E +05 | 1.33E-03 | 8.40E-09 | 8.80E +04 | 1.24E-03 | 1.41E-08 | N.D | | |
| hRS7 | 1.88E +05 | 2.85E-04 | 1.51E-09 | 1.15E +05 | 2.99E-04 | 2.59E-09 | N.D | | |

### 1.3.2Antibody-dependent cellular phagocytosis (ADCP) functional assay

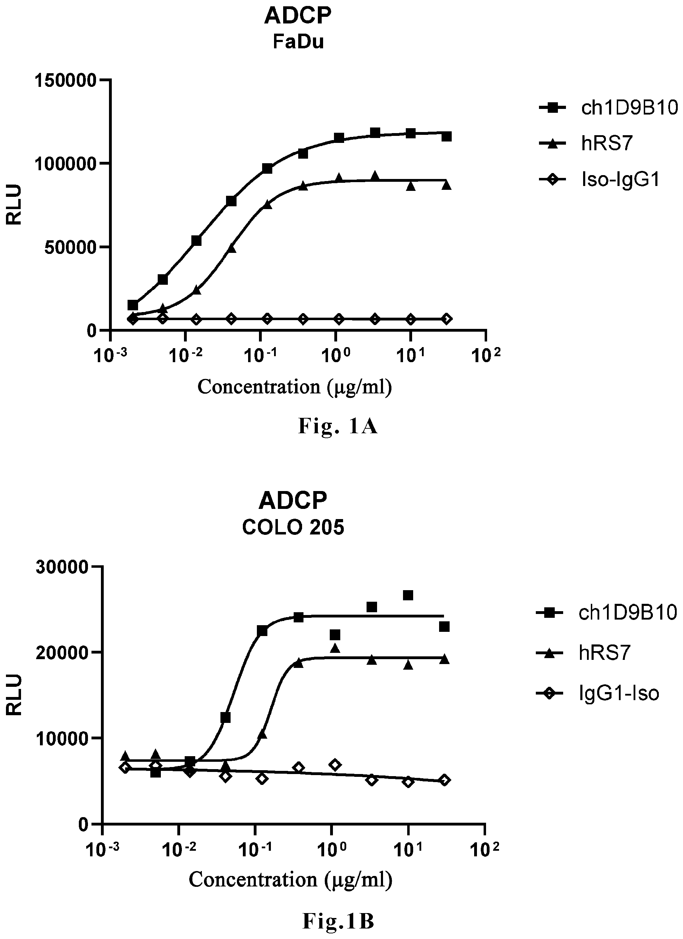

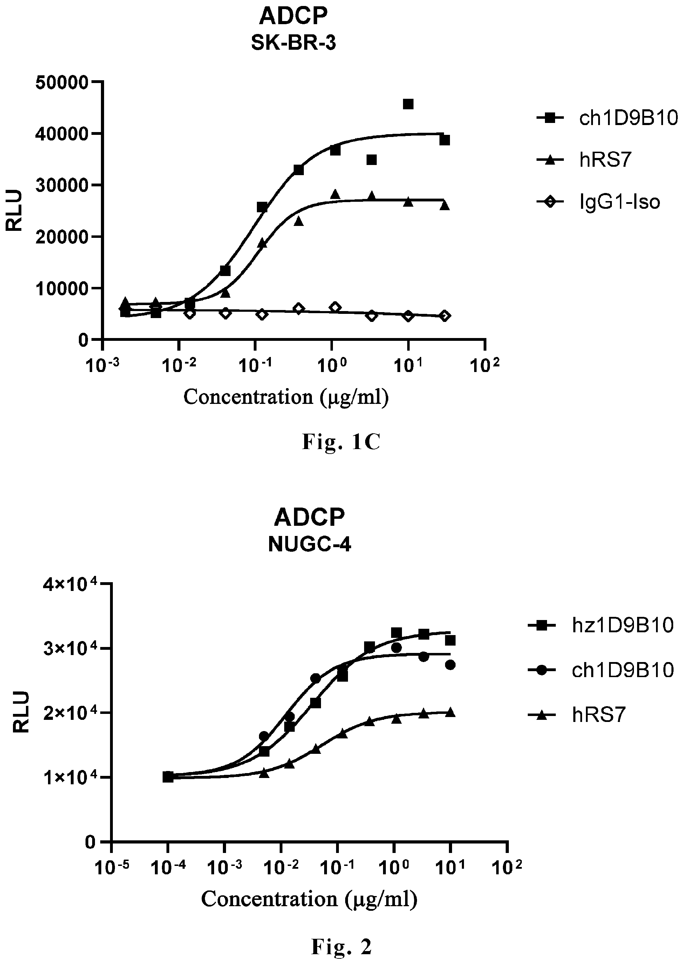

In the present invention, the ADCP (Antibody-dependent cell-mediated phagocytosis) activity of the TROP2 chimeric antibody is detected using FcγRIIa-H reporter cells (G9871, Promega). The cells are engineered Jurkat T cells. When FcγRIIa on the cell surface is bound and activated, luciferase is released into the experimental system through the downstream signal NFAT (Nuclear Factor of Activated T), so that the degree of cell activation can be detected.
The specific method is as follows: a 96-well white flat-bottom cell culture plate is used in which target cells FaDu (HTB-43, ATCC) or COLO 205 (TCHu102, Cell Bank of Chinese Academy of Sciences) or SK-BR-3 (HTB-30, ATCC) were added at 6×10⁴ cells/well, as well as effector cells FcγRIIa-H Cells being added at 3x10⁴ cells/well. Then, the corresponding concentration of TROP2 Monoclonal antibodies were added and the plate is incubated in an incubator at 37 °C for 6 h. The antibody moiety of TROP2 ADC Trodelvy, namely hRS7, was used as a control antibody (see WO2003/074566 for the sequence and source). The culture plate was then taken out, and Bio-Glo (G7940, Promega) was added for wavelength detection using a microplate reader (Spectra, Molecular Devices). The results are shown in FIGs. 1A (FaDu), 1B (COLO 205), and 1C(SK-BR-3), illustrating that clone ch1D9B10 had superior ADCP activity to hRS7 in both the TROP2 high-expression cell line FaDu and the TROP2 moderate-expression cell lines COLO 205 and SK-BR-3, and was suitable for the use in ISAC (Immune-Stimulating Antibody Conjugate).

### Preparation of hRS7 antibody:

Plasmid DNA for the expression of the antibody is obtained according to the publication WO2003/074566 .

Expi293F cells (purchased from Gibco) were cultured in Expi293F medium (Gibco, REF# A14351-01). The cell density (the viability should be greater than 95%) was measured the day before transfection, and the cell density was adjusted to 3 × 10⁶ with fresh Expi293F medium to continue the culture, and the cell density was adjusted to 3×10⁶ cells/ml on the day of transfection.

Opti-MEM medium (Gibco, REF# 31985-070) was used at a volume of 1/10 of the final transfection volume, as a transfection buffer, and the DNA to be transfected was added at a ratio of 1 mg/L, wherein the plasmid ratio of light and heavy chains was 1:1. The mixture was mixed well, and PEIMax^{®} (Polysciences Inc. Cat# 24765-1) was added at a mass ratio of DNA:PEI=1:3, and then the mixture was mixed well and incubated at room temperature for 20 min. Then the mixture was gently poured into the Expi293F cell suspension with shaking. The cells were cultured in a shaker under the conditions of 8% CO₂, 36.5 °C and 120 rpm.

After culturing for 16-18 h, the cell suspension was supplemented with 2% (v/v) Feed at a concentration of 200 g/L (100 g/L Phytone Peptone + 100 g/L Difco Select Phytone), a glucose solution at a final concentration of 5 g/L, and Valproic acid sodium salt (Merk, Cat# P4543-100G) at a final concentration of 2.2 mM, mixed gently, cultured for another 7 days at 8% CO₂, 36.5 °C, and 120 rpm, and then collected. The cell feed liquid was mixed well with diatomaceous earth (Sartorius, Cat. No. 1000037025) (1L of the cell feed liquid was mixed with 40 g of diatomaceous earth), and the mixture was filtered using a 0.22 µm disposable vacuum filtration apparatus.

Purification of target protein by affinity chromatography: A HiTrap MabSelect PrismA (GE Healthcare, Cat# 17549853) affinity chromatography column was selected for affinity capture. Before purification, 10-20 column volumes of 0.1 M NaOH were passed through the pipeline and the affinity chromatography column, and then the pipeline and the column were washed with 10-20 column volumes of distilled water. The packing column was equilibrated with 5 column volumes of 1× PBS (Gibco). The filtered cell feed liquid was passed through the column, and then the packing column was washed with 10 column volumes of 1× PBS to remove nonspecific binding proteins. The packing was washed with 5 column volumes of an elution buffer (100 mM sodium citrate, pH 3.5). The eluent was collected, the pH was adjusted to 6.0 with 2 M Tris, and the mixture was filtered to remove bacteria and used after the purity was determined to be acceptable.

**Table 3. VH and VL sequences of the final clone of murine antibodies**

| **Clone No.** | **Sequence name** | **Sequence No.** |
|---|---|---|
| | VH-DNA | SEQ ID NO: 11 |
| 1D9B10 | VH-amino acid | SEQ ID NO: 12 |
| | VL-DNA | SEQ ID NO: 13 |
| | VL-amino acid | SEQ ID NO: 14 |

### Example.1.4. Humanization of chimeric antibodies

The chimeric antibodies obtained from the hybridomas were humanized according to a conventional method, and the specific steps were as follows:
1) determining the structure of CDR loops;
②searching a human germline sequence database for the closest homologous sequences for each V/J region of the heavy and light chains;
③screening human germlines for the highest match in heavy and light chains with a minimum amount of back mutations;
④constructing the CDR regions of the chimeric antibody onto framework regions of a human antibody;
⑤determining amino acid positions that maintain the CDR functions in the framework regions based on the sequences and structural features;
⑥adding back mutations (back to the input amino acids) at important sequence positions identified;
⑦optimizing amino acids at risk sites.

Thus, a humanized antibody hz1D9B10 was obtained. The CDR sequences, light and heavy chain variable regions, and light and heavy chain amino acid sequences are shown in Table 4 below.

**Table 4. Final Sequences of the Humanized Clone**

| **Region** | **Sequences** |
|---|---|
| HCDR1 | SEQ ID NO 1 |
| HCDR2 | SEQ ID NO 2 |
| HCDR3 | SEQ ID NO 3 |
| LCDR1 | SEQ ID NO 4 |
| LCDR2 | SEQ ID NO 5 |
| LCDR3 | SEQ ID NO 6 |
| hz1D9B10-VH | SEQ ID NO 7 |
| hz1D9B10-VL | SEQ ID NO 8 |
| Heavy Chain | SEQ ID NO 9 |
| Light Chain | SEQ ID NO 10 |

### Example.1.5. Affinity of humanized antibodies for the antigen as determined by ForteBio

The equilibrium dissociation constant (K_{D}) for binding of the antibodies of the present invention to human TROP2 was determined by bio-layer interferometry (ForteBio). The ForteBio affinity assay followed the existing method (Estep, P, et al., High throughput solution Based measurement of antibody-antigen affinity and epitope binning. MAbs, 2013.5(2): p. 270-8).

Briefly, an AHC (Sartorius, 18-5060) sensor was equilibrated offline in an assay buffer for 30 min, and was equilibrated online for 60 s to establish a baseline. The purified antibody obtained as described above was loaded online onto the AHC sensor (ForteBio) for the ForteBio affinity assay. The sensor with the loaded antibodies was then exposed to the antigen human TROP2 before transferring the sensor to the assay buffer for dissociation rate measurement. The K_{D} values were analyzed using ForteBio analysis software.

The detection results of the antibody affinity are shown in Table 5. The humanized antibodies still had high affinity for the antigen TROP2, and had equilibrium dissociation constants K_{D} comparable to those of the corresponding chimeric antibodies against the antigen.

**Table 5. Affinity constant (M) for binding of antigen to antibody by ForteBio assay**

| **Antibody** | **Human TROP2** |
|---|---|
| ch1D9B10 | 4.81E-10 |
| hz1D9B10 | 8.78E-10 |

### Example.1.6. Antibody-dependent cellular phagocytosis (ADCP) functional assay of humanized antibodies

The ADCP activity of the anti-TROP2 humanized antibody was determined using FcγRIIa-H reporter cells, and the target cell was the NUGC-4 cell line (JCRB0834, JCRB CELL TANK) with medium and low TROP2 expression. The determination method was the same as that in Example 1.3.2. The results, as shown in FIG. 2, indicated that the ADCP activity of hz1D9B10 was comparable to that of the corresponding chimeric antibodies, and both were superior to that of the control antibody hRS7, indicating that the humanization process did not affect the ADCP function of the antibodies.

### Example.1.7. Macrophage endocytosis function verification of humanized antibodies

The ADCP activity of the humanized anti-TROP2 antibody clones was verified using induced differentiated human M1 macrophage . Macrophages and target cells were fluorescently labeled, respectively, and when ADCP was generated between the two upon the aid of the antibody, a double-positive cell population could be observed. Induced differentiation of M1 macrophage-: Monocytes were isolated from fresh human peripheral blood mononuclear cells (PBMCs, TPCS) using a human monocyte enrichment kit without CD16 depletion (19058, STEMCELL) and resuspended in AIM V ^{®} Medium CTS (A3021002, Gibco) medium at a density of 1×10⁶ Cells/ml and placed in a cell culture flask and subjected to adherent culture at 37 °C for 4 h. Subsequently the medium was changed to AIM V^{®} Medium CTS (A3021002, Gibco) + 10% inactivated fetal bovine serum (FBS, SH30406.05, Hyclone) +25ng/ml human MCSF1(216-MC-500,R&D) for the culture. In the following seven days, AIM V ^{®} Medium CTS (A3021002, Gibco) + 10% inactivated fetal bovine serum (FBS, SH30406.05, Hyclone) was used for half medium exchange once every third day. After 7 days, media AIM V ^{®} Medium CTS (A3021002, Gibco) + 10% inactivated fetal bovine serum (FBS, SH30406.05, Hyclone) + 100 ng/mL IFNg (285-IF-100/CF, R&D) was used for half medium exchange, and the macrophage endocytosis experiment was performed the next day.

Macrophage endocytosis assay: The induced macrophages were digested with accutase (A6964-500ML, Sigma), and the cell density was adjusted using an RPMI-1640 complete medium (22400-071, Gibco) containing 10% fetal bovine serum (FBS, SH30406.05, Hyclone). The cells were added to a 96-well low-adsorption plate at 1×10⁵ cells per well. Target cells FaDu (HTB-43, ATCC) were labeled with CFSE (C34554, INVITROGEN) at 37 °C for 10 min, washed twice with RPMI-1640 complete medium (22400-071, Gibco) containing 10% fetal bovine serum (FBS, SH30406.05, Hyclone), and added to a 96-well low-adsorption plate at 2.5 × 10⁴ cells per well. The target cells and macrophages were mixed with gradiently diluted anti-TROP2 humanized antibodies, and were pipetted and mixed well, and cultured in a 37 °C incubator for 4 h.

Flow staining: Macrophages were labeled with CD14 PE/Cy7 (557742, BD) and then detected by flow cytometry, and cells in the PE/Cy7 and CFSE double-positive region were identified as the target cell population.

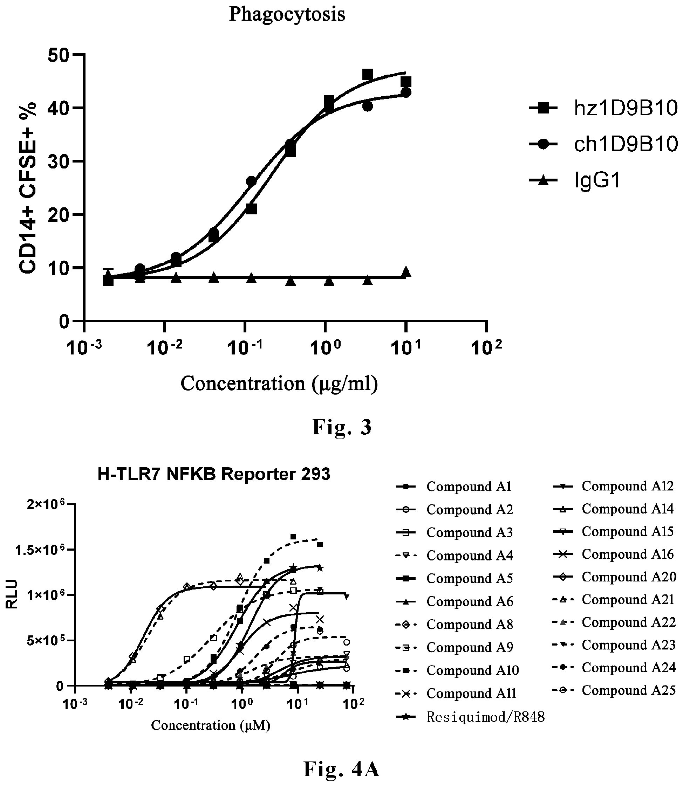

The results, as shown in FIG. 3, indicated that the hz1D9B10 antibody could induce the ADCP effect in M1 macrophage in the presence of human pharyngeal squamous cell carcinoma cell FaDu, with the activity comparable to that of the corresponding chimeric antibody.

### Example 2. Synthesis of ISAC Molecules

### Example 2.1 Synthesis of Compound 1, Compound 2 and Compound 3

### Synthetic route

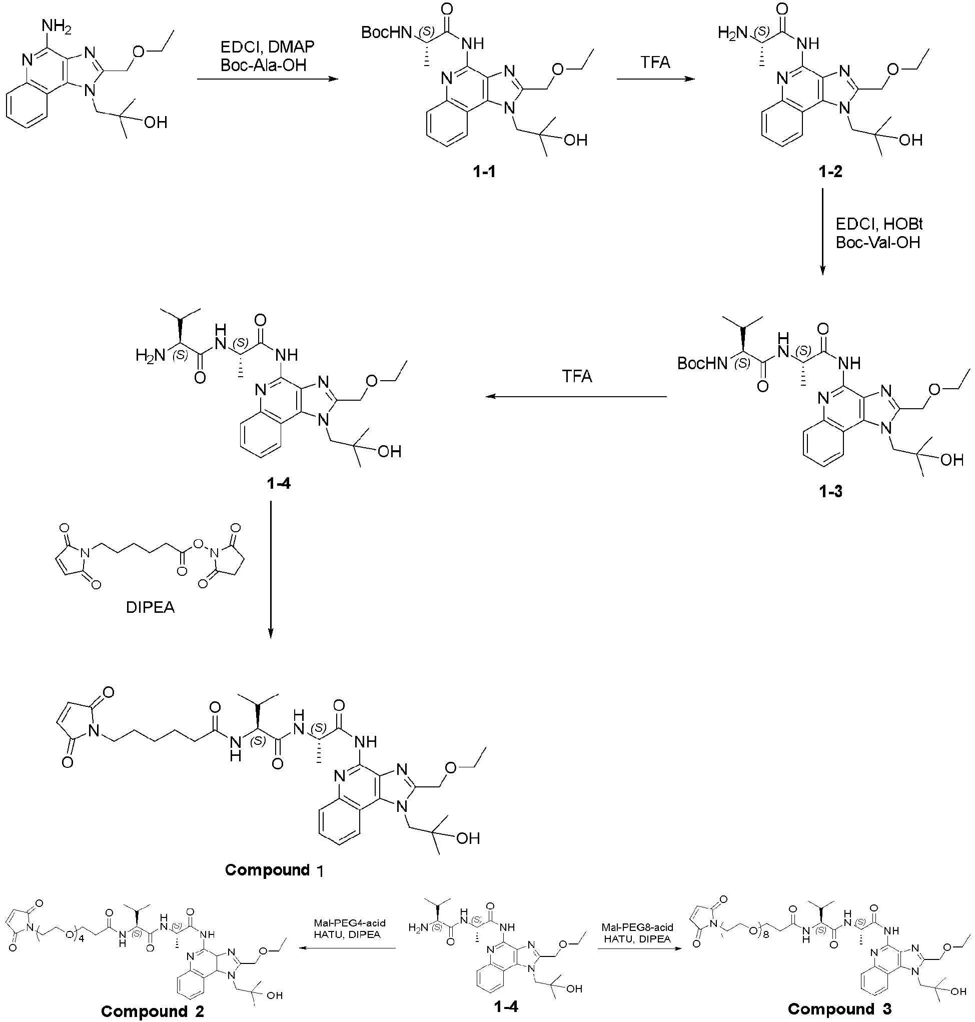

### tert-Butyl (S)-(1-((2-(Ethoxymethyl)-1-(2-hydroxy-2-methylpropyl)-1H-imidazo[4,5-c]quinolin-4-yl)amino)-1-oxopropan-2-yl)carbamate (1-1)

Boc-Ala-OH (0.9 g, 4.77 mmol), DMAP (0.7 g, 5.73 mmol), and EDCI (1.1 g, 5.73 mmol) were added to a solution of resiquimod (1.0 g, 3.18 mmol) in DMF (12 mL). The reaction mixture was stirred at room temperature overnight. The mixture was diluted with EtOAc (50 mL), washed successively with saturated NH₄Cl aqueous solution and brine, dried over anhydrous Na₂SO₄ and concentrated under reduced pressure. The residue was purified by silica gel column chromatography to give the Compound **1-1** as a light yellow oil (0.9 g, 58% yield).

LC-MS (ESI): *m*/*z* 486.3 [M+H]⁺

### (S)-2-amino-N-(2-(ethoxymethyl)-1-(2-hydroxy-2-methylpropyl)-1H-imidazo[4,5-c]quinolin-4-yl)propionamide (1-2)

To a solution of Compound **1-1** (70 mg, 0.144 mmol) in DCM (5 mL) was added TFA (5 mL) at 0 °C. The reaction mixture was stirred for 1 h, then concentrated under reduced pressure to afford Compound **1-2** (70 mg), which was used in the next step without further purification.

LC-MS (ESI):*m*/*z* 386.2 [M+H]⁺

### tert-Butyl ((S)-1-(((S)-1-((2-(ethoxymethyl)-1-(2-hydroxy-2-methylpropyl)-1H-imidazo[4,5-c]quinolin-4-yl)amino)-1-oxopropan-2-yl)amino)-3-methyl-1-oxobutan-2-yl)carbamate (1-3)

To a solution of Compound **1-2** (70 mg, 0.144 mmol) in DCM (10 mL) were added Boc-Val-OH (32.8 mg, 0.152 mmol), HOBt (22 mg, 0.16 mmol), DIPEA (46.4 mg, 0.36 mmol), and EDCI (30.4 mg, 0.16 mmol). The reaction mixture was stirred at room temperature overnight. The mixture was diluted with EtOAc (30 mL), washed successively with saturated NH₄Cl aqueous solution and brine, dried over anhydrous Na₂SO₄ and concentrated under reduced pressure. The residue was purified by silica gel column chromatography to give the Compound **1-3** as a light yellow oil (50 mg, 38% yield over two steps).

LC-MS (ESI):*m*/*z* 585.6 [M+H]⁺.

### (S)-2-Amino-N-((S)-1-((2-(ethoxymethyl)-1-(2-hydroxy-2-methylpropyl)-1H-imidazo[4,5-c]quinolin-4-yl)amino)-1-oxopropan-2-yl)-3-methylbutanamide (1-4)

To a solution of Compound **1-3** (200 mg, 0.34 mmol) in DCM (10 mL) was added TFA (10 mL) at 0 °C. The reaction mixture was stirred for 1 h, then concentrated under reduced pressure to afford Compound **1-4** (200 mg), which was used in the next step without further purification.

LC-MS (ESI):*m*/*z* 485.3 [M+H]⁺.

### 6-(2,5-Dioxo-2,5-dihydro-1H-pyrrol-1-yl)-N-((S)-1-(((S)-1-((2-(ethoxymethyl)-1-(2-hydroxy-2-methylpropyl)-1H-imidazo[4,5-c]quinolin-4-yl)amino)-1-oxopropan-2-yl)amino)-3-methyl-1-oxobutan-2-yl)hexanamide (Compound 1)

To a solution of compound **1-4** (200 mg, 0.34 mmol) in DMF (5 mL) were added N-succinimidyl 6-maleimidohexanoate (105 mg, 0.34 mmol) and DIPEA (132 mg, 1.02 mmol). The reaction mixture was stirred at room temperature overnight. The mixture was diluted with EtOAc (20 mL), washed successively with saturated NH₄Cl aqueous solution and brine, dried over anhydrous Na₂SO₄ and concentrated under reduced pressure. The residue was purified by prep-HPLC to afford **Compound 1** as a white solid (65 mg, 28% yield over two steps).

LC-MS (ESI):*m*/*z* 678.3 [M+H]⁺.

### 1-(2,5-Dioxo-2,5-dihydro-1H-pyrrol-1-yl)-N-((S)-1-(((S)-1-((2-(ethoxymethyl)-1-(2-hydroxy-2-methylpropyl)-1H-imidazo[4,5-c]quinolin-4-yl)amino)-1-oxopropan-2-yl)amino)-3-methyl-1-oxobutan-2-yl)-3,6,9,12-tetraoxapentadecan-15-amide (Compound 2)

To a solution of Compound **1-4** (82 mg, 0.17 mmol) and Mal-PEG4-oic acid (59 mg) in DCM (5 mL) were added HATU (65 mg, 0.17 mmol) and DIPEA (55 mg, 0.43 mmol). The reaction mixture was stirred at room temperature overnight. The mixture was washed successively with saturated aqueous NaHCO₃ (5 mL), saturated aqueous NH₄Cl and brine, dried over anhydrous Na₂SO₄ and concentrated under reduced pressure. The residue was purified by prep-HPLC to afford **Compound 2** as a white solid (37 mg, 27% yield).

LC-MS (ESI):*m*/*z* 812.2 [M+H]⁺.

### 1-(2,5-Dioxo-2,5-dihydro-1H-pyrrol-1-yl)-N-((S)-1-(((S)-1-((2-(ethoxymethyl)-1-(2-hydroxy-2-methylpropyl)-1H-imidazo[4,5-c]quinolin-4-yl)amino)-1-oxopropan-2-yl)amino)-3-methyl-1-oxobutan-2-yl)-3,6,9,12,15,18,21,24-octaoxaheptacosan-27-amide (Compound 3)

To a solution of Compound 1-4 (107 mg, 0.22 mmol) and Mal-PEG8-acid (116 mg) in DCM (8 mL) were added HATU (85 mg, 0.22 mmol) and DIPEA (72 mg, 0.56 mmol). The reaction mixture was stirred at room temperature overnight. The mixture was washed successively with saturated aqueous NaHCO₃ (8 mL), saturated aqueous NH₄Cl and brine, dried over anhydrous Na₂SO₄ and concentrated under reduced pressure. The residue was purified by prep-HPLC to afford **Compound** 3 as a white solid (11 mg, 5% yield).

LC-MS (ESI): m/z 989.0 [M+H]⁺.

### Example 2.2 Synthesis of Compound 4

### Synthetic route

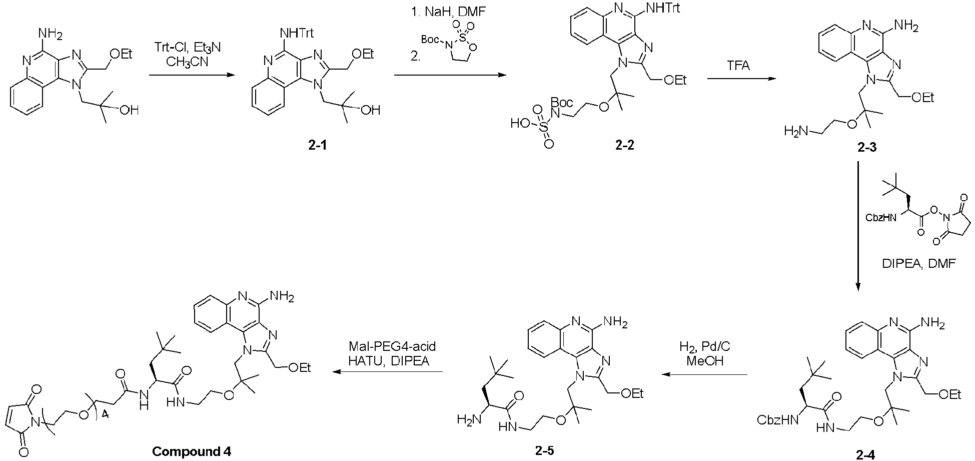

### 1-(2-(Ethoxymethyl)-4-(tritylamino)-1H-imidazo[4,5-c]quinolin-1-yl)-2-methylpropan-2-ol (2-1)

To a solution of resiquimod (0.1 g, 0.3 mmol) in CH₃CN (5 mL) were added Et₃N (76 mg, 0.75 mmol) and trityl chloride (100 mg, 0.36 mmol) under an N₂ atmosphere. The reaction mixture was irradiated at 100 °C for 30 min in a microwave reactor. The mixture was cooled to 0 °C. The precipitate was collected by filtration and washed with cold CH₃CN to afford the Compound **2-1** as a white solid (120 mg, 68% yield).

LC-MS (ESI): m/z 557.5 [M+H]⁺.

### N-(tert-Butoxycarbonyl)-(2-((1-(2-(ethoxymethyl)-4-(tritylamino)-1H-imidazo[4,5-c]quinolin-1-yl)-2-methylpropan-2-yl)oxy)ethyl)sulfamic acid (2-2)

Under a nitrogen atmosphere, a solution of Compound **2-1** (200 mg, 0.36 mmol) in DMF (2 mL) was added dropwise to a suspension of NaH (60% dispersion in oil, 28 mg, 0.72 mmol) in DMF (2 mL). The mixture was stirred at 0 °C for 1 h and at room temperature for 30 min. To this solution was added 1,1-dimethylethyl 2,2-dioxide-1,2,3-oxathiazolidine-3-carboxylate (160 mg, 0.72 mmol) at 0 °C. The mixture was allowed to warm to room temperature and stirred overnight. The mixture was poured into ice water and extracted with EtOAc. The organic layers were combined, washed with water and brine, dried over anhydrous Na₂SO₄ and concentrated under reduced pressure. The residue was purified by silica gel column chromatography (DCM/MeOH=150:1) to afford the Compound **2-2** as a white foam (150 mg, 54% yield).

LC-MS (ESI):*m*/*z* 802.6 [M+Na]⁺.

### 1-(2-(2-Aminoethoxy)-2-methylpropyl)-2-(ethoxymethyl)-1H-imidazo[4,5-c]quinolin-4-amine (2-3)

To a solution of Compound **2-2** (150 mg, 0.19 mmol) in DCM (8 mL) was added Et₃SiH (100 mg), followed by TFA (8 mL). The reaction mixture was stirred at room temperature for 3 h. The mixture was concentrated under reduced pressure to give compound **2-3** (67 mg, 97% yield).

LC-MS (ESI):*m*/*z* 358.4 [M+H]⁺.

### Benzyl (S)-(1-((2-((1-(4-amino-2-(ethoxymethyl)-1H-imidazo[4,5-c]quinolin-1-yl)-2-methylpropan-2-yl)oxy)ethyl)amino)-4,4-dimethyl-1-oxopentan-2-yl)carbamate (2-4)

To a solution of Compound **2-3** (2.7 g, 7.55 mmol) in DMF (15 mL) were added DIPEA (3.9 g, 30.2 mmol) and 2,5-dioxopyrrolidin-1-yl (S)-2-(((benzyloxy)carbonyl)amino)-4,4-dimethylpentanoate (3.4 g, 9.06 mmol). The reaction mixture was stirred at room temperature overnight. The mixture was diluted with EtOAc, washed with 0.5 N HCl and brine, dried over anhydrous Na₂SO₄ and concentrated under reduced pressure. The residue was purified by prep-HPLC to afford the Compound **2-4** as a white foam (2.5 g, 54% yield).

LC-MS (ESI):*m*/*z* 619.5 [M+H]⁺.

### (S)-2-Amino-N-(2-((1-(4-amino-2-(ethoxymethyl)-1H-imidazo[4,5-c]quinolin-1-yl)-2-methylpropan-2-yl)oxy)ethyl)-4,4-dimethylpentanamide (2-5)

To a solution of Compound **2-4** (100 mg, 0.16 mmol) in MeOH (5 mL) was added 10% Pd/C (10 mg) under an N₂ atmosphere. The reaction mixture was stirred at room temperature for 5 h under an H₂ atmosphere. The reaction was filtered and the filtrate was concentrated under reduced pressure to afford the Compound **2-5** as a white solid (NeoR848, 78 mg, quantitative).

LC-MS (ESI):*m*/*z* 485.4 [M+H]⁺.

### (S)-N-(1-((2-((1-(4-Amino-2-(ethoxymethyl)-1H-imidazo[4,5-c]quinolin-1-yl)-2-methylpropan-2-yl)oxy)ethyl)amino)-4,4-dimethyl-1-oxopentan-2-yl)-1-(2-(2,5-dioxo-2,5-dihydro-1H-pyrrol-1-yl)-3,6,9,12-tetraoxapentadecan-15-amide

### (Compound 4)

To a solution of Compound **2-5** (78 mg, 0.16 mmol) and Mal-PEG4-oic acid (56 mg) in DCM (10 mL) were added HATU (61 mg, 0.16 mmol) and DIPEA (41 mg, 0.32 mmol). The reaction mixture was stirred at room temperature overnight. The mixture was washed successively with saturated aqueous NaHCO₃ (5 mL), saturated aqueous NH₄Cl and brine, dried over anhydrous Na₂SO₄ and concentrated under reduced pressure. The residue was purified by Prep-HPLC to afford **Compound 4** as a white foam (19 mg, 15% yield).

LC-MS (ESI): m/z 812.5 [M+H]⁺.

### Example 2.3 Synthesis of Compound 5

### Synthetic route

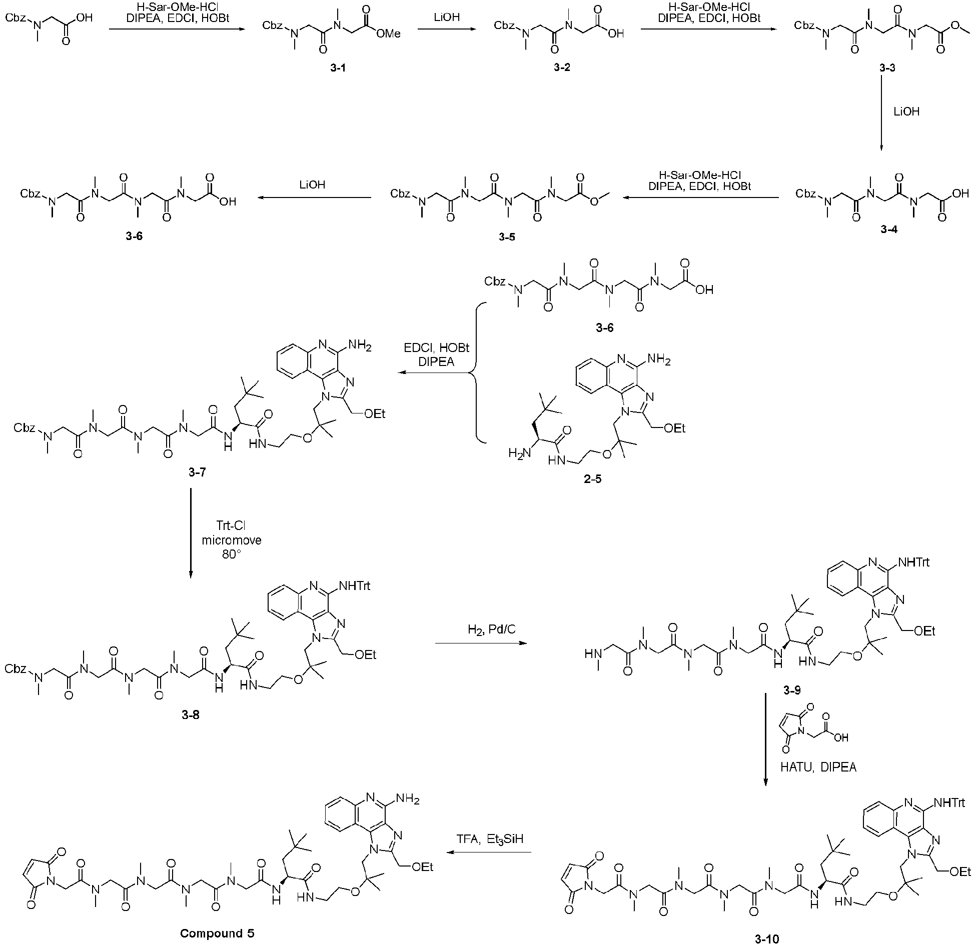

### Methyl N-(N-((benzyloxy)carbonyl)-N-methylglycyl)-N-methylglycinate (3-1)

To a solution of Cbz-Sar-OH (10 g, 44.8 mmol) in DCM (100 mL) were added H-Sar-OMe.HCl (6.9 g, 49.3 mmol), DIPEA (14.4 g, 112 mmol), EDCI (9.4 g, 49.3 mmol), and HOBt (6.7 g, 49.3 mmol). The reaction mixture was stirred at room temperature overnight. The mixture was washed successively with 1N HCl (3 × 100 mL), water (100 mL), saturated aqueous NaHCO₃ (3×100 mL) and brine (100 mL), dried over anhydrous Na₂SO₄ and concentrated under reduced pressure. The residue was purified by silica gel column chromatography (petroleum ether/EtOAc = 100:1 to 30:1) to give the Compound 3-1 as a light yellow oil (11.2 g, 81% yield).

LC-MS (ESI): m/z 309.2 [M+H]⁺.

### N-(N-((Benzyloxy)carbonyl)-N-methylglycyl)-N-methylglycine (3-2)

To a solution of Compound **3-1** (6.0 g, 4.2 mmol) in MeOH (120 mL) was added a solution of LiOH.H.₂O (5.28 g, 126 mmol) in H₂O (30 mL) at 0 °C. The reaction mixture was stirred at the same temperature for 2 h. The mixture was acidified with 3N HCl and extracted with EtOAc. The organic layer was washed with brine, dried over anhydrous Na₂SO₄ and concentrated under reduced pressure. The residue was purified by silica gel column chromatography (DCM/MeOH=150:1) to give the Compound **3-2** as a colorless oil (5.2 g, yield: 90%).

LC-MS (ESI): m/z 317.1 [M+Na] ⁺.

### Methyl 4,7,10-trimethyl-3,6,9-trioxo-1-phenyl-2-oxa-4,7,10-triazadodecan-12-oate (3-3)

To a soultion of Compound **3-2** (5.2 g, 17.67 mmol) in DCM (100 mL) were added H-Sar-OMe.HCl (2.47 g, 17.67 mmol), DIPEA (6.85 g, 53.0 mmol), EDCI (3.74 g, 19.47 mmol) and HOBt (2.63 g, 19.47 mmol). The reaction mixture was stirred at room temperature overnight. The mixture was washed successively with 1N HCl (3 × 100 mL), water (100 mL), saturated aqueous NaHCO₃ (3x100 mL) and brine (100 mL), dried over anhydrous Na₂SO₄ and concentrated under reduced pressure. The residue was purified by silica gel column chromatography (petroleum ether/EtOAc = 100:1 to 30:1) to give the Compound **3-3** as a light yellow oil (3.4 g, 51% yield).

LC-MS (ESI): m/z 402.3 [M+Na]⁺.

### 4,7,10-Trimethyl-3,6,9-trioxo-1-phenyl-2-oxa-4,7,10-triazadodecan-12-oic acid (3-4)

To a solution of Compound **3-3** (3.4 g, 8.98 mmol) in MeOH (15 mL) was added LiOH.H₂O (1.5 g, 35.9 mmol) in H₂O (15 mL) at 0 °C. The reaction mixture was stirred at the same temperature for 2 h. The mixture was acidified with 3N HCl and extracted with EtOAc. The organic layer was washed with brine, dried over anhydrous Na₂SO₄ and concentrated under reduced pressure. The residue was purified by silica gel column chromatography (DCM/MeOH = 100:1 to 50:1) to give the Compound **3-4** as a colorless oil (2.1 g, 64% yield).

LC-MS (ESI): m/z 388.5 [M+Na] ⁺.

### Methyl 4,7,10,13-tetramethyl-3,6,9,12-tetraoxo-1-phenyl-2-oxa-4,7,10,13-tetraazapentadecan-15-oate (3-5)

To a solutuion of Compound **3-4** (3.0 g, 8.2 mmol) in DCM (100 mL) were added H-Sar-OMe.HCl (1.2 g, 8.2 mmol), DIPEA (3.1 g, 24.0 mmol), EDCI (1.7 g, 9.0 mmol) and HOBt (1.2 g, 9.0 mmol). The reaction mixture was stirred at room temperature overnight. The mixture was washed successively with 1N HCl (3 × 100 mL), water (100 mL), saturated aqueous NaHCO₃ (3 × 100 mL) and brine (100 mL), dried over anhydrous Na₂SO₄ and concentrated under reduced pressure. The residue was purified by silica gel column chromatography (petroleum ether/EtOAc = 100:1 to 30:1) to give the Compound **3-5** as a light yellow oil (1.2 g, 32% yield).

LC-MS (ESI): m/z 451.3 [M+H]⁺.

### 4,7,10,13-Tetramethyl-3,6,9,12-tetraoxo-1-phenyl-2-oxa-4,7,10,13-tetraazapentadecan-15-oic acid (3-6)

To a solution of Compound **3-5** (12.3 g, 27.3 mmol) in MeOH (120 mL) was added LiOH.H.₂O (4.6 g, 109.2 mmol) in H₂O (30 mL) at 0 °C. The reaction mixture was stirred at the same temperature for 2 h. The mixture was acidified with 3N HCl and extracted with EtOAc. The organic layer was washed with brine, dried over anhydrous Na₂SO₄ and concentrated under reduced pressure. The residue was purified by silica gel column chromatography (DCM/MeOH = 100:1 to 50:1) to give the Compound **3-6** as a colorless oil (5.3 g, 45% yield).

LC-MS (ESI): m/z 459.2 [M+Na] ⁺.

### Benzyl (S-(20-(4-amino-2-(ethoxymethyl)-1H-imidazo[4,5-c]quinolin-1-yl)-3,6,9,12,15-pentamethyl-13-neopentyl-2,5,8,11,14-pentaoxo-18-oxa-3,6,9,12,15-pentaazaicosyl)(methyl)carbamate (3-7)

To a solution of Compound **3-6** (200 mg, 0.46 mmol) in DCM (10 mL) were added EDCI (114 mg, 0.60 mmol) and HOBt (80 mg, 0.60 mmol) at 0 °C. After stirring for 5 min, Compound **2-5** (Example 2.2, 266 mg, 0.56 mmol) and DIPEA (178 mg, 1.38 mmol) were added. The reaction mixture was stirred at room temperature overnight. The mixture was washed successively with 1N HCl (2 × 10 mL), water (10 mL), NaHCO₃ (2 × 10 mL) and brine (10 mL), dired over anhydrous Na₂SO₄ and concentrated under reduced pressure. The residue was purified by prep-HPLC to afford the Compound **3-7** as a light yellow solid (148 mg, 36% yield).

LC-MS (ESI): m/z 903.7 [M+H] ⁺.

### Benzyl (S-(20-(2-(ethoxymethyl)-4-(tritylamino)-1H-imidazo[4,5-c]quinolin-1-yl)-3,6,9,12,15-pentamethyl-13-neopentyl-2,5,8,11,14-pentaoxo-18-oxa-3,6,9,12,15-pentaazaicosyl)(methyl)carbamate (3-8)

Under an N₂ atmosphere, to a suspension of Compound **3-7** (180 mg, 0.18 mmol) in CH₃CN (5 mL) were added Et₃N (41 mg, 40.5 mmol) and trityl chloride (68 mg, 0.23 mmol). The reaction mixture was irradiated in a microwave reactor at 80 °C for 45 minutes. The mixture was concentrated under reduced pressure. The residue was purified by Prep-TLC (DCM/MeOH=20:1) to afford the Compound **3-8** as a yellow solid (100 mg, 44%).

LC-MS (ESI): m/z 1145.9 [M+H] ⁺.

### (S)-N-(2-((1-(2-(Ethoxymethyl)-4-(tritylamino)-1H-imidazo[4,5-c]quinolin-1-yl)-2-methylpropan-2-yl)oxy)ethyl)-4,4-dimethyl-2-(5,8,11-trimethyl-4,7,10-trioxo-2,5,8,11-tetraazatridecan-13-amido)pentanamide (3-9)

Under an N₂ atmosphere, to a solution of Compound **3-8** (80 mg) in MeOH (5 mL) was added 10% Pd/C (40 mg). The reaction mixture was stirred at room temperature for 4 h under an H₂ atmosphere. The reaction was filtered and the filtrate was concentrated under reduced pressure to afford the Compound **3-9** as a yellow solid (69 mg, 98% yield).

LC-MS (ESI): m/z 1011.6 [M+H] ⁺.

### (S)-2-(14-(2,5-Dioxo-2,5-dihydro-1H-pyrrol-1-yl)-3,6,9,12-tetramethyl-4,7,10,13-tetraoxo-3,6,9,12-tetraazatetradecanamido)-N-(2-((1-(2-(ethoxymethyl)-4-(tritylamino)-1H-imidazo[4,5-c]quinolin-1-yl)-2-methylpropan-2-yl)oxy)ethyl)-4,4-dimethylpentanamide (3-10)

To a solutuion of Compound **3-9** (69 mg, 0.069 mmol) and maleimidoacetic acid (13.8 mg, 0.069 mmol) in DCM (25 mL) were added HATU (33 mg, 0.083 mmol) and DIPEA (19 mg, 0.14 mmol). The reaction mixture was stirred at room temperature overnight. The mixture was washed successively with saturated aqueous NaHCO₃ (20 mL), saturated NH₄Cl aqueous solution (20 mL) and brine (20 mL), dried over anhydrous Na₂SO₄, and concentrated under reduced pressure to afford the Compound **3-10** as a yellow solid (85 mg).

LC-MS (ESI): m/z 1149.0 [M+H] ⁺.

### (S)-N-(2-((1-(4-Amino-2-(ethoxymethyl)-1H-imidazo[4,5-c]quinolin-1-yl)-2-methylpropan-2-yl)oxy)ethyl)-2-(14-(2,5-dioxo-2,5-dihydro-1H-pyrrol-1-yl)-3,6,9,12-tetramethyl-4,7,10,13-tetraoxo-3,6,9,12-tetraazatetradecanamido)-4,4-dimethylpentanamide (Compound 5)

To a solution of Compound 3-10 (85 mg, 0.074 mmol) in DCM (5 mL) were added Et₃SiH (18 mg, 0.148 mmol) and TFA (0.5 mL). The reaction mixture was stirred at room temperature for 4 h. The mixture was concentrated under reduced pressure. The residue was purified by Prep-HPLC to afford the **Compound** 5 as a white foam (11 mg, 18% yield over two steps).

LC-MS (ESI): m/z 906.8 [M+H] ⁺.

### Example 2.4 Synthesis of Compound 6

### Synthetic route

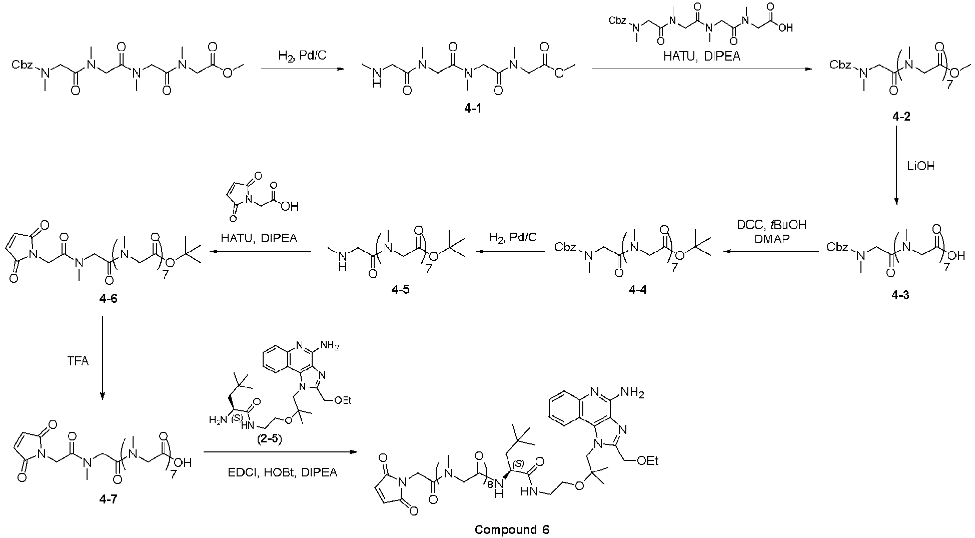

### Methyl N-methyl-N-(N-methyl-N-(N-methyl-N-(methylglycyl)glycyl)glycyl)glycinate (4-1)

To a solution of methyl 4,7,10,13-tetramethyl-3,6,9,12-tetraoxo-1-phenyl-2-oxa-4,7,10,13-tetraazapentadecan-15-oate (5 g, 11.1 mmol) in MeOH (50 mL) was added 10% Pd/C (0.5 mg) under an N₂ atmosphere. The reaction mixture was stirred at room temperature for 6 h under an H₂ atmosphere. The reaction was filtered, the filtrate was concentrated under reduced pressure to afford the Compound **4-1** as a light yellow oil (3.5 g, quantitative).

LC-MS (ESI): m/z 339.1 [M+Na]⁺.

### Methyl 4,7,10,13,16,19,22,25-octamethyl-3,6,9,12,15,18,21,24-octaoxo-1-phenyl-2-oxa-4,7,10,13,16,19,22,25-octaazaheptacosan-27-oate (4-2)

HATU (5.06 g, 13.3 mmol) was added to a solution of Cbz-Sar-Sar-Sar-Sar-OH (4.8 g, 11.1 mmol) in DCM (80 mL) at 10 °C. After stirring for 5 min, a solution of H-Sar-Sar-Sar-Sar-OMe (3.5 g, 11.1 mmol) and DIPEA (2.86 g, 22.2 mmol) in DCM (25 mL) was added. The reaction mixture was stirred at room temperature overnight. The mixture was washed successively with 1N HCl (100 mL), water (100 mL), saturated aqueous NaHCO₃ (100 mL) and brine (100 mL), dried over anhydrous Na₂SO₄ and concentrated under reduced pressure. The residue was purified by silica gel column chromatography (DCM/MeOH=100:1 to 50:1) to give the Compound **4-2** as a light yellow oil (2.2 g, 27% yield).

LC-MS (ESI): m/z 757.7 [M+Na]⁺.

### 4,7,10,13,16,19,22,25-Octamethyl-3,6,9,12,15,18,24-octaoxo-1-phenyl-2-oxa-4,7,10,13,16,19,22,25-octaazaheptacosan-27-oic acid (4-3)

To a solution of Compound **4-2** (2.2 g, 2.99 mmol) in MeOH (15 mL) was added LiOH.H₂O (0.5 g, 11.96 mmol) in H₂O (5 mL) at 0 °C. The reaction mixture was stirred at the same temperature for 3 h. The solvent was removed by concentration. The resulting aqueous solution was acidified with 3N HCl and washed with EtOAc. Water was removed by lyophilization. The residue was purified by prep-HPLC to give the Compound **4-3** as a white solid (601 mg, 28% yield).

LC-MS (ESI): m/z 721.6 [M+H]⁺.

### tert-Butyl 4,7,10,13,16,19,22,25-octamethyl-3,6,9,12,15,18,24-octaoxo-1-phenyl-2-oxa-4,7,10,13,16,19,22,25-octaazaheptacosan-27-oate (4-4)

To a solution of Compound **4-3** (600 mg, 0.83 mmol) in DCM (10 mL) was added tert-butyl N,N'-diisopropylcarbamate (928 mg, 4.63 mmol) at 0 °C. The reaction mixture was stirred at room temperature overnight. The mixture was washed successively with 1N HCl (2 × 5 mL), water (5 mL), saturated aqueous NaHCO₃ (2 x 5 mL) and brine (5 mL), dried over anhydrous Na₂SO₄, concentrated under reduced pressure to yield the Compound **4-4** as a light yellow oil (702 mg, quantitative). LC-MS (ESI): m/z 799.8 [M+H] ⁺.

### tert-Butyl 5,8,11,14,17,20,23-heptamethyl-4,7,10,13,16,19,22-heptaoxo-2,5,8,11,14,17,20,23-octaazapentacosan-25-oate (4-5)

To a solution of Compound **4-4** (700 mg) in MeOH (10 mL) was added 10% Pd/C (100 mg) under an N₂ atmosphere. The reaction mixture was stirred at room temperature for 4 h under an H₂ atmosphere. The reaction was filtered, the filtrate was concentrated under reduced pressure to afford the Compound **4-5** as a light yellow oil (360 mg, 62% yield).

LC-MS (ESI): m/z 643.2 [M+H]⁺.

### tert-Butyl 26-(2,5-dioxo-2,5-dihydro-1H-pyrrol-1-yl)-3,6,9,12,15,18,21,24-octamethyl-4,7,10,13,16,19,22,25-octaoxo-3,6,9,12,15,18,21,24-octaazahexacosanoate (4-6)

To a slolution of Compound **4-5** (360 mg, 0.56 mmol) and maleimidoacetic acid (104 mg, 0.672 mmol) in DCM (10 mL) were added HATU (255 mg, 0.672 mmol) and DIPEA (108 mg, 0.84 mmol). The reaction mixture was stirred at room temperature overnight. The mixture was washed successively with saturated aqueous NaHCO₃ (5 mL), saturated aqueous NH₄Cl (5 mL) and brine (5 mL), dried over anhydrous Na₂SO₄, concentrated under reduced pressure to yield the Compound **4-6** as a light yellow oil (436 mg, quant.).

LC-MS (ESI): m/z 802.3 [M+H] ⁺.

### 26-(2,5-Dioxo-2,5-dihydro-1H-pyrrol-1-yl)-3,6,9,12,15,18,21,24-octamethyl-4,7,10,13,16,19,22,25-octaoxo-3,6,9,12,15,18,21,24-octaazahexacosanoic acid (4-7)

To a solution of Compound **4-6** (435 mg, 0.56 mmol) in DCM (8 mL) was added TFA (4 mL) at 0 °C. The reaction mixture was stirred at room temperature for 4 h. The mixture was concentrated under reduced pressure. The residue was purified by prep-HPLC to afford the Compound **4-7** as a white solid (295 mg, 74% yield).

LC-MS (ESI): m/z 746.4 [M+Na] ⁺.

### (S)-N-(2-((1-(4-Amino-2-(ethoxymethyl)-1H-imidazo[4,5-c]quinolin-1-yl)-2-methylpropan-2-yl)oxy)ethyl)-2-(26-(2,5-dioxo-2,5-dihydro-1H-pyrrol-1-yl)-3,6,9,12,15,18,21,24-octamethyl-4,7,10,13,16,19,22,25-octaoxo-3,6,9,12,15,18,21,24-octaazahexacosanamido)-4,4-dimethylpentanamide (Compound 6)

To a solution of Compound 4-7 (90 mg, 0.12 mmol) in DCM (2 mL) were added EDCI (30 mg, 0.16 mmol) and HOBt (21 mg, 0.16 mmol) at 0 °C. After stirring for 5 min, Compound 2-5 (Example 2.2, 59 mg, 0.13 mmol) was added. The reaction mixture was stirred at room temperature for 4 h. The residue was purified by prep-HPLC to afford **Compound** 6 as a white solid (36 mg, 24% yield).

LC-MS (ESI): m/z 1212.8 [M+Na]⁺.

### Example 2.5 Synthesis of Compound 7 and Compound 8

### Synthetic route

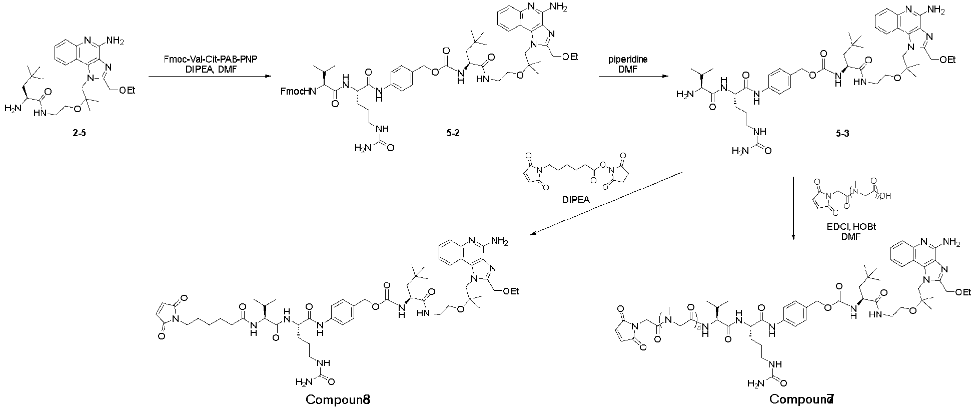

### (9H-Fluoren-9-yl)methyl ((S)-1-(((S)-1-((4-((S)-12-(4-amino-2-(ethoxymethyl))-1H-imidazo[4,5-c]quinolin-1-yl)-11,11-dimethyl-5-neopentyl-3,6-dioxo-2,10-dioxa-4,7-diazadodecyl)phenyl)amino)-1-oxo-5-ureidopentan-2-yl)amino)-3-methyl-1-oxobutan-2-yl)carbamate (5-2)

To a solution of Compound 2-5 (Example 2.2, 250 mg, 0.516 mmol) and DIPEA (272 mg, 2.1 mmol) in DMF (8 mL) was added Fmoc-Val-Cit-PAB-PNP (396 mg, 0.516 mmol). The reaction mixture was stirred at room temperature overnight. The mixture was diluted with EtOAc (20 mL), washed successively with saturated aqueous NaHCO₃ (5 mL), NH₄Cl aq. (5 mL) and brine (5 mL), dried over anhydrous Na₂SO₄ and concentrated under reduced pressure to give the Compound **5-2** (crude, 550 mg).

LC-MS (ESI):*m*/*z* 1112.1 [M+H]⁺.

### 4-((S)-2-((S)-2-Amino-3-methylbutanamido)-5-ureidopentanamido)benzyl ((S)-1-((2-((1-(4-amino-2-(ethoxymethyl)-1H-imidazo[4,5-c]quinolin-1-yl)-2-methylpropan-2-yl)oxy)ethyl)amino)-4,4-dimethyl-1-oxopentan-2-yl)carbamate (5-3)

To a solution of Compound **5-2** (550 mg, 0.19 mmol) in DMF (5 mL) was added piperidine (420 mg). The reaction mixture was stirred at room temperature for 3 h. The mixture was purified by prep-HPLC to give the Compound **5**-**3** (238 mg, 52% yield).

LC-MS (ESI):*m*/*z* 890.3 [M+H]⁺.

### 4-((2S,5S)-20-(2,5-Dioxo-2,5-dihydro-1H-pyrrol-1-yl)-5-isopropyl-9,12,15,18-tetramethyl-4,7,10,13,16,19-hexaoxo-2-(3-ureidopropyl)-3,6,9,12,15,18-hexaazaeicosanamido)benzyl ((S)-1-((2-((1-(4-amino-2-(ethoxymethyl)-1H-imidazo[4,5-c]quinolin-1-yl)-2-methylpropan-2-yl)oxy)ethyl)amino)-4,4-dimethyl-1-oxopentan-2-yl)carbamate (Compound 7)

To a solution of Mal-Sar₄-OH (25 mg, 0.056 mmol) and HOBt (10 mg, 0.07 mmol) in DMF (0.5 mL) was added EDCI (14 mg, 0.07 mmol). The reaction mixture was stirred at room temperature for 2 h under an N₂ atmosphere. The reaction mixture was stirred at room temperature for 10 min under an N₂ atmosphere, then Compound **5-3** (50 mg 0.056 mmol) was added. The reaction mixture was stirred at room temperature for 2 h under an N₂ atmosphere. The mixture was filtered and the filtrate was purified by prep-HPLC to afford the **Compound 7** as a white solid (20 mg, 27% yield).

LC-MS (ESI):*m*/*z* 1311.3 [M+H]⁺.

### 4-((S)-2-((S)-2-(6-(2,5-Dioxo-2,5-dihydro-1H-pyrrol-1-yl)hexanamido)-3-methylbutanamido)-5-ureidopentanamido)benzyl ((S)-1-((2-((1-(4-amino-2-(ethoxymethyl)-1H-imidazo[4,5-c]quinolin-1-yl)-2-methylpropan-2-yl)oxy)ethyl)amino)-4,4-dimethyl-1-oxopentan-2-yl)carbamate (Compound 8)

To a solution of Compound 5-3 (60 mg, 0.067 mmol) in DMF (2 mL) was added DIPEA (8.8 mg, 0.067 mmol) and N-succinimidyl 6-maleimidohexanoate (21 mg, 0.067 mmol). The reaction mixture was stirred at room temperature for 4 h. The mixture was purified by prep-HPLC to afford the Compound 8 as a white solid (20 mg, 27% yield).

LC-MS (ESI):m/z 1083.2 [M+H]⁺.

### Example 2.6 Synthesis of Compound 9

### Synthetic route

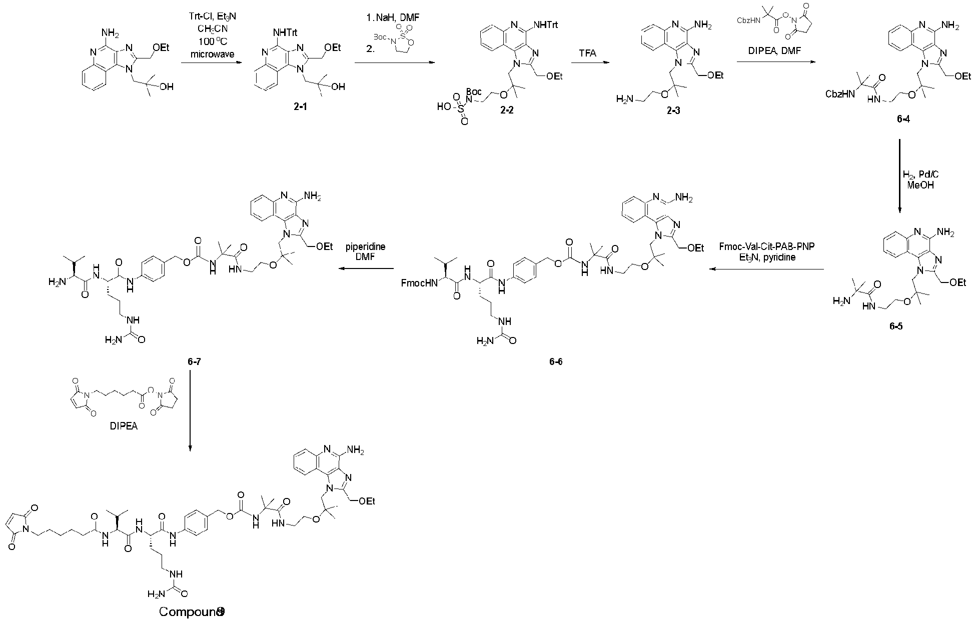

### Benzyl (1-((2-((1-(4-amino-2-(ethoxymethyl)-1H-imidazo[4,5-c]quinolin-1-yl)-2-methylpropan-2-yl)oxy)ethyl)amino)-2-methyl-1-oxopropan-2-yl)carbamate (6-4)

To a solution of Compound 2-3 (see synthesis of **Compound** 4) (600 mg, 1.68 mmol) in DMF (10 mL) were added DIPEA (759 mg, 5.88 mmol) and 2,5-dioxopyrrolidin-1-yl (*S*)-2-(((benzyloxy)-carbonyl)amino)-4,4-dimethylpentanoate (573 mg, 1.7 mmol), and the reaction mixture was stirred at room temperature overnight. The mixture was diluted with EtOAc (50 mL), washed successively with 0.5N HCl and brine, dried over anhydrous Na₂SO₄ and concentrated under reduced pressure. The residue was purified by prep-HPLC to afford the Compound **6-4** as a white foam (560 mg, 58% yield).

LC-MS (ESI):*m*/*z* 577.1 [M+H]⁺.

### 2-Amino-N-(2-((1-(4-amino-2-(ethoxymethyl)-1H-imidazo[4,5-c]quinolin-1-yl)-2-methylpropan-2-yl)oxy)ethyl)-2-methylpropanamide (6-5)

To a solution of Compound **6-4** (560 mg, 0.97 mmol) in MeOH (10 mL) was added 10% Pd/C (500 mg) under an N₂ atmosphere. The reaction mixture was stirred at room temperature for 4 h under an H₂ atmosphere. The reaction was filtered and the filtrate was concentrated under reduced pressure to afford the Compound **6-5** as a white solid (390 mg, 91 % yield).

LC-MS (ESI):*m*/*z* 443.2 [M+H]⁺.

### (9H-Fluoren-9-yl)methyl ((S)-1-(((S)-1-((4-(12-(4-amino-2-(ethoxymethyl))-1H-imidazo[4,5-c]quinolin-1-yl)-5,5,11,11-tetramethyl-3,6-dioxo-2,10-dioxa-4,7-diazadodecyl)phenyl)amino)-1-oxo-5-ureidopentan-2-yl)amino)-3-methyl-1-oxobutan-2-yl)carbamate (6-6)

To a solution of Compound **6-5** (390 mg, 0.88 mmol) and DIPEA (569 mg, 4.4 mmol) in DMF (10 mL) was added Fmoc-Val-Cit-PAB-PNP (675 mg, 0.88 mmol), and the reaction mixture was stirred at room temperature overnight. The mixture was diluted with EtOAc (50 mL), washed successively with saturated aqueous NaHCO₃ (5 mL), NH₄Cl aqueous solution (5 mL) and brine (5 mL), dried over anhydrous Na₂SO₄ and concentrated under reduced pressure to give Compound **6-6** (crude, 958 mg).

LC-MS (ESI):*m*/*z* 1070.3 [M+H]⁺.

### 4-((S)-2-((S)-2-Amino-3-methylbutanamido)-5-ureidopentanamido)benzyl (1-((2-((1-(4-amino-2-(ethoxymethyl))-1H-imidazo[4,5-c]quinolin-1-yl)-2-methylpropan-2-yl)oxy)ethyl)amino)-2-methyl-1-oxopropan-2-yl)carbamate (6-7)

To a sloution of Compound **6-6** (958 mg, 0.88 mmol) in DMF (5 mL) was added piperidine (750 mg) at 0 °C. The reaction mixture was stirred at room temperature for 3 h. The mixture was purified by prep-HPLC to give Compound **6-7** (210 mg, 28% yield).

LC-MS (ESI):*m*/*z* 848.1 [M+H]⁺.

### 4-((S)-2-((S)-2-(6-(2,5-Dioxo-2,5-dihydro-1H-pyrrol-1-yl)hexanamido)-3-methylbutanamido)-5-ureidopentanamido)benzyl (1-((2-((1-(4-amino-2-(ethoxymethyl)-1H-imidazo[4,5-c]quinolin-1-yl)-2-methylpropan-2-yl)oxy)ethyl)amino)-2-methyl-1-oxopropan-2-yl)carbamate (Compound 9)

To a solution of Compound **6-7** (50 mg, 0.056 mmol) in DMF (1 mL) were added DIPEA (7 mg, 0.056 mmol) and N-succinimidyl 6-maleimidohexanoate (18 mg, 0.056 mmol). The reaction mixture was stirred at room temperature for 4 h. The mixture was purified by prep-HPLC to give the **Compound 9** as a white solid (15 mg, 25% yield).

LC-MS (ESI):*m*/*z* 1041.1 [M+H]⁺.

### Example 3. Assay on Activity of TLR7/8 Immune Agonists in H-TLR7 NFKB Reporter 293 and H-TLR8 NFKB Reporter 293 Cell Lines

Uses of the reporter gene cells H-TLR7 NFKB Reporter 293 (GM-C15426, Genomeditech, Shanghai, China), H-TLR8 NFKB Reporter 293 (GM-C15427, Genomeditech) were assayed on activation activity of TLR7 and TLR8 of TLR7/8 immune agonists . After the TLR7 and TLR8 receptors in the cells are bound and activated, luciferase is released into the experimental system through the downstream signal NF-κB, so that the degree of activation of the cells can be measured.

The specific method is as follows: A 96-well white flat-bottom cell culture plate was used, and H-TLR7 NFKB Reporter 293 (GM-C15426, Genomeditech) and H-TLR8 NFKB Reporter 293 (GM-C15427, Genomeditech) were added to each well. at 3×10⁴ cells per well, and subjected to adherent culture at 37 °C overnight. The supernatant was discarded the next day, and corresponding concentrations of TLR7/8 IMMUNOAGONIST were added, respectively. The cells were cultured in an incubator at 37 °C for another 6 hours. The culture plate was then taken out, and Bio-Glo (G7940, Promega) was added for wavelength detection using a microplate reader (Spectra, Molecular Devices).

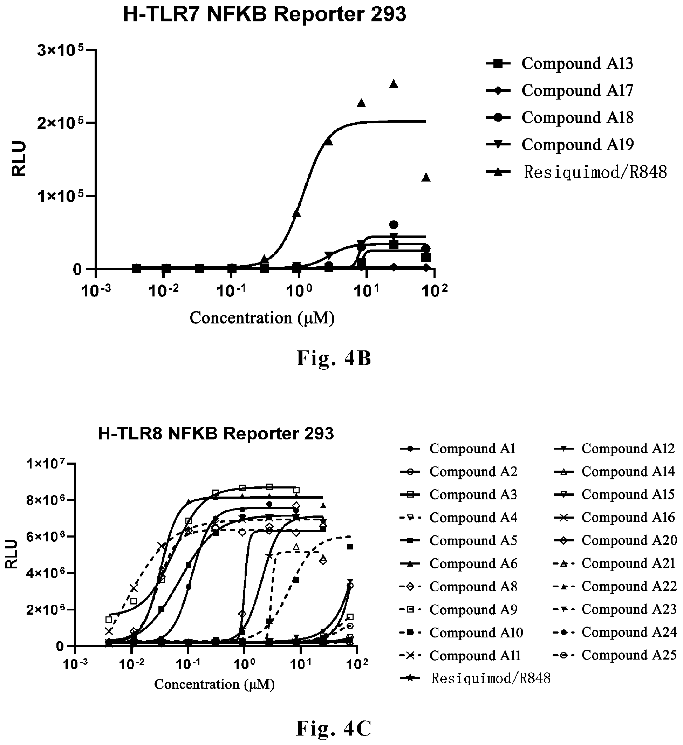

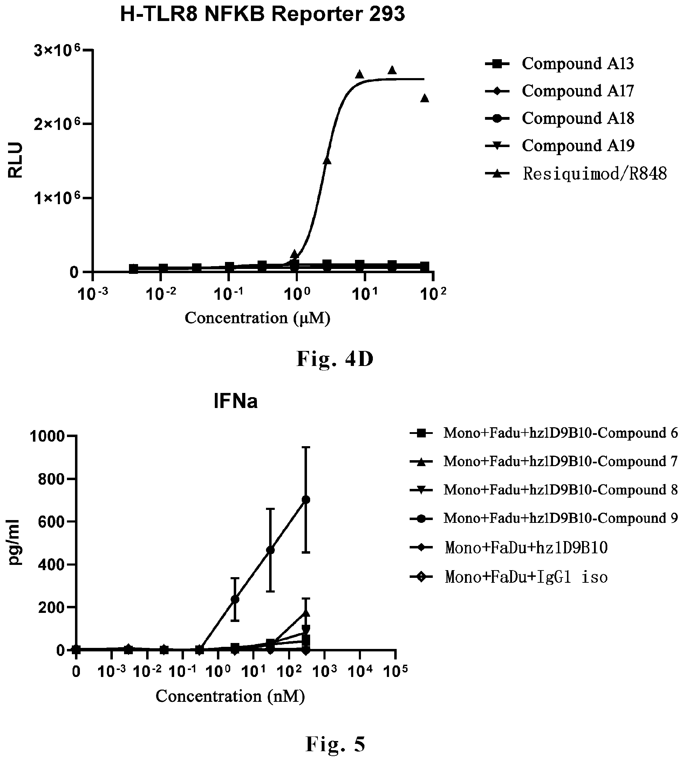

The TLR7 activity of the compounds is shown in FIGs. 4A and 4B, and the TLR8 activity is shown in FIGs. 4C and 4D. As can be seen from FIG. 4A, compounds other than compound A1, compound A3, compound A6, compound A11, compound A8 and compound A4 can all exhibit the activity, as detected on H-TLR7 NFKB Reporter 293, indicating that only compound A1, compound A3, compound A6, compound A11, compound A8, and compound A4 are not TLR7 agonists, and the other compounds are TLR7 agonists. In addition, different compounds have different activation efficacies. For example, compound A20, compound A21, compound A9, compound A10, compound A5, compound A16, and compound A22 have higher activity for TLR7 than Resiquimod (Resiquimod, R848). The TLR7 activity of the other compounds was lower than Resiquimod (Resiquimod, R848). As can be seen from FIG. 4B, compound A13, compound A18 and compound A19 have weak TLR7 activity, and compound A17 has no TLR7 activity. As can be seen from FIG. 4C, compound A11, compound A8, compound A6, compound A5, compound A3, compound A1, compound A20 exhibit hith activity, as detected on H-TLR8 NFKB Reporter 293, with the activity higher than Resiquimod (Resiquimod, R848); and TLR8 activity of compound A21 and compound A10 is less than Resiquimod (Resiquimod, R848), and the rest of the compounds have no TLR8 activity. As can be seen from Fig. 4D, none of compound A13, compound A17, compound A18 and compound A19 had TLR8 activity.

Thus compound A2, compound A12, compound A14, compound A15, compound A16, compound A9, compound A22, compound A23, compound A24, compound A25, compound A13, compound A18, compound A19 are TLR7 agonists, compound A1, compound A3, compound A6, compound A11, compound A8 are TLR8 agonists, compound A5, compound A20, compound A21, compound A10 are TLR7/8 dual agonists, wherein compound A5 is the TLR 7/8 dual agonist showing stronger activity than Resiquimod (Resiquimod, R848).

### Example 4. Preparation of Conjugates

### 4.1 Preparation of hz1D9B10-compound 5

The specific procedures are as follows:
**(**a) Antibody hz1D9B10 was dissolved in PBS buffer.
(b) adding a reducing agent solution (TCEP, Aldrich, Catalog Number 646547, dissolved in water), and leaving the reaction mixture to react at room temperature for 2 h, wherein
   (i) the optimal concentration of hz1D9B10 was 5-10 mg/mL,
   (ii) the optimal molar ratio of TCEP/mAb was 2.0,
   (iii) the optimal temperature for the reaction was 25 °C, and
   (iv) the optimal pH value for the reaction was between 6.0 and 8.0.
(c) adding an excess amount of linker-toxin (compound 5, dissolved in DMSO) to react with the reduced antibody in step (b), and placing the reaction mixture at room temperature for 2 h, wherein
   (i) the optimal molar ratio of compound 5/mAb was 6.0,
   (ii) the optimal temperature for the reaction was 25 °C, and
   A crude ADC product was obtained.
(d) purifying the obtained crude ADC product by spin desalting, ultrafiltration, or dialysis to obtain a final ADC product.
(e) The ADC product was detected by RP-HPLC, LC-MS, and SEC HPLC, and the average DAR value was determined to be 4 and the SEC purity was determined.

### 4.2 Preparation of hz1D9B10-compound 6

The specific procedures are as follows:
(a) Antibody hz1D9B10 was dissolved in PBS buffer.
(b) adding a reducing agent solution (TCEP, Aldrich, Catalog Number 646547, dissolved in water), and leaving the reaction mixture to react at room temperature for 2 h, wherein
   (i) the optimal concentration of hz1D9B10 was 5-10 mg/mL,
   (ii) the optimal molar ratio of TCEP/mAb was 2.0,
   (iii) the optimal temperature for the reaction was 25 °C, and
   (iv) the optimal pH value for the reaction was between 6.0 and 8.0.
(c) adding an excess amount of linker-toxin (compound 6, dissolved in DMSO) to react with the reduced antibody in step (b), and placing the reaction mixture at room temperature for 2 h, wherein
   (i) the optimal molar ratio of compound 6/mAb was 6.0,
   (ii) the optimal temperature for the reaction was 25 °C, and
   A crude ADC product was obtained.
(d) purifying the obtained crude ADC product by spin desalting, ultrafiltration, or dialysis to obtain a final ADC product.
(e) The ADC product was detected by RP-HPLC, LC-MS, and SEC HPLC, and the average DAR value was determined to be 4 and the SEC purity was determined.

### 4.3 Preparation of hz1D9B10-compound 7

The specific procedures are as follows:
(a) Antibody hz1D9B10 was dissolved in PBS buffer.
(b) adding a reducing agent solution (TCEP, Aldrich, Catalog Number 646547, dissolved in water), and leaving the reaction mixture to react at room temperature for 2 h, wherein
   (i) the optimal concentration of hz1D9B10 was 5-10 mg/mL,
   (ii) the optimal molar ratio of TCEP/mAb was 2.0,
   (iii) the optimal temperature for the reaction was 25 °C, and
   (iv) the optimal pH value for the reaction was between 6.0 and 8.0.
(c) adding an excess amount of linker-toxin (compound 7, dissolved in DMSO) to react with the reduced antibody in step (b), and placing the reaction mixture at room temperature for 2 h, wherein
   (i) the optimal molar ratio of compound 7/mAb was 6.0,
   (ii) the optimal temperature for the reaction was 25 °C, and
   A crude ADC product was obtained.
(d) purifying the obtained crude ADC product by spin desalting, ultrafiltration, or dialysis to obtain a final ADC product.
(e) The ADC product was detected by RP-HPLC, LC-MS, and SEC HPLC, and the average DAR value was determined to be 4 and the SEC purity was determined.

### 4.4 Preparation of hz1D9B10-compound 8

The specific procedures are as follows:
(a) Antibody hz1D9B10 was dissolved in PBS buffer.
(b) adding a reducing agent solution (TCEP, Aldrich, Catalog Number 646547, dissolved in water), and leaving the reaction mixture to react at room temperature for 2 h, wherein
   (i) the optimal concentration of hz1D9B10 was 5-10 mg/mL,
   (ii) the optimal molar ratio of TCEP/mAb was 2.0,
   (iii) the optimal temperature for the reaction was 25 °C, and
   (iv) the optimal pH value for the reaction was between 6.0 and 8.0.
(c) adding an excess amount of linker-toxin (compound 8, dissolved in DMSO) to react with the reduced antibody in step (b), and placing the reaction mixture at room temperature for 2 h, wherein
   (i) the optimal molar ratio of compound 8/mAb was 6.0,
   (ii) the optimal temperature for the reaction was 25 °C, and
   A crude ADC product was obtained.
(d) purifying the obtained crude ADC product by spin desalting, ultrafiltration, or dialysis to obtain a final ADC product.
(e) The ADC product was detected by RP-HPLC, LC-MS, and SEC HPLC, and the average DAR value was determined to be 4 and the SEC purity was determined.

### 4.5 Preparation of hz1D9B10-compound 9

The specific procedures are as follows:
(a) Antibody hz1D9B10 was dissolved in PBS buffer.
(b) adding a reducing agent solution (TCEP, Aldrich, Catalog Number 646547, dissolved in water), and leaving the reaction mixture to react at room temperature for 2 h, wherein
   (i) the optimal concentration of hz1D9B10 was 5-10 mg/mL,
   (ii) the optimal molar ratio of TCEP/mAb was 2.0,
   (iii) the optimal temperature for the reaction was 25 °C, and
   (iv) the optimal pH value for the reaction was between 6.0 and 8.0.
(c) adding an excess amount of linker-toxin (compound 9, dissolved in DMSO) to react with the reduced antibody in step (b), and placing the reaction mixture at room temperature for 2 h, wherein
   (i) the optimal molar ratio of compound 9/mAb was 6.0,
   (ii) the optimal temperature for the reaction was 25 °C, and
   A crude ADC product was obtained.
(d) purifying the obtained crude ADC product by spin desalting, ultrafiltration, or dialysis to obtain a final ADC product.
(e) The ADC product was detected by RP-HPLC, LC-MS, and SEC HPLC, and the average DAR value was determined to be 4 and the SEC purity was determined.

### 4.6 Preparation of IgG-compound 6

The specific procedures are as follows:
(a) Antibody isotype control IgG1 was dissolved in PBS buffer.
(b) adding a reducing agent solution (TCEP, Aldrich, Catalog Number 646547, dissolved in water), and leaving the reaction mixture to react at room temperature for 2 h, wherein
   (i) the optimal concentration of isotype control IgG1 was 5-10 mg/mL, and
   (ii) the optimal molar ratio of TCEP/mAb was 2.0,
   (iii) the optimal temperature for the reaction was 25 °C, and
   (iv) the optimal pH value for the reaction was between 6.0 and 8.0.
(c) adding an excess amount of linker-toxin (compound 6, dissolved in DMSO) to react with the reduced antibody in step (b), and placing the reaction mixture at room temperature for 2 h, wherein
   (i) the optimal molar ratio of compound 6/mAb was 6.0,
   (ii) the optimal temperature for the reaction was 25 °C, and
   A crude ADC product was obtained.
(d) purifying the obtained crude ADC product by spin desalting, ultrafiltration, or dialysis to obtain a final ADC product.
(e) The ADC product was detected by RP-HPLC, LC-MS, and SEC HPLC, and the average DAR value was determined to be 4 and the SEC purity was determined.

### 4.7 Preparation of IgG-NT3

The specific procedures are as follows:
(a) Antibody isotype control IgG1 was dissolved in PBS buffer.
(b) adding a reducing agent solution (TCEP, Aldrich, Catalog Number 646547, dissolved in water), and leaving the reaction mixture to react at room temperature for 2 h, wherein
   (i) the optimal concentration of isotype control IgG1 was 5-10 mg/mL, and
   (ii) the optimal molar ratio of TCEP/mAb was 2.0,
   (iii) the optimal temperature for the reaction was 25 °C, and
   (iv) the optimal pH value for the reaction was between 6.0 and 8.0.
(c) adding an excess of linker-toxin (NT3, from WO2021173773A1, dissolved in DMSO) to react with the reduced antibody in step (b), and placing the reaction mixture at room temperature for 2 h, wherein
   (i) the optimal molar ratio of NT3/mAb was 6.0,
   (ii) the optimal temperature for the reaction was 25 °C, and
   A crude ADC product was obtained.
(d) purifying the obtained crude ADC product by spin desalting, ultrafiltration, or dialysis to obtain a final ADC product.
(e) The ADC product was detected by RP-HPLC, LC-MS, and SEC HPLC, and the average DAR value was determined to be 4 and the SEC purity was determined.

### 4.8 Preparation of hRS7-NT3

**hRS7-NT3** is prepared by the following process. 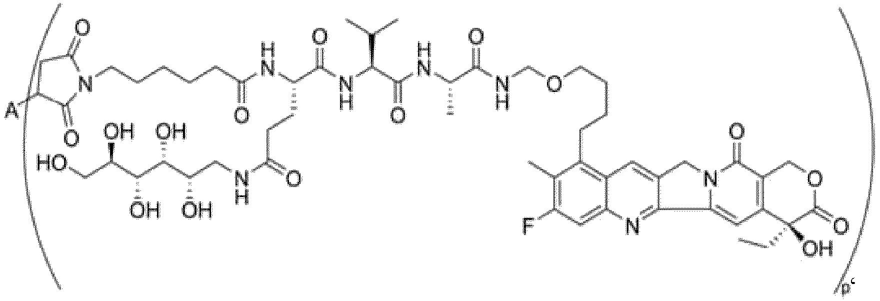 wherein A is hRS7, p' is about 4.3.
(a) The antibody hRS7 was dissolved in a PBS buffer (pH 7.4).
(b) a reducing agent solution (TCEP, Aldrich, Catalog Number 646547, dissolved in water) was added, and the reaction mixture was left to react at room temperature for 2 h, wherein: (i) the optimal concentration of hRS7 was 5 mg/mL, (ii) the optimal molar ratio of TCEP/mAb was 2.0, (iii) the optimal temperature for the reaction was 25 °C, and (iv) the optimal pH value for the reaction was between 6.0 and 8.0;
(c) an excess amount of NT3 (from WO2021173773A1, dissolved in DMSO), and reacted with the reduced antibody in step (a). The reaction mixture was left to stand at room temperature for 2 h, wherein: (i) the optimal molar ratio of NT3/mAb was 6.0, and (ii) the optimal temperature for the reaction was 25 °C, thus obtaining a crude ADC product;
(d) subjecting the obtained crude ADC product to spin desalting, ultrafiltration, or dialysis purification to obtain a final ADC product;
(e) The ADC product was detected by RP-HPLC, LC-MS, and SEC HPLC to determine the average DAR and SEC purity.

The average DAR value was 4.3, and the SEC purity was 98.42.

### Example 5. Activity of Different Immune-Stimulating Antibody Conjugates (ISAC) in Co-Culture System of PBMC and Tumor Cells

Monocytes were isolated from human peripheral blood mononuclear cells (PBMCs, TPCS) using a human monocyte enrichment kit without CD16 depletion (19058, STEMCELL). The cells were resuspended at a density of 1×10⁷ cells/mL in RPMI-1640 complete medium (22400-071, Gibco) containing 10% fetal bovine serum (FBS, SH30406.05, Hyclone) and 0.1% β-mercaptoethanol (21985023, Gibco) and added to a flat-bottom 96-well cell culture plate at 5×10⁵ cells per well. Target cells FaDu (HTB-43, ATCC) were digested and the cell density was adjusted to 2 × 10⁶ Cells/mL, and added at 1×10⁵ cells/well, followed by addition of each respective concentration of TROP2 ISAC or anti-TROP2 monoclonal antibody. The mixture was mixed well and then incubated in an incubator at 37 °C for 18 h. Then the cell culture plate was taken out, and IFNa secretion in the cell-free supernatant was detected using ELISA method. The results are shown in FIG. 5. The amounts of IFNa secreted by hz1D9B10-compound 6, hz1D9B10-compound 7 and hz1D9B10-compound 8 were all less than that by hz1D9B10-compound 9, indicating that the *in vitro* activity of hz1D9B10-compound 6, hz1D9B10-compound 7 and hz1D9B10-compound 8 was weaker than that of hz1D9B10-compound 9.

### Example 6. Therapeutic Effect of TROP2-ISAC in NCI-N87 Model 6.1 Therapeutic effect of hz1D9B10-compound 7 and hz1D9B10-compound 9 in NCI-N87 model

NCI-N87 cells were used for inoculation to CB17-SCID mice in this experiment. The anti-tumor effect of the TROP2-ISAC antibody of the present invention was determined.

### CB17-SCID Mice:

Female CB17-SCID SPF background mice purchased from Beijing Vital River Laboratory Animal Technology Co., Ltd., qualified by Beijing Vital River Laboratory Animal Technology Co., Ltd., with certificate No. 110011221100703563. The study started after the mice were quarantined and adapted for 3 days upon arrival.

### Cells:

NCI-N87 cells were purchased from ATCC (CAT#: CRL-5822), and the cells were subcultured in strict accordance with the instructions for subsequent in vivo assays. The cells were collected by centrifugation and resuspended in sterile PBS, with the cell density adjusted to 10×10⁶ cells/mL. On day 0, 0.2 mL of the cell suspension was subcutaneously inoculated into CB17-SCID in the right abdominal region of the mice. NCI-N87 tumor-bearing mouse models were established.

### Administration:

The tumor volume of each mouse was measured 8 days after tumor cell inoculation, and tumors with a volume of about 78-138 mm³ were selected. Mice were evenly divided into groups by tumor volume (6 mice per group). The dosages and routes of administration are shown in Table 8. h-IgG (purchased from EQUITECH-BIO) was used as a negative control. Administration was performed on day 8 after inoculation. The tumor volume and body weight of the mice were monitored twice a week. The body weight and tumor volume were measured before each administration. On day 26 after inoculation, the relative tumor growth inhibition (TGI%) was calculated by the following formula: TGI% = 100% × (control group tumor volume - treatment group tumor volume)/(control group tumor volume - control group tumor volume before administration).Tumor volume measurement: The maximum length of major axis (L) and maximum length of minor axis (W) of tumors were measured with a vernier caliper, and tumor volume was calculated using the following formula: V = L×W²/2.The mice were weighted using an electronic balance.

**Table 8. Experimental Design**

| Group | Dose of administration | Administration frequency | Mode of administration |
|---|---|---|---|
| h-IgG | 5 mg/kg | Single dose | Intravenous injection |
| hz1D9B10 | 5 mg/kg | Single dose | Intravenous injection |
| hz1D9B10-Compound 7 | 5 mg/kg | Single dose | Intravenous injection |
| hz1D9B10-compound 9 | 5 mg/kg | Single dose | Intravenous injection |

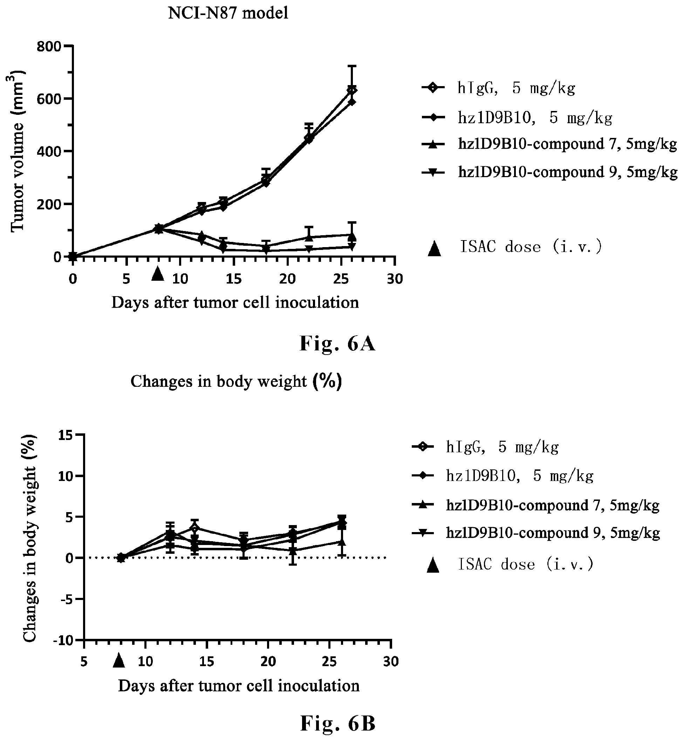

Tumor inhibition rates of hz1D9B10\hz1D9B10-Compound 7\hz1D9B10-Compound 9 group were 8.44% \104% \113%, respectively, and The tumor inhibitory effects of hz1D9B10-Compound 7 and hz1D9B10-compound 9 were comparable, and both significantly inhibited tumor growth. The efficacy results are shown in FIG. 6A and Table 9. Meanwhile, the body weight of the mice was monitored. As shown in FIG. 6B, there was no significant difference in body weight of the mice. Therefore, the TROP2-ISAC drug in the present invention has a significant inhibitory effect on tumors, and mice are in good general condition, which indicates that side effects or off-target effects are not significant.

**Table 9. Tumor growth inhibition on day 26**

| Group | Tumor volume (mm³) | TGI(%) |
|---|---|---|
| h-IgG, 5 mg/kg | 631.47 | N/A |
| hz1D9B10, 5 mg/kg | 587.07 | 8.44 |
| hz1D9B10-compound 7, 5 mg/kg | 82.71 | 104 |
| Hz1D9B10-compound 9, 5 mg/kg | 36.59 | 113 |

### 6.2 Therapeutic effect of hz1D9B10-compound 5, hz1D9B10-compound 8 and hz1D9B10-compound 6 in NCI-N87 model

In this experiment, CB17-SCID mice were inoculated with NCI-N87 cells to determine the anti-tumor effect of the TROP2-ISAC antibody of the present invention.

### CB17-SCID mice:

SPF female CB17-SCID mice, purchased from Beijing Vital River Laboratory Animal Technology Co., Ltd., qualified by Beijing Vital River Laboratory Animal Technology Co., Ltd., with certificate No. 110011221104924762. The study started after the mice were quarantined and adapted for 3 days upon arrival.

### Cells:

NCI-N87 cells were purchased from ATCC (CAT#: CRL-5822) and routinely subcultured strictly according to the instructions for subsequent in vivo experiments. The cells were collected by centrifugation and resuspended in sterile PBS, with the cell density adjusted to 10×10⁶cells/mL. On day 0, 0.2 mL of the cell suspension was subcutaneously inoculated into the right abdominal region of each of the CB17-SCID mice to establish NCI-N87 tumor-bearing mouse models.

### Administration:

The tumor volume of each mouse was measured 8 days after tumor cell inoculation, and tumors with a volume of about 100-122³ mm were selected. Mice were evenly divided into groups by tumor volume (6 mice per group). The dosages and routes of administration are shown in Table 10. h-IgG (purchased from EQUITECH-BIO) was used as a negative control. Administration was performed on day 8 after inoculation. The tumor volume and body weight of the mice were monitored twice a week. The body weight and tumor volume were measured before each administration. On day 25 after inoculation, the relative tumor growth inhibition (TGI%) was calculated by the following formula: TGI% = 100% × (control group tumor volume - treatment group tumor volume)/(control group tumor volume - control group tumor volume before administration).Tumor volume measurement: The maximum length of major axis (L) and maximum length of minor axis (W) of tumors were measured with a vernier caliper, and tumor volume was calculated using the following formula: V = L×W²/2.The mice were weighted using an electronic balance.

**Table 10. Experimental Design**

| Group | Dose of administration | Administration frequency | Mode of administration |
|---|---|---|---|
| h-IgG | 5 mg/kg | Single dose | Intravenous injection |
| hz1D9B10-compound 8 | 5 mg/kg | Single dose | Intravenous injection |
| hz1D9B10-compound 6 | 5 mg/kg | Single dose | Intravenous injection |
| Hz1D9B10-Compound 5 | 5 mg/kg | Single dose | Intravenous injection |

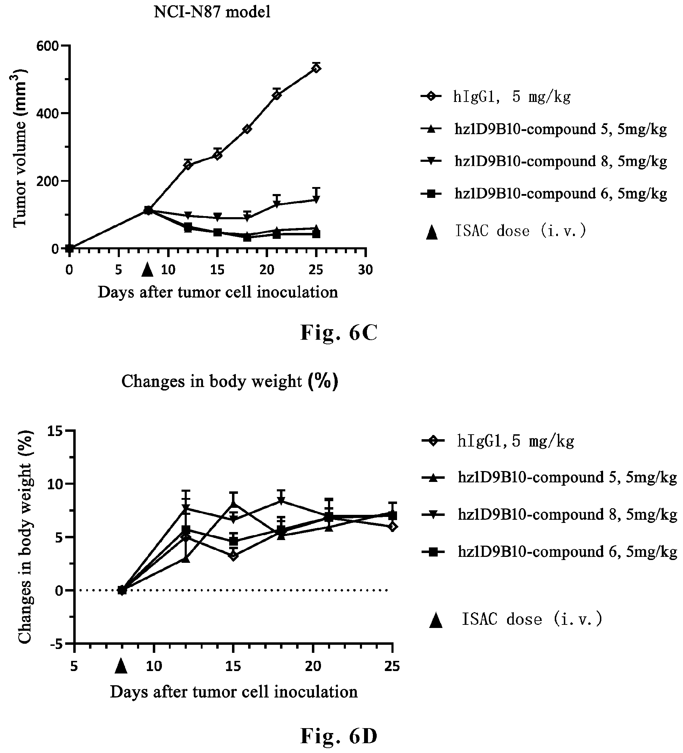

The tumor inhibition rates of the groups hz1D9B10-Compound 8\hz1D9B10-Compound 6\hz1D9B10-compound 5 were 92.67%\116.78%\112.73%, respectively, and the efficacy results are shown in FIG. 6C and Table 11. Meanwhile, the body weight of the mice was monitored. As shown in FIG. 6D, there was no significant difference in body weight of the mice. Therefore, the TROP2-ISAC drug in the present invention has a significant inhibitory effect on tumors, and mice are in good general condition, which indicates that side effects or off-target effects are not significant.

**Table 11. Tumor growth inhibition on day 25**

| Group | Tumor volume (mm³) | TGI(%) |
|---|---|---|
| h-IgG, 5 mg/kg | 532.37 | N/A |
| hz1D9B10-compound 8, 5 mg/kg | 143.69 | 92.67 |
| hz1D9B10-compound 6, 5 mg/kg | 42.56 | 116.78 |
| hz1D9B10-compound 5, 5 mg/kg | 59.53 | 112.73 |

### Example 7. Therapeutic Effect of TROP2-ISAC in BxPC3 Model

In this experiment, CB17-SCID mice were inoculated with BxPC3 cells to determine the anti-tumor effect of the TROP2-ISAC antibody of the present invention.

### CB17-SCID Mice:

Female CB17-SCID SPF background mice were purchased from Beijing Vital River Laboratory Animal Technology Co., Ltd., qualified by Beijing Vital River Laboratory Animal Technology Co., Ltd., with certificate No. 110011221110560000. Before the study, the mice were adapted for 3 days after arrival.

### Cells:

BxPC3 cells were purchased from Cell Bank, Chinese Academy of Sciences (CAT#: TCHu012), and were routinely subcultured strictly according to the instructions for subsequent in vivo experiments. The cells were collected by centrifugation and resuspended in sterile PBS, with the cell density adjusted to 15×10⁶ cells/mL. On day 0, 0.2 mL of the cell suspension was subcutaneously inoculated into CB17-SCID in the right abdominal region of the mice. The BxPC3 tumor-bearing mouse model was established.

### Administration:

11 days after the tumor cell inoculation, the tumor volume in each mouse was measured, and the tumor volume was selected to be about 76-122 mm³. Mice were evenly divided into groups by tumor volume (7 mice per group). The dosages and routes of administration are shown in Table 12. h-IgG (purchased from EQUITECH-BIO) was used as a negative control. Administration was performed on day 11 after inoculation. The tumor volume and body weight of the mice were monitored twice a week. The body weight and tumor volume were measured before each administration. On day 35 after inoculation, the relative tumor growth inhibition (TGI%) was calculated by the following formula: TGI% = 100% × (control group tumor volume - treatment group tumor volume)/(control group tumor volume - control group tumor volume before administration).Tumor volume measurement: The maximum length of major axis (L) and maximum length of minor axis (W) of tumors were measured with a vernier caliper, and tumor volume was calculated using the following formula: V = L×W²/2.The mice were weighted using an electronic balance.

**Table 12. Experimental Design**

| Group | Dose of administration | Administration frequency | Mode of administration |
|---|---|---|---|
| h-IgG | 5 mg/kg | Single dose | Intravenous injection |
| IgG-Compound 6 | 5 mg/kg | Single dose | Intravenous injection |
| hz1D9B10-compound 6 | 5 mg/kg | Single dose | Intravenous injection |
| hz1D9B10-compound 9 | 5 mg/kg | Single dose | Intravenous injection |

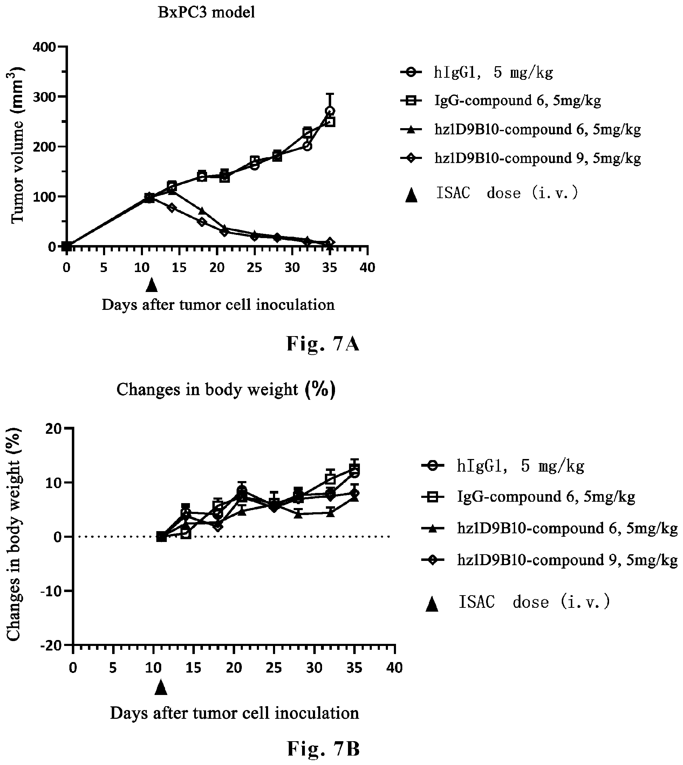

The tumor growth inhibition results are shown in FIG. 7A and Table 13: on day 35 after inoculation, the tumor growth inhibition of the hz1D9B10-compound 6 group and the hz1D9B10-compound 9 group was 155% and 150%, respectively, and the efficacy results are shown in FIG. 7A and Table 13. Meanwhile, the body weight of the mice was monitored. As shown in FIG. 7B, there was no significant difference in body weight of the mice. Therefore, the TROP2-ISAC drug in the present invention has a significant inhibitory effect on tumors, and mice are in good general condition, which indicates that side effects or off-target effects are not significant.

**Table 13. Tumor growth inhibition on day 35**

| Group | Tumor volume (mm³) | TGI(%) |
|---|---|---|
| h-IgG, 5 mg/kg | 271 | N/A |
| IgG-compound 6, 5 mg/kg | 249 | 13 |
| hz1D9B10-compound 6, 5 mg/kg | 0 | 155 |
| hz1D9B10-compound 9, 5 mg/kg | 8.39 | 150 |

### Example 8. Toxicity Studies of TROP2-ISAC in BALB/C Mice 8.1 Toxicity study of hz1D9B10-compound 7 and hz1D9B10-compound 9

In this experiment, BALB/C mice were used. The body temperature changes and deaths of the mice were observed after intravenous injection of TROP2-ISAC twice at an interval of 7 days, and the toxicity of the drug was determined thereby.

### BALB/C mice:

SPF-grade female BALB/C mice purchased from Beijing Vital River Laboratory Animal Technology Co., Ltd., qualified by Beijing Vital River Laboratory Animal Technology Co., Ltd., with certificate No. 110011221105222536. The study started after the mice purchased from Vital River were acclimated for 3 days after arrival.

### Administration:

According to the experimental design shown in Table 14, the mice were grouped and weighed, and injected intravenously with TROP2-ISAC on days 0 and 7. The initial rectal temperature of the mice was measured before administration on day 7, and the rectal temperature of the mice was measured 20 min\40 min\60 min after administration.

**Table 14. Experimental Design**

| Group | Dose of administration | Administration frequency | Mode of administration |
|---|---|---|---|
| h-IgG1 | 5 mg/kg | QWx2 | Intravenous injection |
| hz1D9B10 Mab | 5 mg/kg | QWx2 | Intravenous injection |
| hz1D9B10-Compound 7 | 5 mg/kg | QWx2 | Intravenous injection |
| hz1D9B10-Compound 9 | 5 mg/kg | QWx2 | Intravenous injection |

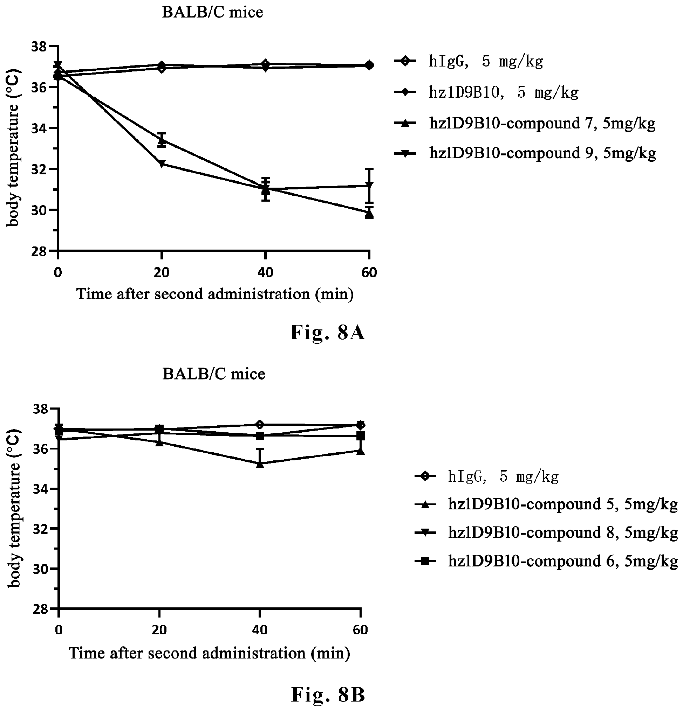

The rectal temperature results are shown in FIG. 8A and Table 15: after the second administration on D7, the rectal temperature of the mice in the TROP2-compound 7 group dropped to 29.88 °C at 60 min, and the rectal temperature of the mice in the TROP2-compound 9 group dropped to 31.18 °C at 60 min.Therefore, the administration of TROP2-compound 7 and TROP2-compound 9 on BALB/C mice at an interval of 7 days had toxic effects.

**Table 15. Rectal temperature of mice before administration on day 7 and after administration**

| Group | Pre-dose | 20 min | 40 min | 60 min |
|---|---|---|---|---|
| h-IgG1, 5 mg/kg | 36.54 | 36.92 | 37.12 | 37.08 |
| hz1D9B10 Mab, 5 mg/kg | 36.72 | 37.10 | 36.94 | 37.04 |
| hz1D9B10 -compound 7, 5 mg/kg | 36.56 | 33.42 | 31.08 | 29.88 |
| hz1D9B10-compound 9, 5 mg/kg | 37.06 | 32.24 | 31.02 | 31.18 |

### 8.2 Toxicity study of hz1D9B10-compound 5, hz1D9B10-compound 8 and hz1D9B10-compound 6

In this experiment, BALB/C mice were used. The body temperature changes and deaths of the mice were observed after intravenous injection of TROP2-ISAC twice at an interval of 7 days, and the toxicity of the drug was determined thereby.

### BALB/C mice:

SPF female BALB/C mice purchased from Beijing Vital River Laboratory Animal Technology Co., Ltd., qualified by Beijing Vital River Laboratory Animal Technology Co., Ltd., with certificate No. 110011221105981185. The study started after the mice purchased from Vital River were acclimated for 3 days after arrival.

### Administration:

According to the experimental design shown in Table 16, the mice were grouped and weighed, and injected intravenously with TROP2-ISAC on days 0 and 7, and then the initial rectal temperature of the mice was measured before administration on day 7 and the rectal temperature of the mice was measured 20 min\40 min\60 min after administration.

**Table 16. Experimental Design**

| Group | Dose of administration | Administration frequency | Mode of administration |
|---|---|---|---|
| h-IgG1 | 5 mg/kg | QWx2 | Intravenous injection |
| hz1D9B10-Compound 5 | 5 mg/kg | QWx2 | Intravenous injection |
| hz1D9B10-Compound 8 | 5 mg/kg | QWx2 | Intravenous injection |
| hz1D9B10-Compound 6 | 5 mg/kg | QWx2 | Intravenous injection |

The rectal temperature results are shown in FIG. 8B and Table 17: after the second administration on D7, only the rectal temperature of the mice in the TROP2-compound 5 group droppped to 35.9°C at 60 min. The other groups did not show significant temperature decrease. Therefore, there was no toxic effect of TROP2-compound 8 and TROP2-compound 6 on BALB/C mice after a 7-day administration interval.

**Table 17. Rectal temperature of mice before administration on day 7 and after administration**

| Group | Pre-dose | 20 min | 40 min | 60 min |
|---|---|---|---|---|
| h-IgG1, 5 mg/kg | 36.98 | 36.95 | 37.2 | 37.18 |
| hz1D9B10 -compound 5, 5 mg/kg | 37.00 | 36.33 | 35.25 | 35.9 |
| hz1D9B10 - compound 8, 5 mg/kg | 36.45 | 36.78 | 36.63 | 37.2 |
| hz1D9B10-compound 6, 5 mg/kg | 36.88 | 37.00 | 36.65 | 36.63 |

### Example 9. Toxic effects of TROP2-ISAC in hTROP2 KI mice

In this experiment, hTROP2 KI mice were used. The body temperature change and death of the mice were observed after intravenous injection of TROP2-ISAC twice to determine the toxicity of the drug thereby.

### hTROP2 KI transgenic mouse:

Female hTROP2 KI (B-hTROP2) mice, SPF grade, purchased from Biocytogen Jiangsu Co., Ltd., were qualified by Suzhou Xishan Biotechnology Co., Ltd., with certificate No. 3207262221100150728. The study started after the mice, purchased from Biocytogen, were acclimated for 3 days after arrival.

### Administration:

According to the experimental design shown in Table 18, the mice were grouped and weighed, injected intravenously with TROP2-ISAC on days 0 and 7, and then the initial rectal temperature of the mice was measured before administration on day 7 and the rectal temperature of the mice was measured 20 min\40 min\60 min after administration.

**Table 18. Experimental Design**

| Group | Dose of administration | Administration frequency | Mode of administration |
|---|---|---|---|
| hz1D9B10 | 10 mg/kg | QWx2 | Intravenous injection |
| hz1D9B10-compound 8 | 10 mg/kg | QWx2 | Intravenous injection |
| hz1D9B10-compound 9 | 10 mg/kg | QWx2 | Intravenous injection |
| hz1D9B10-compound 6 | 10 mg/kg | QWx2 | Intravenous injection |

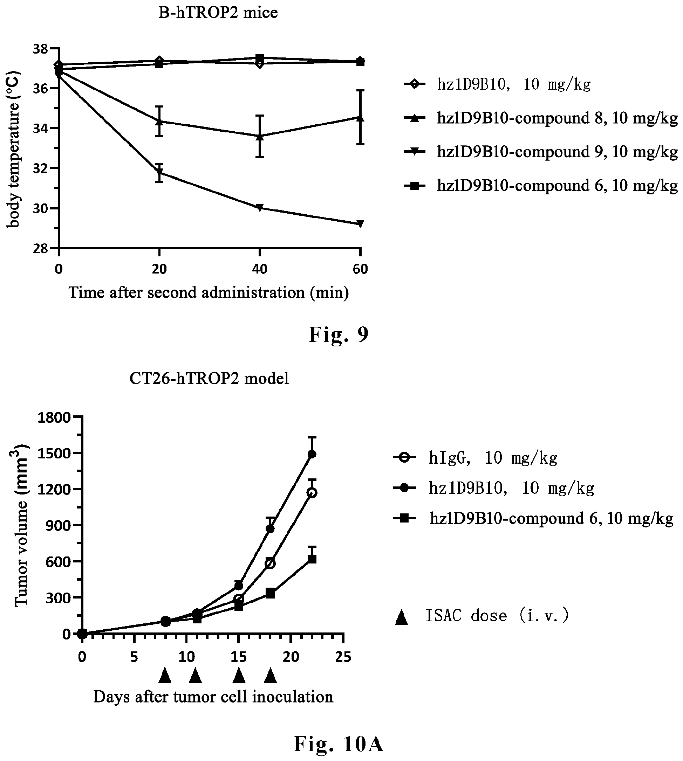

The rectal temperature results are shown in FIG. 9 and Table 19: after the second administration on D7, the rectal temperature of the mice in the TROP2-compound 8 group decreased to 34.55°C at 60 min, the rectal temperature of the mice in the TROP2-compound 9 group dropped to 29.20°C at 60 min, and there was no significant temperature decrease in the TROP2-compound 6 group. Therefore, there was no toxic effect of TROP2-compound 6 administered at 10 mg/kg to hTROP2 KI mice at an interval of 7 days.

**Table 19. Rectal temperature of mice on day 7**

| Group | Pre-dose | 20 min | 40 min | 60 min |
|---|---|---|---|---|
| hz1D9B10, 10 mg/kg | 37.18 | 37.38 | 37.23 | 37.35 |
| hz1D9B10-compound 8, 10 mg/kg | 36.88 | 34.35 | 33.60 | 34.55 |
| hz1D9B10-compound 9, 10 mg/kg | 36.60 | 31.78 | 30.00 | 29.20 |
| hz1D9B10-Compound 6, 10 mg/kg | 36.95 | 37.20 | 37.53 | 37.33 |

### Example 10. Dose finding test of repeated intravenous infusions of hz1D9B10-Compound 6 (hereinafter referred to as Conjugate A) to cynomolgus monkeys for 4 weeks

This study was conducted byJoinn (Suzhou) New Drug Research Center Co., Ltd.The objective of the study was to evaluate the possible toxic response of the conjugate A administered to cynomolgus monkeys by intravenous infusion/subcutaneous injection once every 2 weeks or 1 week for 4 consecutive weeks (3 injections in total), determine the target organ of toxicity, and investigate the immunogenicity and in vivo exposure level of the conjugate A.

A total of 8 cynomolgus monkey were used in this experiment (4 of each gender), and the monkeys were stratified by sex and randomly assigned to four groups based on body weight. The animals in Groups 1 and 2 were administered with conjugate A at 10 mg/kg and 30 mg/kg, respectively, once every 2 weeks. The administration was performed by intravenous infusion for a total of 3 times, with an administration volume of 5 mL/kg, an administration rate of about 10 mL/kg/h, and an administration time of about 30 min. Animals in Group 3 were administered Conjugate A at 15 mg/kg once weekly by intravenous infusion for a total of 4 doses, at a volume of 5 mL/kg, a rate of administration of about 10 mL/kg/h and an administration time of about 30 min.The animals in Group 4 were administered with conjugate A at 30 mg/kg once every 2 weeks by subcutaneous injection for a total of 3 times, with an administration volume of 2 mL/kg. The surviving animals in Groups 1, 2 and 4 were dissected 7 days after the last administration (D36). The surviving animals in the 3rd group were dissected 7 days after the last administration (D29).

During the experiment, the animals were subjected to examinations of clinical observations, body weight, food intake, body temperature, ophthalmology, clinical pathology (blood cell count, blood coagulation function, blood biochemistry), immune cell phenotype, cytokines, circulating immune complex (CIC), anti-drug antibody gross anatomy, organ weighing, and histopathology . Simultaneously, contents of ISAC(Conjugate A), total antibody (Conjugate A total antibody) and small molecules from conjugate A (payload, linker-payload, cys-linker-payload) were determined and toxicokinetic analysis was performed.

Under the conditions of this test, female animals in the groups with the dose of 30 mg/kg (both intravenous infusion and subcutaneous injection) were all moribund or found dead. Decreased body weight was observed in female animals in the 15 mg/kg dose group (intravenous administration QW). In addition, reduced food intake, immune-related toxicity (WBC, Neut, Lymph, CD3⁺, CD3⁺CD4⁺, CD3⁺CD8⁺ and CD20⁺ decrease then increase or recover; Mono, IgG, IgM, MCP-1, IP-10 and IL-6 increase), kidney-related toxicity, and changes in red blood cell-related indexes (continuous decrease in RBC and HGB level), and abnormal coagulation function (pale gums/cheeks, gingival hemorrhage, erythema on the skin, decreased PLT, and undetectable values of coagulation/biochemical indexes in dead/dying animals; increased Retic in other animals, were observed). Highest Non-Severely Toxic Dose (HNSTD) was at 15 mg/kg (intravenous infusion QW).

After the first administration, the peak drug concentrations (Cₘₐₓ) of conjugate A and the total antibody of conjugate A and drug exposure (AUC)ₗₐₛₜ in the serum of the animals in the low-dose group and the high-dose group wereincreased with increasing dose. In the groups in which Conjugate A was administered by subcutaneous injection at 30 mg/kg, and the bioavailability (AUC _{last, subcutaneous group}/ AUC_{last, intravenous group}) of conjugate A in male and female animals after the first administration were 69% and 98%, respectively, and the bioavailability of the total antibody of conjugate A in male and female animals were 60% and 89%, respectively. After the repeated administrations, the plasma concentration decreased relatively rapidly, possibly due to the generation of anti-drug antibodies. The calculated parameters and ratios were for reference only. The peak drug concentration (Cₘₐₓ) and drug exposure (AUCₗₐₛₜ) of conjugate A in the animal after the first administration was substantially consistent with the total antibody of conjugate A.

The toxicokinetic parameters of conjugate A and conjugate A total antibody in each group of animals are as follows:

**Table 20. Toxicokinetic parameters of conjugate A and conjugate A total antibody**

| **Analyte** | **Day** | **Group Dose** | **Cₘₐₓ (µg/mL)** | **AUCₗₐₛₜ* (h·mg/mL)** |
|---|---|---|---|---|
| Conjugate A | D1 | 10 mg/kg, i.v | 262.00 | 11.91 |
| | D1 | 30 mg/kg, i.v | 804.00 | 42.20 |
| | D15 | 10 mg/kg, i.v | 174.50 | 2.46 |
| | D15 | 30 mg/kg, i.v | 318.00 | 2.06 |
| Conjugate A total antibody | D1 | 10 mg/kg, i.v | 278.00 | 13.20 |
| | D1 | 30 mg/kg, i.v | 828.50 | 45.70 |
| | D15 | 10 mg/kg, i.v | 180.50 | 3.73 |
| | D15 | 30 mg/kg, i.v | 329.50 | 2.67 |
| Conjugate A | D1 | 15 mg/kg, i.v | 392.00 | 19.85 |
| | D15 | 15 mg/kg, i.v | 164.50 | 0.75 |
| Conjugate A total antibody | D1 | 15 mg/kg, i.v | 395.50 | 20.35 |
| | D15 | 15 mg/kg, i.v | 173.50 | 1.60 |
| Conjugate A | D1 | 30 mg/kg, s.c. | 381.50 | 34.85 |
| | D15 | 30 mg/kg, s.c. | 97.25 | 3.05 |
| Conjugate A total antibody | D1 | 30 mg/kg, s.c. | 364.00 | 33.70 |
| | D15 | 30 mg/kg, s.c. | 118.35 | 3.68 |

| | | | | |
|---|---|---|---|---|
| Note: AUCₗₐₛₜ* at 15 mg/kg was based on the statistics collected from 0-168 h, and those of 10 mg/kg and 30 mg/kg were based on the statistics collected from 0-336 h. | | | | |

### Example 11. Therapeutic Effect of TROP2-ISAC in CT26-hTROP2 Model

In this experiment, BALB/C mice were inoculated with CT26-hTROP2 cells to determine the anti-tumor effect of TROP2-ISAC of the present invention.

### BALB/C mice:

SPF female BALB/C mice, purchased from Zhejiang Vital River Laboratory Animal Technology Co., Ltd., qualified by Beijing Vital River Laboratory Animal Technology Co., Ltd., with certificate No. 20230510Abzz0619000585. Before the study, the mice were adapted for 3 days after arrival.

### Cells:

CT26-hTROP2 cells were constructed by Innovent Biologics and routinely subcultured for subsequent *in vivo* experiments. The cells were collected by centrifugation and resuspended in sterile PBS, with the cell density adjusted to 5×10⁶cells/mL. On day 0, 0.2 mL of the cell suspension was subcutaneously inoculated into the right abdominal region of the BALB/C mice to establish CT26-hTROP2 tumor-bearing mouse models.

### Administration:

The tumor volume of each mouse was measured 8 days after tumor cell inoculation, and tumors with a volume of about 100 mm³ were selected. The mice were evenly divided into groups by tumor volume (7 mice per group). The dosages and routes of administration are shown in Table 21. h-IgG (purchased from EQUITECH-BIO) was used as a negative control. Administration was performed on days 8, 11, 15, and 18 after inoculation. The tumor volume and body weight of the mice were monitored twice a week. The body weight and tumor volume were measured before each administration. On day 22 after inoculation, the relative tumor growth inhibition (TGI%) was calculated by the following formula: TGI% = 100% × (control group tumor volume - treatment group tumor volume)/(control group tumor volume - control group tumor volume before administration).Tumor volume measurement: The maximum length of major axis (L) and maximum length of minor axis (W) of tumors were measured with a vernier caliper, and tumor volume was calculated using the following formula: V = L×W²/2.The mice were weighted using an electronic balance.

**Table 21. Experimental Design**

| Group | Dose of administration | Administration frequency | Mode of administration |
|---|---|---|---|
| h-IgG | 10 mg/kg | Q3-4Dx4 | Intravenous injection |
| hz1D9B10 | 10 mg/kg | Q3-4Dx4 | Intravenous injection |
| hz1D9B10-compound6 | 10 mg/kg | Q3-4Dx4 | Intravenous injection |

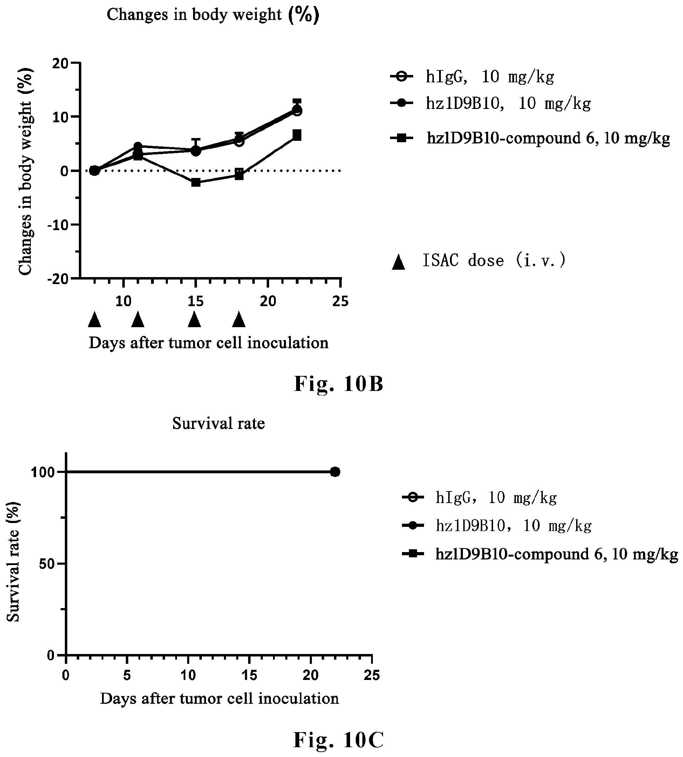

The efficacy results are shown in FIG. 10A and Table 22. On day 22 after inoculation, the TGI of hz1D9B10-compound 6 was 52%. Meanwhile, the body weight of the mice was monitored. As shown in FIG. 10B, there was no significant difference in body weights of the mice. The survival rate results of the mice are shown in FIG. 10C, hz1D9B10-compound6 caused no mortality to the mice. Therefore, in the present invention, the TROP2-ISAC as a medicament has a significant inhibitory effect on tumors, and the mice are in good general condition without being affected by significant side effects.

**Table 22. Tumor growth inhibition on day 22**

| Group | Tumor volume (mm³) | TGI(%) |
|---|---|---|
| h-IgG | 1171 | N/A |
| hz1D9B10 | 1490 | N/A |
| hz1D9B10-compound6 | 618 | 52 |

### Example 12. Therapeutic Effect of TROP2-ISAC in Combination with TROP2-ADC in BxPC3 Model

In this experiment, CB17-SCID mice were inoculated with BxPC3 cells to determine the anti-tumor effect of TROP2-ISAC in combination with TROP2-ADC.

### CB17-SCID mice:

SPF female CB17-SCID mice, purchased from Beijing Vital River Laboratory Animal Technology Co., Ltd., qualified by Beijing Vital River Laboratory Animal Technology Co., Ltd., with certificate No. 110011230103807533. Before the study, the mice were adapted for 3 days after arrival.

### Cells:

BxPC3 cells were purchased from Cell Bank, Chinese Academy of Sciences (CAT#: TCHu012), and were routinely subcultured strictly according to the instructions for subsequent in vivo experiments. The cells were collected by centrifugation and resuspended in sterile PBS, with the cell density adjusted to 1.5×10⁷cells/mL. On day 0, 0.2 mL of the cell suspension was subcutaneously inoculated into the right abdominal region of the CB17-SCID mice to establish BxPC3 tumor-bearing mouse models.

### Administration:

The tumor volume of each mouse was measured 13 days after the tumor cell inoculation, and tumors with a volume of about 100 mm³ were selected. The mice were evenly divided into groups by tumor volume (7 mice per group). The dosages and routes of administration are shown in Table 23. Administration was performed on day 13 after inoculation, and the tumor volume and body weight of the mice were monitored twice a week. The body weight and tumor volume were measured before each administration. On day 59 after inoculation, the relative tumor growth inhibition (TGI%) was calculated by the following formula: TGI% = 100% × (control group tumor volume - treatment group tumor volume)/(control group tumor volume - control group tumor volume before administration).Tumor volume measurement: The maximum length of major axis (L) and maximum length of minor axis (W) of tumors were measured with a vernier caliper, and tumor volume was calculated using the following formula: V = L×W²/2.The mice were weighted using an electronic balance.

**Table 23. Experimental Design**

| Group | Dose of administration | Administration frequency | Mode of administration |
|---|---|---|---|
| IgG-ISAC+IgG-ADC | 1+5 mg/kg | Single dose | Intravenous injection |
| hz1D9B10-compound6 | 1 mg/kg | Single dose | Intravenous injection |
| TROP2 ADC | 1 mg/kg | Single dose | Intravenous injection |
| TROP2 ADC | 5 mg/kg | Single dose | Intravenous injection |
| hz1D9B10-compound6+ TROP2 ADC | 1+1 mg/kg | Single dose | Intravenous injection |
| hz1D9B10-compound6+ TROP2 ADC | 1+5 mg/kg | Single dose | Intravenous injection |

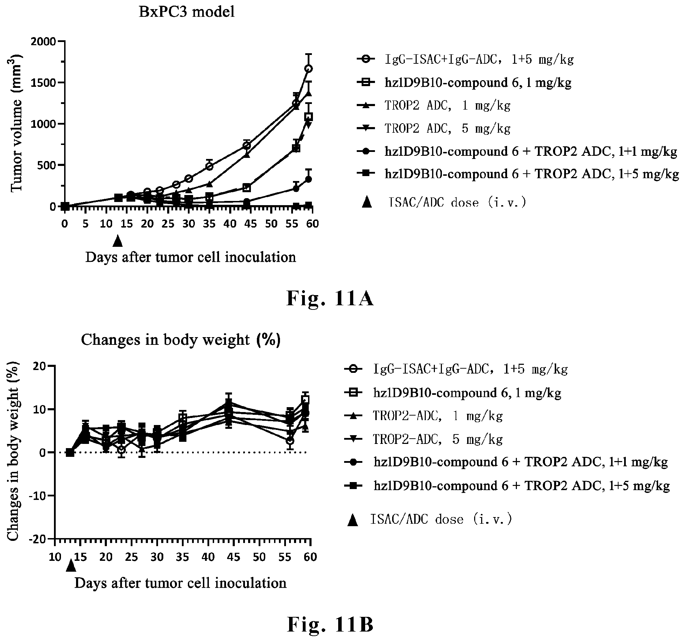

The efficacy results are shown in FIG. 11A and Table 24: on day 59 after inoculation, hz1D9B10-compound 6 /TROP2 ADC (1 mg/kg) /TROP2 ADC (5 mg/kg) /hz1D9B10-Compound 6 + TROP2 ADC (1+1 mg/kg) / hz1D9B10-Compound 6 + TROP2 ADC ( 1+5 mg/kg) realize tumor inhibition rates of 37% / 19% / 44% / 86% / 106%, respectively. The combination hz1D9B10-Compound 6 + TROP2 ADC has an efficacy stronger than that of the single-drug group of hz1D9B10-Compound 6 or TROP2 ADC . Meanwhile, the body weights of the mice were measured. As shown in FIG. 11B, there was no significant difference in body weight of the mice. Therefore, TROP2-ISAC in combination with TROP2-ADC has a significant synergistic inhibitory effect on BxPC3 tumors, and has no significant toxic and side effects on mice.

Wherein, TROP2-ADC is the hRS7-NT3 prepared in Example 4.8 above.

**Table 24. Tumor growth inhibition on day 59**

| Group | Tumor volume (mm³) | TGI(%) |
|---|---|---|
| IgG-ISAC+IgG-ADC, 1+5 mg/kg | 1668 | / |
| hz1D9B10-compound6, 1 mg/kg | 1083 | 37 |
| TROP2 ADC, 1 mg/kg | 1376 | 19 |
| TROP2 ADC, 5 mg/kg | 976 | 44 |
| hz1D9B10-compound6+ TROP2 ADC, 1+1 mg/kg | 330 | 86 |
| hz1D9B10-compound6+ TROP2 ADC, 1+5 mg/kg | 13 | 106 |

### Example 13. Therapeutic Effect of TROP2-ISAC in Combination with TROP2-ADC in LK-2 Model

In this experiment, CB17-SCID mice were inoculated with LK-2 cells to determine the anti-tumor effect of TROP2-ISAC in combination with TROP2-ADC.

### CB17-SCID mice:

Female CB17-SCID mice were purchased from Beijing Vital River Laboratory Animal Technology Co., Ltd., SPF grade, qualified by Beijing Vital River Laboratory Animal Technology Co., Ltd., with certificate No. 110011231104065475.Before the study, the mice were adapted for 3 days after arrival.

### Cells:

LK-2 cells were purchased from Nanjing Cobioer (CAT#: CBP60105) and routinely subcultured strictly according to the instructions for subsequent in vivo experiments.The cells were collected by centrifugation and resuspended in sterile PBS, with the cell density adjusted to 1.5×10⁷cells/mL. On day 0, 0.2 mL of the cell suspension was subcutaneously inoculated into the right abdominal region of each of the CB17-SCID mice to establish LK-2 tumor-bearing mouse models.

### Administration:

The tumor volume of each mouse was measured 9 days after the tumor cell inoculation, and tumors with a volume of about 120 mm³ were selected. The left and right mice were evenly divided into groups by tumor volume (7 mice per group). The dosages and routes of administration are shown in Table 25. h-IgG (purchased from EQUITECH-BIO) was used as a negative control. Administration was performed on days 9, 19 and 29 after inoculation. The tumor volume and body weight of the mice were monitored twice a week. The body weight and tumor volume were measured before each administration. On day 36 after inoculation, the relative tumor growth inhibition (TGI%) was calculated by the following formula: TGI% = 100% × (control group tumor volume - treatment group tumor volume)/(control group tumor volume - control group tumor volume before administration).Tumor volume measurement: The maximum length of major axis (L) and maximum length of minor axis (W) of tumors were measured with a vernier caliper, and tumor volume was calculated using the following formula: V = L×W²/2.The mice were weighted using an electronic balance.

**Table 25. Experimental Design**

| Group | Dose of administration | Administration frequency | Mode of administration |
|---|---|---|---|
| IgG | 10 mg/kg | QWx3 | Intravenous injection |
| hz1D9B10-compound6 | 10 mg/kg | QWx3 | Intravenous injection |
| TROP2 ADC | 10 mg/kg | QWx3 | Intravenous injection |
| hz1D9B10-compound6+ TROP2 ADC | 10+10 mg/kg | QWx3 | Intravenous injection |

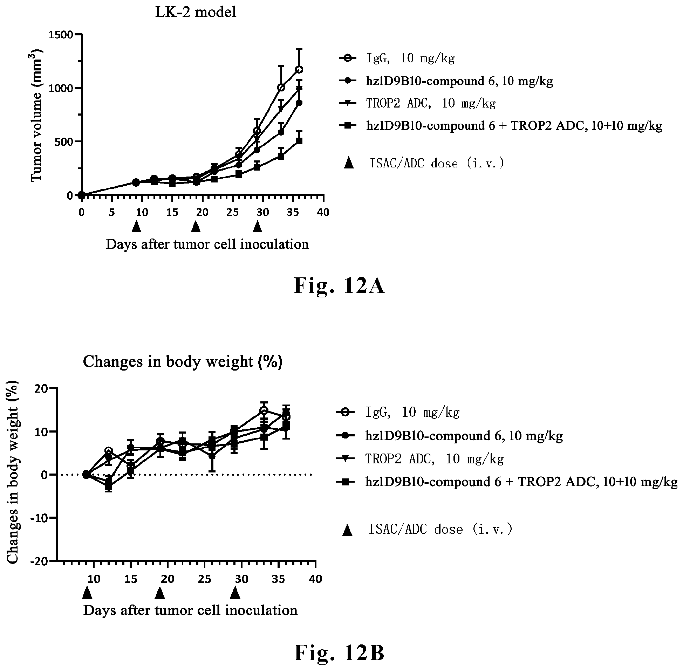

The efficacy results are shown in FIG. 12A and Table 26: on day 36 after inoculation, hz1D9B10-Compound 6, TROP2 ADC and hz1D9B10-Compound 6 +TROP2 ADC realize tumor growth inhibition rates of 29%, 17%, and 63%, respectively. The efficacy of the combination group was stronger than that of the single-drug groups. Meanwhile, the body weights of the mice were measured. As shown in FIG. 12B, there was no significant difference in body weight of the mice. Therefore, TROP2-ISAC in combination with TROP2-ADC has a significant synergistic inhibitory effect on LK-2 tumors, and has no significant toxic or side effects on mice.
wherein TROP2-ADC is the hRS7-NT3 prepared in Example 4.8 above.

**Table 26. Tumor growth inhibition on day 36**

| Group | Tumor volume (mm³) | TGI(%) |
|---|---|---|
| IgG, 10 mg/kg | 1172 | / |
| hz1D9B10-compound6, 10 mg/kg | 861 | 29 |
| TROP2 ADC, 10 mg/kg | 988 | 17 |
| hz1D9B10-compound6+ TROP2 ADC, 10+10 mg/kg | 505 | 63 |

### Example 14. High Dose Toxicity Studyof TROP2-ISAC inBALB/C Mice

In this experiment, BALB/C mice were used. The body temperature changes and deaths of the mice were observed after intravenous injection of TROP2-ISAC twice at an interval of 7 days, and the toxicity of the drug was determined.

### BALB/C mice:

SPF female BALB/C mice purchased from Beijing Vital River Laboratory Animal Technology Co., Ltd., qualified by Beijing Vital River Laboratory Animal Technology Co., Ltd., with certificate No. 110011221111661541. Before the study, the mice were adapted for 3 days after arrival.

### Administration:

According to the experimental design shown in Table 27, the mice were grouped and weighed, with 5 mice in each group. h-IgG (purchased from EQUITECH-BIO) was used as a negative control, and the TROP2-ISAC drug was injected intravenously on days 0 and 7. Then the initial rectal temperature of the mice was measured before the administration on day 7, and the rectal temperature of the mice was measured 30 min\60 min after the administration.

**Table 27. Experimental Design**

| Group | Dose of administration | Administration frequency | Mode of administration |
|---|---|---|---|
| h-IgG | 5 mg/kg | QWx2 | Intravenous injection |
| hz1D9B10-compound6 | 5 mg/kg | QWx2 | Intravenous injection |
| hz1D9B10-compound6 | 15 mg/kg | QWx2 | Intravenous injection |
| hz1D9B10-compound6 | 45 mg/kg | QWx2 | Intravenous injection |

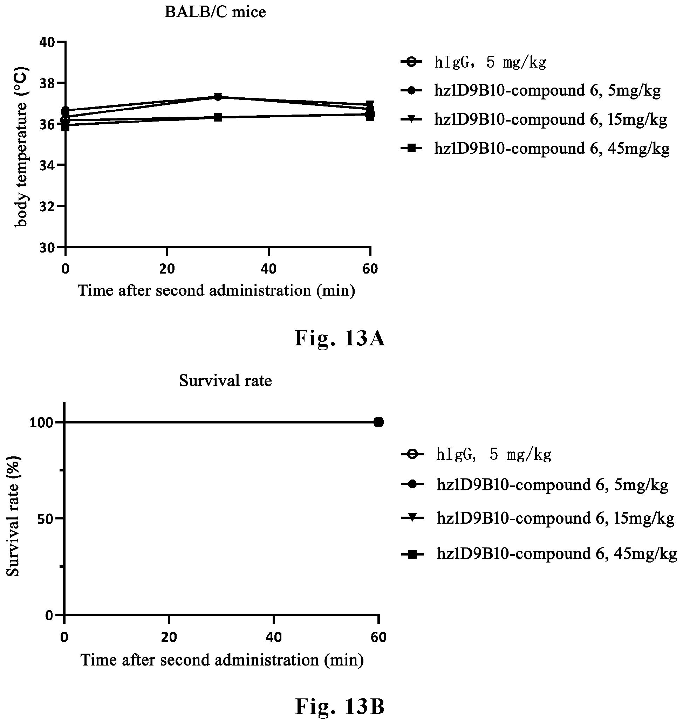

The rectal temperature results are shown in FIG. 13A and Table 28: after the administration on day 7, the rectal temperatures of the groups of 5 mg/kg hz1D9B10-compound 6, 15 mg/kg hz1D9B10-compound 6, and 45 mg/kg hz1D9B10-compound 6 were found not decreased compared with those before the administration. Meanwhile, the survival rates of the mice was counted, and the results are shown in FIG. 13B. Administration of various doses of hz1D9B10-compound 6 caused no mortality to the mice. Therefore, the high dose of hz1D9B10-compound6 TROP2-ISAC at an interval of 7 days brings no toxic effect on BALB/C mice.

**Table 28. Rectal temperature of mice before administration on day 7 and after administration**

| Group | Pre-dose | 30 min | 60 min |
|---|---|---|---|
| h-IgG | 36.18 | 36.54 | 36.46 |
| hz1D9B10-compound6 | 36.66 | 37.32 | 36.74 |
| hz1D9B10-compound6 | 36.34 | 37.30 | 36.93 |
| hz1D9B10-compound6 | 35.94 | 36.32 | 36.46 |

### Example 15. Preparation of Anti-FRα Antibodies Using Display Library

Experimental strategy: Based on the phage display technology, human FOLR1 (FRα) was used to perform two rounds of panning from the proprietory human naïve scFv library of Innovent Biologics, and then the panned library was subjected to plasmid extraction.The library fragments were transferred to a yeast system by PCR to further obtain highly functional molecules.

### 15.1 Phage display antibody panning

Frozen human naïve scFv library recombinant phages were taken out and dissolved at room temperature. The library was synthesized into 6 sublibraries depending on the type of IgM/IgG antibody displayed. PBST (PBS + 0.05% Tween 20) was added to 4% BSA for filtration and sterilization to prepare a blocking solution. 2 × 10¹² of recombinant phages were shaken and blocked at room temperature for 1 h, and Dynabeads^{™} M-280 (Invitrogen) were taken out and also shaken and blocked at room temperature for 1 h. The blocked magnetic beads were added to the blocked recombinant phage, and the mixture was shaken at room temperature for 1 h to achieve the purpose of negative screening. The centrifuge tube was placed on a magnetic rack to adsorb the magnetic beads, the recombinant phages were transferred to a new centrifuge tube by pipetting, and 30 pmol of biotinylated Human FOLR1 (Acro Biosystems) was added for binding by shaking at room temperature for 1.5 h.The blocked magnetic beads were added to the antigen-recombinant phage complex for adsorption, and the mixture was shaken at room temperature for 0.5 h and washed 8 times with PBST buffer. The magnetic beads were collected, 0.5 mL of glycine eluent was added, and the mixture was shaken at room temperature for 10 min and placed on a magnetic rack to adsorb the magnetic beads. The supernatant was removed, and Tris was added for pH neutralization. After TG1 competent cells were infected, 100 µL of the diluted solution was pipetted and plated to determine the size of the library. The excessive medium was removed by centrifugation, and the plate was coated on a 2× YT (2% glucose) large plate for culturing at 30 °C overnight for preparing recombinant phages by library scraping.

### 15.2 Preparation of recombinant phages

The output of the one round of screening was scraped off the culture plate, and a part of the bacterial solution was put into a 2× TY (2% glucose) liquid culture plate to give an OD600 of 0.1, and cultured at 37 °C and 220 rpm to give an OD600 of 0.5. An appropriate amount of helper phage M13K07 (PROGEN) was added for infection for 1 h. The bacteria were collected by centrifugation, resuspended in a 2× TY (2% glucose) medium, and cultured overnight at 30 °C. The bacterial solution cultured overnight was centrifuged to remove the precipitate. 1/4 volumes of PEG/NaCl were added to the supernatant, and the mixture was left to stand on ice for 1 h and centrifuged for 20 min. The supernatant was removed, and the precipitate was retained. PBS was added for resuspension, and the phage titer was determined. The preparation of recombinant phages was completed.

Two rounds of phage panning were performed using human FOLR1, and the library could be enriched to about 10⁹. 570 monoclones were picked for ELISA verification of the binding ability to FOLR1 and cyno FOLR1 showed that the level of the cross binder reached 48%, so that the next step of phage to yeast could be performed. About 8 × 10⁸ bacterial liquid of the library scraper in the second round was collected. The E. coli cells were subjected to plasmid extraction, and the concentration of the plasmids was determined using a NanoDrop^{™} 2000. The plasmid was cryopreserved for subsequent yeast library construction.

### 15.3 Yeast display library construction

Based on human naïve scFv library, hybrid primers were designed according to the antibody sequences in the, and scFv fragments of the IgM/IgG antibody library were amplified and displayed using a library extraction plasmid panned by the phage display technology as a template. The yeast display plasmid pYDC011 was used as a template to amplify upstream and downstream homology arms with a length of about 150 bp. The fragment was amplified with a total length of about 1200 bp using the overlap PCR technique, and the plasmid pYDC011 was treated with a restriction endonuclease BamH I to obtain a linearized vector. The full-length fragment and the linearized vector were simultaneously mixed and transformed into Saccharomyces cerevisiae EBY100 (purchased from ATCC) using an electroporator (BioRad) to obtain a transformed Saccharomyces cerevisiae yeast, which was transferred into a corresponding liquid defect medium for overnight culture. Meanwhile, the mixture was subjected to gradient dilution and coating on a defective medium plate for library size identification, and sent for sequencing for library quality QC. The successfully transformed flora was cultured and screened overnight for library screening.

### 15.4 Yeast Display Library Induction

The yeast library cultured overnight was centrifuged, and the bacterial cells were collected, resuspended in a pre-cooled PBSA (PBS + 0.1% BSA) buffer, centrifuged, and washed twice. The collected bacterial cells were transferred to an appropriate volume of YPGP induction medium (2% galactose, 2% peptone, 1% yeast extract, 0.54% Na₂HPO₄ ,0.86% NaH₂PO₄.H₂O) After inducing overnight at 20 °C for 72 h, the scFv antibody fragments of the library were displayed on the surface of the yeast for subsequent staining and screening of the library.

### 15.5 Yeast display library screening

The yeast library was sorted using a flow cytometer (BD): about 1x10⁸ yeast cells were washed three times with PBSA buffer and incubated in the mixture containing biotin-labeled human FOLR1 antigen and mouse anti Flag M2 (sigma) with shaking at room temperature for 1 h.The medium was discarded, and the cells were eluted twice with PBSA buffer, mixed with SA-PE (Thermo Fisher) and Goat Anti Mouse-647 (Thermo Fisher) reagents, and incubated at room temperature for 20 min while shaking.The cells were eluted twice with pre-cooled PBSA buffer and resuspended in an appropriate volume of PBSA buffer. The cells were transferred to a flow cytometry tube after passing through a 40 µm sieve, and the positive flora was collected by BD FACSAria^{™}. The positive yeast cells were sorted and transferred to a liquid defect medium for culturing overnight.

### 15.6 Clone identification for yeast display library screening

The bacterial flora enriched three times by FACS sorting was applied to a defective culture medium plate and cultured at 30 °C for 72 h until monoclones were generated. A proper amount of monoclones were selected and inoculated into a liquid defective culture medium for activation and amplification. A part of the bacterial liquid was sent for sequencing, and the remaining bacterial liquid was centrifuged to discard the supernatant and transferred to YPGP induction culture for 72 h. The cells were incubated at room temperature for 1 h while shaking using a low-concentration biotin-labeled human FOLR1 antigen and mouse anti Flag M2 (sigma).The medium was discarded, and the cells were eluted twice with PBSA buffer, mixed with SA-PE (Thermo Fisher) and Goat Anti Mouse-647 (Thermo Fisher) reagents, and incubated at room temperature for 20 min while shaking. The cells were eluted twice with pre-cooled PBSA buffer and resuspended in an appropriate volume of PBSA buffer. The cells were sieved through a 40-µm sieve and then transferred to a flow cytometry tube. FACS was used to perform monoclonal signal analysis. The PE signal represents the intensity of binding to human FOLR1 antigen.

### 15.7 Yeast sequencing and protein expression

Single clones of yeast with relatively high signals were selected and sent to Genewiz for sequencing, followed by sequence analysis. The DNA sequences of the potential molecules were amplified into the eukaryotic expression vector pcDNA3.1 and linked to the light chain constant region-encoding gene and the IgG1 heavy chain constant region-encoding gene.The successfully constructed plasmid can be transfected into eukaryotic cells for antibody expression to obtain the full-length antibody.BC1254-A1.

### Example 16. Cysteine mutation engineering and expression of anti-FRα antibody

In order to obtain more stable and potent immunoconjugate molecules, cysteine mutation engineering was performed based on BC1254-A1 in Example 15. Two light chain constant regions of the BC1254-A1 antibody were engineered to a lambda light chain constant region carrying mutations at amino acid positions 160 and 166 (both according to EU numbering) to cysteine.

The main sequences of antibody BC1254-A1 and engineered antibody BC1254-A1-V2 are as follows:

### Antibody sequence information:

| **Clone No.** | **Sequence name** | **Sequence No.** |
|---|---|---|
| BC1254-A1 | HCDR1 | SEQ ID NO:27 |
| | HCDR2 | SEQ ID NO:28 |
| | HCDR3 | SEQ ID NO:29 |
| | LCDR1 | SEQ ID NO:30 |
| | LCDR2 | SEQ ID NO:31 |
| | LCDR3 | SEQ ID NO:32 |
| | VH | SEQ ID NO:33 |
| | VL | SEQ ID NO:34 |
| | Heavy Chain | SEQ ID NO:35 |
| | Light Chain | SEQ ID NO:36 |
| BC1254-A1 V2 | HCDR1 | SEQ ID NO:27 |
| | HCDR2 | SEQ ID NO:28 |
| | HCDR3 | SEQ ID NO:29 |
| | LCDR1 | SEQ ID NO:30 |
| | LCDR2 | SEQ ID NO:31 |
| | LCDR3 | SEQ ID NO:32 |
| | VH | SEQ ID NO:33 |
| | VL | SEQ ID NO:34 |
| | Heavy Chain | SEQ ID NO:35 |
| | Light Chain | SEQ ID NO:37 |

**16.1 Plasmid construction:** the sequences were synthesized by Genewiz and loaded onto a pTT5 vector.

**16.2 Protein expression:** The ExpiCHOTM Expression system (Gibco, A29133) was used to produce proteins. Specifically, ExpiCHO-STM cells (Gibco) were passaged according to a desired transfection volume. The cell density was adjusted to 3.5 × 10⁶ cells/ml one day before transfection.The cell density was adjusted to 6×10⁶ cells/ml on the day of transfection. A transfection buffer reagent OptiPROTM SFM (Gibco, 12309019) was added to a 50 mL centrifuge tube at 8% of the volume of transfected cells, and the total amount of plasmids required was calculated according to 0.8 µg/mL transfected cells, wherein the mass ratio of the light chain to the heavy chain plasmid was 1:1. The transfection buffer containing DNA plasmids was filtered through a 0.22 µm filter membrane into another 50 mL centrifuge tube, and ExpiFectamineTM CHO reagent (Gibco, 100033022) was added to the filtered mixture at a ratio of DNA:Reagent = 1:4. The mixture was mixed well, and then slowly added to the cells immediately after mixing the transfection reagent with the plasmid DNA. The shake flask was gently shaken while the addition was performed, and the incubation time of the transfection reagent with the plasmid was controlled to be no more than 5 min. The mixture was cultured in a shaker at 37 °C with 8% CO₂. After 18-22 h, Enhancer (Gibco, 100033019) was added at 6 µL/mL, and Feed (Gibco, A29101-01) was added at 300 µL/mL. The mixture was incubated at 37 °C, 120 rpm, 8% CO₂The cells were cultured under the condition. On day 7 of continuous culture or when the cell viability was ≤ 60%, the cell feed liquid was collected.The cell feed liquid was mixed well with diatomaceous earth(Sartorius, Cat. No. 1000037025) (1 L of cell feed liquid was added with 40 g ofdiatomaceous earth), and the mixture was filtered using a 0.22 µm disposable vacuum filtration apparatus. The supernatant was used for the next affinity purification.

### 16.3 Purification of protein of interest by affinity chromatography

A HiTrap MabSelect PrismA (GE Healthcare, Cat# 17549853) affinity chromatography column was selected for affinity capture. Before purification, 10-20 column volumes of 0.1 M NaOH were passed through the pipeline and the affinity chromatography column, and then the pipeline and the column were washed with 10-20 column volumes of distilled water. The packing column was equilibrated with 5 column volumes of 1× PBS (Gibco). The filtered cell feed liquid was passed through the column, and then the packing column was washed with 10 column volumes of 1× PBS to remove non-specific binding proteins. The packing was washed with 5 column volumes of an elution buffer (100 mM sodium citrate, pH 3.5), and the eluate was collected, adjusted to pH 6.0 with 2 M Tris, and filtered to remove bacteria.

**16.4 In vitro reductive oxidation of antibodies:** An appropriate amount of GSH was added to the purified antibody, and the reaction system was adjusted to pH 8.0 with 2 M Tris.The reaction system was incubated at room temperature overnight. The reactant was buffer-exchanged into PBS and left to stand at 4 °C for later use.

**16.5 Purification of antibody by ion exchange chromatography:** A Mono S 5/50 GL ion exchange chromatography column (obtained from GE Healthcare) was selected and placed in an AKTApure system (obtained from GE healthcare).The AKTApure system equipped with a Mono S 5/50 GL ion exchange chromatography column was endotoxin-free with 0.5 M NaOH for 2 h, and then the system and the column were washed with distilled water.The column was equilibrated with 5-10 column volumes of loading buffer (20 mM NaPO4, pH 6.6) until the conductivity and pH were stable; the protein obtained by affinity chromatography was diluted 10-fold with the loading buffer before loading; the column was reequilibrated with 5 column volumes of loading buffer; the column was equilibrated with an elution buffer (20 mM NaPO₄, 1 M NaCl, pH 6.6) in a total of 30 column volumes, and the samples were collected according to the UV absorption value.

Collected samples in each fraction tube were analyzed for purity by size exclusion chromatography (SEC). According to the SEC results, the samples in the fraction tubes with a purity greater than 95% were combined.

The purified antibody solution was centrifuged using a 15 mL ultrafiltration centrifuge tube at 4500 rpm for 30 min. The protein was diluted with PBS and then centrifuged again at 4500 rpm for 30 min, and this operation was repeated several times to exchange the buffer. The antibodies after buffer exchange were combined, and the antibody concentration was measured.The composition and content of the antibody were further identified and quantified by a combination of capillary electrophoresis (CE-SDS) and liquid chromatography-mass spectrometry (LC-MS).

### Example 17. Affinity of Antibodies for Antigens as Determined by ForteBio

The binding of the anti-FRα antibodies of the present invention to human, monkey and mouse was determined by bio-layer interferometry (ForteBio).FRαequilibrium dissociation constant (KD).The ForteBio affinity assay was performed according to the prior art (Estep, P, et al., High throughput solution Based measurement of antibody-antigen affinity and epitope binning. MAbs, 2013.5(2): 270-8).

Briefly, an AHC (Sartorius, 18-5060) sensor was equilibrated offline in an assay buffer for 30 min, and was equilibrated online for 60 s to establish a baseline. The purified antibody obtained as described above was loaded online onto the AHC sensor (ForteBio) for the ForteBio affinity assay.The sensor with the loaded antibodies is then exposed to the antigen FRα before transferring the sensor to the assay buffer for dissociation rate measurement.The KD values were analyzed using ForteBio analysis software.The antigen sources were: human (Acro, Cat#FO1-H82E2), monkey (Acro,Cat#FO1-C52H8), mouse (Acro, Cat.#FO1-M5225) and rat (Sino, Cat# 81073-R08H).

The results of the antibody affinity assay are shown in Table 29. Antibody BC1254-A1 had a high affinity for antigen FRα, and the mutated antibody BC1254-A1 V2 maintained an equivalent equilibrium dissociation constant KD for the antigen.

**Table 29. Affinity constants (M) for bindings of antigens and antibodies by ForteBio assay**

| Clone ID | Human FRα | | | Cyno FRα | | | Mouse FRα | Rat FRα |
|---|---|---|---|---|---|---|---|---|
| | ka (1/Ms) | kd (1/s) | K_{D} (M) | ka (1/Ms) | kd (1/s) | K_{D} (M) | K_{D} (M) | K_{D} (M) |
| BC1254-A1 | 4.61E+05 | 7.20E -04 | 1.56E -09 | 4.34E+ 05 | 4.68 E-04 | 1.08E -10 | N D | N B |
| BC1254-A1 V2 | 3.62E+05 | 5.58E -04 | 1.54E -09 | N D | N D | N D | N B | N D |
| mirvetuximab | 7.40E+05 | 9.56E -04 | 1.29E -09 | 7.06E+ 05 | 7.09 E-04 | 1.00E -09 | N D | N B |
| FR57 | 3.38E+05 | 1.03E -03 | 3.03E -09 | 3.72E+ 05 | 5.24 E-04 | 1.41E -09 | N D | N B |

| | | | | | | | | |
|---|---|---|---|---|---|---|---|---|
| Note: N D indicates no data, N B indicates no binding. Mirvetuximab was a control antibody from ImmunoGen, and was antibody of FRα ADC Elahere^{®}. The sequences and sources are shown in U.S. Patent Application Publication No. US20200362029A1 (huMov19 (or M9346A)). FR57 is a control antibody from ImmunoGen, and its sequence and source are shown in U.S. Patent Application Publication No. US20200362029A1 | | | | | | | | |

### Example 18. Antibody-Dependent Cellular Phagocytosis (ADCP) Functional Assay

The ADCP (antibody-dependent cell-mediated phagocytosis) activity of the FRα antibodies was assayed using FcγRIIa-H reporter cells (G9871, Promega), which are engineered Jurkat T cells. After FcγRIIa on the cell surface is bound and activated, luciferase is released into the experimental system through the downstream signal NFAT (Nuclear Factor of Activated T), so that the degree of cell activation can be assayed.

The specific method is as follows: a 96-well white flat-bottom cell culture plate is used in which target cells FaDu (HTB-43, ATCC) or COLO 205 (TCHu102, Cell Bank of Chinese Academy of Sciences) or SK-BR-3 (HTB-30, ATCC) were added at 6×10⁴ cells/well, as well as effector cells FcγRIIa-H Cells being added at 3x10⁴ cells/well. Then, the corresponding concentration of FRα Monoclonal antibodies were added and the plate is incubated in an incubator at 37 °C for 6 h. The culture plate was then taken out, and Bio-Glo (G7940, Promega) was added for wavelength detection using a microplate reader (Spectra, Molecular Devices).

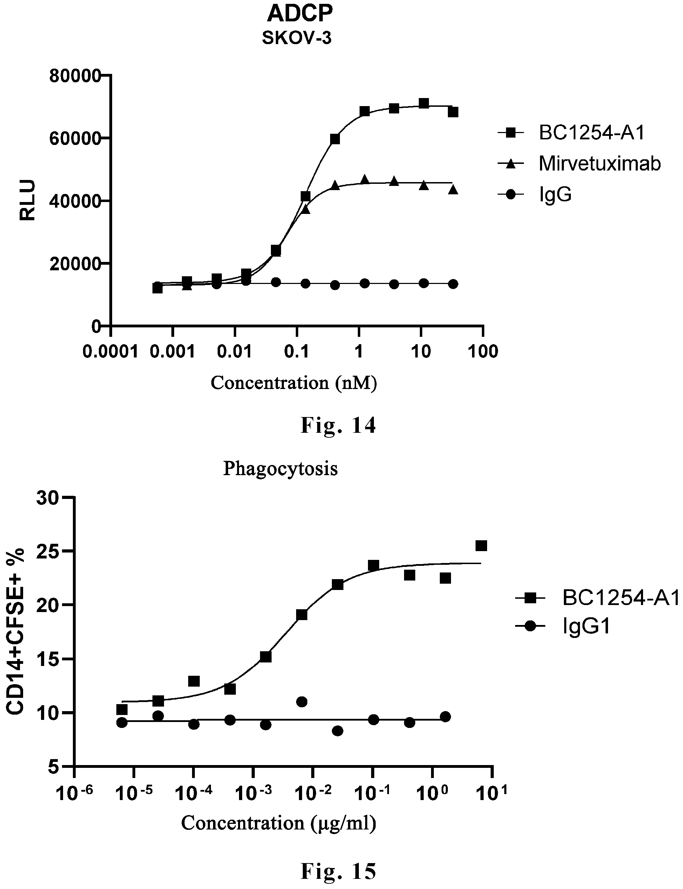

The results are shown in FIG. 14. The ADCP activity of clone BC1254-A1 was superior to that of mirvetuximab, suggesting its suitability for use in the preparation of ISAC(Immune-Stimulating Antibody Conjugate)..

### Example 19. Verification of Macrophage Endocytosis Function of Antibodies

The ADCP activity of anti-FRα antibody clones was verified using induced differentiated human M1-type macrophages. Macrophages and target cells were fluorescently labeled separately, and when ADCP of the two occurred via the antibody, double-positive cell populations could be observed.

Induced differentiation of M1 macrophage: Monocytes were isolated from fresh human peripheral blood mononuclear cells (PBMCs, TPCS) using a human monocyte enrichment kit without CD16 depletion (19058, STEMCELL) and resuspended in AIM V ^{®} Medium CTS (A3021002, Gibco) medium at a density of 1×10⁶ Cells/ml and placed in a cell culture flask and subjected to adherent culture at 37 °C for 4 h. Subsequently the medium was changed to AIM V^{®} Medium CTS (A3021002, Gibco) + 10% inactivated fetal bovine serum (FBS, SH30406.05, Hyclone) +25ng/ml human MCSF1(216-MC-500,R&D) for the culture. In the following seven days, AIM V ^{®} Medium CTS (A3021002, Gibco) + 10% inactivated fetal bovine serum (FBS, SH30406.05, Hyclone) was used for half medium exchange once every third day. After 7 days, media AIM V ^{®} Medium CTS (A3021002, Gibco) + 10% inactivated fetal bovine serum (FBS, SH30406.05, Hyclone) + 100 ng/mL IFNg (285-IF-100/CF, R&D) was used for half medium exchange, and the macrophage endocytosis experiment was performed the next day.

Macrophage endocytosis assay:The induced macrophages were digested with accutase (A6964-500ML, Sigma), and the cell density was adjusted using an RPMI-1640 complete medium (22400-071, Gibco) containing 10% fetal bovine serum (FBS, SH30406.05, Hyclone). The cells were added to a 96-well low-adsorption plate at 1×10⁵ cells per well. Target cells FaDu (HTB-43, ATCC) were labeled with CFSE (C34554, INVITROGEN) at 37 °C for 10 min, washed twice with RPMI-1640 complete medium (22400-071, Gibco) containing 10% fetal bovine serum (FBS, SH30406.05, Hyclone), and added to a 96-well low-adsorption plate at 2.5 × 10⁴ cells per well. The target cells and macrophages were mixed with gradiently diluted anti-TROP2 humanized antibodies, and were pipetted and mixed well, and cultured in a 37 °C incubator for 4 h.

Flow staining: Macrophages were labeled with CD14 PE/Cy7 (557742, BD) and then detected by flow cytometry, and cells in the PE/Cy7 and CFSE double-positive region were identified as the target cell population.

The results are shown in FIG. 15. The BC1254-A1 antibody can induce an ADCP effect on M1-type macrophages in the presence of SKOV-3.

### Example 20. Preparation of Conjugates (FRα ISAC and FRα ADC)

### 20.1 Preparation of BC1254-A1 V2-compound 6

The specific procedures are as follows:
(a) Antibody BC1254-A1 V2 was dissolved in a histidine buffer (20 mM, pH 6.5, Sigma, Lot# K5376054201).
(b) A reducing agent solution (TCEP, Aldrich, Catalog Number 646547, dissolved in water) was added, and the reaction mixture was left to react at room temperature for 2 h, wherein
   (i) the optimal concentration of the BC1254-A1 V2 antibody was 5-15 mg/mL;
   (ii) the optimal molar ratio of TCEP/mAb was 10-20, and
   (iii)the optimal temperature for the reaction was 25 °C;
   (iv)The optimal pH value for the reaction was 6.0-8.0.
(c) After the reduction is completed, the reducing agent is removed by desalting, ultrafiltration, or dialysis purification.
(d) The oxidant dehydroascorbic acid (dhAA, Aldrich, Catalog Number 261556, dissolved in DMSO) (DMSO, Sigma, Catalog Number 276855) was added, and the mixture was oxidized in a water bath for 2-3 h, wherein
   (i) the optimal concentration of the BC1254-A1 V2 antibody was 5-15 mg/mL;
   (ii) the optimal molar ratio of dhAA/mAb was 20-40, and
   (iii)the optimal temperature for the reaction was 20-37 °C;
   (iv)The optimal pH value for the reaction was 6.0-8.0.
(e) adding an excess amount of linker-toxin (compound 6, dissolved in DMSO) to react with the antibody in step (d) with a volume ratio of DMSO in the solution of 10%, and allowing the reaction mixture to stand at room temperature for 1-2 h, wherein
   (i) the optimal molar ratio of compound 6/mAb was 6-8, and
   (ii) The optimal temperature for the reaction was 20-37 °C.

The resulting crude ISAC product was purified by spin desalting, ultrafiltration, or dialysis to give the final ISAC product.

### 20.2. Preparation of BC1254-A1-compound 6

The specific procedures are as follows:
(a) Antibody BC1254-A1 was dissolved in PBS buffer.
(b) A reducing agent solution (TCEP, Aldrich, Catalog No. 646547, dissolved in water) was added, and the reaction mixture was left to react at room temperature for 2 h, wherein
   (i) the optimal concentration of BC1254-A1 antibody was 5-10 mg/mL, and
   (ii) The optimal molar ratio of TCEP/mAb was 2.0,
   (iii)The optimal temperature for the reaction was 25 °C.
   (iv)The optimum pH for the reaction was between 6.0 and 8.0.
(c) adding an excess of linker-toxin (compound 6, dissolved in DMSO) to react with the reduced antibody of step (b),

The reaction mixture was left at room temperature for 2 hours, wherein
(i) The optimal molar ratio of compound 6/mAb was 6.0,
(ii) The optimal temperature for the reaction was 25 °C.

The resulting crude ISAC product was purified by spin desalting, ultrafiltration, or dialysis to give the final ISAC product.

### 20.3 Preparation of FR57-DXd

The specific procedures are as follows:
(a) The antibody FR57 was dissolved in PBS buffer.
(b)A reducing agent solution (TCEP, Aldrich, Catalog No. 646547, dissolved in water) was added, and the reaction mixture was left to react at room temperature for 2 h, wherein
   (i) the optimal concentration of FR57 was 5-10 mg/mL, and
   (ii) The optimal molar ratio of TCEP/mAb was 10-20,
   (iii) The optimal temperature for the reaction was 37 °C.
   (iv) The optimal pH value for the reaction was between 6.0 and 8.0.
(c) An excess of MC-GGFG-DXd (MCE, HY-13631E, dissolved in DMSO) was added to react with the reduced antibody in step (b), and the reaction mixture was left to stand at room temperature for 2 h, wherein
   (i) The optimal molar ratio of MC-GGFG-DXd/mAb was 10-12.
   (ii) The optimal temperature for the reaction was 25 °C.

The resulting ADC crude product was purified by spin desalting, ultrafiltration, or dialysis to obtain the final ADC product.

### Example 21. DAR Analysis of FRα ISAC

BC1254-A1 V2-Compound 6 samples were reduced with DTT (Aladdin, Catalog Number D104860), and the reaction mixtures were allowed to react at 37 °C for 0.5 h.
(i) The final concentration of ISAC is 1 mg/mL,
(ii) The optimal molar ratio of DTT/mAb was 200-300,
(iii) The optimal temperature for the reaction was 25 °C.
(iv) The optimal pH value for the reaction was between 7.0 and 8.0.

The average DAR value of BC1254-A1 V2-Compound 6 was determined by RP-HPLC. The average DAR value was calculated from the peak area of each peak at UV 280 nm. Related parameters are shown in Table 30.

**Table 30**

| Reversed-phase high performance liquid chromatography RP-HPLC | | |
|---|---|---|
| Instrument | Waters, ACQuity H class UPLC | |
| Chromatography column | Waters, BioResolve RP mAb Polyphenyl, 450 Å, 2.7 µm, 2.1 mm × 15 cm | |
| Column temperature | 70°C | |
| Mobile phase | A: 0.1% TFA in H₂O | |
| | B: 0.1% TFA in ACN | |
| Flow rate | 0.6 mL/min | |
| Loading amount | 10 µg | |

| Detection wavelength | 280 nm, 324 nm | |
|---|---|---|
| Whether to use gradient | gradient | |
| Time | 15 min | |
| Time (min) | Mobile phase A% | Mobile phase B% |
| 0 | 70 | 30 |
| 1.5 | 70 | 30 |
| 11.5 | 50 | 50 |
| 12 | 10 | 90 |
| 13 | 10 | 90 |
| 14 | 70 | 30 |
| 15 | 70 | 30 |

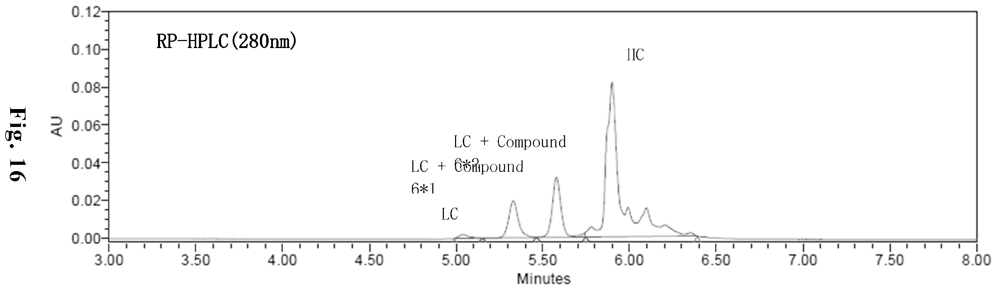

FIG. 16 represents the RP-HPLC profile used for calculation of average DAR values of BC1254-A1 V2-Compound 6, wherein LC represents the light chain component without the linking to compound 6, LC + compound 6*1 represents the light chain component conjugated with one compound 6, and LC + compound 6*2 represents the light chain component conjugated with two compounds 6. HC represents the heavy chain component without the linking to compound 6. The average DAR value of BC1254-A1 V2-Compound 6 was calculated to be 3.07.

The average DAR value of BC1254-A1-Compound 6 was determined by HIC-HPLC, and the average DAR value was calculated according to the peak area of each peak at UV 280 nm. Related parameters are shown in Table 31.

**Table 31**

| High performance liquid chromatography (hydrophobic interaction chromatography) | | |
|---|---|---|
| Instrument | Agilent 1260 | |
| Chromatography column | Tosh, TSKgel Butyl-NPR 4.6mm I.D. × 10cm | |
| Column temperature | 30°C | |
| Mobile phase | A: 1.5 M ammonium sulfate, 50 mM sodium phosphate, pH 7.0 | |
| | B: 50 mM sodium phosphate, pH 7.0 / isopropanol (80:20 v/v) | |
| Flow rate | 0.75 mL/min | |

| Loading amount | 30 µg | |
|---|---|---|
| Detection wavelength | 280 nm, 324 nm | |
| Whether to use gradient | gradient | |
| Time | 30 min | |
| Time (min) | Mobile phase A% | Mobile phase B% |
| 0 | 100 | 0 |
| 3 | 100 | 0 |
| 24 | 0 | 100 |
| 25 | 0 | 100 |
| 26 | 100 | 0 |
| 30 | 100 | 0 |

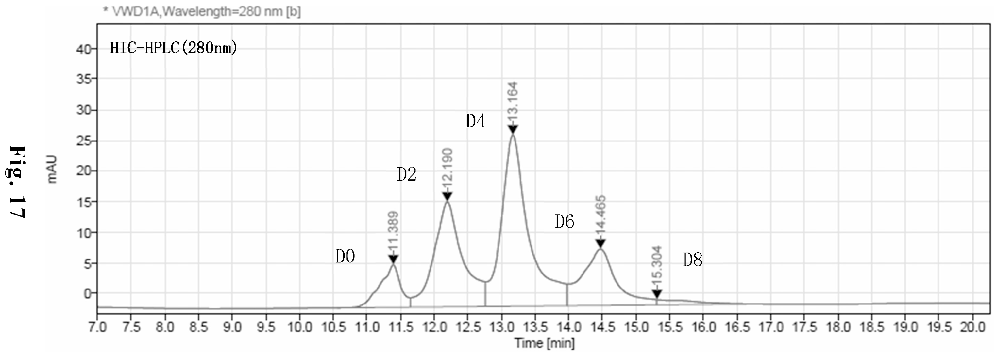

FIG. 17 shows HIC-HPLC profiles for calculation of average DAR values of BC1254-A1-compound 6, wherein D0 represents the antibody component without compound 6 linked thereto, D2 represents the antibody component conjugated to two compounds 6, D4 represents the antibody component conjugated to four compounds 6, D6 represents the antibody component conjugated to six compounds 6, and D8 represents the antibody component conjugated to eight compounds 6. The average DAR value of BC1254-A1-Compound 6 was calculated to be 3.54.

### Example 22. Antibody-Dependent Cellular Phagocytosis (ADCP) Functional Assays Before and After Antibody Engineering and Before and After Conjugation

The ADCP activity of the engineered FRα antibody was determined using FcγRIIa-H reporter cells, wherein the target cell was the cell line IGROV-1 (CTCC-009-0048, Meisen) highly expressing FRα, and the determination method was the same as in Example 18.

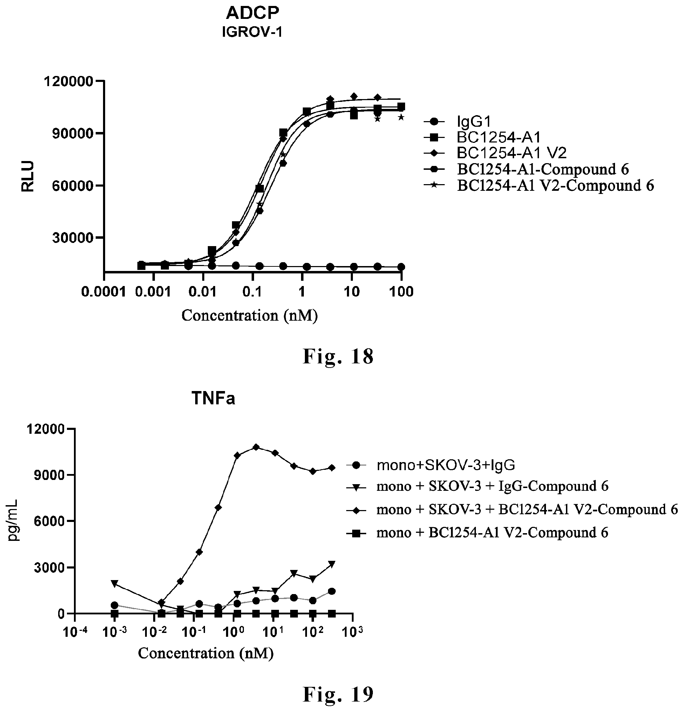

The results, as shown in FIG. 18, demonstrated that the ADCP activity of the engineered BC1254-A1 V2 antibody was comparable to that of the BC1254-A1 antibody before engineering, and the ADCP activity of the BC1254-A1 and BC1254-A1 V2 antibodies before and after conjugation was also comparable, indicating that the engineering of cysteine mutations and the conjugation process did not affect the ADCP function of the antibodies.

### Example 23. Activity of Antibody-Immunoagonist Conjugates (ISAC) in Co-Culture System of PBMCs and Tumor Cells

Monocytes were isolated from human peripheral blood mononuclear cells (PBMCs, TPCS) using a human monocyte enrichment kit without CD16 depletion (19058, STEMCELL). The cells were resuspended at a density of 1×10⁷ cells/mL in RPMI-1640 complete medium (22400-071, Gibco) containing 10% fetal bovine serum (FBS, SH30406.05, Hyclone) and 0.1% β-mercaptoethanol (21985023, Gibco) and added to a flat-bottom 96-well cell culture plate at 5×10⁵ cells per well. Target cells SKOV-3 (HTB-77, ATCC) were digested and the cell density was adjusted to 2 × 10⁶ Cells/mL, and added at 1×10⁵ cells/well, followed by addition of each respective concentration of FRα ISAC. The mixture was mixed well and then incubated in an incubator at 37 °C for 244 h. Then the cell culture plate was taken out, and IFNα and TNFα secretions in the cell-free supernatant were detected using ELISA method.

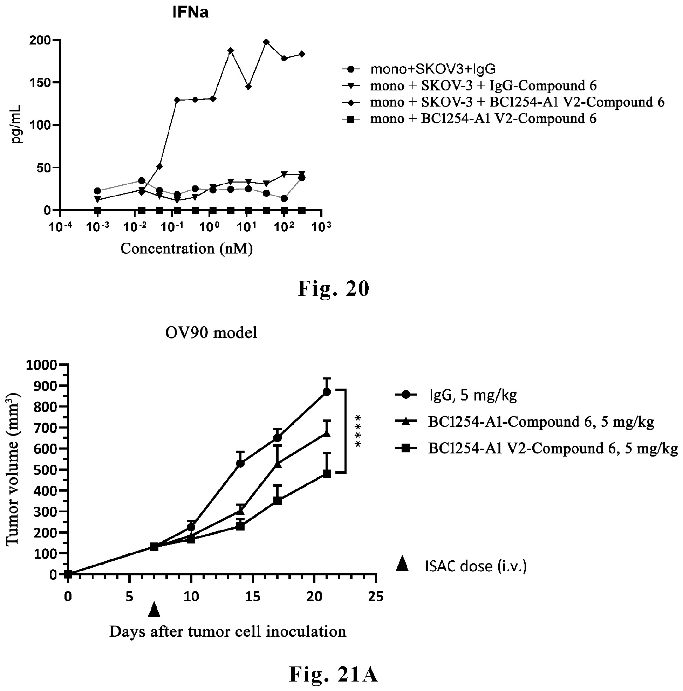

The results are shown in FIGs. 19 and 20. FRα ISAC can stimulate IFNα and TNFα secretion only when target cells SKOV-3 are present, demonstrating that FRα ISAC has antigen-dependent immunostimulatory effect.

### Example 24. Therapeutic Effect of FRα-ISAC in OV90 Model

In this experiment, CB17-SCID mice were inoculated with OV90 cells to determine the anti-tumor effect of FRα-ISAC of the present invention.

### CB17-SCID mice:

Female CB17-SCID Background Mice, SPF grade, purchased from Beijing Vital River Laboratory Animal Technology Co., Ltd., qualified by Beijing Vital River Laboratory Animal Technology Co., Ltd., with certificate No. NO110011241100336441. Before the study, the mice were adapted for 3 days after arrival.

### Cells:

OV90 cells, purchased from Zhejiang Meisen Cell Technology Co., Ltd. (CAT#: CTCC-001-0363) were subcultured in strict accordance with the instructions for subsequent in vivo study. The cells were collected by centrifugation and resuspended in sterile PBS, with the cell density adjusted to 1.5×10⁷ cells/mL. On day 0, 0.2 mL of the cell suspension was subcutaneously inoculated into the right abdominal region of the CB17-SCID mice to establish OV90 tumor-bearing mouse models.

### Administration:

The tumor volume of each mouse was measured 7 days after tumor cell inoculation, and the tumors with a volume of about 130 mm³ were selected. Then the mice were divided into groups by tumor volume (6 mice per group). The dosages and routes of administration are shown in Table 32. IgG (purchased from EQUITECH-BIO) was as a negative control, and the medicaments were administered on day 7 after inoculation, then the tumor volume and body weight of the mice were monitored twice a week. The body weight and tumor volume were measured before each administration. On day 21 after inoculation, the relative tumor growth inhibition (TGI%) was calculated by the following formula: TGI% = 100% × (control group tumor volume - treatment group tumor volume)/(control group tumor volume - control group tumor volume before administration).Tumor volume measurement: The maximum length of major axis (L) and maximum length of minor axis (W) of tumors were measured with a vernier caliper, and tumor volume was calculated using the following formula: V = L×W²/2. The mice were weighted using an electronic balance.

**Table 32. Experimental design**

| Group | Dose of administration | Administration frequency | Mode of administration |
|---|---|---|---|
| IgG | 5 mg/kg | Single dose | Intravenous injection |
| BC1254-A1-Compound 6 | 5 mg/kg | Single dose | Intravenous injection |
| BC1254-A1 V2-Compound 6 | 5 mg/kg | Single dose | Intravenous injection |

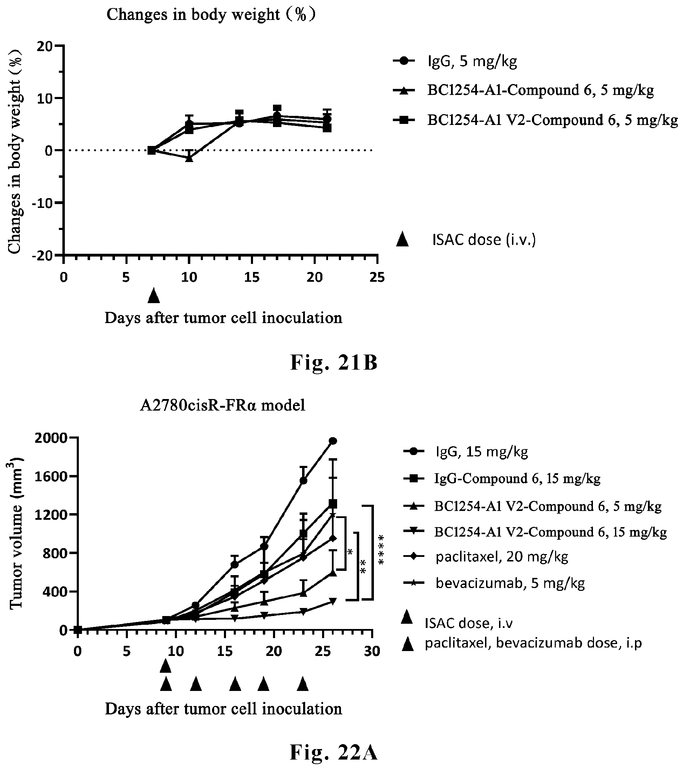

Pharmacodynamic results are shown in Figure 21A and Table 33: at day 21 post-inoculation, BC1254-A1-compound6 and BC1254-A1 V2-compound 6 realize TGIs of 27% and 53%, respectively. Meanwhile, the body weight of the mice was monitored. The results are shown in FIG. 21B. There was no significant difference in mouse body weight. Therefore, in the present invention BC1254-A1 V2-compound6 had a significant inhibitory effect on tumors, and the mice were in good general condition, indicating that side effects or off-target effects were not significant.

**Table 33 Tumor growth inhibition on day 21**

| Group | Tumor volume (mm³) | TGI(%) |
|---|---|---|
| IgG, 5 mg/kg | 870 | N/A |
| BC1254-A1-compound 6, 5 mg/kg | 673 | 27 |
| BC1254-A1 V2-compound 6, 5 mg/kg | 479 | 53 |

### Example 25. Therapeutic Effect of FRα-ISAC in A2780cisR-FRα Model

In this experiment, CB17-SCID mice were inoculated with A2780cisR-FRα cells to determine the anti-tumor effect of FRα-ISAC.

### CB17-SCID mice:

SPF female CB17-SCID mice, purchased from Beijing Vital River Laboratory Animal Technology Co., Ltd., qualified by Beijing Vital River Laboratory Animal Technology Co., Ltd., with certificate No. 20240318Abzz0619000270. Before the study, the mice were adapted for 3 days after arrival.

### Cells:

The A2780cisR-FRα cells were constructed by Innovent Biologics (constructed by introducing stable transfection of FRα into the parent cisplatin-resistant A2780-cisR ovarian cancer cells (CBP60283DR, Nanjing Cobioer) not expressing FRα) and routinely subcultured for subsequent in vivo experiments.The cells were collected by centrifugation and resuspended in sterile PBS, with the cell density adjusted to 2.5×10⁷cells/mL. On day 0, 0.2 mL of the cell suspension was subcutaneously inoculated into the right abdominal region of the CB17-SCID mice to establish A2780cisR-FRα tumor-bearing mouse models.

### Administration:

The tumor volume of each mouse was measured 9 days after tumor cell inoculation, and tumors with a volume of about 100 mm³ were selected. Then the mice were evenly divided into groups by tumor volume (6 mice per group, with 4 mice in the bevacizumab (Genentech) group). The dosages and routes of administration are shown in Table 34. IgG (purchased from EQUITECH-BIO) was used as a negative control. Administration was performed on day 9 after inoculation. The tumor volume and body weight of the mice were monitored twice a week. The body weight and tumor volume were measured before each administration. On day 26 after inoculation, the relative tumor growth inhibition (TGI%) was calculated by the following formula: TGI% = 100% × (control group tumor volume - treatment group tumor volume)/(control group tumor volume - control group tumor volume before administration).Tumor volume measurement: The maximum length of major axis (L) and maximum length of minor axis (W) of tumors were measured with a vernier caliper, and tumor volume was calculated using the following formula: V = L×W²/2.The mice were weighted using an electronic balance.

**Table 34 Experimental Design**

| Group | Dose of administration | Administration frequency | Mode of administration |
|---|---|---|---|
| IgG | 15 mg/kg | Single dose | Intravenous injection |
| IgG-Compound 6 | 15 mg/kg | Single dose | Intravenous injection |
| BC1254-A1 V2-Compound 6 | 5 mg/kg | Single dose | Intravenous injection |
| BC1254-A1 V2-Compound 6 | 15 mg/kg | Single dose | Intravenous injection |
| paclitaxel | 20 mg/kg | Q3-4Dx5 | Intraperitoneal injection |
| bevacizumab | 5 mg/kg | Q3-4Dx5 | Intraperitoneal injection |

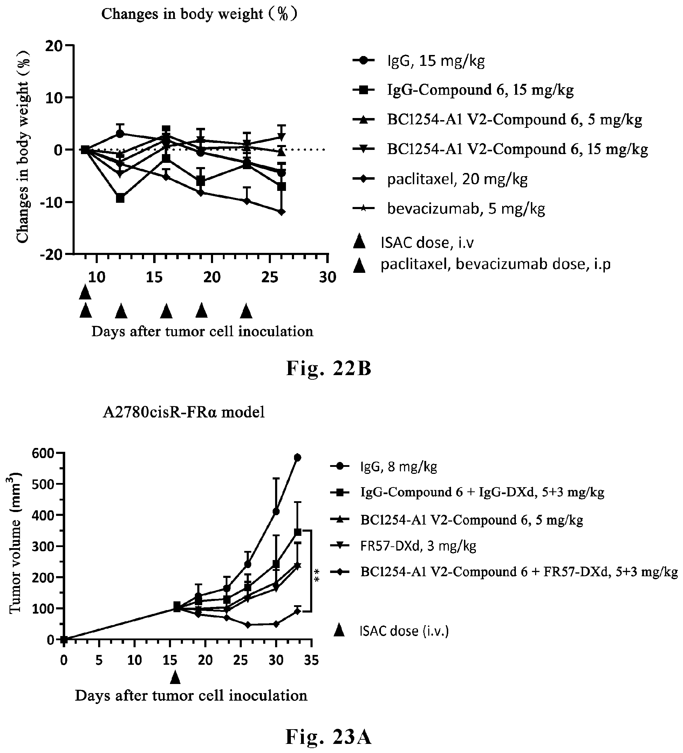

The efficacy results are shown in FIG. 22A and Table 35: on day 26 after inoculation, the groups of low-dose BC1254-A1 V2-compound 6, high-dose BC1254-A1 V2-compound 6, paclitaxel and bevacizumab exhibit TGIs of 74%, 90%, 63%, and 41%, respectively. BC1254-A1 V2-compound 6 group showed a stronger efficacy than the paclitaxel group and the bevacizumab group. Meanwhile, the body weight of the mice was measured. The results are shown in FIG. 22B, except the paclitaxel group, there was no significant difference in body weight of the mice.Thus, BC1254-A1 V2-Compound 6 as a medicament had a significant inhibitory effect on tumors, and the treated mice were all in good general condition, indicating that side effects or off-target effects were not significant.

**Table 35 Tumor growth inhibition on day 26**

| Group | Tumor volume (mm³) | TGI(%) |
|---|---|---|
| IgG, 15 mg/kg | 1965 | NA |
| IgG-compound 6, 15 mg/kg | 1320 | 35 |
| BC1254-A1 V2-compound 6, 5 mg/kg | 597 | 74 |
| BC1254-A1 V2-compound 6, 15 mg/kg | 294 | 90 |
| Paclitaxel, 20 mg/kg | 793 | 63 |
| Bevacizumab, 5 mg/kg | 1203 | 41 |

### Example 26. FRα-ISAC in Combination with FRα-ADC (FR57-DXd ) Therapeutic effect in the A2780cisR-FRα model

In this experiment, CB17-SCID mice were inoculated with A2780cisR-FRα cells to determine the anti-tumor effect of FRα-ISAC in combination with FRα-ADC (FR57-DXd).

### CB17-SCID mice:

SPF female CB17-SCID mice, purchased from Beijing Vital River Laboratory Animal Technology Co., Ltd., qualified by Beijing Vital River Laboratory Animal Technology Co., Ltd., with certificate No. 110011241105958341. Before the study, the mice were adapted for 3 days after arrival.

### Cells:

The A2780cisR-FRα cells were constructed by Innovent Biologics and routinely subcultured for subsequent in vivo experiments. The cells were collected by centrifugation and resuspended in sterile PBS, with the cell density adjusted to 2.5×10⁷cells/mL. On day 0, 0.2 mL of the cell suspension was subcutaneously inoculated into the right abdominal region of the CB17-SCID mice to establish A2780cisR-FRα tumor-bearing mouse models.

### Administration:

16 days after the tumor cell inoculation, the tumor volume in each mouse was measured, and the tumor volume was selected to be about 100 mm³. Then the mice were evenly divided into groups by tumor volume (5 mice per group). The dosages and routes of administration are shown in Table 36. IgG (purchased from EQUITECH-BIO) was used as a negative control. Administration was performed on day 16 after inoculation. The tumor volume and body weight of the mice were monitored twice a week. The body weight and tumor volume were measured before each administration. On day 33 after inoculation, the relative tumor growth inhibition (TGI%) was calculated by the following formula: TGI% = 100% × (control group tumor volume - treatment group tumor volume)/(control group tumor volume - control group tumor volume before administration).Tumor volume measurement: The maximum length of major axis (L) and maximum length of minor axis (W) of tumors were measured with a vernier caliper, and tumor volume was calculated using the following formula: V = L×W²/2.The mice were weighted using an electronic balance.

**Table 36 Experimental Design**

| Group | Dose of administration | Administration frequency | Mode of administration |
|---|---|---|---|
| IgG | 8 mg/kg | Single dose | Intravenous injection |
| IgG-Compound 6 + IgG-DXd | 5+3 mg/kg | Single dose | Intravenous injection |
| BC1254-A1 V2-Compound 6 | 5 mg/kg | Single dose | Intravenous injection |
| FR57-DXd | 3 mg/kg | Single dose | Intravenous injection |
| BC1254-A1 V2-Compound 6 + FR57-DXd | 5+3 mg/kg | Single dose | Intravenous injection |

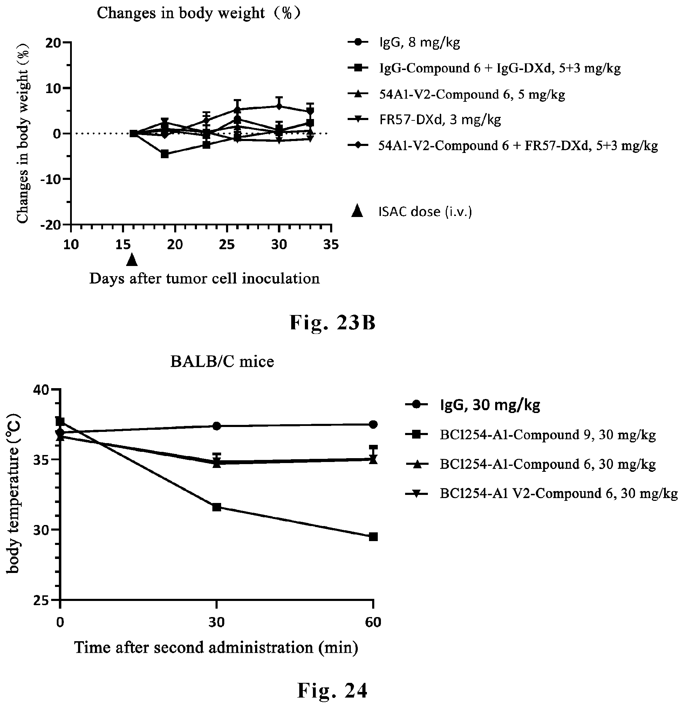

The efficacy results are shown in FIG. 23A and Table 37: on day 33 after inoculation, the tumor growth inhibition rates of the BC1254-A1 V2-compound 6 group, the FR57-DXd group, and the BC1254-A1 V2-compound 6 + FR57-DXd combination group were 71%, 73%, and 102%, respectively. The efficacy of the combination was stronger than that in the single-drug groups. Meanwhile, the body weight of the mice was measured. As shown in FIG. 23B, there was no significant difference in body weight of the mice. Therefore, FRα-ISAC in combination with FRα-ADC has a significant synergistic inhibitory effect on A2780cisR-FRα tumors, and has no significant toxic side effects on mice.

**Table 37Tumor growth inhibition on day 33**

| Group | Tumor volume (mm3) | TGI(%) |
|---|---|---|
| IgG, 8 mg/kg | 585 | NA |
| IgG-compound 6 + IgG-DXd, 5 + 3 mg/kg | 345 | 49 |
| BC1254-A1 V2-compound 6, 5 mg/kg | 243 | 71 |
| FR57-DXd, 3 mg/kg | 233 | 73 |
| BC1254-A1 V2-compound 6 + FR57-DXd, 5 + 3 mg/kg | 91 | 102 |

### Example 27. High Dose Toxicity Study of FRα-ISAC in BALB/c Mice

In this experiment, BALB/c mice were used. The body temperature changes of the mice were observed after intravenous injection of the FRα-ISAC drug twice at an interval of 7 days to determine the toxicity of the drug.

### BALB/c mice:

Female BALB/c background mice, SPF grade, purchased from Beijing Vital River Laboratory Animal Technology Co., Ltd., qualified by Beijing Vital River Laboratory Animal Technology Co., Ltd., with certificate No. NO110011241105136147. The study started after the mice were adapted for 3 days after arrival.

### Administration:

See Table 38. Experimental design: grouping and weighing, 5 mice per group, IgG (purchased from EQUITECH-BIO) as a negative control. The FRα-ISAC drugs (or_control) were injected intravenously on days 0 and 7, and then the initial rectal temperature of the mice was measured before administration on day 7 and the rectal temperature of the mice was also measured 30 min and 60 min after administration.

**Table 38 Experimental Design**

| Group | Dose of administration | Administration frequency | Mode of administration |
|---|---|---|---|
| IgG | 30 mg/kg | QWx2 | Intravenous injection |
| BC1254-A1-Compound 9 | 30mg/kg | QWx2 | Intravenous injection |
| BC1254-A1-Compound 6 | 30 mg/kg | QWx2 | Intravenous injection |
| BC1254-A1 V2-Compound 6 | 30 mg/kg | QWx2 | Intravenous injection |

The rectal temperature results are shown in FIG. 24 and Table 39: after the administration on day 7, the rectal temperature of the BC1254-A1-compound 9 group decreased significantly to 31.62 °C at 30 min after the administration, and further decreased to 29.50 °C at 60 min after the administration. However, the BC1254-A1-compound 6 and BC1254-A1 V2-compound 6 groups did not exhibit significant rectal temperature decreases. Therefore, administering a high dose of BC1254-A1-compound 9 at an interval of 7 days had significant toxic and side effects on BALB/C mice, while the high dose of BC1254-A1-compound6 and BC1254-A1 V2-Compound 6 had no significant toxic side effects on BALB/C mice.

**Table 39 Rectal temperatures of mice before and after administration on day 7**

| Group | Pre-dose (°C) | 30 min (°C) | 60 min (°C) |
|---|---|---|---|
| IgG | 36.92 | 37.38 | 37.5 |
| BC1254-A1-Compound 9 | 37.68 | 31.62 | 29.50 |
| BC1254-A1-Compound 6 | 36.64 | 34.70 | 34.98 |
| BC1254-A1 V2-Compound 6 | 36.62 | 34.84 | 35.04 |

### Sequence information:

| Num. | **Region or Name** | **Sequences** |
|---|---|---|
| Sequence of hz1D9B10 | | |
| 1 | HCDR1 | GYTFTNYWLG |
| 2 | HCDR2 | NIYPGGDYTNYNEKFKG |
| 3 | HCDR3 | EGYDNY |
| 4 | LCDR1 | KSSQSLLNSGNQENYLT |
| 5 | LCDR2 | WASTRES |
| 6 | LCDR3 | QSDYSYPFT |
| 7 | hz1D9B10 -VH | QVQLVQSGAEVKKPGSSVKVSCKASGYTFTNYWLGW VRQAPGQGLEWMGNIYPGGDYTNYNEKFKGRVTLTAD KSTSTAYMELSSLRSEDTAVYYCSKEGYDNYWGQGTLV TVSS |
| 8 | hz1D9B10 -VL | DIVMTQSPDSLAVSLGERATINCKSSQSLLNSGNQENYL TWYQQKPGQPPKLLIYWASTRESGVPDRFSGSGSGTDF TLTISSLQAEDVAVYYCQSDYSYPFTFGQGTKLEIK |
| 9 | Heavy Chain | QVQLVQSGAEVKKPGSSVKVSCKASGYTFTNYWLGW VRQAPGQGLEWMGNIYPGGDYTNYNEKFKGRVTLTAD |
| | | KSTSTAYMELSSLRSEDTAVYYCSKEGYDNYWGQGTLV TVSSASTKGPSVFPLAPSSKSTSGGTAALGCLVKDYFPE PVTVSWNSGALTSGVHTFPAVLQSSGLYSLSSVVTVPSS SLGTQTYICNVNHKPSNTKVDKKVEPKSCDKTHTCPPC PAPELLGGPSVFLFPPKPKDTLMISRTPEVTCVVVDVSH EDPEVKFNWYVDGVEVHNAKTKPREEQYNSTYRVVSV LTVLHQDWLNGKEYKCKVSNKALPAPIEKTISKAKGQP REPQVYTLPPSRDELTKNQVSLTCLVKGFYPSDIAVEWE SNGQPENNYKTTPPVLDSDGSFFLYSKLTVDKSRWQQG NVFSCSVMHEALHNHYTQKSLSLSPGK |
| 10 | Light Chain | DIVMTQSPDSLAVSLGERATINCKSSQSLLNSGNQENYL TWYQQKPGQPPKLLIYWASTRESGVPDRFSGSGSGTDF TLTISSLQAEDVAVYYCQSDYSYPFTFGQGTKLEIKRTVA APSVFIFPPSDEQLKSGTASVVCLLNNFYPREAKVQWK VDNALQSGNSQESVTEQDSKDSTYSLSSTLTLSKADYE KHKVYACEVTHQGLSSPVTKSFNRGEC |
| 11 | VH-DNA | GAGGTTCAGCTGCAGCAGTCTGGAACTGAGCTGGTA AGGCCTGGGACTTCAGTGAAGATATCCTGCAAGGCTT CTGGCTACACCTTCACTAACTACTGGCTAGGTTGGGTA AAGCAGAGGCCTGGACATGGACTTGAGTGGATTGGA AATATTTACCCTGGAGGTGATTATACTAACTATAATGAG AAGTTCAAGGGCAAGGCCACACTGACTGCAGACACA TCCTCCAGCACTGCCTACATGCAACTCAGTAGCCTGA CATCTGAGGACTCTGCTGTCTATTTCTGTTCAAAGGAG GGGTACGACAACTACTGGGGCCAAGGCACCACTCTC ACAGTCTCCTCA |
| 12 | VH-amino acid | EVQLQQSGTELVRPGTSVKISCKASGYTFTNYWLGWV KQRPGHGLEWIGNIYPGGDYTNYNEKFKGKATLTADTS SSTAYMQLSSLTSEDSAVYFCSKEGYDNYWGQGTTLTV SS |
| 13 | VL-DNA | GACATTGTGATGACCCAATCTCCATCCTTCCTGACTGT GACAGCAGGAGAGAAGGTCACTTTGAGCTGCAAGTC |
| | | CAGTCAGAGTCTGTTAAACAGTGGAAATCAAGAGAA CTACTTGACCTGGTATCAGCAGAAACCAGGTCAGCCT CCTAAACAGTTGATCTACTGGGCATCCACTAGGGAAT CTGGGGTCCCTGATCGCTTCACAGGCAGTGGATCTGG AACAGATTTCACTCTCACCATCAGCAGTGTGCAGGCT GAAGACCTGGCAGTTTATTACTGTCAGAGTGATTATAG TTATCCATTCACGTTCGGCTCGGGGACAAAGTTGGAA ATAAAA |
| 14 | VL-amino acid | DIVMTQSPSFLTVTAGEKVTLSCKSSQSLLNSGNQENYL TWYQQKPGQPPKQLIYWASTRESGVPDRFTGSGSGTDF TLTISSVQAEDLAVYYCQSDYSYPFTFGSGTKLEIK |
| 15 | ch1D9B10 -heavy chain | EVQLQQSGTELVRPGTSVKISCKASGYTFTNYWLGWV KQRPGHGLEWIGNIYPGGDYTNYNEKFKGKATLTADTS SSTAYMQLSSLTSEDSAVYFCSKEGYDNYWGQGTTLTV SSASTKGPSVFPLAPSSKSTSGGTAALGCLVKDYFPEPV TVSWNSGALTSGVHTFPAVLQSSGLYSLSSVVTVPSSSL GTQTYICNVNHKPSNTKVDKKVEPKSCDKTHTCPPCPA PELLGGPSVFLFPPKPKDTLMISRTPEVTCVVVDVSHED PEVKFNWYVDGVEVHNAKTKPREEQYNSTYRVVSVLT VLHQDWLNGKEYKCKVSNKALPAPIEKTISKAKGQPRE PQVYTLPPSRDELTKNQVSLTCLVKGFYPSDIAVEWESN GQPENNYKTTPPVLDSDGSFFLYSKLTVDKSRWQQGNV FSCSVMHEALHNHYTQKSLSLSPGK |
| 16 | ch1D9B10 -light chain | DIVMTQSPSFLTVTAGEKVTLSCKSSQSLLNSGNQENY LTWYQQKPGQPPKQLIYWASTRESGVPDRFTGSGSGT DFTLTISSVQAEDLAVYYCQSDYSYPFTFGSGTKLEIKR TVAAPSVFIFPPSDEQLKSGTASVVCLLNNFYPREAKV QWKVDNALQSGNSQESVTEQDSKDSTYSLSSTLTLSK ADYEKHKVYACEVTHQGLSSPVTKSFNRGEC |
| Humanized RS7 mAb, The definition scheme for CDRs is as follows: Kabat+Chothia | | |
| 17 | Humanized RS7 Mab-HCDR1 | GYTFTNYGM |
| 18 | Humanized RS7 Mab-HCDR2 | WINTYTGEPTYTDDFKG |
| 19 | Humanized RS7 Mab-HCDR3 | GGFGSSYWYFDV |
| 20 | Humanized RS7 Mab-LCDR1 | KASQDVSIAVA |
| 21 | Humanized RS7 Mab-LCDR2 | SASYRYT |
| 22 | Humanized RS7 Mab-LCDR3 | QQHYITPLT |
| 23 | Humanized RS7 Mab-VH | QVQLQQSGSELKKPGASVKVSCKASGYTFTNYGMNW VKQAPGQGLKWMGWINTYTGEPTYTDDFKGRFAFSL DTSVSTAYLQISSLKADDTAVYFCARGGFGSSYWYFD VWGQGSLVTVSS |
| 24 | Humanized RS7 Mab-VL | DIQLTQSPSSLSASVGDRVSITCKASQDVSIAVAWYQQ KPGKAPKLLIYSASYRYTGVPDRFSGSGSGTDFTLTISS LQPEDFAVYYCQQHYITPLTFGAGTKVEIK |
| 25 | Humanized RS7 Mab-heavy chain | QVQLQQSGSELKKPGASVKVSCKASGYTFTNYGMNW VKQAPGQGLKWMGWINTYTGEPTYTDDFKGRFAFSL DTSVSTAYLQISSLKADDTAVYFCARGGFGSSYWYFD VWGQGSLVTVSSASTKGPSVFPLAPSSKSTSGGTAALG CLVKDYFPEPVTVSWNSGALTSGVHTFPAVLQSSGLYS LSSVVTVPSSSLGTQTYICNVNHKPSNTKVDKRVEPKS CDKTHTCPPCPAPELLGGPSVFLFPPKPKDTLMISRTPE VTCVVVDVSHEDPEVKFNWYVDGVEVHNAKTKPRE EQYNSTYRVVSVLTVLHQDWLNGKEYKCKVSNKALP APIEKTISKAKGQPREPQVYTLPPSREEMTKNQVSLTC LVKGFYPSDIAVEWESNGQPENNYKTTPPVLDSDGSFF LYSKLTVDKSRWQQGNVFSCSVMHEALHNHYTQKSL SLSPGK |
| 26 | Humanized RS7 Mab-light chain | DIQLTQSPSSLSASVGDRVSITCKASQDVSIAVAWYQQ KPGKAPKLLIYSASYRYTGVPDRFSGSGSGTDFTLTISS LQPEDFAVYYCQQHYITPLTFGAGTKVEIKRTVAAPSV FIFPPSDEQLKSGTASVVCLLNNFYPREAKVQWKVDN ALQSGNSQESVTEQDSKDSTYSLSSTLTLSKADYEKH KVYACEVTHQGLSSPVTKSFNRGEC |

| BC1254-A1 & BC1254-A1 V2 sequences | | |
|---|---|---|
| 27 | HCDR1 | GDSVSSNSAAWN |
| 28 | HCDR2 | RTYYRSKWYNDYSLSVKS |
| 29 | HCDR3 | DWPMDV |
| 30 | LCDR1 | RASHNVDKWLA |
| 31 | LCDR2 | TASSLES |
| 32 | LCDR3 | QQYFTYSLST |
| 33 | VH | EVQLVQSGPGLVEPSQTLSLTCAISGDSVSSNSAAWNW IRQSPSRGLEWLGRTYYRSKWYNDYSLSVKSRITINPD TAKNQFSLHLDSVTPEDTAVYYCARDWPMDVWGQGT MVTVSS |
| 34 | VL | DIQLTQSPSFLSASVGDRVTITCRASHNVDKWLAWYQ QKPGKAPKLLIHTASSLESGVPSRFSGSGSGTEFTLTISS LQPDDFATYYCQQYFTYSLSTFGQGTKVDIK |
| 35 | Heavy Chain | EVQLVQSGPGLVEPSQTLSLTCAISGDSVSSNSAAWNW IRQSPSRGLEWLGRTYYRSKWYNDYSLSVKSRITINPD TAKNQFSLHLDSVTPEDTAVYYCARDWPMDVWGQGT MVTVSSASTKGPSVFPLAPSSKSTSGGTAALGCLVKDY FPEPVTVSWNSGALTSGVHTFPAVLQSSGLYSLSSVVT VPSSSLGTQTYICNVNHKPSNTKVDKKVEPKSCDKTH TCPPCPAPELLGGPSVFLFPPKPKDTLMISRTPEVTCVV VDVSHEDPEVKFNWYVDGVEVHNAKTKPREEQYNST YRVVSVLTVLHQDWLNGKEYKCKVSNKALPAPIEKTI SKAKGQPREPQVYTLPPSRDELTKNQVSLTCLVKGFYP SDIAVEWESNGQPENNYKTTPPVLDSDGSFFLYSKLTV DKSRWQQGNVFSCSVMHEALHNHYTQKSLSLSPGK |
| 36 | Light Chain BC1254-A1 | DIQLTQSPSFLSASVGDRVTITCRASHNVDKWLAWYQ QKPGKAPKLLIHTASSLESGVPSRFSGSGSGTEFTLTISS LQPDDFATYYCQQYFTYSLSTFGQGTKVDIKRTVAAPS VFIFPPSDEQLKSGTASVVCLLNNFYPREAKVQWKVD NALQSGNSQESVTEQDSKDSTYSLSSTLTLSKADYEK HKVYACEVTHQGLSSPVTKSFNRGEC |
| 37 | Light Chain BC1254-A1 V2 | DIQLTQSPSFLSASVGDRVTITCRASHNVDKWLAWYQ QKPGKAPKLLIHTASSLESGVPSRFSGSGSGTEFTLTISS LQPDDFATYYCQQYFTYSLSTFGQGTKVDIKGQPKAA PSVTLFPPSSEELQANKATLVCLISDFYPGAVTVAWKA DSSPVKAGVCTTTPSCQSNNKYAASSYLSLTPEQWKS HRSYSCQVTHEGSTVEKTVAPTECS |
| 38 | Lambda light chain constant region | GQPKAAPSVTLFPPSSEELQANKATLVCLISDFYPGAVT VAWKADSSPVKAGVETTTPSKQSNNKYAASSYLSLTP EQWKSHRSYSCQVTHEGSTVEKTVAPTECS |
| 39 | Lambda light chain constant region (LLC160C (Eu)) | GQPKAAPSVTLFPPSSEELQANKATLVCLISDFYPGAVT VAWKADSSPVKAGVCTTTPSKQSNNKYAASSYLSLTP EQWKSHRSYSCQVTHEGSTVEKTVAPTECS |
| 40 | Lambda light chain constant region (LLC166C (Eu)) | GQPKAAPSVTLFPPSSEELQANKATLVCLISDFYPGAVT VAWKADSSPVKAGVETTTPSCQSNNKYAASSYLSLTP EQWKSHRSYSCQVTHEGSTVEKTVAPTECS |
| 41 | Lambda light chain constant region (LLC160C &166C (Eu)) | GQPKAAPSVTLFPPSSEELQANKATLVCLISDFYPGAVT VAWKADSSPVKAGVCTTTPSCQSNNKYAASSYLSLTP EQWKSHRSYSCQVTHEGSTVEKTVAPTECS |
| 42 | LLC160C (Eu) Sequences near the mutation | VKAGVCTTTPS |
| 43 | LLC166C (Eu) Sequences | TTTPSCQSNNK |
| | near the mutation | |
| 44 | Sequences near LLC160-166 (Eu) mutations | VKAGVCTTTPSCQSNNK |
| 45 | Kappa light chain constant region | |
| 46 | IgG1 heavy chain constant region | |

## Claims

1. An anti-TROP2 antibody or antigen-binding fragment thereof, the antibody comprising:
(i) three complementarity determining regions HCDR1, HCDR2 and HCDR3 contained in the VH set forth in SEQ ID NO: 7, and three complementarity determining regions LCDR1, LCDR2 and LCDR3 contained in the VL set forth in SEQ ID NO: 8; or
(ii) three complementarity determining regions HCDR1, HCDR2, and HCDR3 contained in the VH set forth in SEQ ID NO: 12, and three complementarity determining regions LCDR1, LCDR2, and LCDR3 contained in the VL set forth in SEQ ID NO: 14.

2. An anti-TROP2 antibody or an antigen-binding fragment thereof, comprising: a first heavy chain complementarity-determining region (HCDR1), a second heavy chain complementarity-determining region (HCDR2), a third heavy chain complementarity-determining region (HCDR3), and a first light chain complementarity-determining region (LCDR1), a second light chain complementarity-determining region (LCDR2), and a third light chain complementarity-determining region (LCDR3), wherein the HCDR1, the HCDR2, the HCDR3, the LCDR1, the LCDR2, and the LCDR3 comprise or consist of amino acid sequences set forth in SEQ ID NO: 1, SEQ ID NO: 2, SEQ ID NO: 3, SEQ ID NO: 4, SEQ ID NO: 5, and SEQ ID NO: 6, respectively.

3. The anti-TROP2 antibody or the antigen-binding fragment thereof according to claim 1 or 2, comprising a heavy chain variable region (VH), wherein the heavy chain variable region comprises or consists of an amino acid sequence having at least 90% to at most 100% sequence identity to an amino acid sequence selected from SEQ ID NOs: 7 and 12, or comprises or consists of an amino acid sequence selected from SEQ ID NOs: 7 and 12.

4. The anti-TROP2 antibody or the antigen-binding fragment thereof according to any one of claims 1-3, comprising a light chain variable region (VL), wherein the light chain variable region comprises or consists of an amino acid sequence having at least 90% to at most 100% sequence identity to an amino acid sequence selected from the group consisting of SEQ ID NOs: 8 and 14, or comprises or consists of an amino acid sequence selected from the group consisting of SEQ ID NOs: 8 and 14.

5. The anti-TROP2 antibody or the antigen-binding fragment thereof according to claim 1 or 2, comprising a heavy chain variable region and a light chain variable region, wherein
(i) the heavy chain variable region comprises or consists of an amino acid sequence set forth in SEQ ID NO: 7, and the light chain variable region comprises or consists of an amino acid sequence set forth in SEQ ID NO: 8; or
(ii) the heavy chain variable region comprises or consists of an amino acid sequence set forth in SEQ ID NO: 12, and the light chain variable region comprises or consists of an amino acid sequence set forth in SEQ ID NO: 14.

6. The anti-TROP2 antibody or the antigen-binding fragment thereof according to any one of claims 1-5, comprising an Fc region.

7. The anti-TROP2 antibody or the antigen-binding fragment thereof according to claim 6, wherein the Fc region is an Fc from human IgG, e.g., an Fc region from human IgG1, an Fc region from human IgG2, an Fc region from human IgG3, or an Fc region from human IgG4.

8. The anti-TROP2 antibody or the antigen-binding fragment thereof according to any one of claims 1-7, comprising a heavy chain, wherein the heavy chain
(i) comprises or consists of an amino acid sequence having at least 85%, 90%, 91%, 92%, 93%, 94%, 95%, 96%, 97%, 98%, or 99% identity to the amino acid sequence selected from SEQ ID NO: 9 and 15; or
(ii) comprises or consists of an amino acid sequence selected from SEQ ID NO: 9 and 15.

9. The anti-TROP2 antibody or the antigen-binding fragment thereof according to any one of claims 1-8, comprising a light chain, wherein the light chain
(i) comprises or consists of an amino acid sequence having at least 85%, 90%, 91%, 92%, 93%, 94%, 95%, 96%, 97%, 98%, or 99% identity to the amino acid sequence selected from SEQ ID NO: 10 and 16; or
(ii) comprises or consists of an amino acid sequence selected from SEQ ID NO: 10 and 16.

10. The anti-TROP2 antibody or the antigen-binding fragment thereof according to any one of claims 1-9, comprising a heavy chain and a light chain, wherein the heavy chain comprises or consists of an amino acid sequence set forth in SEQ ID NO: 9, and the light chain comprises or consists of an amino acid sequence set forth in SEQ ID NO: 10.

11. The anti-TROP2 antibody or the antigen-binding fragment thereof according to any one of claims 1-10, wherein the antibody is a monoclonal antibody.

12. The anti-TROP2 antibody or the antigen-binding fragment thereof according to any one of claims 1-11, wherein the antibodyis a humanized antibody or a chimeric antibody.

13. The anti-TROP2 antibody or the antigen-binding fragment thereof according to any one of claims 1-12, wherein the antigen-binding fragment is an antibody fragment selected from: Fab, Fab', Fab'-SH, Fv, single-chain antibody (e.g., scFv), and (Fab')₂, single-domain antibodies such as VHH, dAb (domain antibodies), bivalent antibodies, or linear antibodies.

14. A fusion protein, comprising the anti-TROP2 antibody or the antigen-binding fragment thereof according to any one of claims 1-13.

15. An immunoconjugate, comprising the anti-TROP2 antibody or the antigen-binding fragment thereof according to any one of claims 1-13 and an additional substance, e.g., a toxin, a small molecule drug, a cytotoxic agent, an apoptotic agent, a chelating agent, and an immunomodulatory agent (e.g., an anti-inflammatory agent or an immunosuppressant).

16. The immunoconjugate according to claim 15, comprising an immunomodulatory agent, such as a TLR agonist, e.g., a TLR7/8 agonist.

17. An isolated nucleic acid, encoding the anti-TROP2 antibody or the antigen-binding fragment thereof according to any one of claims 1-13.

18. A vector, comprising the nucleic acid according to claim 17, wherein, preferably, the vector is an expression vector.

19. A host cell, comprising the nucleic acid according to claim 17 or the vector according to claim 18, wherein, preferably, the host cell is prokaryotic or eukaryotic, and more preferably, the host cell is selected from yeast cells, mammalian cells (e.g., 293 cells or CHO cells, such as CHO-K cells or HEK293 cells), or other cells suitable for preparing an antibody or the antigen-binding fragment thereof.

20. A method for preparing an anti-TROP2 antibody or the antigen-binding fragment thereof, comprising:
a) culturing the host cell according to claim 19 under conditions suitable for expressing the nucleic acid encoding the anti-TROP2 antibody or the antigen-binding fragment thereof according to any one of claims 1-13,
b) optionally isolating the antibody or antigen binding fragment thereof,
c) optionally, the method further comprises recovering the anti-TROP2 antibody or the antigen-binding fragment thereof from the host cell, and optionally the antibody is purified, e.g., by Protein A purification.

21. An anti-FRα antibody or the antigen-binding fragment thereof, comprising:
three complementarity-determining regions HCDR1, HCDR2, and HCDR3 contained in a VH set forth in SEQ ID NO: 33, and three complementarity-determining regions LCDR1, LCDR2, and LCDR3 contained in a VL set forth in SEQ ID NO: 34.

22. An anti-FRα antibody or an antigen-binding fragment thereof, comprising a first heavy chain complementarity-determining region (HCDR1), a second heavy chain complementarity-determining region (HCDR2), a third heavy chain complementarity-determining region (HCDR3), and a first light chain complementarity-determining region (LCDR1), a second light chain complementarity-determining region (LCDR2), and a third light chain complementarity-determining region (LCDR3), wherein the HCDR1, HCDR2, HCDR3, LCDR1, LCDR2, and LCDR3 comprise or consist of the amino acid sequences set forth in SEQ ID NO:27, SEQ ID NO:28, SEQ ID NO:29, SEQ ID NO:30, SEQ ID NO:31, and SEQ ID NO:32 respectively.

23. The anti-FRα antibody or the antigen-binding fragment thereof according to claim 21 or 22, comprising a heavy chain variable region (VH), wherein the heavy chain variable region comprises or consists of an amino acid sequence having at least 90%, 91%, 92%, 93%, 94%, 95%, 96%, 97%, 98%, or 99% identity to the amino acid sequence set forth in SEQ ID NO: 33, or comprises or consists of an amino acid sequence set forth in SEQ ID NO: 33.

24. The anti-FRα antibody or the antigen-binding fragment thereof according to any one of claims 21-23, comprising a light chain variable region (VL), wherein the light chain variable region comprises or consists of an amino acid sequence having at least 90%, 91%, 92%, 93%, 94%, 95%, 96%, 97%, 98%, or 99% identity to the amino acid sequence set forth in SEQ ID NO: 34, or comprises or consists of an amino acid sequence set forth in SEQ ID NO: 34.

25. An anti-FRα antibody or an antigen-binding fragment thereof, comprising:
a VH comprising or consisting of an amino acid sequence set forth in SEQ ID NO: 33 or an amino acid sequence having at least 90%, 91%, 92%, 93%, 94%, 95%, 96%, 97%, 98%, or 99% identity thereto, and/or a VL comprising or consisting of an amino acid sequence set forth in SEQ ID NO: 34 or an amino acid sequence having at least 90%, 91%, 92%, 93%, 94%, 95%, 96%, 97%, 98%, or 99% identity thereto.

26. The anti-FRα antibody or the antigen-binding fragment thereof according to claim 21 or 22, comprising a heavy chain variable region and a light chain variable region, wherein
said heavy chain variable region comprises or consists of an amino acid sequence set forth in SEQ ID NO: 33, and the light chain variable region comprises or consists of an amino acid sequence set forth in SEQ ID NO: 34.

27. The anti-FRα antibody or the antigen-binding fragment thereof according to any one of claims 21-26, comprising an Fc region, wherein, for example, the Fc region is from an Fc region of IgG1, IgG2, IgG3, or IgG4, such as an Fc region of human IgG1, IgG2, IgG3, or IgG4.

28. The anti-FRα antibody or the antigen-binding fragment thereof according to any one of claims 21-27, comprising a heavy chain constant region, wherein the heavy chain constant region is from a constant region of IgG1, IgG2, IgG3, or IgG4, e.g., a constant region of human IgG1, IgG2, IgG3, or IgG4, e.g., the IgG1 heavy chain constant region
(i) comprises or consists of an amino acid sequence set forth in SEQ ID NO: 46; or
(ii) comprises an amino acid sequence that is at least 85%, 90%, 91%, 92%, 93%, 94%, 95%, 96%, 97%, 98%, or 99% identical to the amino acid sequence of SEQ ID NO: 46.

29. The anti-FRα antibody or the antigen-binding fragment thereof according to any one of claims 21-28, comprising a light chain constant region, wherein the light chain constant region is a lambda or Kappa light chain constant region, e.g., a human lambda or Kappa light chain constant region; preferably, the light chain constant region
(i) comprises or consists of an amino acid sequence having at least 85%, 90%, 91%, 92%, 93%, 94%, 95%, 96%, 97%, 98%, or 99% identity to an amino acid sequence of any one of SEQ ID NOs: 38-41 and 45; or
(ii) comprises or consists of an amino acid sequence of any one of SEQ ID NOs: 38-41 and 45.

30. The anti-FRα antibody or the antigen-binding fragment thereof according to any one of claims 21-29, wherein the antibody or the antigen-binding fragment thereof comprises one or two lambda light chain constant regions, and comprises a cysteine substitution at position 160 (EU numbering) of the lambda light chain constant region (LLC160C).

31. The anti-FRα antibody or the antigen-binding fragment thereof according to claim 30, wherein the lambda light chain constant region comprising LLC160Cs comprises an amino acid sequence set forth in SEQ ID NO: 39 or an amino acid sequence having at least 90%, 91%, 92%, 93%, 94%, 95%, 96%, 97%, 98%, or 99% sequence identity to the amino acid sequence set forth in SEQ ID NO: 39, and comprises the amino acid sequence VKAGVCTTTPS (SEQ ID NO: 42).

32. The anti-FRα antibody or the antigen-binding fragment thereof according to any one of claims 21-29, wherein the antibody or the antigen-binding fragment thereofcomprises one or two lambda light chain constant regions, and comprises a cysteine substitution at position 166 (EU numbering) of the lambda light chain constant region (LLC166C).

33. The anti-FRα antibody or the antigen-binding fragment thereof according to claim 32, wherein the lambda light chain constant region comprising LLC166C comprises the amino acid sequence set forth in SEQ ID NO: 40 or an amino acid sequence having at least 90%, 91%, 92%, 93%, 94%, 95%, 96%, 97%, 98%, or 99% sequence identity to the amino acid sequence set forth in SEQ ID NO: 40, and wherein said constant region comprises the amino acid sequence TTTPSCQSNNK (SEQ ID NO: 43).

34. The anti-FRα antibody or the antigen-binding fragment thereof according to any one of claims 21-29, wherein the antibody or the antigen-binding fragment thereof comprises one or two lambda light chain constant regions, and comprises cysteine substitutions at positions 160 and 166 (EU numbering) of the lambda light chain constant region (LLC160C& LLC 166C).

35. The anti-FRα antibody or the antigen-binding fragment thereof according to claim 34, wherein the Lambda light chain constant region comprising LLC160C and LLC166C comprises an amino acid sequence set forth in SEQ ID NO: 41 or an amino acid sequence having at least 90%, 91%, 92%, 93%, 94%, 95%, 96%, 97%, 98%, or 99% sequence identity to the amino acid sequence set forth in SEQ ID NO: 41 and comprises the amino acid sequence VKAGVCTTTPSCQSNNK (SEQ ID NO: 44).

36. The anti-FRα antibody or the antigen-binding fragment thereof according to any one of the claims 21 to 35, wherein the antibody or the antigen-binding fragment thereof comprises:
(i) one or two Lamda light chain constant regions having a cysteine mutation at position 160 (Eu numbering); and/or
(ii) one or two Lamda light chain constant regions having a cysteine mutation at position 166 (Eu numbering).

37. The anti-FRα antibody or the antigen-binding fragment thereof according to any one of the claims 21-36, wherein the antibody or the antigen-binding fragment thereof comprises: two identical heavy chains and two identical light chains, wherein
the heavy chain comprises or consists of an amino acid sequence having at least 85%, 90%, 91%, 92%, 93%, 94%, 95%, 96%, 97%, 98%, or 99% identity to an amino acid sequence set forth in SEQ ID NO: 35, and/or
the light chain comprises or consists of an amino acid sequence having at least 85%, 90%, 91%, 92%, 93%, 94%, 95%, 96%, 97%, 98%, or 99% identity to an amino acid sequence set forth in SEQ ID NO: 36.

38. The anti-FRα antibody or the antigen-binding fragment thereof according to any one of claims 21-36, wherein antibody or antigen-binding fragment thereof two identical heavy chains and two identical light chains, wherein
the heavy chain comprises or consists of an amino acid sequence set forth in SEQ ID NO: 35, and/or
the light chain comprises or consists of an amino acid sequence set forth in SEQ ID NO: 36.

39. The anti-FRα antibody or the antigen-binding fragment thereof according to any one of the claims 21-36, wherein the antibody or the antigen-binding fragment thereof comprises: two identical heavy chains and two identical light chains, wherein
the heavy chain comprises or consists of an amino acid sequence having at least 85%, 90%, 91%, 92%, 93%, 94%, 95%, 96%, 97%, 98%, or 99% identity to an amino acid sequence set forth in SEQ ID NO: 35, and/or
the light chain comprises or consists of an amino acid sequence having at least 85%, 90%, 91%, 92%, 93%, 94%, 95%, 96%, 97%, 98%, or 99% identity to the amino acid sequence set forth in SEQ ID NO: 37.

40. The anti-FRα antibody or the antigen-binding fragment thereof according to any one of the claims 21-36, wherein antibody or antigen-binding fragment thereof comprised: two identical heavy chains and two identical light chains, wherein
the heavy chain comprises or consists of an amino acid sequence set forth in SEQ ID NO: 35, and/or
the light chain comprises or consists of an amino acid sequence set forth in SEQ ID NO: 37.

41. The anti-FRα antibody or the antigen-binding fragment thereof according to any one of claims 21-40, wherein the antibody is a humanized antibody or a chimeric antibody.

42. A fusion protein comprising the anti-FRα antibody or the antigen-binding fragments thereof according to any one of claim 21-41.

43. An immunoconjugate, comprising the anti-FRα antibody or the antigen-binding fragment thereof according to any one of claims 21-41, and an additional substance, e.g., a toxin, a small molecule drug, a cytotoxic agent, an apoptotic agent, a chelating agent, and an immunomodulatory agent, e.g., an anti-inflammatory agent or an immunosuppressant.

44. The immunoconjugate according to claim 43, comprising an immunomodulatory agent, such as a TLR agonist, e.g., TLR7/8 agonist.

45. An isolated nucleic acid encoding the anti-FRα antibody or the antigen-binding fragment thereof according to any one of claims 21-41.

46. A vector comprising the nucleic acid of claim 42, preferably the vector is an expression vector.

47. A host cell, comprising the nucleic acid according to claim 45 or the vector according to claim 46, wherein, preferably, the host cell is prokaryotic or eukaryotic, and more preferably, the host cell is selected from yeast cells, mammalian cells (e.g., 293 cells or CHO cells, such as CHO-K cells or HEK293 cells), or other cells suitable for preparing an antibody or an antigen-binding fragment thereof.

48. A method for preparing an anti-FRα antibody or an antigen-binding fragment thereof, comprising:
(a) culturing the host cell according to claim 47 under conditions suitable for expressing the nucleic acid encoding the anti-FRα antibody or the antigen-binding fragment thereof according to any one of claims 21-41,
(b) optionally isolating the antibody or the antigen-binding fragment thereof,
(c) optionally, the method further comprises recovering the anti-FRα antibody or the antigen-binding fragment thereof from the host cell, Optionally, the antibody is purified, e.g., by Protein A.

49. A compound or a pharmaceutically acceptable salt or solvate thereof selected from the group consisting of the following:
(1) a compound of formula (D-1): 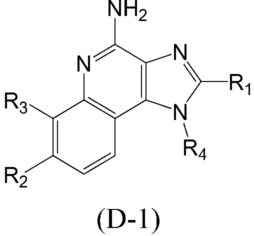 wherein:
R₁ is C₁-C₆alkyl which is unsubstituted or substituted by one or more substituents selected from: hydroxy, halogen, cyano, C₃-C₆cycloalkyl, amino, NH(C₁-C₆alkyl), N(C₁-C₆alkyl)₂, C₁-C₆alkoxy and C₃-C₆cycloalkoxy;
R₂ and R₃ are independently selected from hydrogen, C₁-C₆alkyl, hydroxy, halogen, cyano, amino, C₁-C₆alkoxy, arylC₁-C₆alkoxy and C₁-C₆alkoxycarbonyl, wherein said aryl is optionally substituted by one or more groups selected from C₁-C₆alkyl, hydroxy, halogen, cyano, amino and C₁-C₆alkoxy;
R₄ is C₁-C₆alkyl which is unsubstituted or substituted by one or more substituents selected from: hydroxy, halogen, cyano, C₃-C₆cycloalkyl, arylC₁-C₆alkyl, amino, NH(C₁-C₆alkyl), N(C₁-C₆alkyl)₂, C₁-C₆alkoxy, NHR₅-substituted C₁-C₆alkoxy and NHR₅-substituted C₁-C₆alkylcarbonyloxy; wherein said aryl group is optionally substituted by one or more groups selected from C₁-C₆alkyl, hydroxy, halogen, cyano, amino, C₁-C₆alkoxy and C₁-C₆alkylamino;
R₅ is selected from C₁-C₆alkyl and C₁-C₆alkylcarbonyl, wherein said C₁-C₆alkyl and C₁-C₆alkylcarbonyl are optionally substituted with one or more substituents selected from the group consisting of hydroxy, halo, amino, NH(C₁-C₆alkyl), N(C₁-C₆alkyl)₂, C₁-C₆alkoxy and C₃-C₆cycloalkoxy;
(2) a compound of formula (D-2): 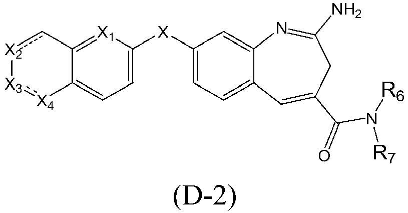 wherein:
X is absent, or is -NH-(C=O)-, -(C=O)-NH-, or C₁-C₆alkylene, wherein said C₁-C₆alkylene is optionally substituted with one or more substituents selected from the group consisting of hydroxy and halogen, and wherein one or more carbon atoms therein may be replaced with a group selected from the group consisting of -NH- and -(C=O)-;
X₁ is selected from CH or N;
X₂ is selected from CH₂ or N;
X₃ is NR₈, wherein R₈ is selected from H and C₁-C₆alkyl, and said C₁-C₆alkyl is optionally substituted with hydroxy, C₁-C₆alkoxy and amino substituted C₁-C₆alkoxy;
X₄ is CH₂ or C(=O);
R₆ and R₇ are each independently selected from H and C₁-C₆alkyl, and said C₁-C₆alkyl is optionally substituted by one or more groups selected from hydroxy, halogen, cyano, C₃-C₆cycloalkyl, amino, NH(C₁-C₆alkyl), N(C₁-C₆alkyl)₂ and C₁-C₆alkoxy; and
- - - - - - represents a single or double bond;
(3) a compound of formula (D-3): 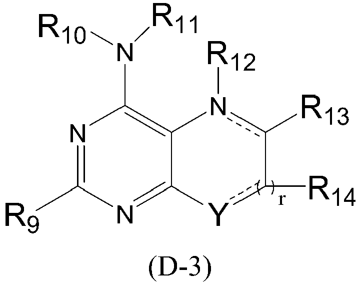 wherein:
R₉ is selected from hydroxyl, amino, NH(C₁-C₆alkyl), N(C₁-C₆alkyl)₂ and C₁-C₆alkoxy;
R₁₀ and R₁₁ are independently selected from hydrogen and C₁-C₈alkyl, and said C₁-C₈alkyl is optionally substituted with one or more substituents selected from the group consisting of hydroxy, halogen, C₁-C₆alkoxy and NR₁₅R₁₆, wherein R₁₅ and R₁₆ are independently selected from H, C₁-C₆acyl and hydroxy-substituted C₁-C₆acyl;
R₁₂ is absent, or is selected from hydrogen and arylC₁-C₆alkyl, wherein said aryl is optionally substituted with one or more substituents selected from hydroxy, halogen, C₁-C₆alkoxy and heterocyclylC₁-C₆alkyl, and said heterocyclyl is optionally substituted with one or more substituents selected from C₁-C₆alkyl, C₁-C₆alkoxy-C₁-C₆alkyl and C₁-C₆acyl;
R₁₃ is selected from hydrogen, halogen, hydroxy and oxo (=O);
R₁₄ is selected from hydrogen, halogen, hydroxyl, C₁-C₆alkyl and C₁-C₆alkoxy;
r is 0 or 1;
Y is selected from C and NR₁₇, wherein R₁₇ is selected from hydrogen or C₁-C₆alkyl optionally substituted with aryl or heterocyclyl, wherein said aryl and heterocyclyl are optionally substituted with one or more substituents selected from C₁-C₆acyl, C₁-C₆alkyl, C₁-C₆alkoxy-C₁-C₆alkyl, hydroxyl-C₁-C₆alkyl and NR₁₈R₁₉-C₁-C₆alkyl, wherein R₁₈ and R₁₉ are independently selected from H, C₁-C₆alkyl and C₁-C₆acyl; and
- - - - - - represents a single or double bond.

50. The compound according to claim 49, having a structure represented by formula (D-1'): 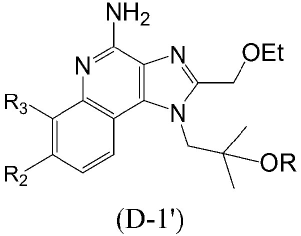
wherein R₂ and R₃ are as defined in claim 21; and
R is selected from H and 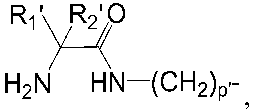
wherein R₁' and R₂' are independently selected from hydrogen and C₁-C₆alkyl, and p' is an integer selected from 1-6.

51. The compound according to claim 49, having the structure represented by formula (D-2) and wherein R₆ and R₇ are selected from C₁-C₆ alkyl, preferably n-propyl.

52. The compound according to claim 49, having a structure represented by formula (D-3'): 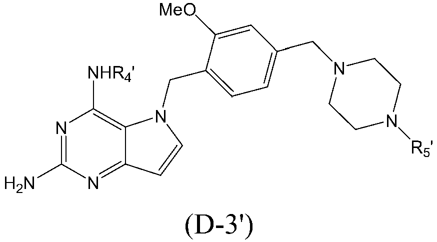 wherein
R₄' represents a C₃-C₆alkyl optionally substituted by one or more groups selected from hydroxy, halogen, amino and C₁-C₆alkoxy, preferably n-pentyl;
R₅' is selected from H, C₁-C₆alkyl, C₁-C₆alkoxy-C₁-C₆alkyl, C₁-C₆acyl, preferably selected from H, acetyl, and methoxyethyl.

53. The compound according to claim 49, having a structure represented by formula (D-3"): 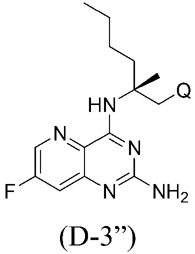 wherein:
Q is selected from OR₆' and NR₇'R₈';
wherein R₆' is selected from H and C₁-C₆alkyl;
R₇' and R₈' are independently selected from H, C₁-C₆acyl, hydroxy-substituted C₁-C₆acyl, preferably, R₇' and R₈' are both H, or one of R₇' and R₈ ' is H and the other is acetyl or hydroxyacetyl.

54. The compound according to claim 49, having a structure represented by formula (D-3"'): 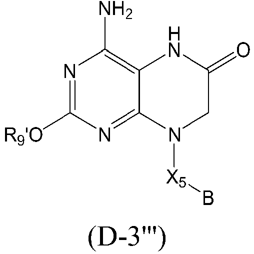 wherein:
R₉' is C₁-C₆ alkyl, preferably straight chain C₃-C₆ alkyl, e.g. n-butyl;
r is 0 or 1;
X₅ is selected from C₁-C₆ alkylene, e.g., -CH₂-, -CH₂-CH₂-, -CH₂-CH₂-CH₂-, - CH₂-CH₂-CH₂-CH₂-, -CH₂-CH₂-CH₂-CH₂-CH₂-, -CH₂-CH₂-CH₂-CH₂-CH₂-CH₂-;
B is selected from the group consisting of aryl and heterocyclyl, e.g., phenyl, tetrahydroisoquinolinyl, and piperidinyl, wherein said aryl and heterocyclyl are optionally substituted with C₁-C₆ acyl, C₁-C₆ alkyl, C₁-C₆ alkoxy-C₁-C₆ alkyl, hydroxyl-C₁-C₆ alkyl or NR'R"-C₁-C₆ alkyl, wherein R' and R" are independently selected from H, C₁-C₆ alkyl and C₁-C₆ acyl.

55. The compound according to claim 49, having a structure represented by formula (D-1"): 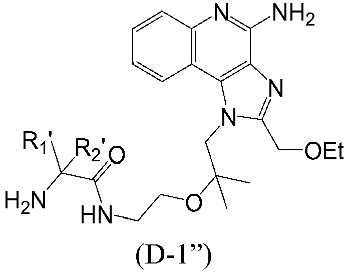 wherein R₁' and R₂' are independently selected from hydrogen and C₁-C₆alkyl, preferably, R₁' and R₂' are both methyl, or R₁' is hydrogen and R₂' is 3,3-dimethylbutyl.

56. The compound according to any one of claims 49 to 55, selected from:

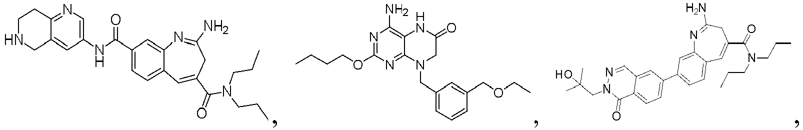

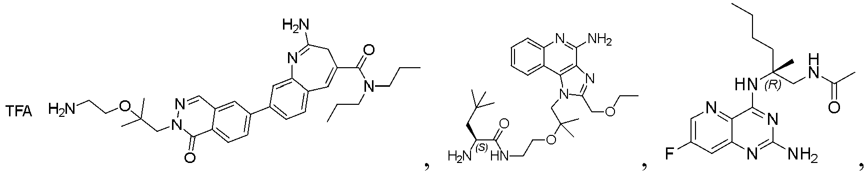

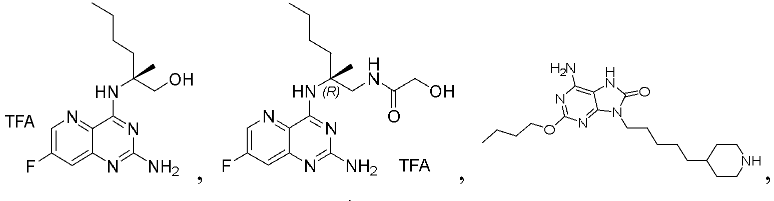

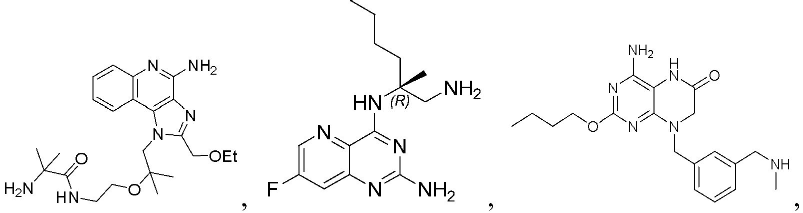

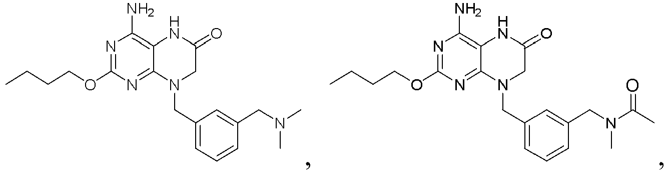

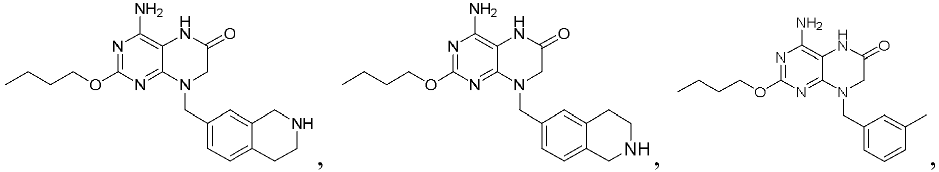

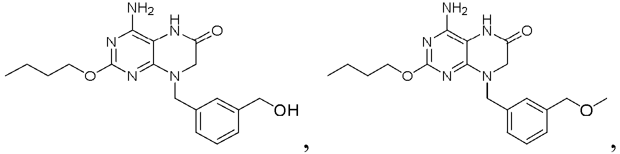

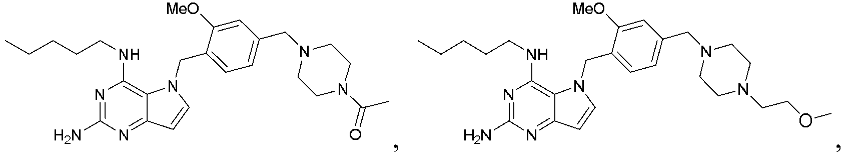

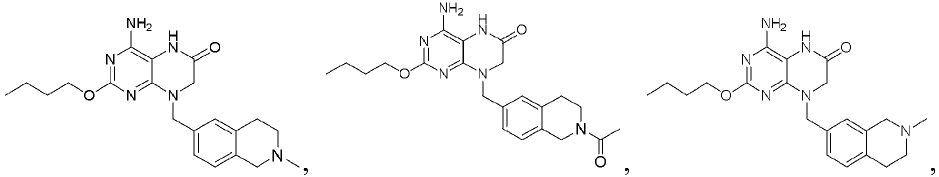

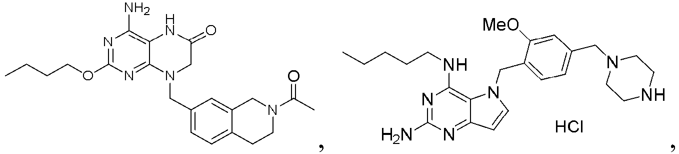

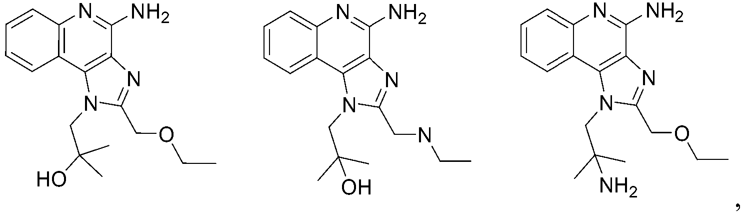

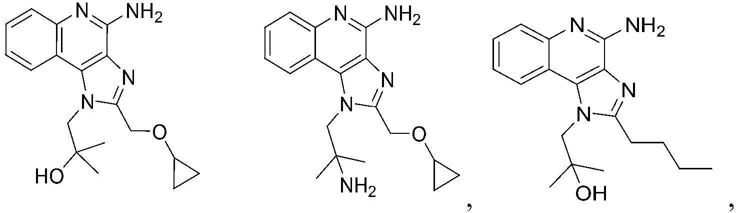

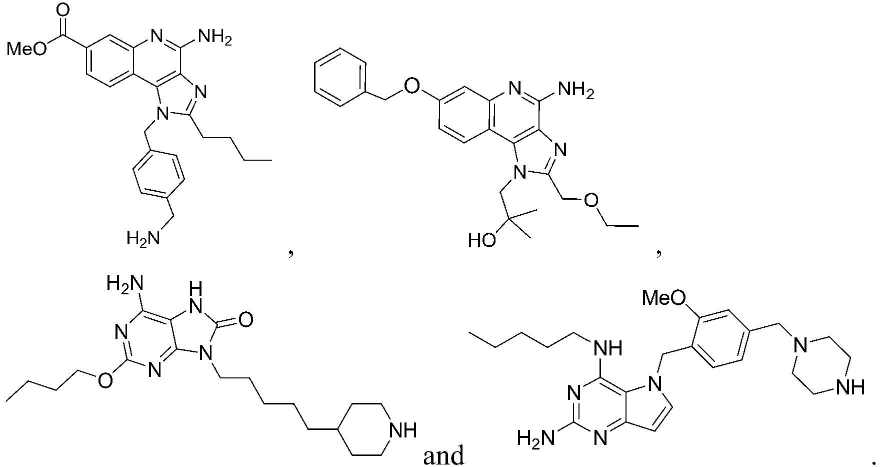

57. A compound of formula (II), or a pharmaceutically acceptable salt or a solvate thereof:
L-(D)ₘ (II)
wherein D is a compound as defined in any one of claims 21-28, or a pharmaceutically acceptable salt or solvate thereof;
L has the following structure:
Z'-L₁₋E-L₂-L₃-
wherein L₃ is linked to D;
Z' is selected from 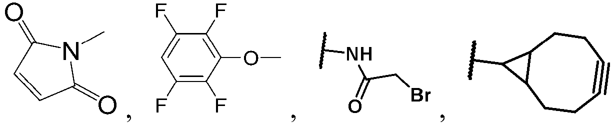 and 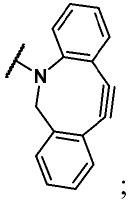
L₁ is -(CH₂)ₙ-C(=O)- or -(CH₂)ₙ-(O(CH₂)₂)ₜ(CH₂)ₙ-C(=O)-;
E is absent, or is a peptide residue comprising 2 to 10 amino acids, wherein the amino acid residue is a natural amino acid residue or a non-natural amino acid residue and is optionally substituted by C₁-C₆alkyl, and the C-terminus of the peptide residue is covalently linked to L₂;
L₂ is absent or is 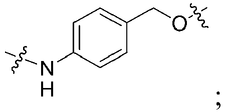
L₃ is absent, or is -C(=O)-*, -C(=O)((CH₂)ₙO)ₜ(CH₂)ₙ-* or -NH(CH₂)-*, wherein * indicates that the terminus is covalently linked to D;
n is an integer from 0 to 10,
t is an integer from 1 to 10,
m is an integer from 1 to 6.

58. The compound or the pharmaceutically acceptable salt or solvate thereof according to claim 57, wherein
E has a structure represented by the following formula:
-X₁-X₂-
wherein X₁ represents -(N(R⁰)C(R⁰)₂C(=O))ₛ-, wherein R⁰ independently of one another represent H or C₁-C₆ alkyl; s=0-10, preferably 2, 4, 6 and 8;
X₂ is absent, or is selected from

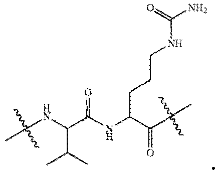

59. The compound or the pharmaceutically acceptable salt or solvate thereof according to claim 58, wherein
E is selected from 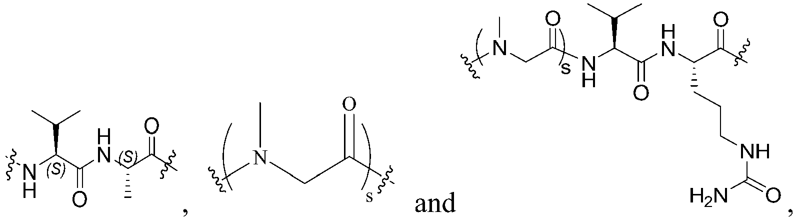
where s = 0-8.

60. The compound or the pharmaceutically acceptable salt or solvate thereof according to any one of claims 57-59, wherein
L-has a structure selected from: 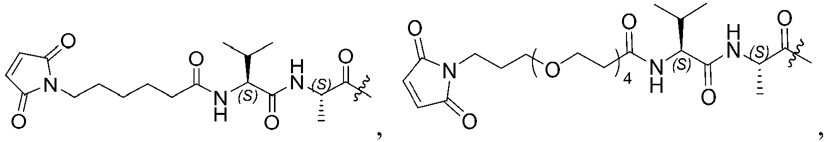 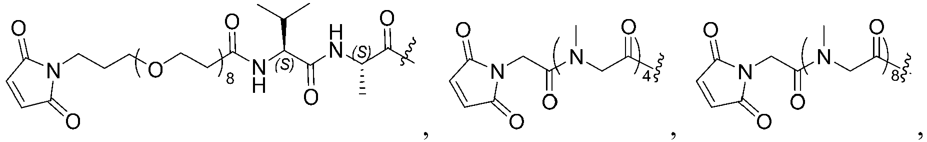 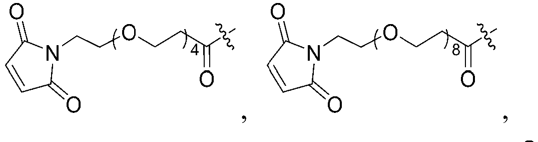 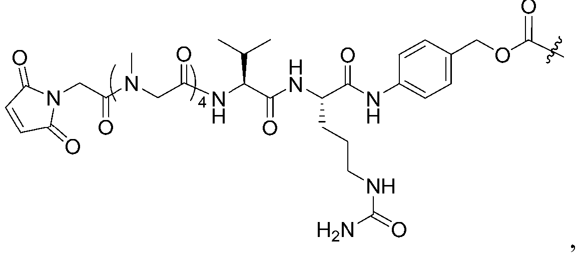 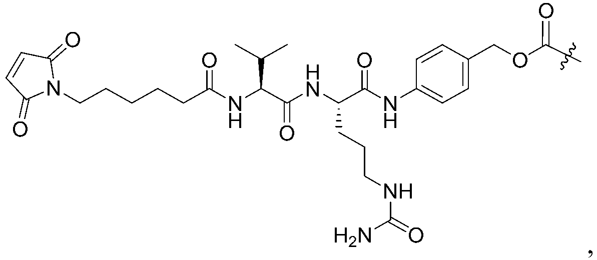 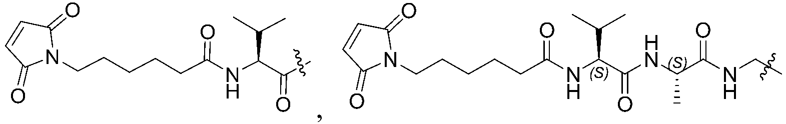 and

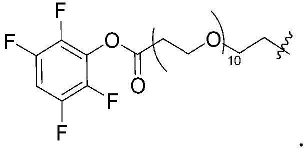

61. The compound or the pharmaceutically acceptable salt or solvate thereof according to claim 57, wherein the compound selected from: 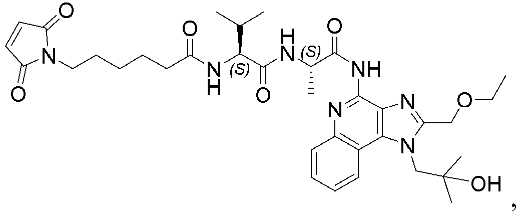 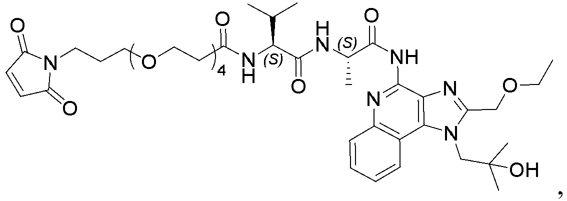 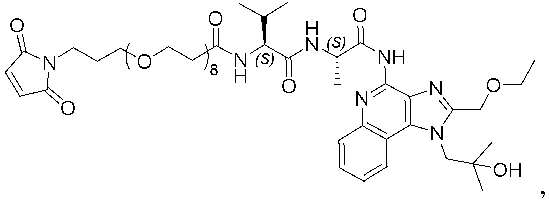 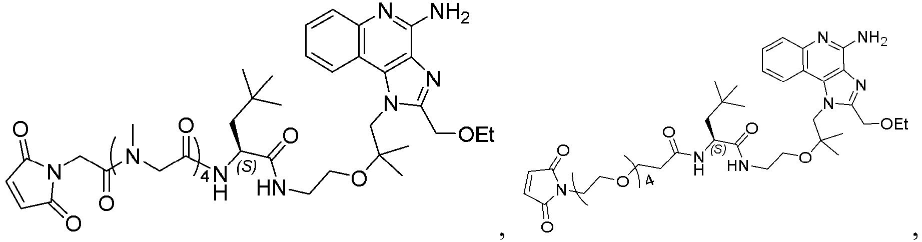 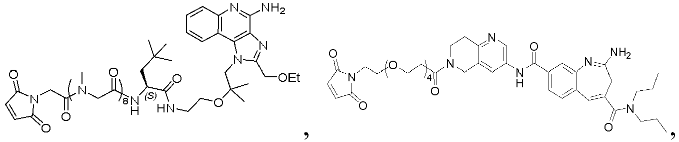 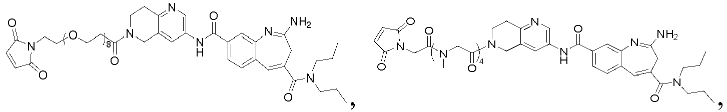 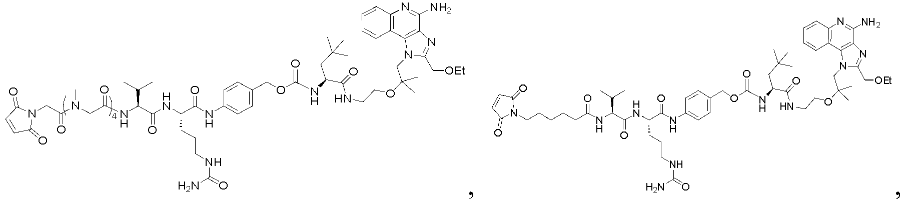 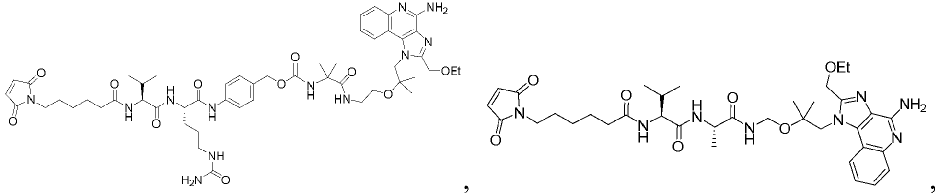 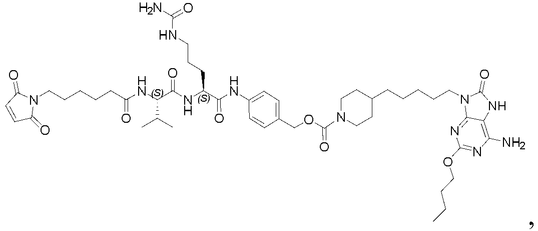 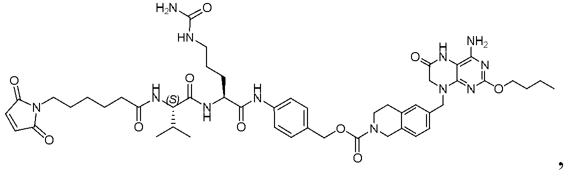 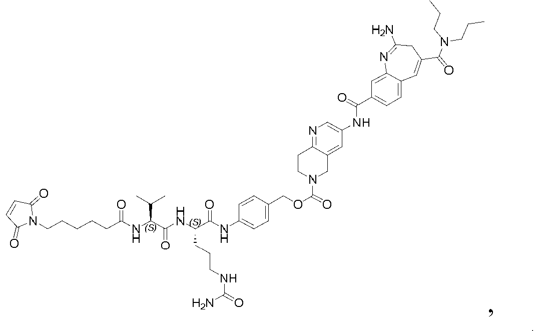 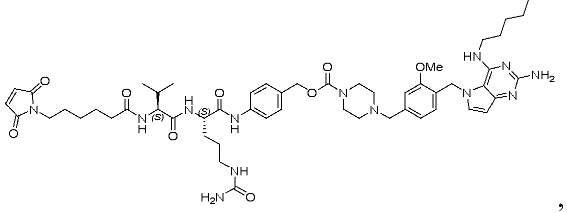 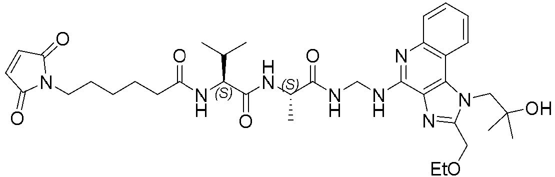 and

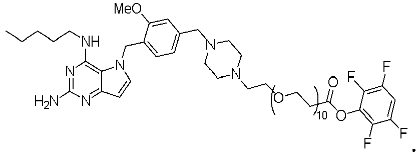

62. An immunoconjugate having a structure of formula (I):
Ab-(L-(D)ₘ)ₚ (I)
or a pharmaceutically acceptable salt or solvate thereof,
wherein:
Ab is a specific antigen-binding protein or a fragment thereof;
L is a linker;
D is a TLR agonist, preferably a TLR 7/8 agonist;
m is an integer from 1 to 6; and
p is an integer from 1 to 16, e.g., 2 to10.

63. The immunoconjugate or the pharmaceutically acceptable salt or solvate thereof according to claim 62, wherein D in formula (I) is a compound as claimed in any one of claims 49-56 or a pharmaceutically acceptable salt or solvate thereof.

64. The immunoconjugate or the pharmaceutically acceptable salt or solvate thereof according to claim 62 or 63, wherein L-(D)ₘ in formula (I) is a compound of Formula (II) as claimed in any one of claims 57-61, or a pharmaceutically acceptable salt or solvate thereof.

65. The immunoconjugate or the pharmaceutically acceptable salt or solvate thereof according to claim 64, wherein -L- has a structure selected from: 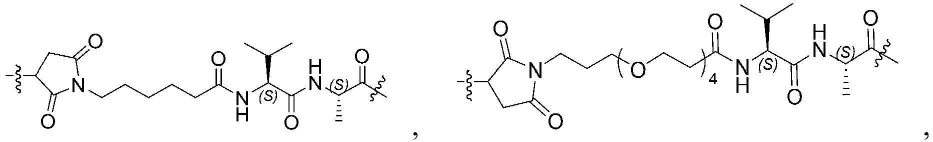 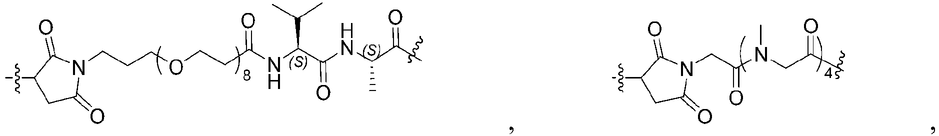 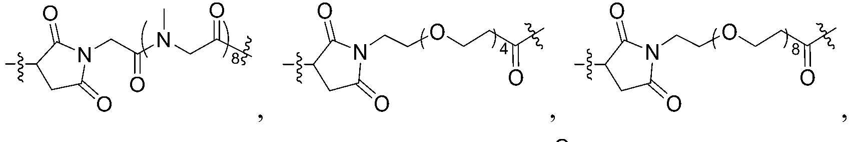 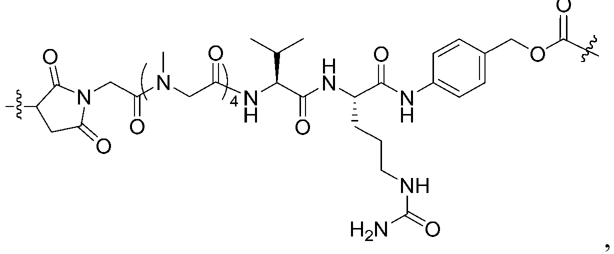 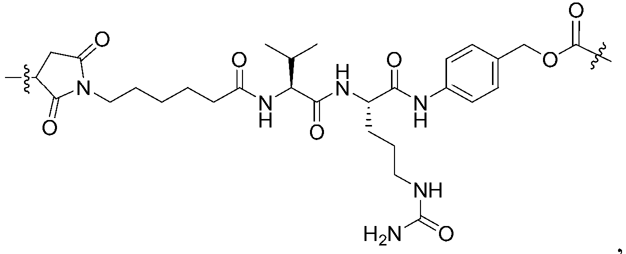 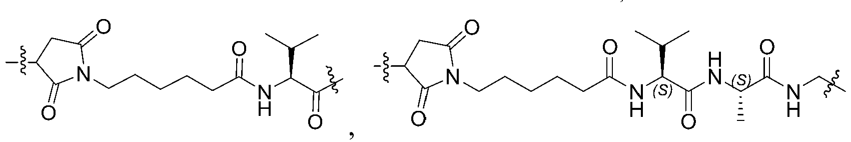 and 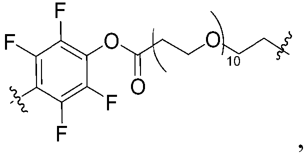 wherein the left side of the structure is linked to Ab, and the right side is linked to D.

66. The immunoconjugate or the pharmaceutically acceptable salt or solvate thereof according to claim 64, wherein L-(D)ₘ has a structure selected from: 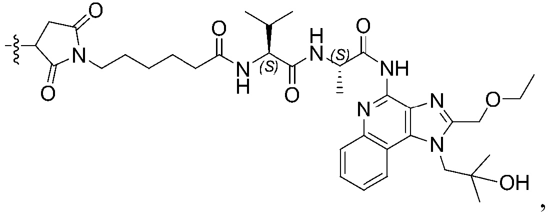 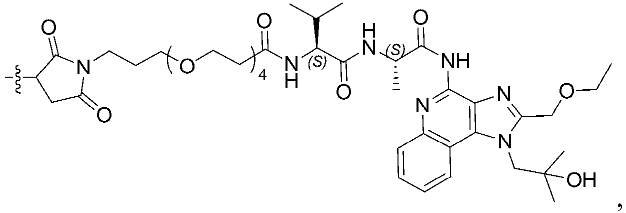 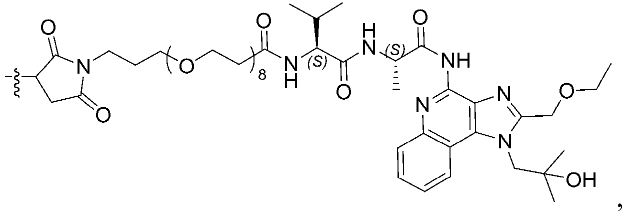 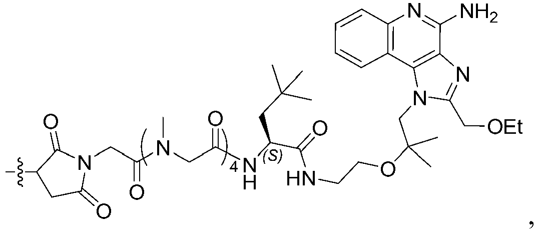 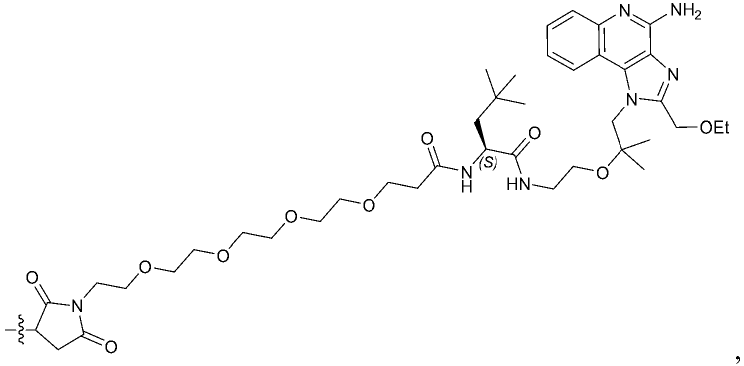 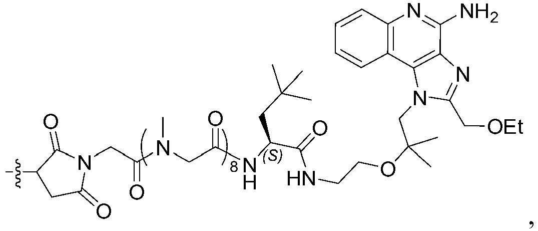 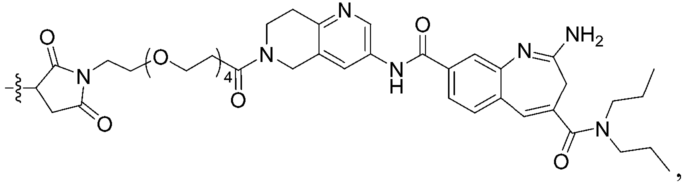 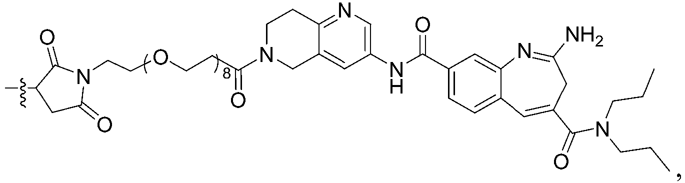 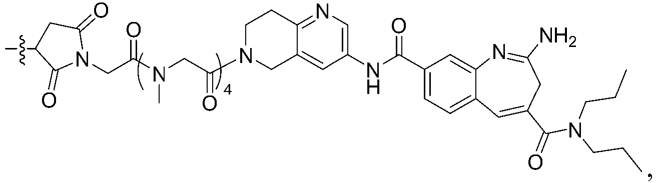 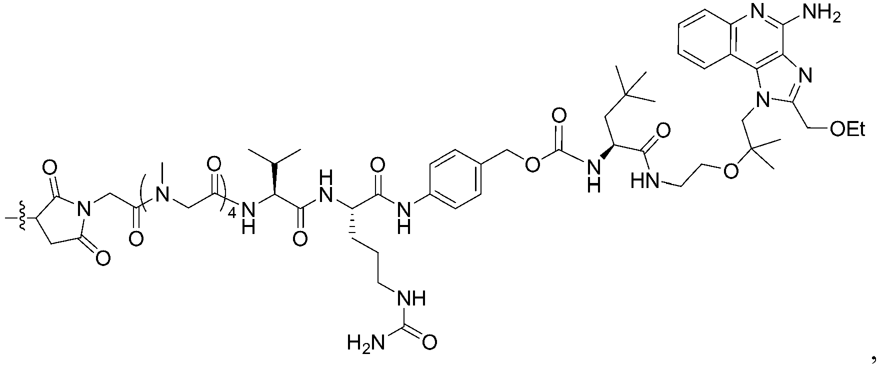 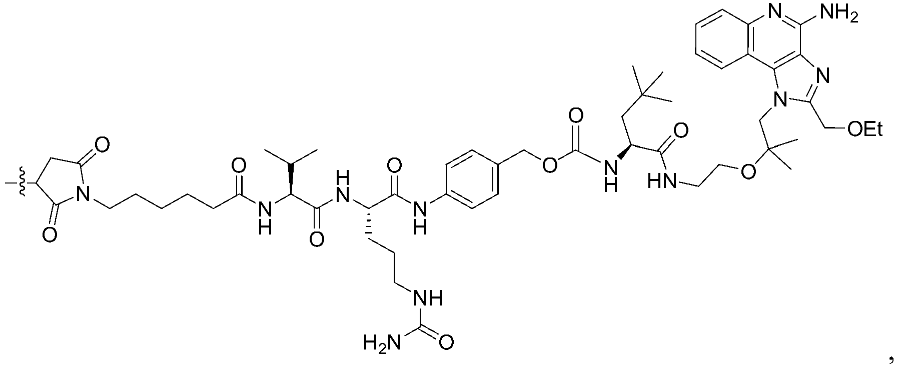 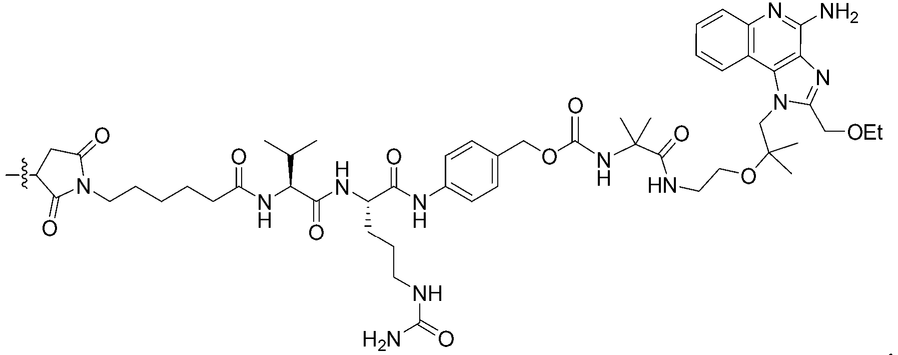 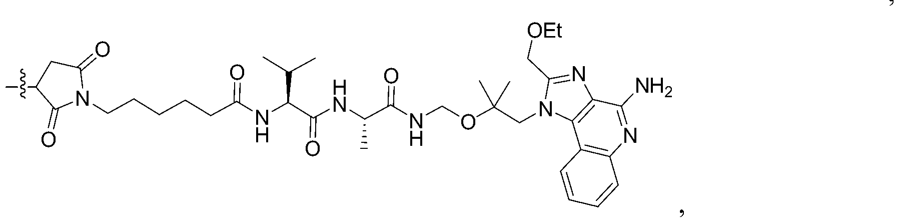 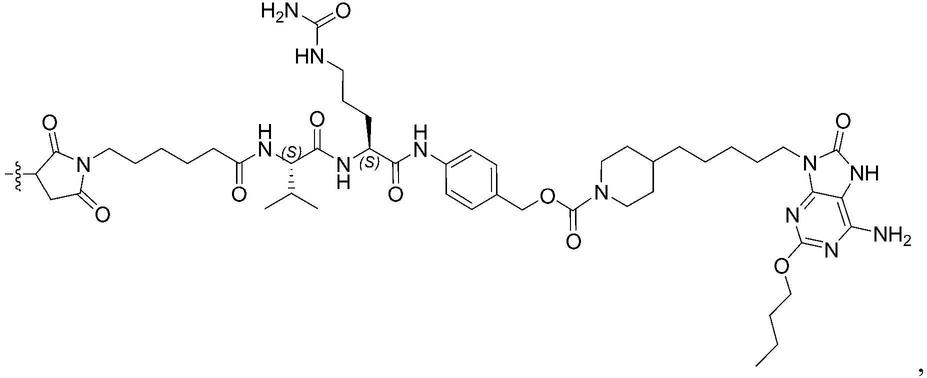 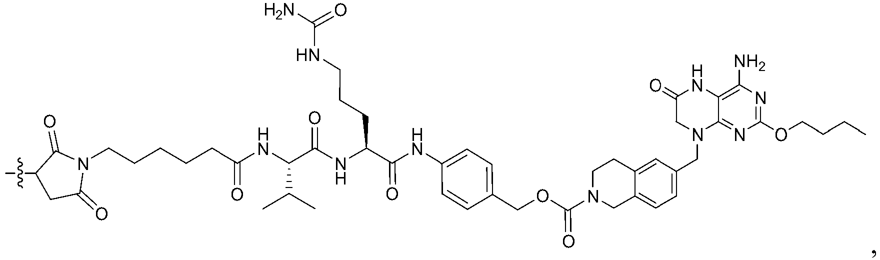 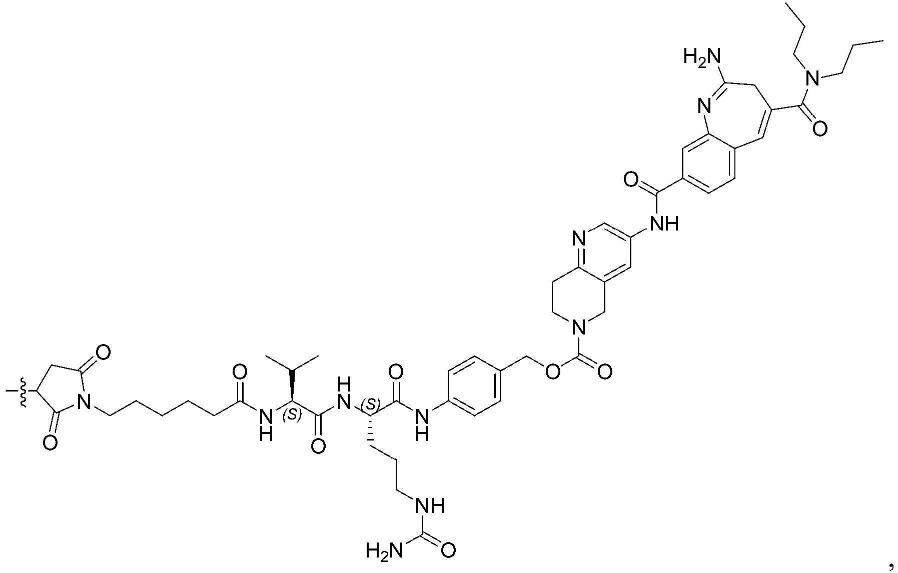 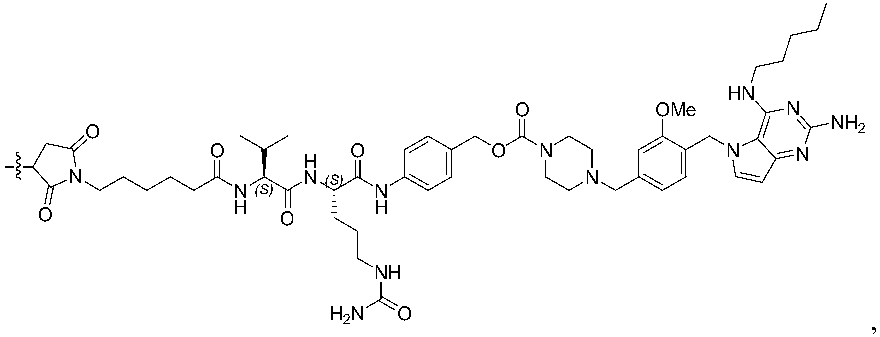 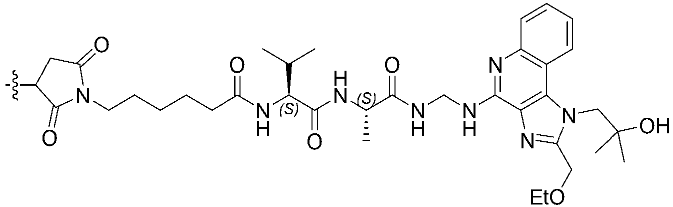 and

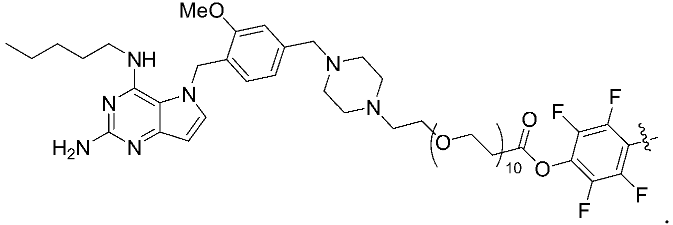

67. The immunoconjugate or the pharmaceutically acceptable salt or solvate thereof according to any one of claims 62-66, having a DAR value of 1 to 10 (e.g., 2 to 8).

68. The immunoconjugate or the pharmaceutically acceptable salt or solvate thereof according to claim 62, which is 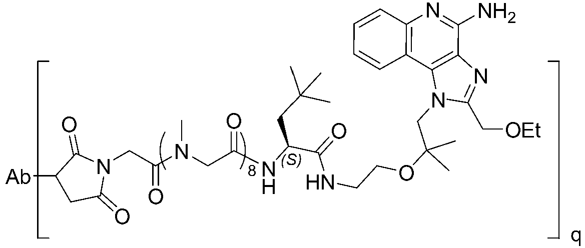 wherein q represents DAR, e.g., 1 to 10, e.g., 2 to 8, e.g., 3, 4, 5, 6, or 7.

69. The immunoconjugate or the pharmaceutically acceptable salt or solvate thereof according to any one of claims 62-68, wherein Ab specifically binds to TROP2.

70. The immunoconjugate or rthe pharmaceutically acceptable salt or solvate thereof according to claim 69, wherein Ab is the anti-TROP2 antibody or antigen-binding fragment thereof according to any one of claims 1-13.

71. The immunoconjugate or the pharmaceutically acceptable salt or solvate thereof according to claim 69 or 70, having a DAR value or an average DAR value of 1 to 10 (e.g., 2 to 8).

72. A pharmaceutical composition, comprising the antibody or the antigen-binding fragment thereof according to any one of claims 1-13, the fusion protein according to claim 14, the immunoconjugate according to claim 15 or 16, the nucleic acid according to claim 17, the vector according to claim 18, the host cell according to claim 19, the compound according to any one of claims 49-56, or the immunoconjugate according to any one of claims 62-71 or the pharmaceutically acceptable salt or solvate thereof,
and optionally one or more additional therapeutic agents, such as a chemotherapeutic agent, an angiogenesis inhibitor, a cytokine, a cytotoxic agent, an additional antibody, a small molecule drug or an immunomodulatory agent, and optionally a pharmaceutical supplementary material.

73. A pharmaceutical combination, comprising the antibody or the antigen-binding fragment thereof according to any one of claims 1-13, the fusion protein according to claim 14, the immunoconjugate according to claim 15 or 16, the nucleic acid according to claim 17, the vector according to claim 18, the host cell according to claim 19, the compound according to any one of claims 49-56, or the immunoconjugate according to any one of claims 62-71 or the pharmaceutically acceptable salt or solvate thereof, or the pharmaceutical composition according to claim 72,
and one or more additional therapeutic agents, such as a chemotherapeutic agent, an angiogenesis inhibitor, a cytokine, a cytotoxic agent, an additional antibody, a small molecule drug, or an immunomodulatory agent.

74. A method for preventing or treating a tumor in a subject, comprising administering to the subject an effective amount of the antibody or the antigen-binding fragment thereof according to any one of claims 1-13, the fusion protein according to claim 14, the immunoconjugate according to claim 15 or 16, the nucleic acid according to claim 17, the vector according to claim 18, the host cell according to claim 19, the compound according to any one of claims 49-56, or the immunoconjugate according to any one of claims 62-71 or the pharmaceutically acceptable salt or solvate thereof, the pharmaceutical composition according to claim 72, or the pharmaceutical combination according to claim 73.

75. The method according to claim 74, wherein the tumor is a cancer; preferably, the cancer has an elevated level (e.g., at a nucleic acid or protein level) of TROP2, e.g., as compared to a healthy individual or healthy tissue adjacent to cancer tissue of the patient.

76. The method according to claim 75, wherein the cancer is selected from the group consisting of non-small cell lung cancer, triple-negative breast cancer, head and neck squamous cell carcinoma, urothelial carcinoma, ovarian cancer, endometrial cancer, gastric cancer, colorectal cancer, and cervical cancer.

77. The method according to any one of claims 74-76, further comprising administering to the patient one or more therapies, such as therapeutic modalities and/or additional therapeutic agents, wherein preferably, the therapeutic modalities comprise radiotherapy or surgery, or the therapeutic agents comprise a chemotherapeutic agent, an angiogenesis inhibitor, a cytokine, a cytotoxic agent, an additional antibody, a small molecule drug, or an immunomodulatory agent.

78. Use of the antibody or the antigen-binding fragment thereof according to any one of claims 1-13, the fusion protein according to claim 14, the immunoconjugate according to claim 15 or 16, the nucleic acid according to claim 17, the vector according to claim 18, the host cell according to claim 19, the compound according to any one of claims 49-56, or the immunoconjugate according to any one of claims 62-71 or the pharmaceutically acceptable salt or a solvate thereof, the pharmaceutical composition according to claim 72, or the pharmaceutical combination according to claim 73 in the preparation of a medicament for the treatment of a tumor.

79. The use according to claim 78, wherein the tumor is a cancer; preferably, the cancer has an elevated level (e.g., at a nucleic acid or protein level) of TROP2; for example, compared with a healthy individual or healthy tissue adjacent to cancer tissue of the patient, more preferably, the cancer is selected from non-small cell lung cancer, triple-negative breast cancer, head and neck squamous cell carcinoma, urothelial carcinoma, ovarian cancer, endometrial cancer, gastric cancer, colorectal cancer, and cervical cancer.

80. The immunoconjugate according to any one of claims 62-68 or the pharmaceutically acceptable salt or solvate thereof, wherein Ab specifically binds to FRα.

81. The immunoconjugate according to claim 80 or the pharmaceutically acceptable salt or solvate thereof, wherein Ab is the anti-FRα antibody or antigen-binding fragment thereof according to any one of claims 21-41.

82. The immunoconjugate according to claim 80 or 81 or the pharmaceutically acceptable salt or a solvate thereof, having a DAR value or an average DAR value of 1 to 10 (e.g., 2 to 8).

83. A pharmaceutical composition, comprising the antibody or the antigen-binding fragment thereof according to any one of claims 21-41, the fusion protein according to claim 42, the immunoconjugate according to claim 43 or 44, the nucleic acid according to claim 45, the vector according to claim 46, the host cell according to claim 47, the compound according to any one of claims 49-56, or the immunoconjugate according to any one of claims 62-68 and 80-82 or the pharmaceutically acceptable salt or solvate thereof,
and optionally one or more additional therapeutic agents, such as a chemotherapeutic agent, an angiogenesis inhibitor, a cytokine, a cytotoxic agent, an additional antibody, a small molecule drug or an immunomodulatory agent, and optionally a pharmaceutical supplementary material.

84. A pharmaceutical combination, comprising the antibody or the antigen-binding fragment thereof according to any one of claims 21-41, the fusion protein according to claim 42, the immunoconjugate according to claim 43 or 44, the nucleic acid according to claim 45, the vector according to claim 46, the host cell according to claim 47, the compound according to any one of claims 49-56, or the immunoconjugate according to any one of claims 62-68 and 80-82 or the pharmaceutically acceptable salt or solvate thereof or the pharmaceutical composition according to claim 83,
and one or more additional therapeutic agents, such as a chemotherapeutic agent, an angiogenesis inhibitor, a cytokine, a cytotoxic agent, an additional antibody, a small molecule drug, or an immunomodulatory agent.

85. The pharmaceutical combination according to claim 84, wherein the additional therapeutic agent is an antibody-drug conjugate targeting FRα, or a stereoisomer, a pharmaceutically acceptable salt, or a solvate thereof.

86. A method for preventing or treating a tumor in a subject, comprising administering to the subject an effective amount of the antibody or the antigen-binding fragment thereof according to any one of claims 21-41, the fusion protein according to claim 42, the immunoconjugate according to claim 43 or 44, the nucleic acid according to claim 45, the vector according to claim 46, the host cell according to claim 47, the compound according to any one of claims 49-56, or the immunoconjugate according to any one of claims 62-68 and 80-82 or the pharmaceutically acceptable salt or solvate thereof, the pharmaceutical composition according to claim 83, or the pharmaceutical combination according to claim 84 or 85.

87. The method according to claim 86, wherein the tumor is a cancer; preferably, the cancer has an elevated level (e.g., at a nucleic acid or protein level) of FRα, e.g., as compared to a healthy individual or healthy tissue adjacent to cancer tissue of the patient.

88. The method according to claim 87, wherein the cancer is selected from the group consisting of non-small cell lung cancer, triple-negative breast cancer, head and neck squamous cell carcinoma, urothelial carcinoma, ovarian cancer, endometrial cancer, gastric cancer, colorectal cancer, and cervical cancer.

89. The method according to any one of claims 86-88, further comprising administering to the patient one or more therapies, such as therapeutic modalities and/or additional therapeutic agents, wherein preferably, the therapeutic modalities comprise radiotherapy or surgery, or the therapeutic agents comprise a chemotherapeutic agent, an angiogenesis inhibitor, a cytokine, a cytotoxic agent, an additional antibody, a small molecule drug, or an immunomodulatory agent.

90. The method according to claim 89, wherein the additional therapeutic agent is the antibody-drug conjugate as defined in claim 85.

91. Use of the antibody or the antigen-binding fragment thereof according to any one of claims 21-41, the fusion protein according to claim 42, the immunoconjugate according to claim 43 or 44, the nucleic acid according to claim 45, the vector according to claim 46, the host cell according to claim 47, the compound according to any one of claims 49-56, or the immunoconjugate according to any one of claims 62-68 and 80-82 or the pharmaceutically acceptable salt or solvate thereof, the pharmaceutical composition according to claim 83, or the pharmaceutical combination according to claim 84 or 85 in the manufacture of a medicament for the treatment of a tumor.

92. The use according to claim 91, wherein the tumor is a cancer; preferably, the cancer has an elevated level (e.g., at a nucleic acid or protein level) of FRα, e.g., as compared to a healthy individual or healthy tissue adjacent to cancer tissue of the patient; more preferably, the cancer is selected from non-small cell lung cancer, triple-negative breast cancer, head and neck squamous cell carcinoma, urothelial carcinoma, ovarian cancer, endometrial cancer, gastric cancer, colorectal cancer, and cervical cancer.
